(19) Europäisches Patentamt
European Patent Office
Office européen des brevets

(11) EP 1 682 178 B1

(12) # EUROPEAN PATENT SPECIFICATION

(45) Date of publication and mention of the grant of the patent:
**07.07.2010 Bulletin 2010/27**

(21) Application number: **04810421.0**

(22) Date of filing: **04.11.2004**

(51) Int Cl.:
***A61K 39/395*** *(2006.01)* ***A61K 38/20*** *(2006.01)*
***A61P 35/02*** *(2006.01)*

(86) International application number:
**PCT/US2004/036958**

(87) International publication number:
**WO 2005/044294 (19.05.2005 Gazette 2005/20)**

(54) **METHODS OF THERAPY FOR CANCERS EXPRESSING THE CD40 ANTIGEN**

VERFAHREN ZUR BEHANDLUNG VON KREBS MIT EXPRESSION DES CD40-ANTIGENS

PROCEDES DE THERAPIE POUR DES CANCERS EXPRIMANT L'ANTIGENE CD40

(84) Designated Contracting States:
**AT BE BG CH CY CZ DE DK EE ES FI FR GB GR HU IE IS IT LI LU MC NL PL PT RO SE SI SK TR**

(30) Priority: **04.11.2003 US 517337 P**
**26.11.2003 US 525579 P**
**27.04.2004 US 565710 P**

(43) Date of publication of application:
**26.07.2006 Bulletin 2006/30**

(73) Proprietor: **Novartis Vaccines and Diagnostics, Inc.**
**Emeryville, CA 94608 (US)**

(72) Inventors:
- **LONG, Li**
  **Emeryville, CA 94662-8097 (US)**
- **LUQMAN, Mohammad**
  **Emeryville, CA 94662-8097 (US)**
- **YABANNAVAR, Asha**
  **Emeryville, CA 94662-8097 (US)**
- **ZAROR, Isabel**
  **Emeryville, CA 94662-8097 (US)**
- **HURST, Deborah**
  **Emeryville, CA 94662-8097 (US)**
- **LOPES DE MENEZES, Daniel, E.**
  **Emeryville, CA 94662-8097 (US)**

(74) Representative: **Marshall, Cameron John et al**
**Carpmaels & Ransford**
**43-45 Bloomsbury Square**
**London WC1A 2RA (GB)**

(56) References cited:
**WO-A-01/83755 WO-A-02/28480**
**WO-A-02/28904 WO-A-02/088186**

- **GISSELBRECHT CHRISTIAN ET AL: "Interleukin-2 treatment in lymphoma: A phase II multicenter study" BLOOD, vol. 83, no. 8, 1994, pages 2081-2085, XP002327659 ISSN: 0006-4971 cited in the application**
- **ROSENBERG S A ET AL: "A PROGRESS REPORT ON THE TREATMENT OF 157 PATIENTS WITH ADVANCES CANCER USING LYMPHOKINE-ACTIVATED KILLER CELLS AND INTERLEUKIN-2 OR HIGH-DOSE INTERLEUKIN-2 ALONE" NEW ENGLAND JOURNAL OF MEDICINE, THE, MASSACHUSETTS MEDICAL SOCIETY, WALTHAM, MA, US, vol. 316, no. 15, 2 April 1987 (1987-04-02), pages 889-897, XP001118518 ISSN: 0028-4793 cited in the application**
- **LITTLE M. ET AL: 'Of mice and men: hybridoma and recombinant antibodies' REVIEW IMMUNOLOGY TODAY vol. 21, no. 8, August 2000, pages 364 - 370**
- **ELLMARK PETER ET AL: 'Modulation of the CD40-CD40 ligand interaction using human anti-CD40 single-chain antibody fragments obtained from the n-CoDeR phage display library' IMMUNOLOGY vol. 106, 2002, pages 456 - 463**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

SEQUENCE LISTING

<110> Hurst, Deborah
Long, Li
Luqman, Mohammad
Lopes de Menezes, Daniel E.
Yabannavar, Asha
Zaror, Isabel
<120> Methods of Therapy for Cancers
Expressing the CD40 Antigen
<130> PP23220.001 (281250)
<150> 60/565,710
<151> 2004-04-27
<150> 60/525,579
<151> 2003-11-26
<150> 60/517,337
<151> 2003-11-04
<160> 18
<170> FastSEQ for Windows Version 4.0
<210> 1
<211> 720
<212> DNA
<213> Artificial Sequence
<220>
<223> Coding sequence for light chain of CHIR-12.12 human anti-CD40 antibody

<221> CDS
<222> (1)...(720)
<400> 1

```
atg gcg ctc cct gct cag ctc ctg ggg ctg cta atg ctc tgg gtc tct    48
Met Ala Leu Pro Ala Gln Leu Leu Gly Leu Leu Met Leu Trp Val Ser
 1               5                  10                  15

gga tcc agt ggg gat att gtg atg act cag tct cca ctc tcc ctg acc    96
Gly Ser Ser Gly Asp Ile Val Met Thr Gln Ser Pro Leu Ser Leu Thr
            20                  25                  30

gtc acc cct gga gag ccg gcc tcc atc tcc tgc agg tcc agt cag agc   144
Val Thr Pro Gly Glu Pro Ala Ser Ile Ser Cys Arg Ser Ser Gln Ser
        35                  40                  45

ctc ctg tat agt aat gga tac aac tat ttg gat tgg tac ctg cag aag   192
Leu Leu Tyr Ser Asn Gly Tyr Asn Tyr Leu Asp Trp Tyr Leu Gln Lys
    50                  55                  60

cca ggg cag tct cca cag gtc ctg atc tct ttg ggt tct aat cgg gcc   240
Pro Gly Gln Ser Pro Gln Val Leu Ile Ser Leu Gly Ser Asn Arg Ala
 65                  70                  75                  80

tcc ggg gtc cct gac agg ttc agt ggc agt gga tca ggc aca gat ttt   288
Ser Gly Val Pro Asp Arg Phe Ser Gly Ser Gly Ser Gly Thr Asp Phe
                85                  90                  95

aca ctg aaa atc agc aga gtg gag gct gag gat gtt ggg gtt tat tac   336
Thr Leu Lys Ile Ser Arg Val Glu Ala Glu Asp Val Gly Val Tyr Tyr
            100                 105                 110

tgc atg caa gct cga caa act cca ttc act ttc ggc cct ggg acc aaa   384
Cys Met Gln Ala Arg Gln Thr Pro Phe Thr Phe Gly Pro Gly Thr Lys
        115                 120                 125

gtg gat atc aga cga act gtg gct gca cca tct gtc ttc atc ttc ccg   432
Val Asp Ile Arg Arg Thr Val Ala Ala Pro Ser Val Phe Ile Phe Pro
    130                 135                 140

cca tct gat gag cag ttg aaa tct gga act gcc tct gtt gtg tgc ctg   480
Pro Ser Asp Glu Gln Leu Lys Ser Gly Thr Ala Ser Val Val Cys Leu
145                 150                 155                 160

ctg aat aac ttc tat ccc aga gag gcc aaa gta cag tgg aag gtg gat   528
Leu Asn Asn Phe Tyr Pro Arg Glu Ala Lys Val Gln Trp Lys Val Asp
                165                 170                 175

aac gcc ctc caa tcg ggt aac tcc cag gag agt gtc aca gag cag gac   576
Asn Ala Leu Gln Ser Gly Asn Ser Gln Glu Ser Val Thr Glu Gln Asp
            180                 185                 190

agc aag gac agc acc tac agc ctc agc agc acc ctg acg ctg agc aaa   624
Ser Lys Asp Ser Thr Tyr Ser Leu Ser Ser Thr Leu Thr Leu Ser Lys
        195                 200                 205

gca gac tac gag aaa cac aaa gtc tac gcc tgc gaa gtc acc cat cag   672
Ala Asp Tyr Glu Lys His Lys Val Tyr Ala Cys Glu Val Thr His Gln
    210                 215                 220

ggc ctg agc tcg ccc gtc aca aag agc ttc aac agg gga gag tgt tag   720
Gly Leu Ser Ser Pro Val Thr Lys Ser Phe Asn Arg Gly Glu Cys  *
225                 230                 235
```

<210> 2
<211> 239
<212> PRT
<213> Artificial Sequence
<220>
<223> Light chain of CHIR-12.12 human anti-CD40 antibody
<400> 2

```
Met Ala Leu Pro Ala Gln Leu Leu Gly Leu Leu Met Leu Trp Val Ser
 1              5                   10                  15
Gly Ser Ser Gly Asp Ile Val Met Thr Gln Ser Pro Leu Ser Leu Thr
            20                  25                  30
Val Thr Pro Gly Glu Pro Ala Ser Ile Ser Cys Arg Ser Ser Gln Ser
        35                  40                  45
Leu Leu Tyr Ser Asn Gly Tyr Asn Tyr Leu Asp Trp Tyr Leu Gln Lys
    50                  55                  60
Pro Gly Gln Ser Pro Gln Val Leu Ile Ser Leu Gly Ser Asn Arg Ala
65                  70                  75                  80
Ser Gly Val Pro Asp Arg Phe Ser Gly Ser Gly Ser Gly Thr Asp Phe
                85                  90                  95
Thr Leu Lys Ile Ser Arg Val Glu Ala Glu Asp Val Gly Val Tyr Tyr
            100                 105                 110
Cys Met Gln Ala Arg Gln Thr Pro Phe Thr Phe Gly Pro Gly Thr Lys
        115                 120                 125
Val Asp Ile Arg Arg Thr Val Ala Ala Pro Ser Val Phe Ile Phe Pro
    130                 135                 140
Pro Ser Asp Glu Gln Leu Lys Ser Gly Thr Ala Ser Val Val Cys Leu
145                 150                 155                 160
Leu Asn Asn Phe Tyr Pro Arg Glu Ala Lys Val Gln Trp Lys Val Asp
                165                 170                 175
Asn Ala Leu Gln Ser Gly Asn Ser Gln Glu Ser Val Thr Glu Gln Asp
            180                 185                 190
Ser Lys Asp Ser Thr Tyr Ser Leu Ser Ser Thr Leu Thr Leu Ser Lys
        195                 200                 205
Ala Asp Tyr Glu Lys His Lys Val Tyr Ala Cys Glu Val Thr His Gln
    210                 215                 220
Gly Leu Ser Ser Pro Val Thr Lys Ser Phe Asn Arg Gly Glu Cys
225                 230                 235
```

<210> 3
<211> 2016
<212> DNA
<213> Artificial Sequence
<220>
<223> Coding sequence for heavy chain of CHIR-12.12 human anti-CD40 antibody (with introns)
<400> 3

```
atggagtttg ggctgagctg ggttttcctt gttgctattt taagaggtgt ccagtgtcag 60
gtgcagttgg tggagtctgg gggaggcgtg gtccagcctg ggaggtccct gagactctcc 120
tgtgcagcct ctggattcac cttcagtagc tatggcatgc actgggtccg ccaggctcca 180
ggcaaggggc tggagtgggt ggcagttata tcatatgagg aaagtaatag ataccatgca 240
gactccgtga agggccgatt caccatctcc agagacaatt ccaagatcac gctgtatctg 300
caaatgaaca gcctcagaac tgaggacacg gctgtgtatt actgtgcgag agatgggggt 360
atagcagcac ctgggcctga ctactggggc cagggaaccc tggtcaccgt ctcctcagca 420
agtaccaagg gcccatccgt cttccccctg gcgcccgcta gcaagagcac ctctgggggc 480
acagcggccc tgggctgcct ggtcaaggac tacttccccg aaccggtgac ggtgtcgtgg 540
aactcaggcg ccctgaccag cggcgtgcac accttcccgg ctgtcctaca gtcctcagga 600
ctctactccc tcagcagcgt ggtgaccgtg ccctccagca gcttgggcac ccagacctac 660
atctgcaacg tgaatcacaa gcccagcaac accaaggtgg acaagagagt tggtgagagg 720
ccagcacagg gagggagggt gtctgctgga agccaggctc agcgctcctg cctggacgca 780
tcccggctat gcagtcccag tccagggcag caaggcaggc cccgtctgcc tcttcacccg 840
gaggcctctg cccgccccac tcatgctcag ggagagggtc ttctggcttt ttccccaggc 900
tctgggcagg cacaggctag gtgcccctaa cccaggccct gcacacaaag gggcaggtgc 960
tgggctcaga cctgccaaga gccatatccg ggaggaccct gcccctgacc taagcccacc 1020
ccaaaggcca aactctccac tccctcagct cggacacctt ctctcctccc agattccagt 1080
aactcccaat cttctctctg cagagcccaa atcttgtgac aaaactcaca catgcccacc 1140
gtgcccaggt aagccagccc aggcctcgcc ctccagctca aggcgggaca ggtgccctag 1200
agtagcctgc atccagggac aggccccagc cgggtgctga cacgtccacc tccatctctt 1260
cctcagcacc tgaactcctg gggggaccgt cagtcttcct cttcccccca aaacccaagg 1320
acaccctcat gatctcccgg acccctgagg tcacatgcgt ggtggtggac gtgagccacg 1380
aagaccctga ggtcaagttc aactggtacg tggacggcgt ggaggtgcat aatgccaaga 1440
caaagccgcg ggaggagcag tacaacagca cgtaccgtgt ggtcagcgtc ctcaccgtcc 1500
tgcaccagga ctggctgaat ggcaaggagt acaagtgcaa ggtctccaac aaagccctcc 1560
cagcccccat cgagaaaacc atctccaaag ccaaaggtgg gacccgtggg gtgcgagggc 1620
cacatggaca gaggccggct cggcccaccc tctgccctga gagtgaccgc tgtaccaacc 1680
tctgtcccta cagggcagcc ccgagaacca caggtgtaca ccctgccccc atcccgggag 1740
gagatgacca agaaccaggt cagcctgacc tgcctggtca aaggcttcta tcccagcgac 1800
atcgccgtgg agtgggagag caatgggcag ccggagaaca actacaagac cacgcctccc 1860
gtgctggact ccgacggctc cttcttcctc tatagcaagc tcaccgtgga caagagcagg 1920
tggcagcagg ggaacgtctt ctcatgctcc gtgatgcatg aggctctgca caaccactac 1980
acgcagaaga gcctctccct gtctccgggt aaatga                          2016
```

<210> 4
<211> 469
<212> PRT
<213> Artificial Sequence
<220>
<223> Heavy chain of CHIR-12.12 human anti-CD40 antibody
<400> 4

```
Met Glu Phe Gly Leu Ser Trp Val Phe Leu Val Ala Ile Leu Arg Gly
 1               5                  10                  15
Val Gln Cys Gln Val Gln Leu Val Glu Ser Gly Gly Gly Val Val Gln
            20                  25                  30
Pro Gly Arg Ser Leu Arg Leu Ser Cys Ala Ala Ser Gly Phe Thr Phe
        35                  40                  45
Ser Ser Tyr Gly Met His Trp Val Arg Gln Ala Pro Gly Lys Gly Leu
    50                  55                  60
Glu Trp Val Ala Val Ile Ser Tyr Glu Glu Ser Asn Arg Tyr His Ala
65                  70                  75                  80
Asp Ser Val Lys Gly Arg Phe Thr Ile Ser Arg Asp Asn Ser Lys Ile
                85                  90                  95
Thr Leu Tyr Leu Gln Met Asn Ser Leu Arg Thr Glu Asp Thr Ala Val
            100                 105                 110
Tyr Tyr Cys Ala Arg Asp Gly Gly Ile Ala Ala Pro Gly Pro Asp Tyr
        115                 120                 125
Trp Gly Gln Gly Thr Leu Val Thr Val Ser Ser Ala Ser Thr Lys Gly
    130                 135                 140
Pro Ser Val Phe Pro Leu Ala Pro Ala Ser Lys Ser Thr Ser Gly Gly
145                 150                 155                 160
Thr Ala Ala Leu Gly Cys Leu Val Lys Asp Tyr Phe Pro Glu Pro Val
                165                 170                 175
Thr Val Ser Trp Asn Ser Gly Ala Leu Thr Ser Gly Val His Thr Phe
            180                 185                 190
Pro Ala Val Leu Gln Ser Ser Gly Leu Tyr Ser Leu Ser Ser Val Val
        195                 200                 205
Thr Val Pro Ser Ser Ser Leu Gly Thr Gln Thr Tyr Ile Cys Asn Val
    210                 215                 220
Asn His Lys Pro Ser Asn Thr Lys Val Asp Lys Arg Val Glu Pro Lys
225                 230                 235                 240
Ser Cys Asp Lys Thr His Thr Cys Pro Pro Cys Pro Ala Pro Glu Leu
                245                 250                 255
Leu Gly Gly Pro Ser Val Phe Leu Phe Pro Pro Lys Pro Lys Asp Thr
            260                 265                 270
Leu Met Ile Ser Arg Thr Pro Glu Val Thr Cys Val Val Val Asp Val
        275                 280                 285
Ser His Glu Asp Pro Glu Val Lys Phe Asn Trp Tyr Val Asp Gly Val
    290                 295                 300
Glu Val His Asn Ala Lys Thr Lys Pro Arg Glu Glu Gln Tyr Asn Ser
305                 310                 315                 320
Thr Tyr Arg Val Val Ser Val Leu Thr Val Leu His Gln Asp Trp Leu
                325                 330                 335
Asn Gly Lys Glu Tyr Lys Cys Lys Val Ser Asn Lys Ala Leu Pro Ala
            340                 345                 350
Pro Ile Glu Lys Thr Ile Ser Lys Ala Lys Gly Gln Pro Arg Glu Pro
        355                 360                 365
Gln Val Tyr Thr Leu Pro Pro Ser Arg Glu Glu Met Thr Lys Asn Gln
    370                 375                 380
Val Ser Leu Thr Cys Leu Val Lys Gly Phe Tyr Pro Ser Asp Ile Ala
385                 390                 395                 400
Val Glu Trp Glu Ser Asn Gly Gln Pro Glu Asn Asn Tyr Lys Thr Thr
                405                 410                 415
Pro Pro Val Leu Asp Ser Asp Gly Ser Phe Phe Leu Tyr Ser Lys Leu
            420                 425                 430
Thr Val Asp Lys Ser Arg Trp Gln Gln Gly Asn Val Phe Ser Cys Ser
        435                 440                 445
Val Met His Glu Ala Leu His Asn His Tyr Thr Gln Lys Ser Leu Ser
    450                 455                 460
Leu Ser Pro Gly Lys
465
```

<210> 5
<211> 469
<212> PRT
<213> Artificial Sequence
<220>
<223> Heavy chain of variant of CHIR-12.12 human anti-CD40 antibody
<400> 5

```
Met Glu Phe Gly Leu Ser Trp Val Phe Leu Val Ala Ile Leu Arg Gly
 1               5                  10                  15
Val Gln Cys Gln Val Gln Leu Val Glu Ser Gly Gly Gly Val Val Gln
            20                  25                  30
Pro Gly Arg Ser Leu Arg Leu Ser Cys Ala Ala Ser Gly Phe Thr Phe
        35                  40                  45
Ser Ser Tyr Gly Met His Trp Val Arg Gln Ala Pro Gly Lys Gly Leu
    50                  55                  60
Glu Trp Val Ala Val Ile Ser Tyr Glu Glu Ser Asn Arg Tyr His Ala
65                  70                  75                  80
Asp Ser Val Lys Gly Arg Phe Thr Ile Ser Arg Asp Asn Ser Lys Ile
                85                  90                  95
Thr Leu Tyr Leu Gln Met Asn Ser Leu Arg Thr Glu Asp Thr Ala Val
            100                 105                 110
Tyr Tyr Cys Ala Arg Asp Gly Gly Ile Ala Ala Pro Gly Pro Asp Tyr
        115                 120                 125
Trp Gly Gln Gly Thr Leu Val Thr Val Ser Ser Ala Ser Thr Lys Gly
    130                 135                 140
Pro Ser Val Phe Pro Leu Ala Pro Ser Ser Lys Ser Thr Ser Gly Gly
145                 150                 155                 160
Thr Ala Ala Leu Gly Cys Leu Val Lys Asp Tyr Phe Pro Glu Pro Val
                165                 170                 175
Thr Val Ser Trp Asn Ser Gly Ala Leu Thr Ser Gly Val His Thr Phe
            180                 185                 190
Pro Ala Val Leu Gln Ser Ser Gly Leu Tyr Ser Leu Ser Ser Val Val
        195                 200                 205
Thr Val Pro Ser Ser Ser Leu Gly Thr Gln Thr Tyr Ile Cys Asn Val
    210                 215                 220
Asn His Lys Pro Ser Asn Thr Lys Val Asp Lys Arg Val Glu Pro Lys
225                 230                 235                 240
Ser Cys Asp Lys Thr His Thr Cys Pro Pro Cys Pro Ala Pro Glu Leu
                245                 250                 255
Leu Gly Gly Pro Ser Val Phe Leu Phe Pro Pro Lys Pro Lys Asp Thr
            260                 265                 270
Leu Met Ile Ser Arg Thr Pro Glu Val Thr Cys Val Val Val Asp Val
        275                 280                 285
Ser His Glu Asp Pro Glu Val Lys Phe Asn Trp Tyr Val Asp Gly Val
    290                 295                 300
Glu Val His Asn Ala Lys Thr Lys Pro Arg Glu Glu Gln Tyr Asn Ser
305                 310                 315                 320
Thr Tyr Arg Val Val Ser Val Leu Thr Val Leu His Gln Asp Trp Leu
                325                 330                 335
Asn Gly Lys Glu Tyr Lys Cys Lys Val Ser Asn Lys Ala Leu Pro Ala
            340                 345                 350
Pro Ile Glu Lys Thr Ile Ser Lys Ala Lys Gly Gln Pro Arg Glu Pro
        355                 360                 365
Gln Val Tyr Thr Leu Pro Pro Ser Arg Glu Glu Met Thr Lys Asn Gln
    370                 375                 380
Val Ser Leu Thr Cys Leu Val Lys Gly Phe Tyr Pro Ser Asp Ile Ala
385                 390                 395                 400
Val Glu Trp Glu Ser Asn Gly Gln Pro Glu Asn Asn Tyr Lys Thr Thr
                405                 410                 415
Pro Pro Val Leu Asp Ser Asp Gly Ser Phe Phe Leu Tyr Ser Lys Leu
            420                 425                 430
Thr Val Asp Lys Ser Arg Trp Gln Gln Gly Asn Val Phe Ser Cys Ser
        435                 440                 445
Val Met His Glu Ala Leu His Asn His Tyr Thr Gln Lys Ser Leu Ser


            450                 455                 460
        Leu Ser Pro Gly Lys
        465
```

<210> 6
<211> 239
<212> PRT
<213> Artificial Sequence

<220>
<223> Light chain of CHIR-5.9 human anti-CD40 antibody
<400> 6

```
Met Ala Leu Leu Ala Gln Leu Leu Gly Leu Leu Met Leu Trp Val Pro
 1               5                  10                  15
Gly Ser Ser Gly Ala Ile Val Met Thr Gln Pro Pro Leu Ser Ser Pro
            20                  25                  30
Val Thr Leu Gly Gln Pro Ala Ser Ile Ser Cys Arg Ser Ser Gln Ser
        35                  40                  45
Leu Val His Ser Asp Gly Asn Thr Tyr Leu Asn Trp Leu Gln Gln Arg
    50                  55                  60
Pro Gly Gln Pro Pro Arg Leu Leu Ile Tyr Lys Phe Phe Arg Arg Leu
65                  70                  75                  80
Ser Gly Val Pro Asp Arg Phe Ser Gly Ser Gly Ala Gly Thr Asp Phe
                85                  90                  95
Thr Leu Lys Ile Ser Arg Val Glu Ala Glu Asp Val Gly Val Tyr Tyr
            100                 105                 110
Cys Met Gln Val Thr Gln Phe Pro His Thr Phe Gly Gln Gly Thr Arg
        115                 120                 125
Leu Glu Ile Lys Arg Thr Val Ala Ala Pro Ser Val Phe Ile Phe Pro
    130                 135                 140
Pro Ser Asp Glu Gln Leu Lys Ser Gly Thr Ala Ser Val Val Cys Leu
145                 150                 155                 160
Leu Asn Asn Phe Tyr Pro Arg Glu Ala Lys Val Gln Trp Lys Val Asp
                165                 170                 175
Asn Ala Leu Gln Ser Gly Asn Ser Gln Glu Ser Val Thr Glu Gln Asp
            180                 185                 190
Ser Lys Asp Ser Thr Tyr Ser Leu Ser Ser Thr Leu Thr Leu Ser Lys
        195                 200                 205
Ala Asp Tyr Glu Lys His Lys Val Tyr Ala Cys Glu Val Thr His Gln
    210                 215                 220
Gly Leu Ser Ser Pro Val Thr Lys Ser Phe Asn Arg Gly Glu Cys
225                 230                 235
```

<210> 7
<211> 474
<212> PRT
<213> Artificial Sequence
<220>
<223> Heavy chain of CHIR-5.9 human anti-CD40 antibody
<400> 7

```
Met Gly Ser Thr Ala Ile Leu Ala Leu Leu Leu Ala Val Leu Gln Gly
 1               5                  10                  15
Val Cys Ala Glu Val Gln Leu Val Gln Ser Gly Ala Glu Val Lys Lys
            20                  25                  30
Pro Gly Glu Ser Leu Lys Ile Ser Cys Lys Gly Ser Gly Tyr Ser Phe
        35                  40                  45
Thr Ser Tyr Trp Ile Gly Trp Val Arg Gln Met Pro Gly Lys Gly Leu
    50                  55                  60
Glu Trp Met Gly Ile Ile Tyr Pro Gly Asp Ser Asp Thr Arg Tyr Ser
65                  70                  75                  80
Pro Ser Phe Gln Gly Gln Val Thr Ile Ser Ala Asp Lys Ser Ile Ser
```

```
                    85                  90                  95
Thr Ala Tyr Leu Gln Trp Ser Ser Leu Lys Ala Ser Asp Thr Ala Met
            100                 105                 110
Tyr Tyr Cys Ala Arg Gly Thr Ala Ala Gly Arg Asp Tyr Tyr Tyr Tyr
        115                 120                 125
Tyr Gly Met Asp Val Trp Gly Gln Gly Thr Thr Val Thr Val Ser Ser
    130                 135                 140
Ala Ser Thr Lys Gly Pro Ser Val Phe Pro Leu Ala Pro Ala Ser Lys
145                 150                 155                 160
Ser Thr Ser Gly Gly Thr Ala Ala Leu Gly Cys Leu Val Lys Asp Tyr
                165                 170                 175
Phe Pro Glu Pro Val Thr Val Ser Trp Asn Ser Gly Ala Leu Thr Ser
            180                 185                 190
Gly Val His Thr Phe Pro Ala Val Leu Gln Ser Ser Gly Leu Tyr Ser
        195                 200                 205
Leu Ser Ser Val Val Thr Val Pro Ser Ser Ser Leu Gly Thr Gln Thr
    210                 215                 220
Tyr Ile Cys Asn Val Asn His Lys Pro Ser Asn Thr Lys Val Asp Lys
225                 230                 235                 240
Arg Val Glu Pro Lys Ser Cys Asp Lys Thr His Thr Cys Pro Pro Cys
                245                 250                 255
Pro Ala Pro Glu Leu Leu Gly Gly Pro Ser Val Phe Leu Phe Pro Pro
            260                 265                 270
Lys Pro Lys Asp Thr Leu Met Ile Ser Arg Thr Pro Glu Val Thr Cys
        275                 280                 285
Val Val Val Asp Val Ser His Glu Asp Pro Glu Val Lys Phe Asn Trp
    290                 295                 300
Tyr Val Asp Gly Val Glu Val His Asn Ala Lys Thr Lys Pro Arg Glu
305                 310                 315                 320
Glu Gln Tyr Asn Ser Thr Tyr Arg Val Val Ser Val Leu Thr Val Leu
                325                 330                 335
His Gln Asp Trp Leu Asn Gly Lys Glu Tyr Lys Cys Lys Val Ser Asn
            340                 345                 350
Lys Ala Leu Pro Ala Pro Ile Glu Lys Thr Ile Ser Lys Ala Lys Gly
        355                 360                 365
Gln Pro Arg Glu Pro Gln Val Tyr Thr Leu Pro Pro Ser Arg Glu Glu
    370                 375                 380
Met Thr Lys Asn Gln Val Ser Leu Thr Cys Leu Val Lys Gly Phe Tyr
385                 390                 395                 400
Pro Ser Asp Ile Ala Val Glu Trp Glu Ser Asn Gly Gln Pro Glu Asn
                405                 410                 415
Asn Tyr Lys Thr Thr Pro Pro Val Leu Asp Ser Asp Gly Ser Phe Phe
            420                 425                 430
Leu Tyr Ser Lys Leu Thr Val Asp Lys Ser Arg Trp Gln Gln Gly Asn
        435                 440                 445
Val Phe Ser Cys Ser Val Met His Glu Ala Leu His Asn His Tyr Thr
    450                 455                 460
Gln Lys Ser Leu Ser Leu Ser Pro Gly Lys
465                 470
```

<210> 8
<211> 474
<212> PRT
<213> Artificial Sequence
<220>
<223> Heavy chain of variant CHIR-5.9 human anti-CD40 antibody
<400> 8

```
Met Gly Ser Thr Ala Ile Leu Ala Leu Leu Leu Ala Val Leu Gln Gly
 1               5                  10                  15
Val Cys Ala Glu Val Gln Leu Val Gln Ser Gly Ala Glu Val Lys Lys
            20                  25                  30
```

Pro Gly Glu Ser Leu Lys Ile Ser Cys Lys Gly Ser Gly Tyr Ser Phe
35 40 45
Thr Ser Tyr Trp Ile Gly Trp Val Arg Gln Met Pro Gly Lys Gly Leu
50 55 60
Glu Trp Met Gly Ile Ile Tyr Pro Gly Asp Ser Asp Thr Arg Tyr Ser
65 70 75 80
Pro Ser Phe Gln Gly Gln Val Thr Ile Ser Ala Asp Lys Ser Ile Ser
85 90 95
Thr Ala Tyr Leu Gln Trp Ser Ser Leu Lys Ala Ser Asp Thr Ala Met
100 105 110
Tyr Tyr Cys Ala Arg Gly Thr Ala Ala Gly Arg Asp Tyr Tyr Tyr Tyr
115 120 125
Tyr Gly Met Asp Val Trp Gly Gln Gly Thr Thr Val Thr Val Ser Ser
130 135 140
Ala Ser Thr Lys Gly Pro Ser Val Phe Pro Leu Ala Pro Ser Ser Lys
145 150 155 160
Ser Thr Ser Gly Gly Thr Ala Ala Leu Gly Cys Leu Val Lys Asp Tyr
165 170 175
Phe Pro Glu Pro Val Thr Val Ser Trp Asn Ser Gly Ala Leu Thr Ser
180 185 190
Gly Val His Thr Phe Pro Ala Val Leu Gln Ser Ser Gly Leu Tyr Ser
195 200 205
Leu Ser Ser Val Val Thr Val Pro Ser Ser Ser Leu Gly Thr Gln Thr
210 215 220
Tyr Ile Cys Asn Val Asn His Lys Pro Ser Asn Thr Lys Val Asp Lys
225 230 235 240
Arg Val Glu Pro Lys Ser Cys Asp Lys Thr His Thr Cys Pro Pro Cys
245 250 255
Pro Ala Pro Glu Leu Leu Gly Gly Pro Ser Val Phe Leu Phe Pro Pro
260 265 270
Lys Pro Lys Asp Thr Leu Met Ile Ser Arg Thr Pro Glu Val Thr Cys
275 280 285
Val Val Val Asp Val Ser His Glu Asp Pro Glu Val Lys Phe Asn Trp
290 295 300
Tyr Val Asp Gly Val Glu Val His Asn Ala Lys Thr Lys Pro Arg Glu
305 310 315 320
Glu Gln Tyr Asn Ser Thr Tyr Arg Val Val Ser Val Leu Thr Val Leu
325 330 335
His Gln Asp Trp Leu Asn Gly Lys Glu Tyr Lys Cys Lys Val Ser Asn
340 345 350
Lys Ala Leu Pro Ala Pro Ile Glu Lys Thr Ile Ser Lys Ala Lys Gly
355 360 365
Gln Pro Arg Glu Pro Gln Val Tyr Thr Leu Pro Pro Ser Arg Glu Glu
370 375 380
Met Thr Lys Asn Gln Val Ser Leu Thr Cys Leu Val Lys Gly Phe Tyr
385 390 395 400
Pro Ser Asp Ile Ala Val Glu Trp Glu Ser Asn Gly Gln Pro Glu Asn
405 410 415
Asn Tyr Lys Thr Thr Pro Pro Val Leu Asp Ser Asp Gly Ser Phe Phe
420 425 430
Leu Tyr Ser Lys Leu Thr Val Asp Lys Ser Arg Trp Gln Gln Gly Asn
435 440 445
Val Phe Ser Cys Ser Val Met His Glu Ala Leu His Asn His Tyr Thr
450 455 460
Gln Lys Ser Leu Ser Leu Ser Pro Gly Lys
465 470

<210> 9
<211> 612
<212> DNA
<213> Homo sapiens
<220>
<221> misc_feature
<222> (0)..(0)
<223> Coding sequence for short isoform of human CD40
<221> CDS
<222> (1)...(612)

<400> 9

```
atg gtt cgt ctg cct ctg cag tgc gtc ctc tgg ggc tgc ttg ctg acc    48
Met Val Arg Leu Pro Leu Gln Cys Val Leu Trp Gly Cys Leu Leu Thr
 1               5                  10                  15

gct gtc cat cca gaa cca ccc act gca tgc aga gaa aaa cag tac cta    96
Ala Val His Pro Glu Pro Pro Thr Ala Cys Arg Glu Lys Gln Tyr Leu
            20                  25                  30

ata aac agt cag tgc tgt tct ttg tgc cag cca gga cag aaa ctg gtg    144
Ile Asn Ser Gln Cys Cys Ser Leu Cys Gln Pro Gly Gln Lys Leu Val
        35                  40                  45

agt gac tgc aca gag ttc act gaa acg gaa tgc ctt cct tgc ggt gaa    192
Ser Asp Cys Thr Glu Phe Thr Glu Thr Glu Cys Leu Pro Cys Gly Glu
    50                  55                  60

agc gaa ttc cta gac acc tgg aac aga gag aca cac tgc cac cag cac    240
Ser Glu Phe Leu Asp Thr Trp Asn Arg Glu Thr His Cys His Gln His
 65                  70                  75                  80

aaa tac tgc gac ccc aac cta ggg ctt cgg gtc cag cag aag ggc acc    288
Lys Tyr Cys Asp Pro Asn Leu Gly Leu Arg Val Gln Gln Lys Gly Thr
                85                  90                  95

tca gaa aca gac acc atc tgc acc tgt gaa gaa ggc tgg cac tgt acg    336
Ser Glu Thr Asp Thr Ile Cys Thr Cys Glu Glu Gly Trp His Cys Thr
            100                 105                 110

agt gag gcc tgt gag agc tgt gtc ctg cac cgc tca tgc tcg ccc ggc    384
Ser Glu Ala Cys Glu Ser Cys Val Leu His Arg Ser Cys Ser Pro Gly
        115                 120                 125

ttt ggg gtc aag cag att gct aca ggg gtt tct gat acc atc tgc gag    432
Phe Gly Val Lys Gln Ile Ala Thr Gly Val Ser Asp Thr Ile Cys Glu
    130                 135                 140

ccc tgc cca gtc ggc ttc ttc tcc aat gtg tca tct gct ttc gaa aaa    480
Pro Cys Pro Val Gly Phe Phe Ser Asn Val Ser Ser Ala Phe Glu Lys
145                 150                 155                 160

tgt cac cct tgg aca agg tcc cca gga tcg gct gag agc cct ggt ggt    528
Cys His Pro Trp Thr Arg Ser Pro Gly Ser Ala Glu Ser Pro Gly Gly
                165                 170                 175

gat ccc cat cat ctt cgg gat cct gtt tgc cat cct ctt ggt gct ggt    576
Asp Pro His His Leu Arg Asp Pro Val Cys His Pro Leu Gly Ala Gly
            180                 185                 190

ctt tat caa aaa ggt ggc caa gaa gcc aac caa taa                    612
Leu Tyr Gln Lys Gly Gly Gln Glu Ala Asn Gln  *
        195                 200
```

<210> 10
<211> 203
<212> PRT
<213> Homo sapiens
<400> 10

```
Met Val Arg Leu Pro Leu Gln Cys Val Leu Trp Gly Cys Leu Leu Thr
```

```
1               5                   10                  15
Ala Val His Pro Glu Pro Pro Thr Ala Cys Arg Glu Lys Gln Tyr Leu
            20                  25                  30
Ile Asn Ser Gln Cys Cys Ser Leu Cys Gln Pro Gly Gln Lys Leu Val
        35                  40                  45
Ser Asp Cys Thr Glu Phe Thr Glu Thr Glu Cys Leu Pro Cys Gly Glu
    50                  55                  60
Ser Glu Phe Leu Asp Thr Trp Asn Arg Glu Thr His Cys His Gln His
65                  70                  75                  80
Lys Tyr Cys Asp Pro Asn Leu Gly Leu Arg Val Gln Gln Lys Gly Thr
                85                  90                  95
Ser Glu Thr Asp Thr Ile Cys Thr Cys Glu Glu Gly Trp His Cys Thr
            100                 105                 110
Ser Glu Ala Cys Glu Ser Cys Val Leu His Arg Ser Cys Ser Pro Gly
        115                 120                 125
Phe Gly Val Lys Gln Ile Ala Thr Gly Val Ser Asp Thr Ile Cys Glu
    130                 135                 140
Pro Cys Pro Val Gly Phe Phe Ser Asn Val Ser Ser Ala Phe Glu Lys
145                 150                 155                 160
Cys His Pro Trp Thr Arg Ser Pro Gly Ser Ala Glu Ser Pro Gly Gly
                165                 170                 175
Asp Pro His His Leu Arg Asp Pro Val Cys His Pro Leu Gly Ala Gly
            180                 185                 190
Leu Tyr Gln Lys Gly Gly Gln Glu Ala Asn Gln
        195                 200
```

<210> 11
<211> 834
<212> DNA
<213> Homo sapiens
<220>
<221> misc_feature
<222> (0)...(0)
<223> Coding sequence for long isoform of human CD40
<221> CDS
<222> (1)...(834)
<400> 11

```
atg gtt cgt ctg cct ctg cag tgc gtc ctc tgg ggc tgc ttg ctg acc    48
Met Val Arg Leu Pro Leu Gln Cys Val Leu Trp Gly Cys Leu Leu Thr
 1               5                  10                  15

gct gtc cat cca gaa cca ccc act gca tgc aga gaa aaa cag tac cta    96
Ala Val His Pro Glu Pro Pro Thr Ala Cys Arg Glu Lys Gln Tyr Leu
            20                  25                  30

ata aac agt cag tgc tgt tct ttg tgc cag cca gga cag aaa ctg gtg    144
Ile Asn Ser Gln Cys Cys Ser Leu Cys Gln Pro Gly Gln Lys Leu Val
        35                  40                  45

agt gac tgc aca gag ttc act gaa acg gaa tgc ctt cct tgc ggt gaa    192
Ser Asp Cys Thr Glu Phe Thr Glu Thr Glu Cys Leu Pro Cys Gly Glu
    50                  55                  60

agc gaa ttc cta gac acc tgg aac aga gag aca cac tgc cac cag cac    240
Ser Glu Phe Leu Asp Thr Trp Asn Arg Glu Thr His Cys His Gln His
 65                  70                  75                  80

aaa tac tgc gac ccc aac cta ggg ctt cgg gtc cag cag aag ggc acc    288
Lys Tyr Cys Asp Pro Asn Leu Gly Leu Arg Val Gln Gln Lys Gly Thr
                85                  90                  95

tca gaa aca gac acc atc tgc acc tgt gaa gaa ggc tgg cac tgt acg    336
```

```
Ser Glu Thr Asp Thr Ile Cys Thr Cys Glu Glu Gly Trp His Cys Thr
        100                 105                 110

agt gag gcc tgt gag agc tgt gtc ctg cac cgc tca tgc tcg ccc ggc    384
Ser Glu Ala Cys Glu Ser Cys Val Leu His Arg Ser Cys Ser Pro Gly
    115                 120                 125

ttt ggg gtc aag cag att gct aca ggg gtt tct gat acc atc tgc gag    432
Phe Gly Val Lys Gln Ile Ala Thr Gly Val Ser Asp Thr Ile Cys Glu
    130                 135                 140

ccc tgc cca gtc ggc ttc ttc tcc aat gtg tca tct gct ttc gaa aaa    480
Pro Cys Pro Val Gly Phe Phe Ser Asn Val Ser Ser Ala Phe Glu Lys
145                 150                 155                 160

tgt cac cct tgg aca agc tgt gag acc aaa gac ctg gtt gtg caa cag    528
Cys His Pro Trp Thr Ser Cys Glu Thr Lys Asp Leu Val Val Gln Gln
            165                 170                 175

gca ggc aca aac aag act gat gtt gtc tgt ggt ccc cag gat cgg ctg    576
Ala Gly Thr Asn Lys Thr Asp Val Val Cys Gly Pro Gln Asp Arg Leu
        180                 185                 190

aga gcc ctg gtg gtg atc ccc atc atc ttc ggg atc ctg ttt gcc atc    624
Arg Ala Leu Val Val Ile Pro Ile Ile Phe Gly Ile Leu Phe Ala Ile
    195                 200                 205

ctc ttg gtg ctg gtc ttt atc aaa aag gtg gcc aag aag cca acc aat    672
Leu Leu Val Leu Val Phe Ile Lys Lys Val Ala Lys Lys Pro Thr Asn
    210                 215                 220

aag gcc ccc cac ccc aag cag gaa ccc cag gag atc aat ttt ccc gac    720
Lys Ala Pro His Pro Lys Gln Glu Pro Gln Glu Ile Asn Phe Pro Asp
225                 230                 235                 240

gat ctt cct ggc tcc aac act gct gct cca gtg cag gag act tta cat    768
Asp Leu Pro Gly Ser Asn Thr Ala Ala Pro Val Gln Glu Thr Leu His
            245                 250                 255

gga tgc caa ccg gtc acc cag gag gat ggc aaa gag agt cgc atc tca    816
Gly Cys Gln Pro Val Thr Gln Glu Asp Gly Lys Glu Ser Arg Ile Ser
        260                 265                 270

gtg cag gag aga cag tga                                            834
Val Gln Glu Arg Gln  *
    275
```

<210> 12
<211> 277
<212> PRT
<213> Homo sapiens
<400> 12

```
Met Val Arg Leu Pro Leu Gln Cys Val Leu Trp Gly Cys Leu Leu Thr
1               5                   10                  15
Ala Val His Pro Glu Pro Pro Thr Ala Cys Arg Glu Lys Gln Tyr Leu
            20                  25                  30
Ile Asn Ser Gln Cys Cys Ser Leu Cys Gln Pro Gly Gln Lys Leu Val
        35                  40                  45
Ser Asp Cys Thr Glu Phe Thr Glu Thr Glu Cys Leu Pro Cys Gly Glu
    50                  55                  60
Ser Glu Phe Leu Asp Thr Trp Asn Arg Glu Thr His Cys His Gln His
65                  70                  75                  80
Lys Tyr Cys Asp Pro Asn Leu Gly Leu Arg Val Gln Gln Lys Gly Thr
                85                  90                  95
```

```
Ser Glu Thr Asp Thr Ile Cys Thr Cys Glu Glu Gly Trp His Cys Thr
            100                 105                 110
Ser Glu Ala Cys Glu Ser Cys Val Leu His Arg Ser Cys Ser Pro Gly
        115                 120                 125
Phe Gly Val Lys Gln Ile Ala Thr Gly Val Ser Asp Thr Ile Cys Glu
    130                 135                 140
Pro Cys Pro Val Gly Phe Phe Ser Asn Val Ser Ser Ala Phe Glu Lys
145                 150                 155                 160
Cys His Pro Trp Thr Ser Cys Glu Thr Lys Asp Leu Val Val Gln Gln
                165                 170                 175
Ala Gly Thr Asn Lys Thr Asp Val Val Cys Gly Pro Gln Asp Arg Leu
            180                 185                 190
Arg Ala Leu Val Val Ile Pro Ile Ile Phe Gly Ile Leu Phe Ala Ile
        195                 200                 205
Leu Leu Val Leu Val Phe Ile Lys Lys Val Ala Lys Lys Pro Thr Asn
    210                 215                 220
Lys Ala Pro His Pro Lys Gln Glu Pro Gln Glu Ile Asn Phe Pro Asp
225                 230                 235                 240
Asp Leu Pro Gly Ser Asn Thr Ala Ala Pro Val Gln Glu Thr Leu His
                245                 250                 255
Gly Cys Gln Pro Val Thr Gln Glu Asp Gly Lys Glu Ser Arg Ile Ser
            260                 265                 270
Val Gln Glu Arg Gln
        275
```

<210> 13
<211> 459
<212> DNA
<213> Homo sapiens
<220>
<221> misc_feature
<222> (0)...(0)
<223> Human IL-2 precursor
<221> CDS
<222> (1)...(459)
<400> 13

```
atg tac agg atg caa ctc ctg tct tgc att gca cta agt ctt gca ctt     48
Met Tyr Arg Met Gln Leu Leu Ser Cys Ile Ala Leu Ser Leu Ala Leu
 1               5                  10                  15

gtc gca aac agt gca cct act tca agt tct aca aag aaa aca cag cta     96
Val Ala Asn Ser Ala Pro Thr Ser Ser Ser Thr Lys Lys Thr Gln Leu
            20                  25                  30

caa ctg gag cat tta ctg ctg gat tta cag atg att ttg aat gga att     144
Gln Leu Glu His Leu Leu Leu Asp Leu Gln Met Ile Leu Asn Gly Ile
        35                  40                  45

aat aat tac aag aat ccc aaa ctc acc agg atg ctc aca ttt aag ttt     192
Asn Asn Tyr Lys Asn Pro Lys Leu Thr Arg Met Leu Thr Phe Lys Phe
    50                  55                  60

tac atg ccc aag aag gcc aca gaa ctg aaa cat ctt cag tgt cta gaa     240
Tyr Met Pro Lys Lys Ala Thr Glu Leu Lys His Leu Gln Cys Leu Glu
65                  70                  75                  80

gaa gaa ctc aaa cct ctg gag gaa gtg cta aat tta gct caa agc aaa     288
Glu Glu Leu Lys Pro Leu Glu Glu Val Leu Asn Leu Ala Gln Ser Lys
                85                  90                  95

aac ttt cac tta aga ccc agg gac tta atc agc aat atc aac gta ata     336
Asn Phe His Leu Arg Pro Arg Asp Leu Ile Ser Asn Ile Asn Val Ile
```

```
            100                 105                 110

gtt ctg gaa cta aag gga tct gaa aca aca ttc atg tgt gaa tat gct    384
Val Leu Glu Leu Lys Gly Ser Glu Thr Thr Phe Met Cys Glu Tyr Ala
        115                 120                 125

gat gag aca gca acc att gta gaa ttt ctg aac aga tgg att acc ttt    432
Asp Glu Thr Ala Thr Ile Val Glu Phe Leu Asn Arg Trp Ile Thr Phe
    130                 135                 140

tgt cag agc atc atc tca aca ctg act                                459
Cys Gln Ser Ile Ile Ser Thr Leu Thr
145                 150
```

<210> 14
<211> 153
<212> PRT
<213> Homo sapiens
<400> 14

```
Met Tyr Arg Met Gln Leu Leu Ser Cys Ile Ala Leu Ser Leu Ala Leu
 1               5                  10                  15
Val Ala Asn Ser Ala Pro Thr Ser Ser Ser Thr Lys Lys Thr Gln Leu
            20                  25                  30
Gln Leu Glu His Leu Leu Leu Asp Leu Gln Met Ile Leu Asn Gly Ile
        35                  40                  45
Asn Asn Tyr Lys Asn Pro Lys Leu Thr Arg Met Leu Thr Phe Lys Phe
    50                  55                  60
Tyr Met Pro Lys Lys Ala Thr Glu Leu Lys His Leu Gln Cys Leu Glu
65                  70                  75                  80
Glu Glu Leu Lys Pro Leu Glu Glu Val Leu Asn Leu Ala Gln Ser Lys
                85                  90                  95
Asn Phe His Leu Arg Pro Arg Asp Leu Ile Ser Asn Ile Asn Val Ile
            100                 105                 110
Val Leu Glu Leu Lys Gly Ser Glu Thr Thr Phe Met Cys Glu Tyr Ala
        115                 120                 125
Asp Glu Thr Ala Thr Ile Val Glu Phe Leu Asn Arg Trp Ile Thr Phe
    130                 135         .       140
Cys Gln Ser Ile Ile Ser Thr Leu Thr
145                 150
```

<210> 15
<211> 399
<212> DNA
<213> Homo sapiens
<220>
<221> misc_feature
<222> (0)...(0)
<223> Mature human IL-2
<221> CDS
<222> (1)...(399)
<400> 15

```
gca cct act tca agt tct aca aag aaa aca cag cta caa ctg gag cat    48
Ala Pro Thr Ser Ser Ser Thr Lys Lys Thr Gln Leu Gln Leu Glu His
 1               5                  10                  15

tta ctg ctg gat tta cag atg att ttg aat gga att aat aat tac aag    96
Leu Leu Leu Asp Leu Gln Met Ile Leu Asn Gly Ile Asn Asn Tyr Lys
            20                  25                  30

aat ccc aaa ctc acc agg atg ctc aca ttt aag ttt tac atg ccc aag    144
```

```
Asn Pro Lys Leu Thr Arg Met Leu Thr Phe Lys Phe Tyr Met Pro Lys
        35                  40                  45

aag gcc aca gaa ctg aaa cat ctt cag tgt cta gaa gaa gaa ctc aaa     192
Lys Ala Thr Glu Leu Lys His Leu Gln Cys Leu Glu Glu Glu Leu Lys
    50                  55                  60

cct ctg gag gaa gtg cta aat tta gct caa agc aaa aac ttt cac tta     240
Pro Leu Glu Glu Val Leu Asn Leu Ala Gln Ser Lys Asn Phe His Leu
65                  70                  75                  80

aga ccc agg gac tta atc agc aat atc aac gta ata gtt ctg gaa cta     288
Arg Pro Arg Asp Leu Ile Ser Asn Ile Asn Val Ile Val Leu Glu Leu
                85                  90                  95

aag gga tct gaa aca aca ttc atg tgt gaa tat gct gat gag aca gca     336
Lys Gly Ser Glu Thr Thr Phe Met Cys Glu Tyr Ala Asp Glu Thr Ala
            100                 105                 110

acc att gta gaa ttt ctg aac aga tgg att acc ttt tgt cag agc atc     384
Thr Ile Val Glu Phe Leu Asn Arg Trp Ile Thr Phe Cys Gln Ser Ile
        115                 120                 125

atc tca aca ctg act                                                 399
Ile Ser Thr Leu Thr
    130
```

<210> 16
<211> 133
<212> PRT
<213> Homo sapiens
<400> 16

```
Ala Pro Thr Ser Ser Ser Thr Lys Lys Thr Gln Leu Gln Leu Glu His
1               5                   10                  15
Leu Leu Leu Asp Leu Gln Met Ile Leu Asn Gly Ile Asn Asn Tyr Lys
            20                  25                  30
Asn Pro Lys Leu Thr Arg Met Leu Thr Phe Lys Phe Tyr Met Pro Lys
        35                  40                  45
Lys Ala Thr Glu Leu Lys His Leu Gln Cys Leu Glu Glu Glu Leu Lys
    50                  55                  60
Pro Leu Glu Glu Val Leu Asn Leu Ala Gln Ser Lys Asn Phe His Leu
65                  70                  75                  80
Arg Pro Arg Asp Leu Ile Ser Asn Ile Asn Val Ile Val Leu Glu Leu
                85                  90                  95
Lys Gly Ser Glu Thr Thr Phe Met Cys Glu Tyr Ala Asp Glu Thr Ala
            100                 105                 110
Thr Ile Val Glu Phe Leu Asn Arg Trp Ile Thr Phe Cys Gln Ser Ile
        115                 120                 125
Ile Ser Thr Leu Thr
    130
```

<210> 17
<211> 396
<212> DNA
<213> Artificial Sequence
<220>
<223> des-alanyl 1, C125S human IL-2 mutein
<221> CDS
<222> (1)...(396)
<400> 17

```
cct act tca agt tct aca aag aaa aca cag cta caa ctg gag cat tta    48
Pro Thr Ser Ser Ser Thr Lys Lys Thr Gln Leu Gln Leu Glu His Leu
 1               5                  10                  15

ctg ctg gat tta cag atg att ttg aat gga att aat aat tac aag aat    96
Leu Leu Asp Leu Gln Met Ile Leu Asn Gly Ile Asn Asn Tyr Lys Asn
            20                  25                  30

ccc aaa ctc acc agg atg ctc aca ttt aag ttt tac atg ccc aag aag   144
Pro Lys Leu Thr Arg Met Leu Thr Phe Lys Phe Tyr Met Pro Lys Lys
        35                  40                  45

gcc aca gaa ctg aaa cat ctt cag tgt cta gaa gaa gaa ctc aaa cct   192
Ala Thr Glu Leu Lys His Leu Gln Cys Leu Glu Glu Glu Leu Lys Pro
    50                  55                  60

ctg gag gaa gtg cta aat tta gct caa agc aaa aac ttt cac tta aga   240
Leu Glu Glu Val Leu Asn Leu Ala Gln Ser Lys Asn Phe His Leu Arg
65                  70                  75                  80

ccc agg gac tta atc agc aat atc aac gta ata gtt ctg gaa cta aag   288
Pro Arg Asp Leu Ile Ser Asn Ile Asn Val Ile Val Leu Glu Leu Lys
                85                  90                  95

gga tct gaa aca aca ttc atg tgt gaa tat gct gat gag aca gca acc   336
Gly Ser Glu Thr Thr Phe Met Cys Glu Tyr Ala Asp Glu Thr Ala Thr
            100                 105                 110

att gta gaa ttt ctg aac aga tgg att acc ttt tct cag agc atc atc   384
Ile Val Glu Phe Leu Asn Arg Trp Ile Thr Phe Ser Gln Ser Ile Ile
        115                 120                 125

tca aca ctg act                                                   396
Ser Thr Leu Thr
    130
```

<210> 18
<211> 132
<212> PRT
<213> Artificial Sequence
<220>
<223> des-alanyl 1, C125S human IL-2 mutein
<400> 18

```
Pro Thr Ser Ser Ser Thr Lys Lys Thr Gln Leu Gln Leu Glu His Leu
 1               5                  10                  15
Leu Leu Asp Leu Gln Met Ile Leu Asn Gly Ile Asn Asn Tyr Lys Asn
            20                  25                  30
Pro Lys Leu Thr Arg Met Leu Thr Phe Lys Phe Tyr Met Pro Lys Lys
        35                  40                  45
Ala Thr Glu Leu Lys His Leu Gln Cys Leu Glu Glu Glu Leu Lys Pro
    50                  55                  60
Leu Glu Glu Val Leu Asn Leu Ala Gln Ser Lys Asn Phe His Leu Arg
65                  70                  75                  80
Pro Arg Asp Leu Ile Ser Asn Ile Asn Val Ile Val Leu Glu Leu Lys
                85                  90                  95
Gly Ser Glu Thr Thr Phe Met Cys Glu Tyr Ala Asp Glu Thr Ala Thr
            100                 105                 110
Ile Val Glu Phe Leu Asn Arg Trp Ile Thr Phe Ser Gln Ser Ile Ile
        115                 120                 125
Ser Thr Leu Thr
    130
```

## Description

FIELD OF THE INVENTION

The present invention relates to methods of combination therapy for cancers comprising neoplastic cells expressing the CD40 antigen, including B cell-related cancers and solid tumors.

BACKGROUND OF THE INVENTION

Over one million Americans are diagnosed with cancer every year. Leukemia, lymphoma, and myeloma strike over 100,000 individuals in the U.S. alone. Solid tumors such as breast cancer, ovarian cancer, and lung cancer, affect an even greater number of individuals. To combat these diseases, immunotherapy has been developed. Generally, immunotherapy boosts the patient's anti-tumor response against target tumor cells. Cytokine therapy is one method of immunotherapy that activates immunosurveillance, especially against tumors and tumor antigens. Alternatively, cancer cells can be directly targeted using antibody therapy. These antibodies are specific to individual cell-surface antigens that are expressed on cancer cells, and the clinical trial results are promising. Successful therapeutic results have been obtained using antibodies that target such antigens as CD20 (expressed on normal B cells and many B cell lymphomas) and HER2 (expressed in many breast carcinomas).

A large percentage of cancer cases are characterized by an outgrowth of neoplastic cells expressing the CD40 antigen. CD40 is a 55 kDa cell-surface antigen present on the surface of both normal and neoplastic human B cells, dendritic cells, other antigen presenting cells (APCs), endothelial cells, monocytic cells, and epithelial cells. Binding of the CD40 ligand to CD40 on the B cell membrane provides a positive costimulatory signal that stimulates B cell activation and proliferation, resulting in B cell maturation into a plasma cell that secretes high levels of soluble immunoglobulin. Transformed cells from patients with low- and high-grade B cell lymphomas, B cell acute lymphoblastic leukemia, multiple myeloma, chronic lymphocytic leukemia, and Hodgkin's disease express CD40. CD40 expression is detected in two-thirds of acute myeloblastic leukemia cases and 50% of AIDS-related lymphomas. Malignant B cells from several tumors of B-cell lineage express a high level of CD40 and appear to depend on CD40 signaling for survival and proliferation.

CD40 expression has also been detected in many non-B cell cancers. CD40-expressing carcinomas include urinary bladder carcinoma (Paulie et al. (1989) J. Immunol. 142:590-595; Braesch-Andersen et al. (1989) J. Immunol. 142:562-567), breast carcinoma (Hirano et al. (1999) Blood 93:2999-3007; Wingett et al. (1998) Breast Cancer Res. Treat. 50:27-36); prostate cancer (Rokhlin et al. (1997) Cancer Res. 57:1758-1768), renal cell carcinoma (Kluth et al. (1997) Cancer Res. 57:891-899), undifferentiated nasopharyngeal carcinoma (LTNPC) (Agathanggelou et al. (1995) Am. J. Pathol. 147:1152-1160), squamous cell carcinoma (SCC) (Amo et al. (2000) Eur. J. Dermatol. 10:438-442; Posner et al. (1999) Clin. Cancer Res. 5:2261-2270), thyroid papillary carcinoma (Smith et al. (1999) Thyroid 9:749-755), cutaneous malignant melanoma (van den Oord et al. (1996) Am. J. Pathol. 149:1953-1961), ovarian cancer (Ciaravino et al. (2004) Eur. J. Gynaecol. Oncol. 25:27-32), lung cancer (Sabel et al. (2000) Cancer Immunol. Immunother. 49:101-8), cervical cancer (Altenberg et al. (1999) J. Immunol. 162:4140-4147), gastric carcinoma (Yamaguchi et al. (2003) Int. J. Oncol. 23(6):1697-702), sarcomas (see, for example, Lollini et al. (1998) Clin. Cancer Res. 4(8):1843-849, discussing human osteosarcoma and Ewing's sarcoma), and liver carcinoma (see, for example, Sugimoto et al. (1999) Hepatology30 (4):920-26, discussing human hepatocellular carcinoma). The role of CD40 in these non-B cell cancers is not well understood. Some studies have shown that ligation of CD40 in transformed cells can result in cell death by apoptosis. However, in some cancers such as malignant melanoma and lung cancer, expression of CD40 can be a negative prognostic indicator (see, for example, Sabel et al. (2000) Cancer Immuno. Immunother. 49(2):101-108; Ottaiano et al. (2004) Clin. Cancer Res. 10(8):2824-2831).

Interleukin-2 (IL-2) is a cytokine that is a potent stimulator of natural killer (NK) and T-cell proliferation and function (see, for example, Morgan et al. (1976) Science 193:1007-1011; Weil-Hillman et al. (1989) Cancer Res. 49(13): 3680-3688). IL-2 has anti-tumor activity against a variety of malignancies either alone or when combined with lymphokine-activated killer (LAK) cells or tumor-infiltrating lymphocytes (TIL) (see, for example, Rosenberg et al. (1987) N. Engl. J. Med. 316:889-897; Rosenberg (1988) Ann. Surg. 208:121-135; Topalian et al. (1988) J. Clin. Oncol. 6:839-853; Rosenberg et al. (1988) N. Engl. J. Med. 319:1676-1680; and Weber et al. (1992) J. Clin. Oncol. 10:33-40). Although the anti-tumor activity of IL-2 has best been described in patients with metastatic melanoma and renal cell carcinoma, other diseases, including lymphoma, also appear to respond to treatment with IL-2 (see, for example, Dutcher and Wiernik (1993) Sem. Oncol. 20(6 Suppl. 9):33-40). However, high doses of IL-2 used to achieve positive therapeutic results with respect to tumor growth frequently cause severe side effects, including capillary leakage, hypotension, and neurological changes (see, for example, Duggan et al. (1992) J. Immunotherapy 12:115-122; Gisselbrecht et al. (1994) Blood 83: 2081-2085; and Sznol and Parkinson 1994) Blood 83:2020-2022). Moreover, clinical responses to IL-2 are variable. For example, the median survival rate for renal cell carcinoma patient subgroups treated with IL-2 can be between 28 and 5 months depending on risk factors (Palmer et al. (1992) Ann. Oncol. 3:475-80).

Although any one immunotherapeutic agent may provide a benefit to the patient, further methods are needed to reduce toxicity, improve efficacy, and improve treatment outcomes. In addition, cancer can often become refractory to treatment with single-agent oncotherapy, either as a result of initial resistance to a single antibody therapy or single cytokine therapy, or as a result of resistance that develops during one or more time courses of therapy with the single antibody or the single cytokine.

Consequently, the discovery of a combination immunotherapy that can simultaneously improve the treatment outcomes relative to single-agent oncotherapy and reduce the toxicity of any one immunotherapeutic can greatly reduce the mortality and morbidity of cancer patients especially those suffering from solid tumors, myelomas, leukemias, and lymphomas.

WO 01/83755 WO 02/28480 and WO 02/28904 describe antibodies capable of binding to CD40, methods for producing these antibodies and methods for their use.

Ellmark et al. (Immunology 2002, 106, 456-463) reports on the modulation of the CD40-CD40 ligand interaction using human anti-CD40 single-chain antibody fragments.

## BRIEF SUMMARY OF THE INVENTION

The invention provides an anti-CD40 antibody or antigen-binding fragment thereof, for use in a method of treating a human subject for a cancer comprising neoplastic cells expressing CD40 antigen, said method comprising administering to said subject combination therapy, said therapy comprising administration of an effective amount of the anti-CD40 antibody or antigen-binding fragment thereof in combination with an interleukin-2 (IL-2) or biologically active variant thereof,
wherein said anti-CD40 antibody or antigen-binding fragment thereof is free of significant agonist activity when bound to CD40 antigen and is selected from the group consisting of:

a) an antibody or antigen-binding fragment thereof that binds to an epitope capable of binding the monoclonal antibody CHIR-5.9 obtainable from the hybridoma cell line deposited with the ATCC as Patent Deposit No. PTA-5542 or the monoclonal antibody CHIR-12.12 obtainable from the hybridoma cell line deposited with the ATCC as Patent Deposit No. PTA-5543;
b) an antibody or antigen-binding fragment thereof that binds to an epitope comprising residues 82-87 of the human CD40 sequence shown in SEQ ID NO:10 or SEQ ID NO:12;
c) an antibody or antigen-binding fragment thereof that binds to an epitope comprising residues 82-89 of the human CD40 sequence shown in SEQ ID NO:10 or SEQ ID NO:12; and
d) an antibody or antigen-binding fragment thereof that competes with the monoclonal antibody CHIR-5.9 obtainable from the hybridoma cell line deposited with the ATCC as Patent Deposit No. PTA-5542 or the monoclonal antibody CHIR-12.12 obtainable from the hybridoma cell line deposited with the ATCC as Patent Deposit No. PTA-5543 in a competitive binding assay

The invention also provides the use of an effective amount of an anti-CD40 antibody or antigen-binding fragment thereof in combination with an interleukin-2 (IL-2) or biologically active variant thereof in the manufacture of a medicament for treating a human subject for a cancer comprising neoplastic cells expressing CD40 antigen by combination therapy, wherein said anti-CD40 antibody or antigen-binding fragment thereof is free of significant agonist activity when bound to CD40 antigen and is selected from the group consisting of:

a) an antibody or antigen-binding fragment thereof that binds to an epitope capable of binding the monoclonal antibody CHIR-5.9 obtainable from the hybridoma cell line deposited with the ATCC as Patent Deposit No. PTA-5542 or the monoclonal antibody CHIR-12.12 obtainable from the hybridoma cell line deposited with the ATCC as Patent Deposit No. PTA-5543;
b) an antibody or antigen-binding fragment thereof that binds to an epitope comprising residues 82-87 of the human CD40 sequence shown in SEQ ID NO:10 or SEQ ID NO:12;
c) an antibody or antigen-binding fragment thereof that binds to an epitope comprising residues 82-89 of the human CD40 sequence shown in SEQ ID NO:10 or SEQ ID NO:12; and
d) an antibody or antigen-binding fragment thereof that competes with the monoclonal antibody CHIR-5.9 obtainable from the hybridoma cell line deposited with the ATCC as Patent Deposit No. PTA-5542 or the monoclonal antibody CHIR-12.12 obtainable from the hybridoma cell line deposited with the ATCC as Patent Deposit No. PTA-5543 in a competitive binding assay

Other aspects of the invention are set out in the appended claims.

BRIEF SUMMARY OF THE DISCLOSURE

Methods of treating a subject for a cancer comprising neoplastic cells expressing the CD40 cell surface antigen are disclosed. The methods comprise combination therapy with interleukin-2 (IL-2) or biologically active variant thereof and at least one anti-CD40 antibody or antigen-binding fragment thereof. Administering these two agents in combination provides for greater effectiveness than either agent alone, resulting in a positive therapeutic response. In some embodiments, a synergistic therapeutic effect occurs, making the disclosure especially useful for treating cancers that are refractory to single-agent therapy.

In accordance with the methods disclosed herein, an individual in need thereof is administered a combination of an IL-2 or biologically active variant thereof and an antagonist anti-CD40 antibody or antigen-binding fragment thereof. Suitable interleukin molecules include human IL-2 and biologically active variants thereof, such as the IL-2 mutein aldesleukin (des-alanyl-1, serine-125 human interleukin-2). Suitable antagonist anti-CD40 antibodies for use in the methods disclosed herein include monoclonal antibodies or antigen-binding fragments thereof that are capable of specifically binding to human CD40 antigen expressed on the surface of a human cell. These antagonist anti-CD40 antibodies are free of significant agonist activity but exhibit antagonist activity when bound to CD40 antigen on human cells, particularly when bound to CD40 antigen on neoplastic human cells. Suitable monoclonal anti-CD40 antibodies have human constant regions; preferably they also have wholly or partially humanized framework regions; and most preferably are fully human antibodies or antigen-binding fragments thereof.

Examples of such monoclonal antagonist anti-CD40 antibodies are the antibodies designated herein as CHIR-5.9 and CHIR-12.12, which can be recombinantly produced; the monoclonal antibodies produced by the hybridoma cell lines designated 131.2F8.5.9 (referred to herein as the cell line 5.9) and 153.8E2.D10.D6.12.12 (referred to herein as the cell line 12.12); a monoclonal antibody comprising an amino acid sequence selected from the group consisting of the sequence shown in SEQ ID NO:6, the sequence shown in SEQ ID NO:7, the sequence shown in SEQ ID NO:8, both the sequence shown in ID NO:6 and SEQ ID NO:7, and both the sequence shown in SEQ ID NO:6 and SEQ ID NO:8; a monoclonal antibody comprising an amino acid sequence selected from the group consisting of the sequence shown in SEQ ID NO:2, the sequence shown in SEQ ID NO:4, the sequence shown in SEQ ID NO:5, both the sequence shown in SEQ ID NO:2 and SEQ ID NO:4, and both the sequence shown in SEQ ID NO:2 and SEQ ID NO:5; a monoclonal antibody comprising an amino acid sequence encoded by a nucleic acid molecule comprising a nucleotide sequence selected from the group consisting of the sequence shown in SEQ ID NO:1, the sequence shown in SEQ ID NO:3, and both the sequence shown in SEQ ID NO:1 and SEQ ID NO:3; and antigen-binding fragments of these monoclonal antibodies that retain the capability of specifically binding to human CD40, and which are free of significant agonist activity but exhibit antagonist activity when bound to CD40 antigen on human cells. Examples of such monoclonal anti-CD40 antibodies also include a monoclonal antibody that binds to an epitope capable of binding the monoclonal antibody produced by the hybridoma cell line 12.12 or that produced by the hybridoma cell line 5.9; a monoclonal antibody that binds to an epitope comprising residues 82-87 of the amino acid sequence shown in SEQ ID NO:10 or SEQ ID NO:12; a monoclonal antibody that competes with the monoclonal antibody CHIR-12.12 or CHIR-5.9 in a competitive binding assay; and a monoclonal antibody that is an antigen-binding fragment of the CHIR-12.12 or CHIR-5.9 monoclonal antibody or any of the foregoing monoclonal antibodies, where the fragment retains the capability of specifically binding to human CD40 antigen.

In some embodiments, these two therapeutic agents are concurrently administered as two separate pharmaceutical compositions, one containing IL-2 or biologically active variant thereof, the other containing the antagonist anti-CD40 antibody or suitable antigen-binding fragment thereof, wherein each is administered according to a particular dosing regimen. The pharmaceutical composition comprising the antagonist anti-CD40 antibody or antigen-binding fragment thereof is administered according to a weekly dosing schedule, or alternatively is dosed once every two, three, or four weeks. The antagonist anti-CD40 antibody or antigen-binding fragment thereof can be dosed throughout a treatment period or for a fixed duration within a treatment period. The pharmaceutical composition comprising IL-2 or biologically active variant thereof is administered according to a constant IL-2 dosing regimen, or is administered according to a two-level IL-2 dosing regimen.

The constant IL-2 dosing regimen comprises a time period during which a constant total weekly dose of IL-2 or biologically active variant thereof is administered to the subject followed by a time period off of IL-2 dosing. One or more cycles of a constant IL-2 dosing regimen are administered to a subject in need thereof. The total weekly dose to be administered during each cycle of the constant IL-2 dosing regimen can be administered as a single dose. Alternatively, the total weekly dose administered during each cycle of the constant IL-2 dosing regimen can be partitioned into a series of equivalent doses that are administered according to a two-, three-, four-, five-, six- or seven-times-a-week dosing schedule.

The two-level IL-2 dosing regimen comprises a first time period of IL-2 dosing, wherein a higher total weekly dose of IL-2 or biologically active variant thereof is administered to the subject, followed by a second time period of IL-2 dosing, wherein a lower total weekly dose of IL-2 or biologically active variant thereof is administered to the subject.

The total weekly dose of IL-2 or biologically active variant thereof during the second time period of IL-2 dosing is lower than the total weekly dose of IL-2 or biologically active variant thereof administered during the first time period of IL-2 dosing. The total weekly dose to be administered during the first time period and/or during the second time period of IL-2 dosing can be administered as a single dose. Alternatively, the total weekly dose administered during either or both of the first and second time periods of IL-2 dosing can be partitioned into a series of equivalent doses that are administered according to a two-, three-, four-, five-, six- or seven-times-a-week dosing schedule. The methods disclosed herein also provide for an interruption in the two-level dosing regimen of IL-2, where the subject is given a time period off of IL-2 administration, and, optionally a time period off of the anti-CD40 antibody dosing, between the first and second time periods of the two-level IL-2 dosing regimen. Concurrent therapy with these two therapeutic agents can comprise administering one or more cycles of a two-level IL-2 dosing regimen in combination with the recommended dosing regimen for the antagonist anti-CD40 antibody or suitable antigen-binding fragment thereof.

The methods disclosed herein are useful for treating individuals for a cancer comprising neoplastic cells expressing the CD40 cell surface antigen. Examples of such cancers include, but are not limited to, solid tumors, such as lung, ovarian, bladder, kidney, liver, gastric, prostate, skin, and breast cancer, and sarcomas, and B cell-related cancers, such as non-Hodgkin's lymphomas (high-grade lymphomas, intermediate-grade lymphomas, and low-grade lymphomas), Hodgkin's disease, acute lymphoblastic leukemias, myelomas, chronic lymphocytic leukemias, and myeloblastic leukemias.

## BRIEF DESCRIPTION OF THE DRAWINGS

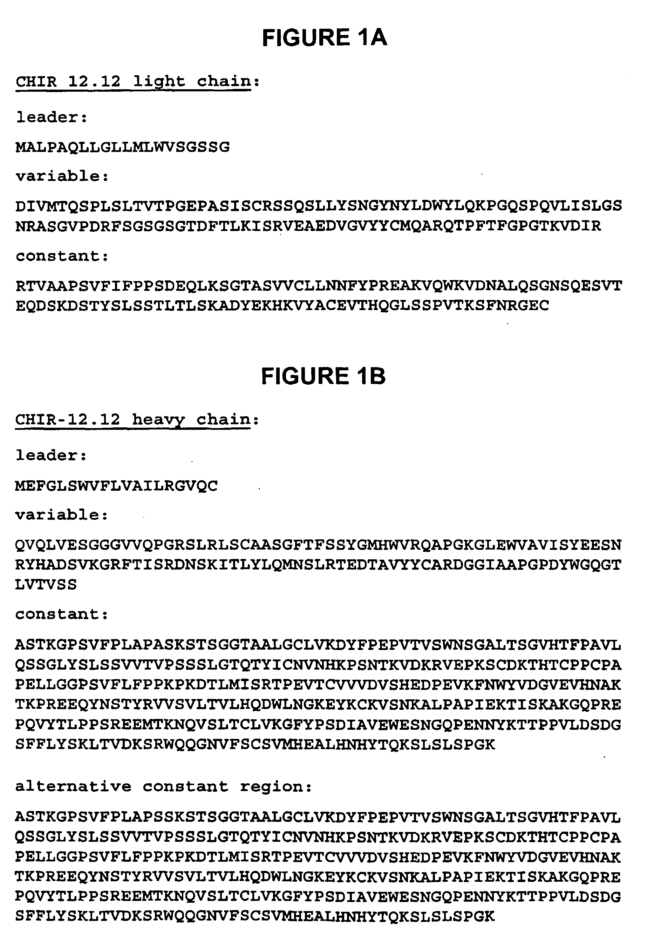

Figure 1 sets forth the amino acid sequences for the light and heavy chains of the mAb CHIR-12.12. The leader (residues 1-20 of SEQ ID NO:2), variable (residues 21-132 of SEQ ID NO:2), and constant (residues 133-239 of SEQ ID NO:2) regions of the light chain are shown in Figure 1A. The leader (residues 1-19 of SEQ ID NO:4), variable (residues 20-139 of SEQ ID NO:4), and constant (residues 140-469 of SEQ ID NO:4) regions of the heavy chain are shown in Figure 1B. The alternative constant region for the heavy chain of the mAb 12.12 shown in Figure 1B reflects a substitution of a serine residue for the alanine residue at position 153 of SEQ ID NO:4. The complete sequence for this variant of the heavy chain of the mAb CHIR-12.12 is set forth in SEQ ID NO:5.

Figure 2 shows the coding sequence for the light chain (Figure 2A; SEQ ID NO:1) and heavy chain (Figure 2B; SEQ ID NO:3) for the mAb CHIR-12.12.

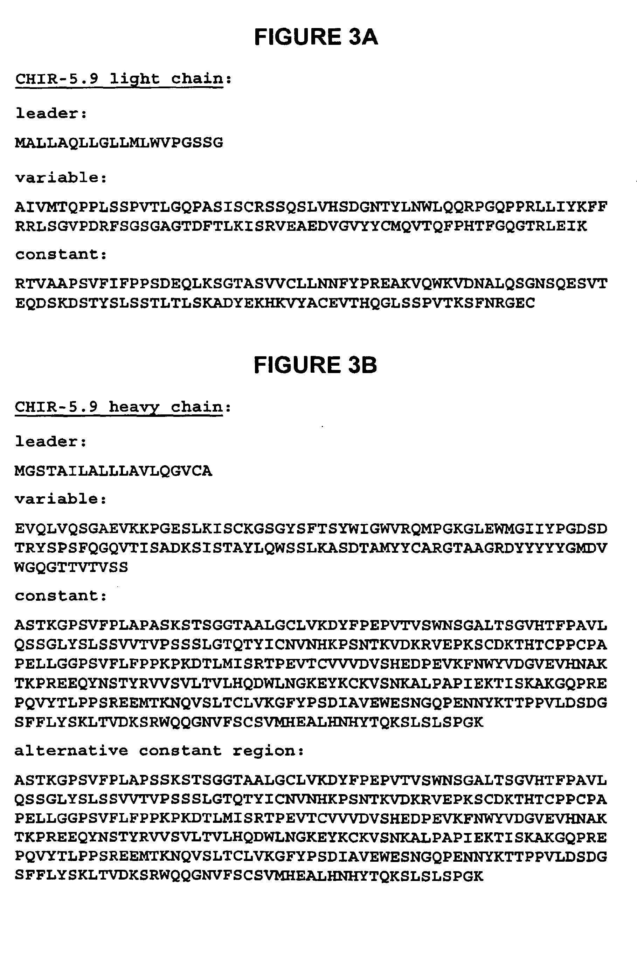

Figure 3 sets forth the amino acid sequences for the light and heavy chains of mAb CHIR-5.9. The leader (residues 1-20 of SEQ ID NO:6), variable (residues 21-132 of SEQ ID NO:6), and constant (residues 133-239 of SEQ ID NO:6) regions of the light chain are shown in Figure 3A. The leader (residues 1-19 of SEQ ID NO:7), variable (residues 20-144 of SEQ ID NO:7), and constant (residues 145-474 of SEQ ID NO:7) regions of the heavy chain are shown in Figure 3B. The alternative constant region for the heavy chain of the mAb CHIR-5.9 shown in Figure 3B reflects a substitution of a serine residue for the alanine residue at position 158 of SEQ ID NO:7. The complete sequence for this variant of the heavy chain of the mAb CHIR-5.9 is set forth in SEQ ID NO:8.

Figure 4 shows the coding sequence (Figure 4A; SEQ ID NO:9) for the short isoform of human CD40 (amino acid sequence shown in Figure 4B; SEQ ID NO:10), and the coding sequence (Figure 4C; SEQ ID NO:11) for the long isoform of human CD40 (amino acid sequence shown in Figure 4D; SEQ ID NO:12).

Figure 5 demostrates enhanced *in vivo* anti-tumor activity of combination treatment with the monoclonal antibody CHIR-12.12 and IL-2 using a human non-Hodgkin's lymphoma (NHL) Namalwa xenograft model.

Figure 6 shows thermal melting temperature of CH1R-12.12 in different pH formulations measured by differential scanning calorimetry (DSC).

## DETAILED DESCRIPTION

The present invention relates to methods for treating a human subject for a cancer comprising neoplastic cells expressing CD40 antigen, including, but not limited to, solid tumors, lymphomas, such as B-cell lymphoma, leukemias, and myelomas. The methods comprise combination therapy with interleukin-2 (IL-2) or biologically active variant thereof and at least one antagonist anti-CD40 antibody or antigen-binding fragment thereof, each of which is administered according to a particular dosing regimen disclosed herein. In some embodiments of the invention, these dosing regimens are followed until the subject is taken off anti-CD40 antibody therapy, for example, when the subject exhibits a complete response or exhibits one or more symptoms of anti-CD40 antibody toxicity, or until the subject is taken off IL-2 therapy due to development of IL-2 toxicity symptoms noted herein below. Where the subject is taken off IL-2 therapy due to toxicity, the anti-CD40 antibody therapy can be continued, following the recommended antibody dosing regimen, or discontinued; and once IL-2 toxicity symptoms have subsided or been resolved, the IL-2 therapy, or the IL-2 therapy and the anti-CD40 antibody therapy where the antibody therapy was discontinued, can be reinstated using the same IL-2 dosing regimen and dose of IL-2 or biologically active variant thereof, the same IL-2 dosing regimen with a lower dose of this therapeutic agent, or a different IL-2 dosing regimen disclosed herein.

In other embodiments of the invention, the dosing regimen for the anti-CD20 antibody therapy is followed for a fixed time period, for example, 2 weeks to 16 weeks, and administered in combination an IL-2 dosing regimen disclosed herein, wherein a given treatment period comprises an overlapping time period in which the subject is receiving both anti-CD40 antibody therapy and IL-2 therapy in accordance with the dosing regimens and doses disclosed herein. In such embodiments, generally the duration of anti-CD20 antibody administration is about 4 weeks to about 12 weeks, including 4, 5, 6, 7, 8, 9, 10, 11, or 12 weeks. The duration of IL-2 administration is a function of the IL-2 dosing regimen used.

Combination therapy with these two therapeutic agents provides for anti-tumor activity. By "anti-tumor activity" is intended a reduction in the rate of cell proliferation, and hence a decline in growth rate of an existing tumor or in a tumor that arises during therapy, and/or destruction of existing neoplastic (tumor) cells or newly formed neoplastic cells, and hence a decrease in the overall size of a tumor during therapy. Subjects undergoing therapy with a combination of IL-2 (or variant thereof) and at least one antagonist anti-CD40 antibody (or antigen-binding fragment thereof) experience a physiological response that is beneficial with respect to treatment of a cancer comprising neoplastic cells expressing CD40 antigen, including, but not limited to, B cell-related cancers and solid tumors as noted herein below.

While the methods disclosed herein are directed to treatment of an existing cancer, it is recognized that the methods may be useful in preventing further tumor outgrowths arising during therapy. Combination therapy with IL-2 (or biologically active variant thereof) and an antagonist anti-CD40 antibody (or antigen-binding fragment thereof) provides a therapeutic benefit that is greater than that provided by the use of either of these therapeutic agents alone. In addition, these two therapeutic agents can be used in combination to treat tumors that are refractory to treatment with either of these agents alone (i.e., single-agent therapy), either as a result of initial resistance to the single antibody therapy or single cytokine therapy, or as a result of resistance that develops during one or more time courses of therapy with the single antibody or the single cytokine. In yet other embodiments, combination therapy with these two therapeutic agents has a synergistic therapeutic effect against tumors that are refractory or non-refractory (i.e., responsive) to single-agent therapy.

The term "oncotherapy" is intended to mean any cancer treatment, including chemotherapy, radiation therapy, immunotherapy, combinations thereof, and the like. Agents used in oncotherapy are referred to herein as "oncotherapeutic agents." The term "immunotherapy" is applicable to any cancer therapy where the immune system is modulated, and includes cytokine administration, antibody administration, antigen administration/vaccination, immune cell administration, immune cell priming, combinations thereof, and the like.

"Tumor," as used herein, refers to all neoplastic cell growth and proliferation, whether malignant or benign, and all pre-cancerous and cancerous cells and tissues. "Neoplastic," as used herein, refers to any form of dysregulated or unregulated cell growth, whether malignant or benign, resulting in abnormal tissue growth. By "CD40 expressing neoplastic cell" is intended any neoplastic cell, whether malignant or benign, that expresses the CD40 cell surface antigen. Methods for detecting CD40 expression in cells are well known in the art and include, but are not limited to, PCR techniques, immunohistochemistry, flow cytometry, Western blot, ELISA, and the like.

The terms "cancer" and "cancerous" refer to or describe the physiological condition in mammals that is typically characterized by unregulated cell growth. Examples of cancer include, but are not limited to, lymphoma, leukemia, myeloma, and solid tumors, including, but not limited to, lung cancer, breast cancer, ovarian cancer, renal carcinomas, hepatic carcinomas, gastric carcinomas, colon cancer, prostate cancer, skin cancer, sarcomas, and the like.

By "refractory" in the context of a cancer is intended the particular cancer is resistant to, or non-responsive to, therapy with a particular therapeutic agent. A cancer can be refractory to therapy with a particular therapeutic agent either from the onset of treatment with the particular therapeutic agent (i.e., non-responsive to initial exposure to the therapeutic agent), or as a result of developing resistance to the therapeutic agent, either over the course of a first treatment period with the therapeutic agent or during a subsequent treatment period with the therapeutic agent.

The term "interleukin-2" (IL-2) as used herein refers to a cytokine with a reported molecular weight in the range of 13,000 to 17,000 daltons (Gillis and Watson (1980) J. Exp. Med. 159:1709) and with an isoelectric point in the range of 6-8.5. IL-2 is naturally produced by normal T cells and is present in the body at low concentrations. IL-2 was first described by Morgan et al. (1976) Science 193:1007-1008 and originally called T cell growth factor because of its ability to induce proliferation of stimulated T lymphocytes. "Antibodies" and "immunoglobulins" (Igs) are glycoproteins having the same structural characteristics. The terms are used synonymously. In some instances the antigen specificity of the immunoglobulin may be known.

The term "antibody" is used in the broadest sense and covers fully assembled antibodies, antibody fragments that can bind antigen (*e.g.*, Fab, $F(ab')_2$, Fv, single chain antibodies, diabodies, antibody chimeras, hybrid antibodies, bispecific antibodies, humanized antibodies, and the like), and recombinant peptides comprising the forgoing.

The terms "monoclonal antibody" and "mAb" as used herein refer to an antibody obtained from a substantially homogeneous population of antibodies, i.e., the individual antibodies comprising the population are identical except for possible naturally occurring mutations that may be present in minor amounts. As used herein, "anti-CD40 antibody" encompasses any antibody that specifically recognizes the CD40 cell surface antigen, including polyclonal antibodies, monoclonal antibodies, single-chain antibodies, and fragments thereof such as Fab, $F(ab')_2$, $F_v$, and other fragments that retain the antigen-binding function of the parent anti-CD40 antibody. Of particular interest for practicing the methods disclosed herein are anti-CD40 antibodies or antigen-binding fragments thereof that have the binding properties exhibited by the CHIR-5.9 and CHIR-12.12 human anti-CD40 monoclonal antibodies described herein below.

"Native antibodies" and "native immunoglobulins" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain ($V_H$) followed by a number of constant domains. Each light chain has a variable domain at one end ($V_L$) and a constant domain at its other end; the constant domain of the light chain is aligned with the first constant domain of the heavy chain, and the light chain variable domain is aligned with the variable domain of the heavy chain. Particular amino acid residues are believed to form an interface between the light and heavy-chain variable domains.

The term "variable" refers to the fact that certain portions of the variable domains differ extensively in sequence among antibodies. Variable regions confer antigen-binding specificity. However, the variability is not evenly distributed throughout the variable domains of antibodies. It is concentrated in three segments called complementarity determining regions (CDRs) or hypervariable regions, both in the light chain and the heavy-chain variable domains. The more highly conserved portions of variable domains are celled in the framework (FR) regions. The variable domains of native heavy and light chains each comprise four FR regions, largely adopting a β-pleated-sheet configuration, connected by three CDRs, which form loops connecting, and in some cases forming part of, the β-pleated-sheet structure. The CDRs in each chain are held together in close proximity by the FR regions and, with the CDRs from the other chain, contribute to the formation of the antigen-binding site of antibodies (see, Kabat et al. (1991) NIH Publ. No. 91-3242, Vol. I, pages 647-669).

The constant domains are not involved directly in binding an antibody to an antigen, but exhibit various effector functions, such as Fc receptor (FcR) binding, participation of the antibody in antibody-dependent cellular toxicity, opsonization, initiation of complement dependent cytotoxicity, and mast cell degranulation.

The term "hypervariable region," when used herein, refers to the amino acid residues of an antibody that are responsible for antigen-binding. The hypervariable region comprises amino acid residues from a "complementarity determining region" or "CDR" (i.e., residues 24-34 (L1), 50-56 (L2), and 89-97 (L3) in the light-chain variable domain and 31-35 (H1), 50-65 (H2), and 95-102 (H3) in the heavy-chain variable domain; Kabat et al. (1991) Sequences of Proteins of Immunological Interest, 5th Ed. Public Health Service, National Institute of Health, Bethesda, MD) and/or those residues from a "hypervariable loop" (i.e., residues 26-32 (L1), 50-52 (L2), and 91-96 (L3) in the light-chain variable domain and (H1), 53-55 (H2), and 96-101 (H3) in the heavy chain variable domain; Clothia and Lesk (1987) J. Mol. Biol., 196:901-917). "Framework" or "FR" residues are those variable domain residues other than the hypervariable region residues, as herein deemed.

"Antibody fragments" comprise a portion of an intact antibody, preferably the antigen-binding or variable region of the intact antibody. Examples of antibody fragments include Fab, Fab, F(ab')2, and Fv fragments; diabodies; linear antibodies (Zapata et al. (1995) Protein Eng. 10:1057-1062); single-chain antibody molecules; and multispecific antibodies formed from antibody fragments. Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')2 fragment that has two antigen-combining sites and is still capable of cross-linking antigen.

"Fv" is the minimum antibody fragment that contains a complete antigen recognition and binding site. This region consists of a dimer of one heavy- and one light-chain variable domain in tight, non-covalent association. It is in this configuration that the three CDRs of each variable domain interact to define an antigen-binding site on the surface of the $V_H$-$V_L$ dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain ($C_H1$) of the heavy chain. Fab fragments differ from Fab' fragments by the addition of a few residues at the carboxy terminus of the heavy chain $C_H1$ domain including one or more cysteines from the antibody hinge region. Fab'-SH is the designation herein for Fab' in which the cysteine residue(s) of the constant domains bear a free thiol group. Fab' fragments are produced by reducing the F(ab')2 fragment's heavy chain disulfide bridge. Other chemical couplings of antibody fragments are also known.

The "light chains" of antibodies (immunoglobulins) from any vertebrate species can be assigned to one of two clearly distinct types, called kappa (κ) and lambda (λ), based on the amino acid sequences of their constant domains.

Depending on the amino acid sequence of the constant domain of their heavy chains, immunoglobulins can be assigned to different classes. There are five major classes of human immunoglobulins: IgA, IgD, IgE, IgG, and IgM, and several of these may be further divided into subclasses (isotypes), e.g., IgG1, IgG2, IgG3, IgG4, IgA1, and IgA2. The heavy-chain constant domains that correspond to the different classes of immunoglobulins are called alpha, delta, epsilon, gamma, and mu, respectively. The subunit structures and three-dimensional configurations of different classes of immunoglobulins are well known. Different isotypes have different effector functions. For example, human IgG1 and IgG3 isotypes mediate ADCC (antibody dependent cell-mediated cytotoxicity) activity.

The word "label," when used herein, refers to a detectable compound or composition that is conjugated directly or indirectly to the antibody so as to generate a "labeled" antibody. The label may be detectable by itself (e.g., radioisotope labels or fluorescent labels) or, in the case of an enzymatic label, may catalyze chemical alteration of a substrate compound or composition that is detectable. Radionuclides that can serve as detectable labels include, for example, I-131, I-123, I-125,Y-90, Re-188, Re-186, At-211, Cu-67, Bi-212, and Pd-109. The label might also be a non-detectable entity such as a toxin.

The term "antagonist" is used in the broadest sense, and includes any molecule that partially or fully blocks, inhibits, or neutralizes a biological activity of a native target disclosed herein or the transcription or translation thereof.

"Carriers," as used herein, include pharmaceutically acceptable carriers, excipients, or stabilizers that are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, succinate, and other organic acids; antioxidants including ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN, polyethylene glycol (PEG), and Pluronics. Administration "in combination with" one or more further therapeutic agents includes simultaneous (concurrent) and consecutive (i.e., sequential) administration in any order.

A "host cell," as used herein, refers to a microorganism or a eukaryotic cell or cell line cultured as a unicellular entity which can be, or has been, used as a recipient for a recombinant vector or other transfer polynucleotides, and include the progeny of the original cell which has been transfected. It is understood that the progeny of a single cell may not necessarily be completely identical in morphology or in genomic or total DNA complement as the original parent, due to natural, accidental, or deliberate mutation.

"Human effector cells" are leukocytes that express one or more FcRs and perform effector functions. Preferably, the cells express at least FcγRIII and carry out antigen-dependent cell-mediated cyotoxicity (ADCC) effector function. Examples of human leukocytes that mediate ADCC include peripheral blood mononuclear cells (PBMC), natural killer (NK) cells, monocytes, macrophages, eosinophils, and neutrophils; with PBMCs and NK cells being preferred. Antibodies that have ADCC activity are typically of the IgG1 or IgG3 isotype. Note that in addition to isolating IgG1 and IgG3 antibodies, such ADCC-mediating antibodies can be made by engineering a variable region from a non-ADCC antibody or variable region fragment onto an IgG1 or IgG3 isotype constant region.

The terms "Fc receptor" or "FcR" are used to describe a receptor that binds to the Fc region of an antibody. The preferred FcR is a native sequence human FcR. Moreover, a preferred FcR is one that binds an IgG antibody (a gamma receptor) and includes receptors of the FcγRI, FcγRII, and FcγRIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. See, Daeron (1997) Annu. Rev. Immunol. 15:203-234. FcRs are reviewed in Ravetch and Kinet (1991) Annu. Rev. Immunol 9:457-92; Capel et al. (1994) Immunomethods 4:25-34; and de Haas et al. (1995) J. Lab. Clin. Med. 126: 330-41. Other FcRs, including those to be identified in the future, are encompassed by the term "FcR" herein. The term also includes the neonatal receptor, FcRn, which is responsible for the transfer of maternal IgGs to the fetus (Guyer et al. (1976) J. Immunol. 117:587 and Kim et al. (1994). J. Immunol. 24:249).

The term "synergy" is used to describe a combined effect of two or more active agents that is greater than the sum of the individual effects of each respective active agent. Thus, where the combined effect of two or more agents results in "synergistic inhibition" of an activity or process, for example, tumor growth, it is intended that the inhibition of the activity or process is greater than the sum of the inhibitory effects of each respective active agent. The term "synergistic therapeutic effect" refers to a therapeutic effect observed with a combination of two or more therapies wherein the therapeutic effect (as measured by any of a number of parameters) is greater than the sum of the individual therapeutic effects observed with the respective individual therapies.

The terms "therapeutically effective dose," "therapeutically effective amount," or "effective amount" are intended to mean an amount of the antagonist anti-CD40 antibody (or antigen-binding fragment thereof) or interleukin-2 (or variant thereof) that, when administered as a part of a combination therapy comprising at least these two agents, brings about a positive therapeutic response with respect to treatment of a subject for a cancer comprising neoplastic cells.

Combination Therapy with Antagonist Anti-CD40 Antibodies and IL-2

The present invention relates to methods for treating a subject having a cancer characterized by neoplastic cell growth. The methods disclosed herein encompass combination therapy with an antagonist anti-CD40 antibody or antigen-binging fragment thereof and IL-2 or biologically active variant thereof. The methods disclosed herein are especially useful for the treatment of cancers comprising neoplastic cells expressing the CD40 cell surface antigen, such as many solid tumors and B cell lymphomas.

Solid tumors that can be treated using the methods disclosed herein include, but are not limited to, ovarian, lung (for example, non-small cell lung cancer of the squamous cell carcinoma, adenocarcinoma, and large cell carcinoma types, and small cell lung cancer), breast, colon, kidney (including, for example, renal cell carcinomas), bladder, liver (including, for example, hepatocellular carcinomas), gastric, cervical, prostate, nasopharyngeal, thyroid (for example, thyroid papillary carcinoma), and skin cancers such as melanoma, and sarcomas (including, for example, osteosarcomas and Ewing's sarcomas). B cell-related cancers such as lymphomas include low-, intermediate-, and high-grade B cell lymphomas, immunoblastic lymphomas, non-Hodgkin's lymphomas, Hodgkin's disease, Epstein-Barr Virus (EBV) induced lymphomas, and AIDS-related lymphomas, as well as B cell acute lymphoblastic leukemias, myelomas, chronic lymphocytic leukemias, acute myeloblastic leukemias, and the like, as noted herein below.

Thus, the methods disclosed herein find use in the treatment of non-Hodgkin's lymphomas related to abnormal, uncontrollable B cell proliferation or accumulation. For purposes of the present invention, such lymphomas will be referred to according to the *Working Formulation* classification scheme, that is those B cell lymphomas categorized as low grade, intermediate grade, and high grade (see "The Non-Hodgkin's Lymphoma Pathologic Classification Project," Cancer 49 (1982):2112-2135). Thus, low-grade B cell lymphomas include small lymphocytic, follicular small-cleaved cell, and follicular mixed small-cleaved and large cell lymphomas; intermediate-grade lymphomas include follicular large cell, diffuse small cleaved cell, diffuse mixed small and large cell, and diffuse large cell lymphomas; and high-grade lymphomas include large cell immunoblastic, lymphoblastic, and small non-cleaved cell lymphomas of the Burkitt's and non-Burkitt's type.

It is recognized that the methods disclosed herein are useful in the therapeutic treatment of B cell lymphomas that are classified according to the Revised European and American Lymphoma Classification (REAL) system. Such B cell lymphomas include, but are not limited to, lymphomas classified as precursor B cell neoplasms, such as B lymphoblastic leukemia/lymphoma; peripheral B cell neoplasms, including B cell chronic lymphocytic leukemia/small lymphocytic lymphoma, lymphoplasmacytoid lymphoma/immunocytoma, mantle cell lymphoma (MCL), follicle center lymphoma (follicular) (including diffuse small cell, diffuse mixed small and large cell, and diffuse large cell lymphomas), marginal zone B cell lymphoma (including extranodal, nodal, and splenic types), hairy cell leukemia, plasmacytoma/ myeloma, diffuse large cell B cell lymphoma of the subtype primary mediastinal (thymic), Burkitt's lymphoma, and Burkitt's like high-grade B cell lymphoma; acute leukemias; acute lymphocytic leukemias; myeloblastic leukemias; acute myelocytic leukemias; promyelocytic leukemia; myelomonocytic leukemia; monocytic leukemia; erythroleukemia; granulocytic leukemia (chronic myelocytic leukemia); chronic lymphocytic leukemia; polycythemia vera; multiple myeloma; Waldenstrom's macroglobulinemia; heavy chain disease; and unclassifiable low-grade or high-grade B cell lymphomas.

In particular, the methods disclosed herein are useful for treating solid tumors comprising neoplastic cells expressing the CD40 antigen and B cell lymphomas, including those listed above. "Treatment" is herein defined as the application or administration of an antagonist anti-CD40 antibody or antigen-binding fragment thereof to a subject, or application or administration of an antagonist anti-CD40 antibody or antigen-binding fragment thereof to an isolated tissue or cell line from a subject, in combination with the application or administration of IL-2 (or biologically active variant thereof) to the subject, or to an isolated tissue or cell line from the subject, where the subject has a disease, a symptom of a disease, or a predisposition toward a disease, where the purpose is to cure, heal, alleviate, relieve, alter, remedy, ameliorate, improve, or affect the disease, the symptoms of the disease, or the predisposition toward the disease. By "treatment" is also intended the combination of the antagonist anti-CD40 antibody (or antigen-binding fragment thereof) and IL-2 (or biologically active variant thereof) can be applied or administered to the subject, or to the isolated tissue or cell line from the subject, as part of a single pharmaceutical composition, or alternatively as part of individual pharmaceutical compositions, each comprising either the antagonist anti-CD40 antibody (or antigen-binding fragment thereof) or IL-2 (or biologically active variant thereof), where the subject has a disease, a symptom of a disease, or a predisposition toward a disease, where the purpose is to cure, heal, alleviate, relieve, alter, remedy, ameliorate, improve, or affect the disease, the symptoms of the disease, or the predisposition toward the disease.

*Brief description of the oncotherapeutic agents*. Antagonist anti-CD40 antibodies suitable for use in the methods disclosed herein specifically bind a human CD40 antigen expressed on the surface of a human cell and are free of significant agonist activity but exhibit antagonist activity when bound to the CD40 antigen on a human CD40-expressing cell, including normal and neoplastic (whether malignant or benign) human cells. In some embodiments, their binding to CD40 displayed on the surface of human cells results in inhibition of proliferation and differentiation of these human cells. Thus the antagonist anti-CD40 antibodies suitable for use in the methods disclosed herein include those monoclonal antibodies that can exhibit antagonist activity toward normal and neoplastic human cells expressing the cell-surface CD40 antigen. These anti-CD40 antibodies and antigen-binding fragments thereof are referred to herein as "antagonist anti-CD40 antibodies." Such antibodies include, but are not limited to, the fully human monoclonal antibodies CHIR-5.9 and CHIR-12.12 described below and monoclonal antibodies having the binding characteristics of monoclonal antibodies CHIR-5.9 and CHIR-12.12. These monoclonal antibodies, which can be recombinantly produced, are described below.

In addition to the monoclonal antibodies CHIR-5.9 and CHIR-12.12, other anti-CD40 antibodies that would be useful in practicing the methods described herein include, but are not limited to: (1) the monoclonal antibodies produced by the hybridoma cell lines designated 131.2F8.5.9 (referred to herein as the cell line 5.9) and 153.8E2.D10.D6.12.12 (referred to herein as the cell line 12.12), deposited with the ATCC as Patent Deposit No. PTA-5542 and Patent Deposit No. PTA-5543, respectively; (2) a monoclonal antibody comprising an amino acid sequence selected from the group consisting of the sequence shown in SEQ ID NO:2, the sequence shown in SEQ ID NO:4, the sequence shown in SEQ ID NO:5, both the sequences shown in SEQ ID NO:2 and SEQ ID NO:4, and both the sequences shown in SEQ ID NO: 2 and SEQ ID NO:5; (3) a monoclonal antibody comprising an amino acid sequence selected from the group consisting of the sequence shown in SEQ ID NO:6, the sequence shown in SEQ ID NO:7, the sequence shown in SEQ ID NO:8, both the sequences shown in SEQ ID NO:6 and SEQ ID NO:7, and both the sequences shown in SEQ ID NO:6 and SEQ ID NO:8; (4) a monoclonal antibody having an amino acid sequence encoded by a nucleic acid molecule comprising a nucleotide sequence selected from the group consisting of the nucleotide sequence shown in SEQ ID NO:1, the nucleotide sequence shown in SEQ ID NO:3, and both the sequences shown in SEQ ID NO:1 and SEQ ID NO:3; (5) a monoclonal antibody that binds to an epitope capable of binding the monoclonal antibody produced by the hybridoma cell line 5.9 or the hybridoma cell line 12.12; (6) a monoclonal antibody that binds to an epitope comprising residues 82-87 of the amino acid sequence shown in SEQ ID NO:10 or SEQ ID NO:12; (7) a monoclonal antibody that competes with the monoclonal antibody CHIR-5.9 or CHIR-12.12 in a competitive binding assay; and (8) a monoclonal antibody that is an antigen-binding fragment of the CHIR-12.12 or CHIR-5.9 monoclonal antibody or the foregoing monoclonal antibodies in preceding items (1)-(7), where the fragment retains the capability of specifically binding to the human CD40 antigen. Those skilled in the art recognize that the antibodies and antigen-binding fragments of these antibodies suitable for use in the methods disclosed herein include antibodies and antigen-binding fragments thereof that are produced recombinantly using methods well known in the art and described herein below, and include, for example, monoclonal antibodies CHIR-5.9 and CHIR-12.12 that have been recombinantly produced.

Fragments of the anti-CD40 antibodies disclosed herein are suitable for use in the methods disclosed herein so long as they retain the desired affinity of the full-length antibody. Thus, a fragment of an anti-CD40 antibody will retain the ability to bind to the CD40 B-cell surface antigen. Such fragments are characterized by properties similar to the corresponding full-length antibody. For example, antagonist anti-CD40 antibody fragments will specifically bind a human CD40 antigen expressed on the surface of a human cell, and are free of significant agonist activity but exhibit antagonist activity when bound to a CD40 antigen on a human CD40-expressing cell. Such fragments are referred to herein as "antigen-binding" fragments.

In addition to using the antagonist anti-CD40 antibodies mentioned above, and described more fully herein below, the methods disclosed herein can be practiced using antibodies that have the binding characteristics of monoclonal antibodies CHIR-5.9 or CHIR-12.12 and which competitively interfere with binding of these antibodies to their respective antigens or bind the same epitopes. One of skill in the art could determine whether an antibody competitively interferes with CHIR-5.9 or CHIR-12.12 binding using standard methods.

The IL-2, or biologically active variant thereof, for use in the methods disclosed herein may be from any source, but preferably is recombinant IL-2, more particularly recombinant human IL-2. By "recombinant IL-2" is intended IL-2 that has comparable biological activity to native-sequence IL-2 and that has been prepared by recombinant DNA techniques as described, for example, by Taniguchi et al. (1983) Nature 302:305-310 and Devos (1983) Nucleic Acids Res. 11:4307-4323 or mutationally altered IL-2 as described by Wang et al. (1984) Science 224:1431-1433. In general, the gene coding for IL-2 is cloned and then expressed in transformed organisms, preferably a microorganism, for example *E. coli,* as described herein. The host organism expresses the foreign gene to produce IL-2 under expression conditions. Synthetic recombinant IL-2 can also be made in eukaryotes, such as yeast or human cells. Processes for growing, harvesting, disrupting, or extracting the IL-2 from cells are substantially described in, for example, U.S. Patent Nos. 4,604,377; 4,738,927; 4,656,132; 4,569,790; 4,748,234; 4,530,787; 4,572,798; 4,748,234; and 4,931,543.

For examples of variant IL-2 proteins, see European Patent Application EP 0136489; European Patent Application EP 0091539; European Patent Application EP 0088195; the recombinant IL-2 muteins described in European Patent Application EP 0109748, which is the equivalent to Belgian Patent No. 893,016, commonly owned U.S. Patent No. 4,518,584; the muteins described in U.S. Patent No. 4,752,585 and WO 99/60128; and the IL-2 mutein des-alanyl-1, serine-125 human IL-2 (also referred to as "aldesleukin") used in the examples herein and described in U.S. Patent No. 4,931,543, as well as the other IL-2 muteins described in this U.S. patent. Additionally, IL-2 can be modified with polyethylene glycol to provide enhanced solubility and an altered pharmokinetic profile (see U.S. Patent No. 4,766,106, ). See also below where suitable variants of IL-2 are more fully discussed.

In some embodiments, the methods disclosed herein comprise combination therapy with IL-2, for example, human IL-2, and the anti-CD40 monoclonal antibody CHIR-12.12. In other embodiments, the methods disclosed herein comprise combination therapy with IL-2, for example, human IL-2, and the anti-CD40 monoclonal antibody CHIR-5.9. In yet other embodiments, the methods disclosed herein comprise combination therapy with IL-2, for example, human IL-2, and an antigen-binding fragment of the anti-CD40 monoclonal antibody CHIR-12.2 or CHIR-5.9.

In alternative embodiments, the methods disclosed herein comprise combination therapy with a biologically active variant of IL-2, for example, the IL-2 mutein des-alanyl-1, serine-125 human IL-2 (also referred to as "aldesleukin," which is available commercially as a formulation that is marketed under the tradename Proleukin® (Chiron Corporation, Emeryville, California)), and the anti-CD40 monoclonal antibody CHIR-12.12 or CHIR-5.9. In other embodiments, the methods disclosed herein comprise combination therapy with a biologically active variant of IL-2, for example, the IL-2 mutein des-alanyl-1, serine-125 human IL-2, and an antigen-binding fragment of the anti-CD40 monoclonal antibody CHIR-12.12 or CHIR-5.9. The combination therapy described herein may comprise other variations, as long the CD40 antigen is targeted in the treatment process.

*Measurements of clinical efficacy*. In accordance with the methods disclosed herein, IL-2 or biologically active variant thereof and at least one antagonist anti-CD40 antibody or antigen-binding fragment thereof as defined elsewhere herein are used in combination to promote a positive therapeutic response with respect to a cancer comprising neoplastic cells expressing the CD40 antigen. By "positive therapeutic response" is intended an improvement in the disease in association with the combined anti-tumor activity of these administered therapeutic agents, and/or an improvement in the symptoms associated with the disease. Thus, for example, a positive therapeutic response would refer to one or more of the following improvements in the disease: (1) a reduction in tumor size; (2) a reduction in the number of cancer (i.e., neoplastic) cells; (3) an increase in neoplastic cell death; (4) inhibition of neoplastic cell survival; (4) inhibition (i.e., slowing to some extent, preferably halting) of tumor growth; (5) inhibition (i.e., slowing to some extent, preferably halting) of cancer cell infiltration into peripheral organs; (6) inhibition (i.e., slowing to some extent, preferably halting) of tumor metastasis; (7) the prevention of further tumor outgrowths; (8) an increased patient survival rate; and (9) some extent of relief from one or more symptoms associated with the cancer. Such therapeutic responses may be further characterized as to degree of improvement. Thus, for example, an improvement in the disease may be characterized as a complete response. By "complete response" is intended an absence of clinically detectable disease with normalization of any previously abnormal radiographic studies, bone marrow, and cerebrospinal fluid (CSF). Such a response must persist for at least one month following treatment according to the methods disclosed herein. A complete response can be unconfirmed if no repeat evaluation of tumor status is done at least one month after the initial response is evaluated. Alternatively, an improvement in the disease may be categorized as being a partial response. By "partial response" is intended at least about a 50% decrease in all measurable tumor burden (i.e., the number of tumor cells present in the subject) in the absence of new lesions and persisting for at least one month. Such a response is applicable to measurable tumors only.

Multiple parameters can be indicative of treatment efficacy. These include, but are not limited to, a reduction in the size of the tumor mass; a reduction in metastatic invasiveness of the tumor; a reduction in the rate of tumor growth; a decrease in severity or incidence of tumor related sequelae such as cachexia and ascites production; a decrease and/or prevention of tumor related complications such as pathologic bone fractures, autoimmune hemolytic anemia, prolymphocytic transformation and Richter's syndrome, etc.; sensitization of the tumor to chemotherapy and other treatments; an increased patient survival rate; an increase in observed clinical correlates of improved prognosis such as increased tumor infiltrating lymphocytes and decreased tumor vascularization; and the like. Thus, in some embodiments, administration of the therapeutic combination will result in an improvement of one or more of these parameters in a patient (i.e., subject) undergoing treatment. In other embodiments, the improvements in the patient will be synergistic with regard to some parameters, but additive with regard to others.

Tumor response can be assessed for changes in tumor morphology (i.e., overall tumor burden, tumor size, and the like) using screening techniques such as magnetic resonance imaging (MRI) scan, x-radiographic imaging, computed tomographic (CT) scan, flow cytometry or fluorescence-activated cell sorter (FACS) analysis, bioluminescent imaging, for example, luciferase imaging, bone scan imaging, and tumor biopsy sampling including bone marrow aspiration (BMA). In addition to these positive therapeutic responses, the subject undergoing therapy may experience the beneficial effect of an improvement in the symptoms associated with the disease. Thus, for B cell tumors, the subject may experience a decrease in the so-called B symptoms, i.e., night sweats, fever, weight loss, and/or urticaria.

One method of predicting clinical efficacy is to measure the effects of combination therapy with these two therapeutic agents in a suitable model, for example, the use of the combination of IL-2 or biologically active variant thereof and an antagonist anti-CD40 antibody or antigen-binding fragment thereof in one or more murine cancer models. For lymphomas, these models include, for example, the nude mouse xenograft tumor models such as those using the human Burkitt's lymphoma cell lines known as Namalwa and Daudi. Thus, in some embodiments, anti-tumor activity of the combination of these two therapeutic agents is assayed in a human NHL Namalwa xenograft model as described in the examples herein below. The Namalwa cell line gives rise to aggressive rituximab-resistant tumors when implanted in nude mice.

In other embodiments, anti-tumor activity of the combination of these two therapeutic agents is assayed in a staged nude mouse xenograft tumor model using the Daudi human lymphoma cell line. A staged nude mouse xenograft tumor model is generally more effective at distinguishing the therapeutic efficacy of a given therapeutic protocol than is an unstaged model, as in the staged model therapeutic dosing is initiated only after the tumor has reached a measurable size. In the unstaged model, therapeutic dosing is initiated generally within about 1 day of tumor inoculation and before a palpable tumor is present. The ability of a combination therapeutic regimen to exhibit increased anti-tumor activity in a staged model is a strong indication that the combination therapeutic regimen will be therapeutically effective.

Other tumor models are well known in the art. For example, for multiple myeloma (MM), tumor models include, but are not limited to, those in which cell lines are grown in immunodeficient mice and the mice are then treated with the therapeutics of interest, and those in which patient myeloma cells are implanted along with fetal bone in immunodeficient mice and the mice are then treated with the therapeutics of interest. Thus, for example, anti-tumor activity of the combination of these two therapeutic agents can be assayed *in vivo* in a human MM IM-9 xenograft model, which uses the human MM cell line IM-9. In this manner, efficacy of combination therapy with IL-2 or biologically active variant thereof and an antagonist anti-CD40 antibody or antigen-binding fragment thereof can be evaluated using an unstaged conditional survival model, where treatment with these therapeutic agents begins one day after intravenous inoculation of the IM-9 tumor cells. Alternatively, efficacy of this combination therapy can be evaluated using a staged subcutaneous model, where the IM-9 cells are injected SC into SCID nice, and then combination treatment with these therapeutic agents begins once the tumor reaches a measurable size.

Anti-tumor activity of combination therapy with IL-2 or biologically active variant thereof and an antagonist anti-CD40 antibody or antigen-binding fragment thereof can be evaluated in a murine solid tumor model. Examples include, but are not limited to, a xenograft colon cancer model using the human colon carcinoma cell line HCT 116, which expresses CD40; an unstaged (prophylactic) orthotopic murine model of ovarian cancer using the ovarian cancer cell line SKOV3i.p.1; and a staged (therapeutic) murine model of ovarian cancer using the ovarian cancer cell line SKOV3i.p.1.

*Modes of administration*. For purposes of carrying out the combination therapy described herein, pharmaceutical compositions comprising these two classes of therapeutic agents (i.e., the cytokine and the anti-CD40 antibody or antigen-binding fragment thereof) as therapeutically active components may be administered using any acceptable method known in the art. Thus, for example, a pharmaceutical composition comprising IL-2 (or biologically active variant thereof), a pharmaceutical composition comprising antagonist anti-CD40 antibody (or antigen-binding fragment thereof), or a pharmaceutical composition comprising IL-2 (or variant thereof) and antagonist anti-CD40 antibody (or antigen-binding fragment thereof) can be administered by any form of injection, including intravenous (IV), intratumoral (IT), intraperitoneal (IP), intramuscular (IM), or subcutaneous (SC) injection. In some embodiments, a pharmaceutical composition comprising IL-2 (or biologically active variant thereof) is administered by IV, IM, IP, or SC injection, and a pharmaceutical composition comprising antagonist anti-CD40 antibody (or antigen-binding fragment thereof) is administered by IV, IP, or SC injection. In other embodiments, a pharmaceutical composition comprising IL-2 (or biologically active variant thereof) is administered by IV, IT, or SC injection, and a pharmaceutical composition comprising antagonist anti-CD40 antibody (or antigen-binding fragment thereof) is administered by IV or SC injection.

In some embodiments disclosed herein, a pharmaceutical composition comprising IL-2 or variant thereof is administered by SC injection and a pharmaceutical composition comprising the anti-CD40 antibody or antigen-binding fragment thereof is administered intravenously. When administered intravenously, the pharmaceutical composition comprising the anti-CD40 antibody or antigen-binding fragment thereof can be administered by infusion over a period of about 1 to about 10 hours. In some embodiments, infusion occurs over a period of about 2 to about 8 hours, over a period of about 3 to about 7 hours, over a period of about 4 to about 6 hours, or over a period of about 6 hours, depending upon the antagonist anti-CD40 antibody being administered.

*Combination therapy*. The methods disclosed herein comprise using combination therapy. The term "combination" is used in its broadest sense and means that a subject is treated with at least two therapeutic regimens. Thus, "combination therapy" is intended to mean a subject is treated with at least two oncotherapeutic regimens, more particularly, with at least IL-2 or biologically active variant thereof in combination with at least one antagonist anti-CD40 antibody or antigen-binding fragment thereof, but the timing of administration of the different oncotherapeutic regimens can be varied so long as the beneficial effects of the combination of these two therapeutic agents is achieved.

Treatment with IL-2 or biologically active variant thereof in combination with an antagonist anti-CD40 antibody or antigen-binding fragment thereof can be simultaneous (concurrent), consecutive (sequential, in either order), or a combination thereof. Therefore, a subject undergoing combination therapy can receive treatment with both of these therapeutic agents at the same time (i.e., simultaneously) or at different times (i.e., sequentially, in either order, on the same day, or on different days), so long as the therapeutic effect of the combination of both substances is caused in the subject undergoing therapy. In some embodiments, the combination of IL-2 or biologically active variant thereof and anti-CD40 antibody or antigen-binding fragment thereof will be given simultaneously for one dosing, but other dosings will include sequential administration, in either order, on the same day, or on different days. Sequential administration may be performed regardless of whether the subject responds to the first therapeutic agent administered. Where these two therapeutic agents are administered simultaneously, they can be administered as separate pharmaceutical compositions, each comprising either the IL-2 (or biologically active variant thereof) or the antagonist anti-CD40 antibody (or antigen-binding fragment thereof), or they can be administered as a single pharmaceutical composition comprising both of these therapeutic agents.

The effect of the combination therapy can also be optimized by varying the timing of administration of either IL-2 (or biologically active variant thereof) and/or the antagonist anti-CD40 antibody (or antigen-binding fragment thereof). Thus, in some embodiments, the IL-2 (or biologically active variant thereof, such as des-alanyl C125S human IL-2 mutein) will be administered simultaneously with the antagonist anti-CD40 antibody, such as the monoclonal antibody CHIR-12.12. or CHIR-5.9 (or antigen-binding fragment thereof). In other embodiments, the IL-2 (or biologically active variant thereof, such as des-alanyl C125S human IL-2 mutein) will be administered first and then the antagonist anti-CD40 antibody, such as the monoclonal antibody CHIR-12.12 or CHIR-5.9 (or antigen-binding fragment thereof), will be administered next. In yet other embodiments, the antagonist anti-CD40 antibody, such as the monoclonal antibody CHIR-12.12 or CHIR-5.9 (or antigen-binding fragment thereof), will be administered first, and the IL-2 (or biologically active variant thereof, such as des-alanyl C125S human IL-2 mutein) will be administered next. In some embodiments, the combination of the IL-2 (or biologically active variant thereof, such as des-alanyl C125S human IL-2 mutein) and antagonist anti-CD40 antibody, such as monoclonal antibody CHIR-12.12 or CHIR-5.9 (or antigen-binding fragment thereof), will be given concurrently for one dosing, but other dosings will include sequential administration, in either order, on the same day, or on different days. As previously noted, where the IL-2 (or biologically active variant thereof, such as des-alanyl C125S human IL-2 mutein) and the antagonist anti-CD40 antibody, such as the monoclonal antibody CHIR-12.12 or CHIR-5.9 (or antigen-binding fragment thereof), are administered simultaneously, they can be administered as separate pharmaceutical compositions, each comprising either the IL-2 (or biologically active variant thereof) or the antagonist anti-CD40 antibody (or antigen-binding fragment thereof), or can be administered as a single pharmaceutical composition comprising both of these therapeutic agents.

Therapy with an effective amount of the combination of IL-2 (or biologically active variant thereof) and at least one antagonist anti-CD40 antibody (or antigen-binding fragment thereof) promotes a positive therapeutic response for a cancer comprising neoplastic cells expressing the CD40 antigen, including B-cell related lymphomas and solid tumors as defined elsewhere herein. Concurrent therapy with both of these anti-tumor agents potentiates the anti-tumor activity of each of these agents, thereby providing a positive therapeutic response that is improved with respect to that observed with administration of IL-2 (or biologically active variant thereof) alone or administration of antagonist anti-CD40 antibody (or antigen-binding fragment thereof) alone. Improvement of the positive therapeutic response may be additive in nature or synergistic in nature. Where synergistic, concurrent therapy with IL-2 (or biologically active variant thereof) and at least one antagonist anti-CD40 antibody (or antigen-binding fragment thereof) results in a positive therapeutic response that is greater than the sum of the positive therapeutic responses achieved with the separate IL-2 (or biologically active variant thereof) and antagonist anti-CD40 antibody (or antigen-binding fragment thereof) components.

Moreover, the treatment can be accomplished with varying doses as well as dosing regimens, as long as the combination of these doses is effective at treating any one or more of a number of therapeutic parameters. These treatment regimens are based on doses and dosing schedules that maximize therapeutic effects, such as those described below. Those skilled in the art recognize that a dose of any one monoclonal antibody or any one cytokine such as IL-2 may not be therapeutically effective when administered individually, but will be therapeutically effective when administered in combination with the other agent. Thus, in some embodiments, the therapeutically effective dose of a combination of IL-2 and antagonist anti-CD40 antibody may comprise doses of individual active agents that, when administered alone, would not be therapeutically effective or would be less therapeutically effective than when administered in combination with each other.

Because the combined administration of these two therapeutic agents potentiates the effectiveness of both of these agents, a positive therapeutic response that is similar to that achieved with a particular dose of IL-2 alone can be achieved with lower doses of this agent. The significance of this is two-fold. First, the potential therapeutic benefits of treatment of B cell malignancies or solid tumors with IL-2 or variant thereof can be realized at IL-2 doses that minimize toxicity responses normally associated with high-dose IL-2 administration. Such toxicity responses include, but are not limited to, chronic fatigue, nausea, hypotension, fever, chills, weight gain, pruritis or rash, dysprea, azotemia, confusion, thrombocytopenia, myocardial infarction, gastrointestinal toxicity, and vascular leak syndrome (see, for example, Allison et al. (1989) J. Clin. Oncol. 7(1):75-80). Secondly, targeting of specific molecules on a tumor cell surface by monoclonal antibodies can select for clones that are not recognized by the antibody or are not affected by its binding, resulting in tumor escape, and loss of effective therapeutic treatment. Improved anti-tumor activity of antagonist anti-CD40 antibody (or a fragment thereof) administered in combination with IL-2 (or variant thereof) may translate into increased potency of monoclonal antibodies, less frequent administration of monoclonal antibodies, and/or lower dose of monoclonal antibodies, thereby lessening the potential for tumor escape. Further, monoclonal antibody ADCC activity may also arise from activity from the neutrophils and monocytes/macrophage polulation. See, for example, Hernandez-Illzaliturri et al. (2003) Clin. Cancer Res. 9(16, Pt 1):5866-5873; and Uchida et al. (2004) J. Exp. Med. 199(12):1659-1669). As IL-2 is known to activate these cell populations, combination therapy of the antagonist anti-CD40 antibody with IL-2 or biologically active variant thereof may improve ADCC-mediated anti-tumor activity of the antibody.

The amount of at least one antagonist anti-CD40 antibody (or antigen-binding fragment thereof) to be administered in combination with an amount of IL-2 (or variant thereof) and the amount of either of these therapeutic agents needed to potentiate the effectiveness of the other therapeutic agent are readily determined by one of ordinary skill in the art without undue experimentation given the disclosure set forth herein. Factors influencing the mode of administration and the respective amount of IL-2 (or variant thereof) and antagonist anti-CD40 antibody (or antigen-binding fragment thereof) administered in combination include, but are not limited to, the particular lymphoma or solid tumor undergoing therapy, the severity of the disease, the history of the disease, and the age, height, weight, health, and physical condition of the individual undergoing therapy. Similarly, the amount of these therapeutic agents to be administered in combination therapy will be dependent upon the mode of administration and whether the subject will undergo a single dose or multiple doses of each of these therapeutic agents. Generally, a higher dosage of the antibody agent is preferred with increasing weight of the subject undergoing therapy.

Exemplary Protocols for Combination IL-2/Antagonist Anti-CD40 Antibody Therapy

In some embodiments of the disclosure, combination therapy is achieved by administering recommended total weekly doses of a pharmaceutical composition comprising IL-2 (or biologically active variant thereof) in combination with recommended therapeutically effective doses of a pharmaceutical composition comprising the antagonist anti-CD40 antibody (or antigen-binding fragment thereof), each being administered according to a particular dosing regimen. By "therapeutically effective dose or amount" is intended an amount of one of these two therapeutic agents that, when administered with a therapeutically effective dose or amount of the other of these two therapeutic agents, brings about a positive therapeutic response with respect to treatment of cancers comprising neoplastic cells expressing the CD40 cell surface antigen. As previously noted above, administration of the separate pharmaceutical compositions can be at the same time or at different times, so long as the therapeutic effect of the combination of both substances is caused in the subject undergoing therapy.

In accordance with some embodiments of the disclosure, the human subject undergoing treatment with one or more weekly doses of antagonist anti-CD40 antibody or antigen-binding fragment thereof as defined herein below is also administered IL-2 or biologically active variant thereof as defined herein below according to a constant IL-2 dosing regimen or according to a two-level IL-2 dosing regimen. An important aspect of this combination therapy is an overlapping period of time during which both of these therapeutic agents are being administered to the subject, each according to the particular dosing regimen disclosed herein. Accordingly, where combination therapy comprises this overlapping time period of dosing with these two therapeutic agents, the combination therapy is also referred to as "concurrent therapy." The first therapeutically effective dose administered to the subject can be the antagonist anti-CD40 antibody (or antigen-binding fragment thereof) or can be the IL-2 (or biologically active variant thereof). On those days where both an antagonist anti-CD40 antibody (or antigen-binding fragment thereof) and IL-2 (or biologically active variant thereof) are scheduled to be administered to the subject, these therapeutic agents can be administered either at the same time (i.e., simultaneous administration) or at different times (i.e., sequential administration, in either order). Although the following discussion of preferred dosing regimens refers to dosing of an antagonist anti-CD40 antibody as defined herein in combination with IL-2 as defined herein, it is recognized that the protocols (i.e., initiation of treatment, duration of antibody and IL-2 treatment, antibody and IL-2 dosing regimens, interruption of IL-2 dosing, subsequent courses of combination IL-2/antagonist antiCD40 antibody therapy, IL-2 dosing schedule, and the like) are equally applicable to dosing with an antigen-binding fragment of an antagonist anti-CD40 antibody as defined herein in combination with IL-2 as defined herein, dosing with an antigen-binding fragment of an antagonist anti-CD40 antibody as defined herein in combination with a biologically active variant of IL-2 as defined herein, or dosing with an antagonist anti-CD40 antibody as defined herein in combination with a biologically active variant of IL-2 as defined herein.

*Concurrent therapy: initiation of treatment*. Where concurrent therapy with IL-2 and antagonist anti-CD40 antibody comprises one or more cycles of a constant IL-2 dosing regimen, the initial therapeutic agent to be administered to the subject at the start of a treatment period can be either the IL-2 or the antagonist anti-CD40 antibody, so long as the subject has an overlapping period of time during which both therapeutic agents are being administered to the subject, each according to the particular dosing regimen disclosed herein.

Thus, in one embodiment, concurrent therapy with these two therapeutic agents comprises intitating the constant IL-2 dosing regimen prior to initiating administration of therapeutically effective doses of antagonist anti-CD40 antibody, which are administered weekly, or, alternatively, once every two, three, or four weeks. In this manner, a first dose of IL-2 is administered up to one month before the first dose of antagonist anti-CD40 antibody is administered. By "up to one month" is intended the first dose of IL-2 is administered at least one day before initiating antagonist anti-CD40 antibody administration, but not more than one month (i.e., 30 days) before initiating antagonist anti-CD40 antibody administration. Thus, IL-2 administration can begin, for example, 1 day, 2 days, 3 days, 4 days, 5 days, 6 days, 7 days (i.e., 1 week), 10 days, 14 days (i.e., two weeks), 17 days, 21 days (i.e., 3 weeks), 24 days, 28 days (4 weeks), or up to one month (i.e., 30 or 31 days) before administering the first therapeutically effective dose of the antagonist anti-CD40 antibody.

In other embodiments, the constant IL-2 dosing regimen and antagonist anti-CD40 antibody administration begin concurrently on the same day, either at the same time (i.e., simultaneous administration) or at different times (i.e., sequential administration, in either order). Thus, for example, in one embodiment where concurrent therapy with these two therapeutic agents begins on day 1 of a treatment period, a first therapeutically effective dose of antagonist anti-CD40 antibody and a first dose of IL-2 would both be administered on day 1 of this treatment period. In one such embodiment, the dose of antagonist anti-CD40 antibody is administered first, followed by administration of the dose of IL-2 within about 10 minutes to about 4 hours of the completion of administering the dose of antagonist anti-CD40 antibody, such as within about 10, 15, 20, 25, 30, 45, 60, 90, 120, 150, 180, 210, or 240 minutes.

In alternative embodiments, the initial therapeutic agent to be administered to the subject at the start of a treatment period is the antagonist anti-CD40 antibody, while the first cycle of constant IL-2 dosing is initiated by administering a first dose of IL-2 subsequently, for example, within 10 days following administration of the first therapeutically effective dose of the antagonist anti-CD40 antibody, for example, within 1, 2, 3, 4, 5, 6, 7, 8, 9, or 10 days. In some embodiments, the first cycle of constant IL-2 dosing is initiated by administering a first dose of IL-2 within 7 days of administering the first therapeutically effective dose of antagonist anti-CD40 antibody, such as within 1, 2, 3, 4, 5, 6, or 7 days. Thus, for example, in one embodiment, a therapeutically effective dose of the antagonist anti-CD40 antibody is administered on day 1 of a treatment period, and the first cycle of constant IL-2 dosing is initiated 7 days later, i.e., by administering the initial dose of IL-2 on day 8 of the treatment period.

Following completion of the first cycle of constant IL-2 dosing, a human subject that is receiving therapeutically effective doses of the antagonist anti-CD40 antibody accordingly to a weekly dosing schedule, or, alternatively, once every two, three, or four weeks, can be administered one or more subsequent cycles of constant IL-2 dosing. During the second and all subsequent cycles of constant IL-2 dosing, generally the first therapeutic agent to be administered to the subject is the antagonist anti-CD40 antibody, with the second or subsequent cycle of constant IL-2 dosing being initiated by administering a first dose of IL-2 within 24 hours, such as within 0.5, 1, 2, 4, 8, 12, 16, 20, or 24 hours of administering the dose of antagonist anti-CD40 antibody. On those days where both antagonist anti-CD40 antibody and IL-2 are scheduled to be administered to the subject, these therapeutic agents can be administered either at the same time (i.e., simultaneous administration) or at different times (i.e., sequential administration, in either order). In one such embodiment, the dose of antagonist anti-CD40 antibody is administered first, followed by administration of the dose of IL-2 within about 10 minutes to about 4 hours of the completion of administering the dose of antagonist anti-CD40 antibody, such as within about 10, 15, 20, 25, 30, 45, 60, 90, 120, 150, 180, 210, or 240 minutes.

Where concurrent therapy with IL-2 and antagonist anti-CD40 antibody comprises one or more cycles of a two-level IL-2 dosing regimen, the initial therapeutic agent to be administered to the subject at the start of a treatment period can be either the IL-2 or the antagonist antiCD40 antibody, so long as the subject has an overlapping period of time during which both therapeutic agents are being administered to the subject, each according to the particular dosing regimen disclosed herein.

Thus, in one embodiment, concurrent therapy with these two therapeutic agents comprises initiating the two-level IL-2 dosing regimen prior to initiating administration of therapeutically effective doses of antagonist anti-CD40 antibody, where a therapeutically effective dose of the antibody is administered according to a weekly dosing schedule, or, alternatively, once every two, three, or four weeks. In this manner, a first dose of IL-2 is administered up to one month before the first dose of antagonist anti-CD40 antibody is administered. By "up to one month" is intended the first dose of IL-2 is administered at least one day before initiating antagonist anti-CD40 antibody administration, but not more than one month (i.e., 30 days) before initiating antagonist anti-CD40 antibody administration. Thus, IL-2 administration can begin, for example, 1 day, 2 days, 3 days, 4 days, 5 days, 6 days, 7 days (i.e., 1 week), 10 days, 14 days (i.e., two weeks), 17 days, 21 days (i.e., 3 weeks), 24 days, 28 days (4 weeks), or up to one month (i.e., 30 or 31 days) before administering the first therapeutically effective dose of the antagonist anti-CD40 antibody.

In other embodiments, the two-level IL-2 dosing regimen and antagonist anti-CD40 antibody administration begin concurrently on the same day, either at the same time (i.e., simultaneous administration) or at different times (i.e., sequential administration, in either order). Thus, for example, in one embodiment where concurrent therapy with these two therapeutic agents begins on day 1 of a treatment period, a first therapeutically effective dose of antagonist anti-CD40 antibody and a first dose of IL-2 would both be administered on day 1 of this treatment period. In one such embodiment, the dose of antagonist anti-CD40 antibody is administered first, followed by administration of the dose of IL-2 within about 10 minutes to about 4 hours of the completion of administering the dose of antagonist anti-CD40 antibody, such as within about 10, 15, 20, 25, 30, 45, 60, 90, 120, 150, 180, 210, or 240 minutes.

In alternative embodiments, a first therapeutically effective dose of antagonist anti-CD40 antibody is administered to the subject, for example, on day 1 of a treatment period, and the two-level IL-2 dosing regimen is initiated by administering a first dose of IL-2 within 10 days of administering the first therapeutically effective dose of antagonist anti-CD40 antibody. In such embodiments, preferably the two-level IL-2 dosing regimen is initiated by administering a first dose of IL-2 within 7 days of administering the first therapeutically effective dose of antagonist anti-CD40 antibody, such as within 1, 2, 3, 4, 5, 6, or 7 days.

Depending upon the severity of the disease, the patient's health, and prior history of the patient's disease, one or more cycles of a two-level IL-2 dosing regimen can be administered concurrently with antagonist anti-CD40 antibody therapy, where therapeutically effective doses of the antibody are administered weekly, or, alternatively, once every two, three, or four weeks.

*Duration of antibody and IL-2 treatment.* In accordance with the methods disclosed herein , a therapeutically effective dose of antagonist anti-CD40 antibody is administered weekly, or is administered once every two, three, or four weeks, in combination with one or more cycles of a constant IL-2 dosing regimen or in combination with one or more cycles of a two-level IL-2 dosing regimen. When a subject is undergoing concurrent therapy with these two therapeutic agents in the manner set forth herein, the duration of antagonist anti-CD40 antibody administration and the duration of any given cycle of either of the IL-2 dosing regimens will depend upon the subject's overall health, history of disease progression, and tolerance of the particular antagonist anti-CD40 antibody/IL-2 administration protocol.

Thus, in some embodiments, therapeutically effective doses of antagonist anti-CD40 antibody are administered weekly (i.e., once a week), or once every two to four weeks, for example, once every two weeks, once every three weeks, or once every four weeks, throughout a treatment period, where the treatment period includes one or more cycles of a constant IL-2 dosing regimen or one or more cycles of a two-level IL-2 dosing regimen.

Alternatively, the anti-CD40 antibody therapy can have a fixed duration within a treatment period. In this manner, a therapeutically effective dose of antagonist anti-CD40 antibody is administered weekly, or is administered once every two, three, or four weeks, for a fixed period of about 4 weeks to about 16 weeks, including 4, 5, 6, 7, 8, 9, 10, 11, 12, 13, 14, 15, or 16 weeks, in combination with one or more cycles of a constant IL-2 dosing regimen or in combination with one or more cycles of a two level-IL-2 dosing regimen. Thus, for example, where the antibody is administered weekly for a duration of 4 weeks or 8 weeks, the subject would receive four or eight therapeutically effective doses of the anti-CD40 antibody, respectively, during concurrent therapy with IL-2. Similarly, where the antibody is administered once every two weeks for a duration of 4 weeks or 8 weeks, for example, the subject would receive two or four therapeutically effective doses of the antagonist anti-CD40 antibody, respectively, during concurrent therapy with IL-2. Where the antibody is administered once every three weeks for a duration of 6 weeks, 9 weeks, 12 weeks, or 15 weeks, for example, the subject would receive two, three, four, or five therapeutically effective doses of the antagonist anti-CD40 antibody, respectively, during concurrent therapy with IL-2. Similarly, where the antibody is administered once every four weeks for a duration of 8 weeks, 12 weeks, or 16 weeks, the subject would receive two, three, or four therapeutically effective doses fo the antagonist anti-CD40 antibody, respectively, during concurrent therapy with IL-2.

The duration of IL-2 administration during concurrent therapy with these two therapeutic agents is a function of the IL-2 dosing regimen used. Generally, IL-2 is administered according to the disclosed protocols, and a subject can repeat one or more cycles of a constant or two-level IL-2 dosing regimen as needed, unless IL-2 toxicity symptoms develop. Should such toxicity symptoms develop, the subject can be taken off of IL-2 dosing until complete resolution of any observed toxicity symptoms. Such IL-2 toxicity responses include but are not limited to, chronic fatigue, nausea, hypotension, fever, chills, weight gain, pruritis or rash, dysprea, azotemia, confusion, thrombocytopenia, myocardial infarction, gastrointestinal toxicity, and vascular leak syndrome (see, for example, Allison et al. (1989) J. Clin. Oncol. 7 (1):75-80). The subject may resume concurrent therapy with these two therapeutic agents as needed following resolution of signs and symptoms of these IL-2 toxicity symptoms. Resumption of concurrent therapy with these two therapeutic agents can entail either of the antagonist anti-CD40 antibody/IL-2 administration protocols disclosed herein (i.e., antagonist anti-CD40 antibody with the constant IL-2 dosing regimen or antagonist anti-CD40 antibody with the two-level IL-2 dosing regimen) depending upon the overall health of the subject, relevant disease state, and tolerance for the particular antagonist anti-CD40 antibody/IL-2 administration protocol.

*Constant IL-2 dosing regimen*. In some embodiments of the disclosure, the subject undergoing concurrent therapy with these two therapeutic agents is administered one or more cycles of a constant IL-2 dosing regimen in combination with the antagonist anti-CD40 antibody dosing schedule disclosed herein (i.e., therapeutically effective doses of antagonist anti-CD40 antibody administered weekly, or administered once every two, three, or four weeks, throughout a treatment period or for a fixed duration within the treatment period as noted herein above). By "constant IL-2 dosing regimen" is intended the subject undergoing concurrent therapy with IL-2 and antagonist anti-CD40 antibody is administered a constant total weekly dose of IL-2 over the course of any given cycle of IL-2 administration. One complete cycle of a constant IL-2 dosing regimen comprises administering a constant total weekly dose of IL-2 for a period of about 2 weeks to about 12 weeks, such as about 2, 3, 4, 5, 6, 7, 8, 9, 10, 11, or 12 weeks, followed by a time period off of IL-2 dosing, which has a duration of about 1 week to about 4 weeks, including 1, 2, 3, or 4 weeks. Thus, each cycle of a constant IL-2 dosing regimen comprises a first time period during which the subject is administered a constant total weekly dose of IL-2, and a second time period during which IL-2 dosing is withheld (i.e., a "rest" period or "holiday" from IL-2 administration). Preferably the subject is administered the constant total weekly dose of IL-2 for at least 4 weeks up to about 12 weeks, at which time IL-2 dosing is withheld for a period of about 1 week to about 4 weeks. As noted herein above, either of these therapetuc agents can be administered first in order to intitate this concurrent therapy protocol.

Where the subject is to receive antagonist anti-CD40 antibody therapy throughout a treatment period (i.e., a time period during which the subject in undergoing concurrent therapy with these two therapeutic agents), during each cycle of the constant IL-2 dosing regimen, the subject remains on the recommended dosing regimen for the antagonist anti-CD40 antibody, and thus receives a therapeutically effective dose of antagonist anti-CD40 antibody according to a weekly dosing schedule, or according to a once every two weeks, once every three weeks, or once every four weeks dosing schedule.

Alternatively, where the subject is to receive antagonist anti-CD40 antibody therapy for a fixed duration throughout a treatment period, the treatment period comprises administering a therapeutically effective dose of the anti-CD40 antibody for a fixed duration of about 4 weeks to about 16 weeks, where the antibody is administered once every week, once every two weeks, once every three weeks, or once every four weeks, and administering one or more cycles of the constant IL-2 dosing regimen. Thus, for example, in some embodiments, the subject is administered a therapeutically effective dose of the antagonist anti-CD40 antibody once per week for a fixed duration of 4 weeks or 8 weeks, in combination with at least one cycle of a constant IL-2 dosing regimen. In such embodiments, each complete cycle of the constant IL-2 dosing regimen comprises administering a constant total weekly dose of IL-2 for a period of about 2 weeks to about 12 weeks, such as about 2, 3, 4, 5, 6, 7, 8, 9, 10, 11, or 12 weeks, followed by a time period off of IL-2 dosing, which has a duration of about 1 week to about 4 weeks, including 1, 2, 3, or 4 weeks.

At the discretion of the managing physician, the subject undergoing weekly antagonist anti-CD40 antibody administration, or antagonist anti-CD40 antibody administration once every two, three, or four weeks, can continue receiving the constant total weekly dose of IL-2 for an extended period of time beyond 12 weeks, for example, for an additional 1-8 weeks, providing the antagonist anti-CD40 antibody/constant IL-2 dosing protocol is well tolerated and the subject is exhibiting minimal signs of either antagonist anti-CD40 antibody and/or IL-2 toxicity symptoms. In such an embodiment, conclusion of IL-2 dosing would be followed by a period of about 1 week to about 4 weeks during which IL-2 dosing would be withheld to allow the subject time off of this therapeutic agent before starting a subsequent cycle of the constant IL-2 dosing regimen.

In one embodiment, a subject in need of concurrent therapy with antagonist anti-CD40 antibody and IL-2 is administered a therapeutically effective dose of antagonist anti-CD40 antibody once per week throughout a treatment period, or is administered a therapeutically effective dose of antagonist anti-CD40 antibody once every three weeks throughout this treatment period, beginning on day 1 of this treatment period, and the first cycle of a constant IL-2 dosing regimen is initiated beginning on day 3, 4, 5, 6, 7, 8, 9, or 10 of the same treatment period. In one such embodiment, the first cycle of the constant IL-2 dosing regimen begins on day 8 (i.e., at the start of week 2) of the treatment period, and has a duration of IL-2 administration of about 4 weeks to about 12 weeks, followed by a time period off of IL-2 administration that has a duration of about 1 week to about 4 weeks. At the conclusion of this first cycle of the constant IL-2 dosing regimen, the subject receives one or more subsequent cycles of the constant IL-2 dosing regimen as noted herein above.

In another embodiment, the subject is administered a therapeutically effective dose of antagonist anti-CD40 antibody once per week throughout a treatment period, or is administered a therapeutically effective dose of antagonist anti-CD40 antibody once every three weeks throughout this treatment period, beginning on day 1 of this treatment period, and a first cycle of the constant IL-2 dosing regimen begins on day 8 (i.e., at the start of week 2) of the treatment period, and has a duration of IL-2 administration of 4 weeks, followed by a time period off of IL-2 administration having a duration of 1 week. At the discretion of the managing physician, the subject is then administered one or more subsequent cycles of this constant IL-2 dosing regimen, i.e., administration of the constant total weekly dose of IL-2 for 4 weeks, followed by 1 week off of IL-2 administration. During the entire treatment period, the subject continues to receive the therapeutically effective dose of antagonist anti-CD40 antibody according to the once-a-week dosing schedule, or the once-every-three-weeks dosing schedule, with the proviso that the subject does not exhibit symptoms of antagonist anti-CD40 antibody toxicity. Thus, for example, if the subject undergoes three cycles of the constant IL-2 dosing regimen in combination with weekly (i.e., once-per-week) administration of antagonist anti-CD40 antibody, with the first cycle of IL-2 administration beginning on day 8 of a treatment period (i.e., day 1 of week 2), therapeutically effective doses of the antagonist anti-CD40 antibody would be administered to this subject on day 1 of each week of treatment (i.e., day 1 of weeks 1-16), and a constant total weekly dose of IL-2 would be administered during weeks 2-5, weeks 7-10, and weeks 12-15, with time off from IL-2 dosing occurring during weeks 6, 11, and 16.

In alternative embodiments, a subject in need of concurrent therapy with antagonist anti-CD40 antibody and IL-2 is administered a therapeutically effective dose of antagonist anti-CD40 antibody once per week, or is administered a therapeutically effective dose of antagonist anti-CD40 antibody once every two weeks, for a fixed duration of 4 weeks or 8 weeks beginning on day 1 of a treatment period, and the first cycle of a constant IL-2 dosing regimen is initiated beginning on day 3, 4, 5, 6, 7, 8, 9, or 10 of the same treatment period. In one such embodiment, the first cycle of the constant IL-2 dosing regimen begins on day 8 (i.e., at the start of week 2) of the treatment period, and has a duration of IL-2 administration of about 4 weeks to about 12 weeks, followed by a time period off of IL-2 administration that has a duration of about 1 week to about 4 weeks. At the conclusion of this first cycle of the constant IL-2 dosing regimen, the subject receives one or more subsequent cycles of the constant IL-2 dosing regimen as noted herein above.

In another embodiment, the subject is administered a therapeutically effective dose of antagonist anti-CD40 antibody once per week, or once every two weeks, for a fixed duration of 4 weeks or 8 weeks beginning on day 1 of a treatment period, and a first cycle of the constant IL-2 dosing regimen begins on day 8 (i.e., at the start of week 2) of the treatment period, and has a duration of IL-2 administration of 4 weeks, followed by a time period off of IL-2 administration having a duration of 1 week. At the discretion of the managing physician, the subject is then administered one or more subsequent cycles of this constant IL-2 dosing regimen, i.e., administration of the constant total weekly dose of IL-2 for 4 weeks, followed by 1 week off of IL-2 administration. Thus, for example, if the subject undergoes three cycles of the constant IL-2 dosing regimen in combination with weekly (i.e., once-per-week) administration of antagonist anti-CD40 antibody for a fixed duration of 4 weeks, with the first cycle of IL-2 administration beginning on day 8 of a treatment period (i.e., day 1 of week 2), therapeutically effective doses of the antagonist anti-CD40 antibody would be administered to this subject on day 1 of weeks 1-4 of the treatment period, and a constant total weekly dose of IL-2 would be administered during weeks 2-5, weeks 7-10, and weeks 12-15, with time off from IL-2 dosing occurring during weeks 6, 11, and 16. Alternatively, if the subject undergoes three cycles of the constant IL-2 dosing regimen in combination with weekly (i.e., once-per-week) administration of antagonist anti-CD40 antibody for a fixed duration of 8 weeks, with the first cycle of IL-2 administration beginning on day 8 of a treatment period (i.e., day 1 of week 2), therapeutically effective doses of the antagonist anti-CD40 antibody would be administered to this subject on day 1 of weeks 1-8 of the treatment period, and a constant total weekly dose of IL-2 would be administered during weeks 2-5, weeks 7-10, and weeks 12-15, with time off from IL-2 dosing occurring during weeks 6, 11, and 16.

*Two-level IL-2 dosing regimen*. In other embodiments of the disclosure, concurrent therapy with antagonist anti-CD40 antibody and IL-2 comprises administering a therapeutically effective dose of antagonist anti-CD40 antibody once per week, or once every two, three, or four weeks, throughout a treatment period or for a fixed duration within the treatment period, in combination with one or more cycles of a "two-level IL-2 dosing regimen" during the course of this treatment period. By "two-level IL-2 dosing regimen" is intended the subject undergoing concurrent therapy with IL-2 and antagonist anti-CD40 antibody is administered IL-2 during two time periods of IL-2 dosing, which have a combined duration of about 2 weeks to about 16 weeks, including, 2, 3, 4, 5, 6, 7, 8, 9, 10, 11, 12, 13, 14, 15, or 16 weeks. In one embodiment, the two-level IL-2 dosing regimen has a combined duration of about 4 weeks to about 12 weeks; in other embodiments, the two-level IL-2 dosing regimen has a combined duration of about 4 weeks to about 8 weeks, including about 4, 5, 6, 7, or 8 weeks. The total weekly dose of IL-2 that is to be administered during the first and second time periods of the two-level IL-2 dosing regimen is chosen such that a higher total weekly dose of IL-2 is given during the first time period and a lower total weekly dose of IL-2 is given during the second time period.

The duration of the individual first and second time periods of any given cycle of the two-level IL-2 dosing regimen can vary, depending upon the health of the individual and history of disease progression. Generally, the subject is administered higher total weekly doses of IL-2 for at least 1 week out of the 4-week to 16-week two-level IL-2 dosing regimen. In one embodiment, higher total weekly doses of IL-2 are administered during the first half of the two-level IL-2 dosing regimen, with lower total weekly doses being administered during the second half of the two-level IL-2 dosing regimen. Thus, for example, where one complete cycle of the two-level IL-2 dosing regimen has a combined duration of 8 weeks, the higher total weekly doses of IL-2 would be administered for the first 4 weeks of IL-2 dosing, and the lower total weekly doses of IL-2 would be administered for the second 4 weeks of IL-2 dosing.

Where the subject is to receive antagonist anti-CD40 antibody therapy throughout a treatment period (i.e., a time period during which the subject in undergoing concurrent therapy with these two therapeutic agents), during each cycle of the two-level IL-2 dosing regimen, the subject remains on the recommended dosing regimen for the antagonist anti-CD40 antibody, and thus receives a therapeutically effective dose of antagonist anti-CD40 antibody according to a weekly dosing schedule, or according to a once every two weeks, once every three weeks, or once every four weeks dosing schedule.

Alternatively, where the subject is to receive antagonist anti-CD40 antibody therapy for a fixed duration throughout a treatment period, the treatment period comprises administering a therapeutically effective dose of the anti-CD40 antibody for a fixed duration of about 4 weeks to about 16 weeks, where the antibody is administered once every week, once every two weeks, once every three weeks, or once every four weeks, and administering one or more cycles of the two-level IL-2 dosing regimen. Thus, for example, in some embodiments, the subject is administered a therapeutically effective dose of the antagonist anti-CD40 antibody once per week for a fixed duration of 4 weeks or 8 weeks, in combination with at least one cycle of a two-level IL-2 dosing regimen as defined herein above.

Though specific dosing regimens are disclosed herein below, it is recognized that the disclosure encompasses any administration protocol that provides for concurrent therapy with an antagonist anti-CD40 antibody and one or more cycles of a two-level IL-2 dosing regimen that provides for initial exposure to higher total weekly doses of IL-2, and subsequent exposure to lower total weekly doses of IL-2. While not being bound by theory, it is believed that administering a higher dose of IL-2 during the initial stages of IL-2 dosing provides for an initial stimulation ofNK cell activity that can be maintained by a lower dose during the subsequent weeks of IL-2 dosing. As IL-2 side effects are dose-related, the lowered dose of IL-2 will increase tolerability of this therapeutic agent during the extended treatment period.

Thus, the methods disclosed herein contemplate treatment regimens where a therapeutically effective dose of at least one antagonist anti-CD40 antibody is administered once a week throughout a treatment period, or is administered once every two, three, or four weeks throughout this treatment period, in combination with a two-level IL-2 dosing having a combined duration of about 2 weeks to about 16 weeks, including 2, 3, 4, 5, 6, 7, 8, 9, 10, 11, 12, 13, 14, 15, or 16 weeks. Either agent could be administered first, as explained above for this two-level IL-2 dosing regimen. For example, in one embodiment, a therapeutically effective dose of antagonist anti-CD40 antibody is administered first, for example, on day 1 of a treatment period, followed by initiation of the two-level IL-2 dosing regimen within 10 days, preferably within 7 days of the first administration of the antagonist anti-CD40 antibody, for example, within 1, 2, 3, 4, 5, 6, or 7 days. During the two-level IL-2 dosing regimen, a higher total weekly dose of IL-2 is administered in the first time period of the two-level IL-2 dosing regimen, for example, over the first 1-4 weeks of IL-2 administration, and lower total weekly doses of IL-2 are administered during the second time period of the two-level IL-2 dosing regimen (i.e., over the remaining course of the two-level IL-2 dosing regimen).

In one embodiment, the methods disclosed herein provide for administering a therapeutically effective dose of a pharmaceutical composition comprising at least one antagonist anti-CD40 antibody weekly (i.e., once per week) throughout a treatment period, or administering this therapeutically effective dose of antagonist anti-CD40 antibody once every three weeks throughout this treatment period, in combination with one or more cycles of a two-level IL-2 dosing regimen during the course of this treatment period, where each cycle of the two-level IL-2 dosing regimen has a combined duration of 4 weeks to 8 weeks, including 4, 5, 6, 7, or 8 weeks. Thus, for example, where the antagonist anti-CD40 antibody is to be dosed once per week, a therapeutically effective dose of at least one antagonist anti-CD40 antibody is administered on day 1 of each week of the treatment period, and the first cycle of a 4-week to 8-week two-level IL-2 dosing regimen is initiated beginning on day 3, 4, 5, 6, 7, 8, 9, or 10 of the same treatment period.

In one such embodiment, therapeutically effective doses of the pharmaceutical composition comprising the antagonist anti-CD40 antibody are administered weekly beginning on day 1 of a treatment period and continuing throughout the treatment period, and a first cycle of the two-level IL-2 dosing regimen begins on day 8 of the same treatment period and continues for 8 weeks (i.e., during weeks 2-9 of the treatment period).

Alternatively, the methods disclosed herein contemplate treatment regimens for a subject in need thereof where a therapeutically effective dose of at least one antagonist anti-CD40 antibody is administered once per week, or is administered once every two weeks, for a fixed duration of 4 weeks or 8 weeks, in combination with a two-level IL-2 dosing regimen having a combined duration of about 2 weeks to about 16 weeks, including 2, 3, 4, 5, 6, 7, 8, 9, 10, 11, 12, 13, 14, 15, or 16 weeks. Either agent could be administered first, as explained above for this two-level IL-2 dosing regimen. For example, in one embodiment, a therapeutically effective dose of antagonist anti-CD40 antibody is administered first, for example, on day 1 of a treatment period, followed by initiation of the two-level IL-2 dosing regimen within 10 days, preferably within 7 days of the first administration of the antagonist anti-CD40 antibody, for example, within 1, 2, 3, 4, 5, 6, or 7 days. During the two-level IL-2 dosing regimen, a higher total weekly dose of IL-2 is administered in the first time period of the two-level IL-2 dosing regimen, for example, over the first 1-4 weeks of IL-2 administration, and lower total weekly doses of IL-2 are administered during the second time period of the two-level IL-2 dosing regimen (i.e., over the remaining course of the two-level IL-2 dosing regimen).

In one embodiment, the methods disclosed herein provide for administering a therapeutically effective dose of a pharmaceutical composition comprising at least one antagonist anti-CD40 antibody weekly (i.e., once per week) or once every two weeks for a fixed duration of 4 weeks or 8 weeks, in combination with one or more cycles of a two-level IL-2 dosing regimen during the course of this treatment period, where each cycle of the two-level IL-2 dosing regimen has a combined duration of 4 weeks to 8 weeks, including 4, 5, 6, 7, or 8 weeks. Thus, for example, where the antagonist anti-CD40 antibody is to be dosed once per week for a duration of 4 weeks, a therapeutically effective dose of at least one antagonist anti-CD40 antibody is administered on day 1 of weeks 1-4 of the treatment period, and the first cycle of a 4-week to 8-week two-level IL-2 dosing regimen is initiated beginning on day 3, 4, 5, 6, 7, 8, 9, or 10 of the same treatment period.

In one such embodiment, therapeutically effective doses of the pharmaceutical composition comprising the antagonist anti-CD40 antibody are administered weekly for a fixed duration of 4 weeks beginning on day 1 of a treatment period, and a first cycle of the two-level IL-2 dosing regimen begins on day 8 of the same treatment period and continues for 8 weeks (i.e., during weeks 2-9 of the treatment period).

*Interruption of IL-2 dosing*. The methods disclosed herein also contemplate embodiments where a subject undergoing antagonist anti-CD40 antibody administration according to the dosing schedule recommended herein in combination with administration of one or more cycles of a two-level IL-2 dosing regimen is given a "drug holiday" or a time period off from IL-2 dosing, or from the IL-2 dosing and the antagonist anti-CD40 antibody dosing, between the conclusion of the first time period of any given cycle of the two-level IL-2 dosing regimen and the initiation of the second time period of that particular cycle of the two-level IL-2 dosing regimen. In these embodiments, the two-level IL-2 dosing regimen is interrupted such that IL-2 dosing is withheld for a period of about 1 week to about 4 weeks following conclusion of the first time period of a given cycle of the two-level IL-2 dosing regimen during which the higher total weekly dose has been administered. During this time period off of IL-2 dosing, the subject may continue to receive a therapeutically effective dose of antagonist anti-CD40 antibody according to the dosing schedule recommended herein (i.e., once per week, or once every two, three, or four weeks) or, alternatively, antagonist anti-CD40 antibody dosing can also be stopped. The length of this interruption in IL-2 dosing will depend upon the health of the subject, history of disease progression, and responsiveness of the subject to the initial IL-2/antibody therapy received during the first time period of any given cycle of the two-level IL-2 dosing regimen. Generally, IL-2 dosing is interrupted for a period of about 1 week to about 4 weeks, at which time the subject is administered the second time period of the two-level IL-2 dosing regimen, where lower total weekly doses of IL-2 are administered in combination with the weekly administration of therapeutically effective doses of the antagonist anti-CD40 antibody. In order to complete any given cycle of a two-level IL-2 dosing regimen, a subject must be administered both the first period of higher total weekly dosing and the second period of lower total weekly dosing.

During this drug holiday (i.e., time period off of IL-2 administration, or time period off of IL-2 and antagonist anti-CD40 antibody administration), natural-killer (NK) cell counts are monitored to determine when the two-level IL-2 dosing regimen, or the two-level IL-2 dosing regimen and weekly administration of antagonist anti-CD40 antibody, are to be resumed. NK cell levels above 200 cells/μl may be an important factor influencing positive clinical response to antagonist anti-CD40 antibody therapy. Therefore, monitoring of NK cell count during, at the conclusion, and following any given cycle of IL-2 dosing provides a means for determining when and if IL-2 therapy should be reinstated following a time off of IL-2 dosing, which may or may not include a time off of antagonist anti-CD40 antibody administration.

In this manner, NK cell counts are measured bi-weekly or monthly during the two-level IL-2 dosing regimen, and at the conclusion of the first time period of the two-level IL-2 dosing regimen before the drug holiday is initiated. Where NK cell count exceeds an acceptable threshold level, the two-level IL-2 dosing regimen can be interrupted. By "acceptable threshold level" is intended the subject undergoing treatment has an NK cell count that is about 150 cells/μl or greater, preferably 200 cells/μl or greater. Following discontinuance of the IL-2 dosing, which may or may not include discontinuance of antagonist anti-CD40 antibody administration, NK cell counts are then measured once per week or twice per week thereafter, or can be monitored less frequently, for example, once every two weeks or once every three weeks. An NK cell count falling below the acceptable threshold level of about 150 cells/μl, for example, an NK cell count of less than 150 cells/μl, is indicative of the necessity to resume the two-level IL-2 dosing regimen, or the two-level IL-2 dosing regimen and the antagonist anti-CD40 antibody dosing regimen where the drug holiday also includes time off of antagonist anti-CD40 antibody administration. Preferably the two-level IL-2 dosing regimen is resumed when NK cell count falls below a threshold level of about 200 cells/μl, i.e., an NK cell count of less than 200 cells/μl. At this time, the subject is administered the second time period of the two-level IL-2 dosing regimen, where lower total weekly doses of IL-2 are administered in combination with the antagonist anti-CD40 antibody, wherein a therapeutically effective dose of the antibody is administered once per week or is administered once every two, three, or four weeks.

Depending upon the overall health of the subject, clinical response, and tolerability of concurrent therapy with these two therapeutic agents, following completion of a first cycle of a two-level IL-2 dosing regimen, the subject can be administered one or more subsequent cycles of a two-level IL-2 dosing regimen in combination with administration of therapeutically effective doses of antagonist anti-CD40 antibody, where a therapeutically effective dose of the antibody is administered once per week or is administered once every two, three, or four weeks. In such embodiments, the managing physician can allow for a drug holiday or time period off of IL-2 administration between successive cycles. Thus, upon completion of any given cycle of a two-level IL-2 dosing regimen, which itself may or may not include a time period off of IL-2 administration between the first and second periods of IL-2 dosing, the subject can be given a break in IL-2 administration before initiating a subsequent cycle of the two-level IL-2 dosing regimen. Generally, the time period off of IL-2 administration between any given cycle of two-level IL-2 dosing is about 1 week to about 4 weeks, including about 1, 2, 3, or 4 weeks. During IL-2 drug holidays, the subject may continue to receive therapeutically effective doses of antagonist anti-CD40 antibody, which can be administered once per week, or once every two, three, or four weeks, or, alternatively, the antagonist anti-CD40 antibody dosing can also be stopped.

*Subsequent courses of combination IL-2/antagonist anti-CD40 antibody therapy.*

Where a subject undergoing therapy in accordance with the previously mentioned dosing regimens exhibits a partial response, or a relapse following a prolonged period of remission, subsequent courses of concurrent therapy may be needed to achieve complete remission of the disease. Thus, subsequent to a period of time off from a first treatment period, which may have comprised a constant IL-2 dosing regimen or a two-level IL-2 dosing regimen in combination with anti-CD40 antibody therapy throughout the treatment period or for a fixed duration within the treatment period, a subject may receive one or more additional treatment periods comprising either constant or two-level IL-2 dosing regimens in combination with antagonist anti-CD40 antibody administration, where the antibody can be administered throughout the additional treatment period(s) or administered for a fixed duration within the treatment period. Such a period of time off between treatment periods is referred to herein as a time period of discontinuance. It is recognized that the length of the time period of discontinuance is dependent upon the degree of tumor response (i.e., complete versus partial) achieved with any prior treatment periods of concurrent therapy with these two therapeutic agents.

Thus, for example, where a subject is undergoing concurrent therapy with weekly doses of antagonist anti-CD40 antibody and a two-level IL-2 dosing regimen, which may or may not include a drug holiday between the first and second time periods of the two-level IL-2 dosing regimen, their treatment regimen may include multiple treatment sessions, each of which comprises concurrent therapy with weekly doses of antagonist anti-CD40 antibody and a two-level IL-2 dosing regimen. These multiple treatment sessions are referred to herein as maintenance cycles, where each maintenance cycle comprises antagonist anti-CD40 antibody administration in combination with a completed two-level IL-2 dosing regimen. By "completed two-level IL-2 dosing regimen" is intended the subject has been administered both the first period of higher total weekly dosing and the second period of lower total weekly dosing. The necessity for multiple maintenance cycles can be assessed by monitoring NK cell count in a manner similar to that used to determine when a drug holiday is warranted, and when such a drug holiday must be concluded. Thus, upon completion of the two-level IL-2 dosing regimen in any given maintenance cycle, the treating physician obtains a measurement of NK cell count. This indicator is then measured at monthly intervals (i.e., once a month) following completion of any given two-level IL-2 dosing regimen. As with drug holidays, an NK cell count falling below an acceptable threshold level (i.e., below about 150 cells/μl, preferably below about 200 cells/μl) is indicative of the need for administering another maintenance cycle to the subject. The duration between maintenance cycles can be about 1 month to about 6 months, including 1 month, 1.5 months, 2 months, 2.5 months, 3 months, 3.5 months, 4 months, 4.5 months, 5 months, 5.5 months, 6 months, or other such time periods falling within the range of about 1 month to about 6 months.

Thus, the administration methods disclosed herein provide an improved means for managing cancers that comprise CD40-expressing cells, for example, B-cell related cancers such as lymphomas, myelomas, and leukemia, and solid cancers, such as kidney (including, for example, renal cell carcinomas), bladder, liver (including, for example, hepatocellular carcinomas), gastric, cervical, prostate, nasopharyngeal, thyroid (for example, thyroid papillary carcinoma), and skin cancers such as melanoma, and sarcomas (including, for example, osteosarcomas and Ewing's sarcomas). Without being bound by theory, the constant IL-2 dosing schedule with interruptions between provides an intermittent dosing schedule that allows for less frequent administration of the IL-2 during antagonist anti-CD40 antibody therapy, and better tolerability of long-term IL-2 therapy. The two-level IL-2 dosing regimen offers the opportunity to provide a patient with higher total weekly doses of IL-2, which provide for expansion of NK cell numbers that can be maintained by a lower dose during the subsequent weeks of IL-2 dosing. This administration protocol has the additional attraction of providing IL-2 drug holidays between the higher and lower total weekly IL-2 dosing schedules, as well as IL-2 drug holidays between completed cycles of the two-level IL-2 dosing regimen, again contributing to increased tolerability of concurrent therapy with antagonist anti-CD40 antibody and IL-2.

It is recognized that where a subject undergoing therapy in accordance with the previously mentioned dosing regimens exhibits a partial response, or a relapse following a prolonged period of remission, the subject could receive the antagonist anti-CD40 antibody as a single therapeutic agent, or in combination with another oncotherapy. Thus, a subject that has undergone previous concurrent therapy with IL-2 and antagonist anti-CD40 antibody for treatment of a cancer comprising CD40-expressing neoplastic cells and who is need of further treatment for the cancer can undergo therapy with an antagonist anti-CD40 antibody alone, or an antagonist anti-CD40 antibody and at least one other oncotherapy prior to, or as an alternative to, further concurrent therapy with antagonist anti-CD40 antibody and IL-2. Other types of oncotherapy include, but are not limited to, those described herein below.

*IL-2 dosing schedule*. For human subjects undergoing concurrent therapy with antagonist anti-CD40 antibody according to the administration schedule recommended herein in combination with one or more cycles of a constant IL-2 dosing regimen or one or more cycles of a two-level IL-2 dosing regimen, the total weekly dose of IL-2 to be administered during periods of IL-2 dosing can be administered as a single dose, or can be partitioned into a series of equivalent doses that are administered according to a two-three-, four-, five-, six-, or seven-times-a-week dosing schedule. Where higher total weekly doses are to be administered during a first time period, and lower total weekly doses are to be administered during a second time period, it is not necessary that the total weekly dose be administered in the same manner over the course of both dosing periods. Thus, for example, the higher total weekly dose during the first time period of a two-level IL-2 dosing regimen can be administered as a single dose, or can be partitioned into a series of equivalent doses that are administered according to a two- three-, four-, five-, six-, or seven-times-a-week dosing schedule. Similarly, the lower total weekly dose during the second time period of a two-level IL-2 dosing regimen can be administered as a single dose, or can be partitioned into a series of equivalent doses that are administered according to a two-, three-, four-, five-, six-, or seven-times-a-week dosing schedule.

For purposes of the present invention, a "two-, three-, four-, five-, six-, or seven-times-a-week dosing schedule" is intended to mean that the total weekly dose is partitioned into two, three, four, five, six, or seven equivalent doses, respectively, which are administered to the subject over the course of a 7-day period, with no more than one equivalent dose being administered per 24-hour period. The series of equivalent doses can be administered on sequential days, or can be administered such that one or more days occur between any two consecutive doses, depending upon the total number of equivalent doses administered per week.

Thus, for example, where a series of two equivalent doses of IL-2 are administered per week (i.e., over a 7-day period) and the first equivalent dose of that week is administered on day 1, the second equivalent dose of IL-2 can be administered on day 2, 3, 4, 5, 6, or 7 of that week. In one embodiment, the total weekly dose of IL-2 is partitioned into two equivalent doses that are administered to the subject within a 7-day period, allowing for a minimum of 72 hours between doses and a maximum of 96 hours between doses.

Similarly, where a series of three equivalent doses of IL-2 are administered per week and the first equivalent dose of that week is administered on day 1, the second equivalent dose can be administered on day 2, 3, 4, 5, or 6 of that week, and the third equivalent dose can be administered on day 3, 4, 5, 6, or 7 of that week, so long as about 24 hours occur between administration of the second and third equivalent doses. In one embodiment, the total weekly dose of IL-2 is partitioned into three equivalent doses that are administered to the subject within a 7-day period, allowing for a minimum of 25 hours between doses and a maximum of 72 hours between doses.

Where a series of four equivalent doses of IL-2 are administered per week and the first equivalent dose of that week is administered on day 1, the second equivalent dose can be administered on day 2, 3, 4, or 5 of that week, the third equivalent dose can be administered on day 3, 4, 5, or 6 of that week, and the fourth equivalent dose can be administered on day 4, 5, 6, or 7 of that week, so long as about 24 hours occur between administration of any two consecutive doses (i.e., between the first and second equivalent doses, between the second and third equivalent doses, and between the third and fourth equivalent doses).

Where a series of five equivalent doses are administered per week and the first equivalent dose of that week is administered on day 1, the second equivalent dose can be administered on day 2, 3, or 4 of that week, the third equivalent dose can be administered on day 3, 4, or 5 of that week, the fourth equivalent dose can be administered on day 4, 5, or 6 of that week, and the fifth equivalent dose can be administered on day 5, 6, or 7 of that week, so long as about 24 hours occur between administration of any two consecutive doses (i.e., between the first and second equivalent doses, between the second and third equivalent doses, between the third and fourth equivalent doses, and between the fourth and fifth equivalent doses).

Where a series of six equivalent doses of IL-2 are administered per week and the first equivalent dose of that week is administered on day 1, the second equivalent dose can be administered on day 2 or 3 of that week, the third equivalent dose can be administered on day 3 or 4 of that week, the fourth equivalent dose can be administered on day 4 or 5 of that week, the fifth equivalent dose can be administered on day 5 or 6 of that week, and the sixth equivalent dose can be administered on day 6 or 7 of that week, so long as about 24 hours occur between administration of any two consecutive doses (i.e., between the first and second equivalent doses, between the second and third equivalent doses, between the third and fourth equivalent doses, between the fourth and fifth equivalent doses, and between the fifth and sixth equivalent doses).

In one embodiment, the total weekly dose of IL-2 is partitioned into seven equivalent doses, which are administered daily over the 7-day period, with about 24 hours occurring between each consecutive dose.

It is not necessary that the same dosing schedule be followed throughout a constant IL-2 dosing regimen, or that the same dosing schedule be followed for both the first and second periods of the two-level IL-2 dosing regimen. Thus, the dosing schedule can be adjusted to accommodate an individual's tolerance of prolonged IL-2 therapy in combination with antagonist anti-CD40 antibody therapy, and to reflect the individual's responsiveness to concurrent therapy with these two therapeutic agents. The preferred dosing schedule during the constant IL-2 dosing regimen and the two time periods of the two-level IL-2 dosing regimen is readily determined by the managing physician given the patient's medical history and the guidance provided herein.

## Antagonist Anti-CD40 Antibody Dose Ranges for Use in Combination IL-2/Antagonist Anti-CD40 Antibody Therapy

For purposes of the present invention, the therapeutically effective dose of an antagonist anti-CD40 antibody or antigen-binding fragment thereof as defined elsewhere herein to be administered in combination with IL-2 therapy as disclosed herein ranges from about 0.003 mg/kg to about 50 mg/kg, from about 0.01 mg/kg to about 40 mg/kg, from about 0.01 mg/kg to about 30 mg/kg, from about 0.1 mg/kg to about 30 mg/kg, from about 0.5 mg/kg to about 30 mg/kg, from about 1 mg/kg to about 30 mg/kg, from about 3 mg/kg to about 30 mg/kg, from about 3 mg/kg to about 25 mg/kg, from about 3 mg/kg to about 20 mg/kg, from about 5 mg/kg to about 15 mg/kg, or from about 7 mg/kg to about 12 mg/kg. Thus, for example, the dose of any one antagonist anti-CD40 antibody or antigen-binding fragment thereof, for example the anti-CD40 monoclonal antibody CHIR-12.12 or CHIR-5.9 or antigen-binding fragment thereof, can be 0.003 mg/kg, 0.01 mg/kg, 0.03 mg/kg, 0.1 mg/kg, 0.3 mg/kg, 0.5 mg/kg, 1 mg/kg, 1.5 mg/kg, 2 mg/kg, 2.5 mg/kg, 3 mg/kg, 5 mg/kg, 7 mg/kg, 10 mg/kg, 15 mg/kg, 20 mg/kg, 25 mg/kg, 30 mg/kg, 35 mg/kg, 40 mg/kg, 45 mg/kg, 50 mg/kg, or other such doses falling within the range of about 0.003 mg/kg to about 50 mg/kg. The same therapeutically effective dose of an antagonist anti-CD40 antibody or antigen-binding fragment thereof can be administered throughout each week of antibody dosing. Alternatively, different therapeutically effective doses of an antagonist anti-CD40 antibody or antigen-binding fragment thereof can be used over the course of a treatment period.

Thus, in some embodiments, the initial therapeutically effective dose of an antagonist anti-CD40 antibody or antigen-binding fragment thereof as defined elsewhere herein can be in the lower dosing range (i.e., about 0.003 mg/kg to about 20 mg/kg) with subsequent doses falling within the higher dosing range (i.e., from about 20 mg/kg to about 50 mg/kg).

In alternative embodiments, the initial therapeutically effective dose of an antagonist anti-CD40 antibody or antigen-binding fragment thereof as defined elsewhere herein can be in the upper dosing range (i.e., about 20 mg/kg to about 50 mg/kg) with subsequent doses falling within the lower dosing range (i.e., 0.003 mg/kg to about 20 mg/kg). Thus, in one embodiment, the initial therapeutically effective dose of the antagonist anti-CD40 antibody or antigen-binding fragment thereof is about 20 mg/kg to about 35 mg/kg, including about 20 mg/kg, about 25 mg/kg, about 30 mg/kg, and about 35 mg/kg, and subsequent therapeutically effective doses of the antagonist anti-CD40 antibody or antigen binding fragment thereof are about 5 mg/kg to about 15 mg/kg, including about 5 mg/kg, 8 mg/kg, 10 mg/kg, 12 mg/kg, and about 15 mg/kg.

In some embodiments of the invention, antagonist anti-CD40 antibody therapy is initiated by administering a "loading dose" of the antibody or antigen-binding fragment thereof to the subject in need of IL-2/antagonist anti-CD40 antibody combination therapy. By "loading dose" is intended an initial dose of the antagonist anti-CD40 antibody or antigen-binding fragment thereof that is administered to the subject, where the dose of the antibody or antigen-binding fragment thereof administered falls within the higher dosing range (i.e., from about 20 mg/kg to about 50 mg/kg). The "loading dose" can be administered as a single administration, for example, a single infusion where the antibody or antigen-binding fragment thereof is administered IV, or as multiple administrations, for example, multiple infusions where the antibody or antigen-binding fragment thereof is administered IV, so long as the complete "loading dose" is administered within about a 24-hour period. Following administration of the "loading dose," the subject is then administered one or more additional therapeutically effective doses of the antagonist anti-CD40 antibody or antigen-binding fragment thereof. Subsequent therapeutically effective doses can be administered according to a weekly dosing schedule, or once every two weeks, once every three weeks, or once every four weeks as noted herein above. In such embodiments, the subsequent therapeutically effective doses generally fall within the lower dosing range (i.e., 0.003 mg/kg to about 20 mg/kg). Alternatively, in some embodiments, following the "loading dose, " the subsequent therapeutically effective doses of the antagonist anti-CD40 antibody or antigen-binding fragment thereof are administered according to a "maintenance schedule," wherein the therapeutically effective dose of the antibody or antigen-binding fragment thereof is administered once a month, once every 6 weeks, once every two months, once every 10 weeks, once every three months, once every 14 weeks, once every four months, once every 18 weeks, once every five months, once every 22 weeks, once every six months, once every 7 months, once every 8 months, once every 9 months, once every 10 months, once every 11 months, or once every 12 months. In such embodiments, the therapeutically effective doses of the antagonist anti-CD40 antibody or antigen-binding fragment thereof fall within the lower dosing range (i.e., 0.003 mg/kg to about 20 mg/kg), particularly when the subsequent doses are administered at more frequent intervals, for example, once every two weeks to once every month, or within the higher dosing range (i.e., from about 20 mg/kg to about 50 mg/kg), particularly when the subsequent doses are administered at less frequent intervals, for example, where subsequent doses are administered about one month to about 12 months apart.

IL-2 Dose Ranges for Use in Combination IL-2/Antagonist Anti-CD40 Antibody Therapy

For purposes of the following discussion of therapeutically effective doses of IL-2 or biologically active variant thereof to be administered in combination with antagonist-antiCD40 antibody therapy, the IL-2 pharmaceutical composition commercially available under the tradename Proleukin® (Chiron Corporation, Emeryville, California) is used as the reference IL-2 standard. By "reference IL-2 standard" is intended the formulation of IL-2 that serves as the basis for determination of the therapeutically effective doses of IL-2 or biologically active variant thereof to be administered to a human subject with a cancer comprising CD40-expressing cells in combination with antagonist anti-CD40 antibody therapy. The active moiety used in this IL-2 product is the recombinant human IL-2 mutein aldesleukin (referred to as des-alanyl-1, serine-125 human interleukin-2; see U.S. Patent No. 4,931,543).

Thus, for example, where the source of the IL-2 is Proleukin®, the total weekly dose of Proleukin® IL-2 to be administered in combination with antagonist-antiCD40 antibody therapy is in the range from about 18 million international units (MIU) to about 54 MIU, depending upon the medical history of the patient undergoing therapy and the recommended IL-2 dosing regimen (for example, constant versus two-level IL-2 dosing regimen). Where higher doses of IL-2 are to be administered, the total weekly dose of Proleukin® IL-2 can be in the range from about 42.0 MIU to about 54.0 MIU, including about 42.0 MIU, 43.5 MIU, 45.0 MIU, 46.5 MIU, 48.0 MIU, 49.5 MIU, 51.0 MIU, 52.5 MIU, and 54.0 MIU, and other such values falling within this range. Where intermediate doses of IL-2 are to be administered, the total weekly dose of Proleukin® IL-2 can be in the range from about 30.0 MIU to about 42.0 MIU, including about 30.0 MIU, 31.5 MIU, 33.0 MIU, 34.5 MIU, 36.0 MIU, 37.5 MIU, 39.0 MIU, 40.5 MIU, and 42.0 MIU, and other such values falling within this range. Where lower doses of IL-2 are to be administered, the total weekly dose of Proleukin® IL-2 can be in the range from about 18.0 MIU to about 30.0 MIU, including about 18.0 MIU, 19.5 MIU, 21.0 MIU, 22.5 MIU, 24.0 MIU, 25.5 MIU, 27.0 MIU, 28.5 MIU, and 30.0 MIU, and other such values falling within this range.

*Recommended doses for constant IL-2 dosing regimen*. Where Proleukin® IL-2 is to be administered according to a constant IL-2 dosing regimen in combination with antagonist-antiCD40 antibody therapy, the total weekly dose of this reference IL-2 standard is about 18.0 MIU to about 54.0 MIU. Thus, for example, in some embodiments, the total amount of Proleukin® IL-2 that is to be administered per week as part of a constant IL-2 dosing regimen is about 18.0 MIU, 20 MIU, 22.0 MIU, 24.0 MIU, 26.0 MIU, 28.0 MIU, 30.0 MIU, 32.0 MIU, 34.0 MIU, 36.0 MIU, 38.0 MIU, 40.0 MIU, 42.0 MIU, 44.0 MIU, 46.0 MIU, 48.0 MIU, 50.0 MIU, 52.0 MIU, or 54.0 MIU, and other such doses falling within the range of about 18.0 MIU to about 54.0 MIU. In some embodiment, the total weekly dose of Proleukin® IL-2 falls within the range of about 42.0 MIU to about 54.0 MIU, for example, 42.0 MIU, 44.0 MIU, 46.0 MIU, 48.0 MIU, 50.0 MIU, 52.0 MIU, or 54.0 MIU. In other embodiments, the total weekly dose of Proleukin® IL-2 falls within the range of about 30.0 MIU to about 42.0 MIU, for example, about 30.0 MIU, 32.0 MIU, 34.0 MIU, 36.0 MIU, 38.0 MIU, 40.0 MIU, or 42.0 MIU. In alternative embodiments, the total weekly dose of Proleukin® IL-2 is about 18.0 MIU to about 30.0 MIU, for example, 18.0 MIU, 20 MIU, 22.0 MIU, 24.0 MIU, 26.0 MIU, 28.0 MIU, or 30.0 MIU.

As previously noted, the total weekly dose of IL-2 during a constant IL-2 dosing regimen can be administered as a single dose, or can be partitioned into a series of equivalent doses that are administered according to a two-, three-, four-, five-, six- or seven-times-a-week dosing schedule. Thus, for example, where the total weekly dose of Proleukin® IL-2 is 54.0 MIU, the three equivalent doses of this IL-2 source to be administered during each week would be 18.0 MIU, and the two equivalent doses of this IL-2 source to be administered during each week would be 27.0 MIU. Similarly, where the total weekly dose of Proleukin® IL-2 is 42.0 MIU, the three equivalent doses of this IL-2 source to be administered during each week would be 14.0 MIU, and the two equivalent doses of this IL-2 source to be administered during each week would be 21.0 MIU. Where the total weekly dose of Proleukin® IL-2 is 30.0 MIU, the three equivalent doses of this IL-2 source to be administered during each week of IL-2 dosing would be 10.0 MIU, and the two equivalent doses of this IL-2 source to be administered during each week of IL-2 dosing would be 15 MIU. Similarly, where the total weekly dose of Proleukin®IL-2 is 18.0 MIU, the three equivalent doses of this IL-2 source to be administered during each week would be 6.0 MIU, and the two equivalent doses of this IL-2 source to be administered during each week would be 9.0 MIU.

In accordance with the methods disclosed herein, the subject is administered this constant IL-2 dosing regimen in combination with therapeutically effective doses of the antagonist anti-CD40 antibody or antigen-binding fragment thereof, where the therapeutically effective dose of the antibody or antigen-binding fragment thereof is administered according to a dosing regimen disclosed herein (i.e., once per week, once every two weeks, once every three weeks, or once every four weeks throughout a treatment period or for a fixed duration within a treatment period). The therapeutically effective dose of anti-CD40 antibody or antigen-binding fragment thereof to be administered is in the range from about 0.003 mg/kg to about 50 mg/kg, including about 0.5 mg/kg to about 30 mg/kg. Thus, for example, in some embodiments, the total amount of antagonist anti-CD40 antibody or antigen-binding fragment thereof is about 0.003 mg/kg, 0.01 mg/kg, 0.03 mg/kg, 0.1 mg/kg, 0.3 mg/kg, 0.5 mg/kg, 1 mg/kg, 1.5 mg/kg, 2 mg/kg, 2.5 mg/kg, 3 mg/kg, 5 mg/kg, 7 mg/kg, 10 mg/kg, 15 mg/kg, 20 mg/kg, 25 mg/kg, 30 mg/kg, 35 mg/kg, 40 mg/kg, 45 mg/kg, 50 mg/kg, or other such doses falling within the range of about 0.003 mg/kg to about 50 mg/kg.

Thus, for example, in some embodiments, the total amount of Proleukin® IL-2 that is to be administered per week as part of a constant IL-2 dosing regimen is about 42.0 MIU, 44.0 MIU, 46.0 MIU, 48.0 MIU, 50.0 MIU, 52.0 MIU, or 54.0 MIU, and the therapeutically effective dose of antagonist anti-CD40 antibody or antigen-binding fragment thereof that is to be administered in combination with the constant IL-2 dosing regimen is about 0.5 mg/kg, 1 mg/kg, 1.5 mg/kg, 2 mg/kg, 2.5 mg/kg, 3 mg/kg, 5 mg/kg, 7 mg/kg, 10 mg/kg, 15 mg/kg, 20 mg/kg, 25 mg/kg, 30 mg/kg, 35 mg/kg, 40 mg/kg, 45 mg/kg, or 50 mg/kg, where this dose of the antagonist anti-CD40 antibody or antigen-binding fragment thereof is administered once per week, once every two weeks, once every three weeks, or once every four weeks, throughout a treatment period or for a fixed duration within a treatment period. In one such embodiment, the total amount of Proleukin® IL-2 that is to be administered per week as part of a constant IL-2 dosing regimen is about 42.0 MIU, and the the therapeutically effective dose of antagonist anti-CD40 antibody or antigen-binding fragment thereof that is to be administered in combination with the constant IL-2 dosing regimen is about 0.5 mg/kg, 1 mg/kg, 5 mg/kg, 7 mg/kg, 10 mg/kg, 15 mg/kg, 20 mg/kg, 25 mg/kg, 30 mg/kg, or 35 mg/kg, where this dose of the antagonist anti-CD40 antibody or antigen-binding fragment thereof is administered once per week, once every two weeks, once every three weeks, or once every four weeks, throughout a treatment period or for a fixed duration within a treatment period. In preferred embodiments, this total weekly dose of 42.0 MIU Proleukin® IL-2 is partitioned into two or three equivalent doses that are administered according to a two- or three-times-a-week dosing schedule, respectively. In this manner, the two equivalent doses of this IL-2 source to be administered during each week of the constant IL-2 dosing regimen would be 21.0 MIU, and the three equivalent doses of this IL-2 source to be administered during each week of the constant IL-2 dosing regimen would be 14.0 MIU.

In other embodiments, the total amount of Proleukin® IL-2 that is to be administered per week as part of a constant IL-2 dosing regimen is about 30.0 MIU, 32.0 MIU, 34.0 MIU, 36.0 MIU, 38.0 MIU, 40.0 MIU, or 42.0 MIU, and the therapeutically effective dose of antagonist anti-CD40 antibody or antigen-binding fragment thereof that is to be administered in combination with the constant IL-2 dosing regimen is about 0.5 mg/kg, 1 mg/kg, 1.5 mg/kg, 2 mg/kg, 2.5 mg/kg, 3 mg/kg, 5 mg/kg, 7 mg/kg, 10 mg/kg, 15 mg/kg, 20 mg/kg, 25 mg/kg, 30 mg/kg, 35 mg/kg, 40 mg/kg, 45 mg/kg, or 50 mg/kg, where this dose of the antagonist anti-CD40 antibody or antigen-binding fragment thereof is administered once per week, once every two weeks, once every three weeks, or once every four weeks, throughout a treatment period or for a fixed duration within a treatment period. In one such embodiment, the total amount of Proleukin® IL-2 that is to be administered per week as part of a constant IL-2 dosing regimen is about 30.0 MIU, and the therapeutically effective dose of antagonist anti-CD40 antibody or antigen-binding fragment thereof that is to be administered in combination with the constant IL-2 dosing regimen is about 0.5 mg/kg, 1 mg/kg, 5 mg/kg, 7 mg/kg, 10 mg/kg, 15 mg/kg, 20 mg/kg, 25 mg/kg, 30 mg/kg, or 35 mg/kg, where this dose of the antagonist anti-CD40 antibody or antigen-binding fragment thereof is administered once per week, once every two weeks, once every three weeks, or once every four weeks, throughout a treatment period or for a fixed duration within a treatment period. In preferred embodiments, this total weekly dose of 30.0 MIU Proleukin® IL-2 is partitioned into two or three equivalent doses that are administered according to a two- or three-times-a-week dosing schedule, respectively. In this manner, the two equivalent doses of this IL-2 source to be administered during each week of the constant IL-2 dosing regimen would be 15.0 MIU, and the three equivalent doses of this IL-2 source to be administered during each week of the constant IL-2 dosing regimen would be 10.0 MIU.

In yet other embodiments, the total amount of Proleukin®IL-2 that is to be administered per week as part of a constant IL-2 dosing regimen is about 18.0 MIU, 20 MIU, 22.0 MIU, 24.0 MIU, 26.0 MIU, 28.0 MIU, or 30.0 MIU, and the therapeutically effective dose of antagonist anti-CD40 antibody or antigen-binding fragment thereof that is to be administered in combination with the constant IL-2 dosing regimen is about 0.5 mg/kg, 1 mg/kg, 1.5 mg/kg, 2 mg/kg, 2.5 mg/kg, 3 mg/kg, 5 mg/kg, 7 mg/kg, 10 mg/kg, 15 mg/kg, 20 mg/kg, 25 mg/kg, 30 mg/kg, 35 mg/kg, 40 mg/kg, 45 mg/kg, or 50 mg/kg, where this dose of the antagonist anti-CD40 antibody or antigen-binding fragment thereof is administered once per week, once every two weeks, once every three weeks, or once every four weeks, throughout a treatment period or for a fixed duration within a treatment period. In one such embodiment, the total amount of Proleukin® IL-2 that is to be administered per week as part of a constant IL-2 dosing regimen is about 18.0 MIU, and the therapeutically effective dose of antagonist anti-CD40 antibody or antigen-binding fragment thereof that is to be administered in combination with the constant IL-2 dosing regimen is about 0.5 mg/kg, 1 mg/kg, 5 mg/kg, 7 mg/kg, 10 mg/kg, 15 mg/kg, 20 mg/kg, 25 mg/kg, 30 mg/kg, or 35 mg/kg, where this dose of the antagonist anti-CD40 antibody or antigen-binding fragment thereof is administered once per week, once every two weeks, once every three weeks, or once every four weeks, throughout a treatment period or for a fixed duration within a treatment period. In preferred embodiments, this total weekly dose of 18.0 MIU Proleukin®IL-2 is partitioned into two or three equivalent doses that are administered according to a two- or three-times-a-week dosing schedule, respectively. In this manner, the two equivalent doses of this IL-2 source to be administered during each week of the constant IL-2 dosing regimen would be 9.0 MIU, and the three equivalent doses of this IL-2 source to be administered during each week of the constant IL-2 dosing regimen would be 6.0 MIU.

*Recommended doses for two-level IL-2 dosing regimen.* Where Proleukin® IL-2 is to be administered according to a two-level IL-2 dosing regimen in combination with antagonist anti-CD40 antibody therapy, the higher total weekly dose of this reference IL-2 standard that is administered during the first time period of this IL-2 dosing regimen is about 30.0 MIU to about 54.0 MIU, and the lower total weekly dose that is administered during the second time period of this IL-2 dosing regimen is about 18.0 MIU to about 39.0 MIU. As previously noted, the total weekly dose administered during the first time period of the two-level IL-2 dosing regimen, for example, during the first half of this dosing regimen, is always higher than the total weekly dose administered during the second time period of the two-level IL-2 dosing regimen, for example, during the second half of this dosing regimen.

Thus, in some embodiments, the higher total weekly dose of Proleukin® IL-2 that is administered during the first time period of the two-level IL-2 dosing regimen is about 30.0 MIU to about 54.0 MIU, including about 30.0 MIU, 32.0 MIU, 35.0 MIU, 37.0 MIU, 40.0 MIU, 42.0 MIU, 45.0 MIU, 47.0 MIU, 50.0 MIU, 52.0 MIU, or 54.0 MIU, and other such values falling within this higher dosing range; and the lower total weekly dose of Proleukin® IL-2 is about 18.0 MIU to about 39.0 MIU, including 18.0 MIU, 20.0 MIU, 23.0 MIU, 25.0 MIU, 27.0 MIU, 30.0 MIU, 32 MIU, 35.0 MIU, 37.0 MIU, or 39.0 MIU, and other such values falling within this lower dosing range.

As previously noted, the total weekly dose of IL-2 during the first and second time periods of a two-level IL-2 dosing regimen is administered as a single dose, or is partitioned into a series of equivalent doses that are administered according to a two-, three-, four-, five-, six-, or seven-times-a-week dosing schedule. Thus, for example, where the total weekly dose of Proleukin® IL-2 during the first period of the two-level IL-2 dosing regimen is 42.0 MIU, the three equivalent doses of this reference IL-2 standard to be administered during each week would be 14.0 MIU, and the two equivalent doses of this reference IL-2 standard to be administered during each week would be 21.0 MIU. Similarly, where the total weekly dose of Proleukin® IL-2 during the second period of the two-level IL-2 dosing regimen is 30.0 MIU, the three equivalent doses of this reference IL-2 standard to be administered during each week would be 10.0 MIU, and the two equivalent doses of this reference IL-2 standard to be administered during each week would be 15.0 MIU.

In accordance with the methods disclosed herein, the subject is administered this two-level IL-2 dosing regimen in combination with therapeutically effective doses of the antagonist anti-CD40 antibody or antigen-binding fragment thereof, where the therapeutically effective dose of the antibody or antigen-binding fragment thereof is administered once per week, once every two weeks, once every three weeks, or once every four weeks throughout a treatment period or for a fixed duration within a treatment period. The therapeutically effective dose of anti-CD40 antibody or antigen-binding fragment thereof to be administered is in the range from about 0.003 mg/kg to about 50 mg/kg, including about 0.1 mg/kg to about 30 mg/kg. Thus, for example, in some embodiments, the therapeutically effective dose of antagonist anti-CD40 antibody or antigen-binding fragment thereof is about 0.003 mg/kg, 0.01 mg/kg, 0.03 mg/kg, 0.1 mg/kg, 0.3 mg/kg, 0.5 mg/kg, 1 mg/kg, 1.5 mg/kg, 2 mg/kg, 2.5 mg/kg, 3 mg/kg, 5 mg/kg, 7 mg/kg, 10 mg/kg, 15 mg/kg, 20 mg/kg, 25 mg/kg, 30 mg/kg, 35 mg/kg, 40 mg/kg, 45 mg/kg, 50 mg/kg, or other such doses falling within the range of about 0.003 mg/kg to about 50 mg/kg.

In one embodiment, therapeutically effective doses of the antagonist anti-CD40 antibody or antigen-binding fragment thereof as noted above are administered once per week, once every two weeks, once every three weeks, or once every four weeks throughout a treatment period or for a fixed duration within a treatment period, and the two-level IL-2 dosing regimen that is to be administered in combination with this antagonist anti-CD40 antibody dosing regimen has a combined duration of 4 weeks to 8 weeks, where the higher total weekly dose of Proleukin® IL-2 that is administered during the first time period of the two-level IL-2 dosing regimen is about 30.0 MIU to about 54.0 MIU, such as 30.0 MIU, 32.0 MIU, 34.0 MIU, 36.0 MIU, 38.0 MIU. 40.0 MIU, 42.0 MIU, 45.0 MIU, 47.0 MIU, 50.0 MIU, 52.0 MIU, or 54.0 MIU, and the lower total weekly dose of Proleukin® IL-2 that is administered during the second time period of the two-level IL-2 dosing regimen is about 18.0 MIU to about 39.0 MIU, such as 18.0, 20.0, 22.0, 24.0, 26.0, 28.0, 30.0 MIU, 32.0 MIU, 35.0 MIU, 37.0 MIU, or 39.0 MIU. In one such embodiment, the higher total weekly dose of Proleukin® IL-2 that is administered during the first time period of the two-level IL-2 dosing regimen is 42.0 MIU and the lower total weekly dose of Proleukin® IL-2 that is administered during the second time period of the two-level IL-2 dosing regimen is 30.0 MIU.

Thus, for example, in some embodiments, the subject undergoes concurrent therapy with weekly administration of antagonist anti-CD40 antibody or antigen-binding fragment thereof, and a two level-IL-2 dosing regimen having a combined duration of 8 weeks. For this particular embodiment, the IL-2 dosing begins on day 1 of week 2 (i.e., on day 8 following the first dosing with antagonist anti-CD40 antibody or antigen-binding fragment thereof on day 1 of this treatment period). In this manner, the total weekly dose of Proleukin® IL-2 to be administered during weeks 2-5 of the treatment period is in the range of about 30.0 MIU to about 54.0 MIU, such as 30.0 MIU, 32.0 MIU, 35.0 MIU, 37.0 MIU, 40.0 MIU, 42.0 MIU, 45.0 MIU, 47.0 MIU, 50.0 MIU, 52.0 MIU, or 54.0 MIU, or other such values falling within this higher dosing range. In this embodiment, the total weekly dose of Proleukin® IL-2 to be administered during weeks 6-9 of the treatment period is in the range of about 18.0 MIU to about 39.0 MIU, such as 18.0,20.0,22.0,24.0,26.0,28.0,30.0 MIU, 32.0 MIU, 35.0 MIU, 37.0 MIU, or 39.0 MIU, and other such values falling within this range. As previously noted, the total weekly dose to be administered during the first and second periods of the two-level IL-2 dosing regimen are chosen from within these ranges such that a higher total weekly dose is administered during the first period of the two-level IL-2 dosing regimen (for example, weeks 2-5 of a 9-week treatment period), and a lower total weekly dose is administered during the second period of the two-level IL-2 dosing regimen (for example, weeks 6-9 of the same 9-week treatment period). Thus, for example, where the total weekly dose of IL-2 during the first period of the two-level IL-2 dosing regimen is about 42.0 MIU to about 54.0 MIU, the total weekly dose of Proleukin® IL-2 during the second period of IL-2 dosing preferably falls within the range of about 18.0 MIU to about 39.0 MIU.

Thus, where the duration of a treatment period with concurrent therapy with weekly antagonist anti-CD40 antibody dosing and a two-level IL-2 dosing regimen is about 9 weeks, in one embodiment the total weekly dose of Proleukin® IL-2 during weeks 2-5 of the treatment period is about 30.0 MIU to about 54.0 MIU or about 30.0 MIU to about 42.0 MIU, and the total weekly dose of Proleukin® IL-2 during weeks 6-9 of this treatment period is about 18.0 MIU to about 30.0 MIU.

In one embodiment, the total weekly dose of Proleukin® IL-2 during weeks 2-5 is about 42.0 MIU, and the total weekly dose of Proleukin® IL-2 during weeks 6-9 is about 30.0 MIU. In this embodiment, each of the higher and lower total weekly doses of Proleukin® IL-2 are partitioned into two equivalent doses that are administered according to a two-times-a-week dosing schedule, where the two equivalent doses are administered to the subject within a 7-day period, allowing for a minimum of 72 hours between doses and a maximum of 96 hours between doses. In an alternative embodiment, each of the higher and lower total weekly doses of Proleukin® IL-2 are partitioned into three equivalent doses that are administered according to a three-times-a-week dosing schedule, where the three equivalent doses are administered to the subject within a 7-day period, allowing for a minimum of 25 hours between doses and a maximum of 72 hours between doses. As noted above, the therapeutically effective dose of antagonist anti-CD40 antibody or antigen-binding fragment thereof that is to be administered in combination with the two-level IL-2 dosing regimen is about 0.003 mg/kg, 0.01 mg/kg, 0.03 mg/kg, 0.1 mg/kg, 0.3 mg/kg, 0.5 mg/kg, 1 mg/kg, 1.5 mg/kg, 2 mg/kg, 2.5 mg/kg, 3 mg/kg, 5 mg/kg, 7 mg/kg, 10 mg/kg, 15 mg/kg, 20 mg/kg, 25 mg/kg, 30 mg/kg, 35 mg/kg, 40 mg/kg, 45 mg/kg, 50 mg/kg, or other such doses falling within the range of about 0.003 mg/kg to about 50 mg/kg, where this dose of the antagonist anti-CD40 antibody or antigen-binding fragment thereof is administered weekly, either throughout the treatment period or for a fixed duration of 4 weeks to 8 weeks within the treatment period. In an alternative embodiment, this therapeutically effective dose of the antibody or antigen-binding fragment thereof is administered once every two weeks, once every three weeks, or once every four weeks, throughout a treatment period, or is administered once every two weeks for a fixed duration of 4 weeks to 8 weeks within the treatment period, wherein the subject is also administered the two-level IL-2 dosing regimen described above.

*Relative versus absolute doses for IL-2 dosing*. The foregoing total weekly doses of Proleukin® IL-2 are expressed in terms of MIU, which represent total amounts or absolute doses that are to be administered to a human subject on a weekly basis. The corresponding relative total weekly dose of Proleukin® IL-2 to be administered to a person to can readily be calculated. The average person has a body surface area of approximately 1.7 $m^2$. Thus, where the absolute total weekly dose of Proleukin® IL-2 to be administered is about 42.0 MIU to about 54.0 MIU, the corresponding relative total weekly dose of Proleukin® IL-2 is about 24.7 MIU/$m^2$ to about 31.8 MIU/$m^2$. Similarly, when the absolute total weekly dose is about 30.0 MIU to 42.0 MIU, the corresponding relative total weekly dose is about 17.6 MIU/$m^2$ to about 24.7 MIU/$m^2$. When the absolute total weekly dose is about 18.0 MIU to about 30.0 MIU, the corresponding relative total weekly dose is about 10.6 MIU/$m^2$ to about 17.6 MIU/$m^2$.

The international unit (IU) for IL-2 biological activity was established in 1988 by the World Health Organization (WHO) International Laboratory for Biological Standards. The IL-2 biological reference materials provided by the National Institute for Biological Standards and Control (NIBSC), which belongs to WHO, has 100 international units per ampoule of native human, Jurkat-derived IL-2. Activity of an IL-2 product can be measured against this international standard in an *in vitro* potency assay by HT-2 cell proliferation. Thus, for example, Proleukin® IL-2 has a biological activity of about 16.36 MIU per mg of this IL-2 product as determined by an HT-2 cell proliferation assay (see, for example, Gearing and Thorpe (1988) J. Immunological Methods 114:3-9; Nakanishi et al. (1984) J. Exp. Med. 160(6):1605-1621). The active moiety used in this product is the recombinant human IL-2 mutein aldesleukin (referred to as des-alanyl-1, serine-125 human interleukin-2; see U.S. Patent No. 4,931,543 ). Using this information, one can calculate the recommended therapeutically effective absolute dose of Proleukin® IL-2 in micrograms.

Hence, where the absolute total weekly dose of Proleukin® IL-2 is about 18.0 MIU to about 54.0 MIU, the corresponding absolute total weekly dose of Proleukin® IL-2 in micrograms is about 1100 μg to about 3300 μg of this product. Similarly, where the absolute total weekly dose in MIU is about 18.0 MIU to about 30.0 MIU, the corresponding absolute total weekly dose of Proleukin® IL-2 in μg is about 1100 μg to about 1834 μg. Where the absolute total weekly dose of Proleukin® IL-2 in MIU is about 30.0 MIU to about 42.0 MIU, the corresponding absolute total weekly dose in μg is about 1833 μg to about 2567 μg. Thus, given an absolute total weekly dose of Proleukin® IL-2 expressed in MIU, one of skill in the art can readily compute the corresponding absolute total weekly dose expressed in μg of this particular IL-2 product.

*Calculation of IL-2 doses for different aldesleukin-formulations*. For purposes of describing this invention, the doses of IL-2 have been presented using Proleukin® IL-2 as the reference IL-2 standard. One of skill in the art can readily determine what the corresponding doses would be for different aldesleukin formulations and routes of administration using a conversion factor based on comparative pharmacokinetic (PK) data and the serum concentration-time curve (AUC) for PK data collected during a 24-hour period for Proleukin® IL-2. Using PK data, the IL-2 exposure in human subjects that were administered a single dose of the reference IL-2 standard was determined. These subjects were selected such that they had not previously received exogenous IL-2 therapy (i.e., these subjects were naive to IL-2 therapy). By "exogenous IL-2 therapy" is intended any intervention whereby a subject has been exposed to an exogenous source of IL-2, as opposed to exposure that occurs with the body's production of naturally occurring IL-2. Some of these subjects had received a single dose of 4.5 MIU of the reference IL-2 standard, while others had received a single dose of 7.5 or 18.0 MIU of the reference IL-2 standard.

Following administration of the single dose of the reference IL-2 standard, the IL-2 exposure in the blood serum was monitored over the first 10 to 12 hours post-injection, then extrapolated to 24 hours, and the resulting area under the serum concentration-time curve (AUC) for data collected during that 24-hour period was calculated. This area under the serum concentration-time curve is referred to herein as the $AUC_{0-24}$. Methods for measuring IL-2 exposure in this manner are well known in the art. See, for example, Gustavson (1998) J Biol. Response Modifiers 1998:440-449; Thompson et al. (1987) Cancer Research 47:4202-4207; Kirchner et al. (1998) Br. J. Clin. Pharmacol. 46:5-10; Piscitelli et al. (1996) Pharmacotherapy 16(5):754-759; and Example 8 below. Thus, for those subjects receiving a dose of 4.5 MIU (275 μg) of Proleukin® IL-2, the $AUC_{0-24}$ value was 51 IU∗hr/ml (SD = 14); for those subjects receiving a dose of 7.5 MIU (458 μg) of Proleukin® IL-2, the $AUC_{0-24}$ value was 79 IU∗hr/ml (SD = 29); and for those subjects receiving the 18.0 MIU (1100 μg) dose of Proleukin® IL-2, the $AUC_{0-24}$ value was 344 IU∗hr/ml (SD = 127). When such $AUC_{0-24}$ data is determined for the reference IL-2 standard, Proleukin® IL-2, the therapeutically effective doses described herein result in an IL-2 exposure within a range from about 23 IU∗hour/ml serum to about 598 IU∗hour/ml serum (see Example 8 below).

The sum of individual $AUC_{0-24}$ from individual doses will comprise the total weekly $AUC_{0-24}$ in partitioned individual doses. For example, if a dose of 18 MIU is administered three-times-a-week, the individual $AUC_{0-24}$ is estimated at 344 IU∗hr/ml, and the total weekly $AUC_{0-24}$ will be 1032 IU∗hr/ml based on linear assumption of increased $AUC_{0-24}$ with dose as shown in the Table 1 below.

Table 1: $AUC_{0-24}$ values obtained after administration of Proleukin® IL-2.

| Proleukin® IL-2 Dose (MIU/μg) | $AUC_{0-24}$ (IU∗hr/ml) |
|---|---|
| | |
| 18/1100 | 344 |
| 30/1834 | 574 |
| 42/2567 | 803 |
| 54/3300 | 1032 |

The same total weekly $AUC_{0-24}$ of 1032 IU∗hr/ml could also be obtained by dosing two-times-a-week at 27 MIU or dosing five-times-a-week at about 11 MIU.

For any other aldesleukin formulation, a comparable recommended dose for use in the methods disclosed herein can be determined based on this $AUC_{0-24}$ data for Proleukin® IL-2. In this manner, a single dose of the aldesleukin formulation of interest is administered to a human subject, and the level of IL-2 in the serum following this initial IL-2 exposure is determined by collecting PK data and generating an $AUC_{0-24}$ for the aldesleukin formulation of interest. By "initial IL-2 exposure" is intended the subject used to measure IL-2 exposure has not previously undergone therapy with an exogenous source of IL-2 as noted above. This $AUC_{0-24}$ is then compared to the $AUC_{0-24}$ for Proleukin® IL-2 to determine a conversion factor that can be used to calculate a dose of the aldesleukin formulation of interest that is comparable to the recommended dose for Proleukin® IL-2. See, for example, the calculations for a representative monomeric IL-2 formulation, L2-7001, that are shown in Example 8 below. Thus, for any other aldesleukin formulation used in the methods disclosed herein, the total weekly dose of IL-2 to be administered during a constant IL-2 dosing regimen, or during a two-level IL-2 dosing regimen, is in an amount equivalent to the recommended total weekly dose of the reference IL-2 standard, i.e., Proleukin® IL-2, as determined by the area under the serum concentration-time curve from human PK data.

*Calculation of IL-2 doses for different IL-2 molecules*. Biologically active variants of IL-2 may have altered intrinsic biological activity. To estimate the dose of an IL-2 variant other than aldesleukin or the dose of native-sequence IL-2 that will be comparable to the doses of aldesleukin disclosed herein, the relative biological activity of the IL-2 variant or native-sequence IL-2 should be determined by testing its biological effect.

The relevant biological activity of an IL-2 or variant thereof according to the present invention is the ability to activate and/or achieve expansion of human NK cells to mediate lymphokine activated killer (LAK) activity and antibody-dependent cellular cytotoxicity (ADCC). Such activity is measured using a standard assay such as that disclosed in Nagler et al. (1989) J. Immunol. 143:3183-3191. To determine suitable doses of IL-2 variants that are not aldesleukin or suitable doses of native-sequence IL-2, a series of doses of the IL-2 molecule of interest (i.e., an IL-2 variant other than aldesleukin or native-sequence IL-2) are administered to human subjects by the same route of administration and freshly isolated PBMC (peripheral blood mononuclear cells) from the subjects are tested for NK cytoxicity against K562 cells as described, for example, by Nagler *et al.* (1989) *supra*. The NK stimulatory effect of the IL-2 molecule being tested is compared with that of the reference IL-2 standard (i.e., aldesleukin as formulated in Proleukin®) when administered in the same μg amounts by the same route of administration using the same procedure described for the IL-2 molecule being tested. Alternatively, suitable doses could be determined *in vitro,* using untreated human PBMC, subjected to a series of doses of the IL-2 molecule of interest. The IL-2 molecule of interest (i.e., an IL-2 variant other than aldesleukin or native-sequence IL-2) will have an NK cell stimulatory potency (i.e., activity) that is preferably at least 100% that of the same amount of the reference IL-2 standard (i.e., aldesleukin as formulated in Proleukin®), or 95%, 90%, 80%, 70%, or 60% that of the same amount of the reference IL-2 standard. The IL-2 variant should be substantially biologically active, as defined as having at least 50% of the NK stimulatory activity of the same dose of the reference IL-2 standard, administered by the same route.

Thus, where a subject is to receive combination therapy with antagonist anti-CD40 antibody and a constant IL-2 dosing regimen, the total weekly dose of any IL-2 or biologically active variant thereof is an amount equivalent to a total weekly dose of the reference IL-2 standard (i.e., aldesleukin as formulated in Proleukin®) in the range of about 1100 μg (i.e., 18.0 MIU) to about 3300 μg (i.e., about 54.0 MIU, preferably in the range of about 1100 μg to about 2567 μg (i.e., about 42.0 MIU), wherein the total weekly dose of the IL-2 or biologically active variant thereof is an amount that provides at least 50% of the NK stimulatory activity of the total weekly dose of the reference IL-2 standard (i.e., aldesleukin as formulated in Proleukin®).

In some embodiments, the total weekly dose of the IL-2 or biologically active variant thereof to be administered in the constant IL-2 dosing regimen is an amount that provides at least 60% of the NK stimulatory activity of a total weekly dose of the reference IL-2 standard administered in the range of about 1100 μg to about 3300 μg, for example, at least 60% of the NK stimulatory activity of a total weekly dose of the reference IL-2 standard administered at about 1100 μg (18.0 MIU), 1222 μg (20.0 MIU), 1345 μg (22.0 MIU), 1467 μg (24.0 MIU), 1589 μg (26.0 MIU), 1711 μg (28.0 MIU), 1834 μg (30.0 MIU), 1956 μg (32.0 MIU), 2078 μg (34.0 MIU), 2200 μg (36.0 MIU), 2323 μg (38.0 MIU), 2445 μg (40.0 MIU), 2567 μg (42.0 MIU), 2689 μg (44.0 MIU), 2812 μg (46.0 MIU), 2934 μg (48.0 MIU), 3056 μg (50.0 MIU), 3178 μg (52.0 MIU), or 3300 μg (54.0 MIU). In other embodiments, the total weekly dose of the IL-2 or biologically active variant thereof to be administered in the constant IL-2 dosing regimen is an amount that provides at least 70% of the NK stimulatory activity of a total weekly dose of the reference IL-2 standard administered in the range of about 1100 μg to about 3300 μg, for example, at least 70% of the NK stimulatory activity of a total weekly dose of the reference IL-2 standard administered at about 1100 μg (18.0 MIU), 1222 μg (20.0 MIU), 1345 μg (22.0 MIU), 1467 μg (24.0 MIU), 1589 μg (26.0 MIU), 1711 μg (28.0 MIU), 1834 μg (30.0 MIU), 1956 μg (32.0 MIU), 2078 μg (34.0 MIU), 2200 μg (36.0 MIU), 2323 μg (38.0 MIU), 2445 μg (40.0 MIU), 2567 μg (42.0 MIU), 2689 μg (44.0 MIU), 2812 μg (46.0 MIU), 2934 μg (48.0 MIU), 3056 μg (50.0 MIU), 3178 μg (52.0 MIU), or 3300 μg (54.0 MIU).

In some embodiments, the total weekly dose of the IL-2 or biologically active variant thereof to be administered in the constant IL-2 dosing regimen is an amount that provides at least 80% of the NK stimulatory activity of a total weekly dose of the reference IL-2 standard administered in the range of about 1100 μg to about 3300 μg, for example, at least 80% of the NK stimulatory activity of a total weekly dose of the reference IL-2 standard administered at about 1100 μg (18.0 MIU), 1222 μg (20.0 MID), 1345 μg (22.0 MIU), 1467 μg (24.0 MIU), 1589 μg (26.0 MIU), 1711 μg (28.0 MIU), 1834 μg (30.0 MIU), 1956 μg (32.0 MIU), 2078 μg (34.0 MIU), 2200 μg (36.0 MIU), 2323 μg (38.0 MIU), 2445 μg (40.0 MIU), 2567 μg (42.0 MIU), 2689 μg (44.0 MIU), 2812 μg (46.0 MIU), 2934 μg (48.0 MIU), 3056 μg (50.0 MIU), 3178 μg (52.0 MIU), or 3300 μg (54.0 MIU). In alternative embodiments, the total weekly dose of the IL-2 or biologically active variant thereof to be administered in the constant IL-2 dosing regimen is an amount that provides at least 90% of the NK stimulatory activity of a total weekly dose of the reference IL-2 standard administered in the range of about 1100 μg to about 3300 μg, for example, at least 90% of the NK stimulatory activity of a total weekly dose of the reference IL-2 standard administered at about 1100 μg (18.0 MIU), 1222 μg (20.0 MIU), 1345 μg (22.0 MIU), 1467 μg (24.0 MIU), 1589 μg (26.0 MIU), 1711 μg (28.0 MIU), 1834 μg (30.0 MIU), 1956 μg (32.0 MIU), 2078 μg (34.0 MIU), 2200 μg (36.0 MIU), 2323 μg (38.0 MIU), 2445 μg (40.0 MIU), 2567 μg (42.0 MIU), 2689 μg (44.0 MIU), 2812 μg (46.0 MIU), 2934 μg (48.0 MIU), 3056 μg (50.0 MIU), 3178 μg (52.0 MIU), or 3300 μg (54.0 MIU).

In yet other embodiments, the total weekly dose of the IL-2 or biologically active variant thereof to be administered in the constant IL-2 dosing regimen is an amount that provides at least 95% of the NK stimulatory activity of a total weekly dose of the reference IL-2 standard administered in the range of about 1100 μg to about 3300 μg, for example, at least 95% of the NK stimulatory activity of a total weekly dose of the reference IL-2 standard administered at about 1100 μg (18.0 MIU), 1222 μg (20.0 MIU), 1345 μg (22.0 MIU), 1467 μg (24.0 MIU), 1589 μg (26.0 MIU), 1711 μg (28.0 MIU), 1834 μg (30.0 MIU), 1956 μg (32.0 MIU), 2078 μg (34.0 MIU), 2200 μg (36.0 MIU), 2323 μg (38.0 MIU), 2445 μg (40.0 MIU), 2567 μg (42.0 MIU), 2689 μg (44.0 MIU), 2812 μg (46.0 MIU), 2934 μg (48.0 MIU), 3056 μg (50.0 MIU), 3178 μg (52.0 MIU), or 3300 μg (54.0 MIU).

In some embodiments, the total weekly dose of the IL-2 or biologically active variant thereof to be administered in the constant IL-2 dosing regimen is an amount that provides 100% of the NK stimulatory activity of a total weekly dose of the reference IL-2 standard administered in the range of about 1100 μg to about 3300 μg, for example, 100% of the NK stimulatory activity of a total weekly dose of the reference IL-2 standard administered at about 1100 μg (18.0 MIU), 1222 μg (20.0 MIU), 1345 μg (22.0 MIU), 1467 μg (24.0 MIU), 1589 μg (26.0 MIU), 1711 μg (28.0 MIU), 1834 μg (30.0 MIU), 1956 μg (32.0 MIU), 2078 μg (34.0 MIU), 2200 μg (36.0 MIU), 2323 μg (38.0 MIU), 2445 μg (40.0 MIU), 2567 µg (42.0 MIU), 2689 µg (44.0 MIU), 2812 µg (46.0 MIU), 2934 µg (48.0 MIU), 3056 µg (50.0 MIU), 3178 µg (52.0 MIU), or 3300 µg (54.0 MIU).

In a similar manner, where a subject is to receive combination therapy with antagonist anti-CD40 antibody and a two-level IL-2 dosing regimen, the total weekly dose of any IL-2 or biologically active variant thereof to be administered during the first period or during the second period of the two-level IL-2 dosing regimen can be expressed as an amount equivalent to the total weekly dose of the IL-2 reference standard that is administered during the two-level IL-2 dosing regimen. Thus, the total weekly dose of any IL-2 or biologically active variant thereof to be administered during the first period of the two-level IL-2 dosing regimen is an amount equivalent to a total weekly dose of the reference IL-2 standard (i.e., aldesleukin as formulated in Proleukin®) in the range of about 1834 µg (i.e., 30.0 MIU) to about 3300 µg (i.e., about 54.0 MIU), wherein the total weekly dose of the IL-2 or biologically active variant thereof is an amount that provides at least 50% of the NK stimulatory activity of the total weekly dose of the reference IL-2 standard (i.e., aldesleukin as formulated in Proleukin®). Similarly, the total weekly dose of any IL-2 or biologically active variant thereof to be administered during the second period of the two-level IL-2 dosing regimen is an amount equivalent to a total weekly dose of the reference IL-2 standard (i.e., aldesleukin as formulated in Proleukin®) in the range of about 1100 µg (i.e., 18.0 MIU) to about 2384 µg (i.e., about 39.0 MIU), wherein the total weekly dose of the IL-2 or biologically active variant thereof is an amount that provides at least 50% of the NK stimulatory activity of the total weekly dose of the reference IL-2 standard (i.e., aldesleukin as formulated in Proleukin®). As previously noted, the total weekly dose administered during the first time period of the two-level IL-2 dosing regimen, for example, during the first half of this dosing regimen, is always higher than the total weekly dose administered during the second time period of the two-level IL-2 dosing regimen, for example, during the second half of this dosing regimen.

Thus, in some embodiments, the total weekly dose of the IL-2 or biologically active variant thereof to be administered in the first period of the two-level IL-2 dosing regimen is an amount that provides at least 60% of the NK stimulatory activity of a total weekly dose of the reference IL-2 standard administered in the range of about 1834 µg (i.e., 30.0 MIU) to about 3300 µg (i.e., about 54.0 MIU), for example, at least 60% of the NK stimulatory activity of a total weekly dose of the reference IL-2 standard administered at about 1834 µg (30.0 MIU), 1956 µg (32.0 MIU), 2078 µg (34.0 MIU), 2200 µg (36.0 MIU), 2323 µg (38.0 MIU), 2445 µg (40.0 MIU), 2567 µg (42.0 MIU), 2689 µg (44.0 MIU), 2812 µg (46.0 MIU), 2934 µg (48.0 MIU), 3056 µg (50.0 MIU), 3178 µg (52.0 MIU), or 3300 µg (54.0 MIU). For these embodiments, the total weekly dose of the IL-2 or biologically active variant thereof to be administered in the second period of the two-level IL-2 dosing regimen is an amount that provides at least 60% of the NK stimulatory activity of a total weekly dose of the reference IL-2 standard administered in the range of about 1100 µg (i.e., 18.0 MIU) to about 2384 µg (i.e., about 39.0 MIU), for example, at least 60% of the NK stimulatory activity of a total weekly dose of the reference IL-2 standard administered at about 1100 µg (18.0 MIU), 1222 µg (20.0 MIU), 1406 µg (23.0 MIU), 1528 µg (25.0 MIU), 1650 µg (27.0 MIU), 1834 µg (30.0 MIU), 1956 µg (32 MIU), 2139 µg (35.0 MIU), 2262 µg (37.0 MIU), or 2384 µg (39.0 MIU). Alternatively, the total weekly dose of the IL-2 or biologically active variant thereof to be administered in the second period of the two-level IL-2 dosing regimen is an amount that provides at least 70%, at least 80%, at least 90%, at least 95%, or even 100% of the NK stimulatory activity of a total weekly dose of the reference IL-2 standard administered in the range of about 1100 µg (i.e., 18.0 MIU) to about 2384 µg (i.e., about 39.0 MIU), for example, at least 70%, at least 80%, at least 90%, at least 95%, or even 100% of the NK stimulatory activity of a total weekly dose of the reference IL-2 standard administered at about 1100 µg (18.0 MIU), 1222 µg (20.0 MIU), 1406 µg (23.0 MIU), 1528 µg (25.0 MIU), 1650 µg (27.0 MIU), 1834 µg (30.0 MIU), 1956 µg (32 MIU), 2139 µg (35.0 MIU), 2262 µg (37.0 MIU), or 2384 µg (39.0 MIU).

In other embodiments, the total weekly dose of the IL-2 or biologically active variant thereof to be administered in the first period of the two-level IL-2 dosing regimen is an amount that provides at least 70% of the NK stimulatory activity of a total weekly dose of the reference IL-2 standard administered in the range of about 1834 µg (i.e., 30.0 MIU) to about 3300 µg (i.e., about 54.0 MIU), for example, at least 70% of the NK stimulatory activity of a total weekly dose of the reference IL-2 standard administered at about 1834 µg (30.0 MIU), 1956 µg (32.0 MIU), 2078 µg (34.0 MIU), 2200 µg (36.0 MIU), 2323 µg (38.0 MIU), 2445 µg (40.0 MIU), 2567 µg (42.0 MIU), 2689 µg (44.0 MIU), 2812 µg (46.0 MIU), 2934 µg (48.0 MIU), 3056 µg (50.0 MIU), 3178 µg (52.0 MIU), or 3300 µg (54.0 MIU). For these embodiments, the total weekly dose of the IL-2 or biologically active variant thereof to be administered in the second period of the two-level IL-2 dosing regimen is an amount that provides at least 60% of the NK stimulatory activity of a total weekly dose of the reference IL-2 standard administered in the range of about 1100 µg (i.e., 18.0 MIU) to about 2384 µg (i.e., about 39.0 MIU), for example, at least 60% of the NK stimulatory activity of a total weekly dose of the reference IL-2 standard administered at about 1100 µg (18.0 MIU), 1222 µg (20.0 MIU), 1406 µg (23.0 MIU), 1528 µg (25.0 MIU), 1650 µg (27.0 MIU), 1834 µg (30.0 MIU), 1956 µg (32 MIU), 2139 µg (35.0 MIU), 2262 µg (37.0 MIU), or 2384 µg (39.0 MIU). Alternatively, the total weekly dose of the IL-2 or biologically active variant thereof to be administered in the second period of the two-level IL-2 dosing regimen is an amount that provides at least 70%, at least 80%, at least 90%, at least 95%, or even 100% of the NK stimulatory activity of a total weekly dose of the reference IL-2 standard administered in the range of about 1100 µg (i.e., 18.0 MIU) to about 2384 µg (i.e., about 39.0 MIU), for example, at least 70%, at least 80%, at least 90%, at least 95%, or even 100% of the NK stimulatory activity of a total weekly dose of the reference IL-2 standard administered at about 1100 μg (18.0 MIU), 1222 μg (20.0 MIU), 1406 μg (23.0 MIU), 1528 μg (25.0 MIU), 1650 μg (27.0 MIU), 1834 μg (30.0 MIU), 1956 μg (32 MIU), 2139 μg (35.0 MIU), 2262 μg (37.0 MIU), or 2384 μg (39.0 MIU).

In yet other embodiments, the total weekly dose of the IL-2 or biologically active variant thereof to be administered in the first period of the two-level IL-2 dosing regimen is an amount that provides at least 80% of the NK stimulatory activity of a total weekly dose of the reference IL-2 standard administered in the range of about 1834 μg (i.e., 30.0 MIU) to about 3300 μg (i.e., about 54.0 MIU), for example, at least 80% of the NK stimulatory activity of a total weekly dose of the reference IL-2 standard administered at about 1834 μg (30.0 MIU), 1956 μg (32.0 MIU), 2078 μg (34.0 MIU), 2200 μg (36.0 MIU), 2323 μg (38.0 MIU), 2445 μg (40.0 MIU), 2567 μg (42.0 MIU), 2689 μg (44.0 MIU), 2812 μg (46.0 MIU), 2934 μg (48.0 MIU), 3056 μg (50.0 MIU), 3178 μg (52.0 MIU), or 3300 μg (54.0 MIU). For these embodiments, the total weekly dose of the IL-2 or biologically active variant thereof to be administered in the second period of the two-level IL-2 dosing regimen is an amount that provides at least 60% of the NK stimulatory activity of a total weekly dose of the reference IL-2 standard administered in the range of about 1100 μg (i.e., 18.0 MIU) to about 2384 μg (i.e., about 39.0 MIU), for example, at least 60% of the NK stimulatory activity of a total weekly dose of the reference IL-2 standard administered at about 1100 μg (18.0 MIU), 1222 μg (20.0 MIU), 1406 μg (23.0 MIU), 1528 μg (25.0 MIU), 1650 μg (27.0 MIU), 1834 μg (30.0 MIU), 1956 μg (32 MIU), 2139 μg (35.0 MIU), 2262 μg (37.0 MIU), or 2384 μg (39.0 MIU). Alternatively, the total weekly dose of the IL-2 or biologically active variant thereof to be administered in the second period of the two-level IL-2 dosing regimen is an amount that provides at least 70%, at least 80%, at least 90%, at least 95%, or even 100% of the NK stimulatory activity of a total weekly dose of the reference IL-2 standard administered in the range of about 1100 μg (i.e., 18.0 MIU) to about 2384 μg (i.e., about 39.0 MIU), for example, at least 70%, at least 80%, at least 90%, at least 95%, or even 100% of the NK stimulatory activity of a total weekly dose of the reference IL-2 standard administered at about 1100 μg (18.0 MIU), 1222 μg (20.0 MIU), 1406 μg (23.0 MIU), 1528 μg (25.0 MIU), 1650 μg (27.0 MIU), 1834 μg (30.0 MIU), 1956 μg (32 MIU), 2139 μg (35.0 MIU), 2262 μg (37.0 MIU), or 2384 μg (39.0 MIU).

In alternative embodiments, the total weekly dose of the IL-2 or biologically active variant thereof to be administered in the first period of the two-level IL-2 dosing regimen is an amount that provides at least 90% of the NK stimulatory activity of a total weekly dose of the reference IL-2 standard administered in the range of about 1834 μg (i.e., 30.0 MIU) to about 3300 μg (i.e., about 54.0 MIU), for example, at least 90% of the NK stimulatory activity of a total weekly dose of the reference IL-2 standard administered at about 1834 μg (30.0 MIU), 1956 μg (32.0 MIU), 2078 μg (34.0 MIU), 2200 μg (36.0 MIU), 2323 μg (38.0 MIU), 2445 μg (40.0 MIU), 2567 μg (42.0 MIU), 2689 μg (44.0 MIU), 2812 μg (46.0 MIU), 2934 μg (48.0 MIU), 3056 μg (50.0 Mru), 3178 μg (52.0 MIU), or 3300 μg (54.0 MIU). For these embodiments, the total weekly dose of the IL-2 or biologically active variant thereof to be administered in the second period of the two-level IL-2 dosing regimen is an amount that provides at least 60% of the NK stimulatory activity of a total weekly dose of the reference IL-2 standard administered in the range of about 1100 μg (i.e., 18.0 MIU) to about 2384 μg (i.e., about 39.0 MIU), for example, at least 60% of the NK stimulatory activity of a total weekly dose of the reference IL-2 standard administered at about 1100 μg (18.0 MIU), 1222 μg (20.0 MIU), 1406 μg (23.0 MIU), 1528 μg (25.0 MIU), 1650 μg (27.0 MIU), 1834 μg (30.0 MIU), 1956 μg (32 MIU), 2139 μg (35.0 MIU), 2262 μg (37.0 MIU), or 2384 μg (39.0 MIU). Alternatively, the total weekly dose of the IL-2 or biologically active variant thereof to be administered in the second period of the two-level IL-2 dosing regimen is an amount that provides at least 70%, at least 80%, at least 90%, at least 95%, or even 100% of the NK stimulatory activity of a total weekly dose of the reference IL-2 standard administered in the range of about 1100 μg (i.e., 18.0 MIU) to about 2384 μg (i.e., about 39.0 MIU), for example, at least 70%, at least 80%, at least 90%, at least 95%, or even 100% of the NK stimulatory activity of a total weekly dose of the reference IL-2 standard administered at about 1100 μg (18.0 MIU), 1222 μg (20.0 MIU), 1406 μg (23.0 MIU), 1528 μg (25.0 MIU), 1650 μg (27.0 MIU), 1834 μg (30.0 MIU), 1956 μg (32 MIU), 2139 μg (35.0 MIU), 2262 μg (37.0 MIU), or 2384 μg (39.0 MIU).

In still other embodiments, the total weekly dose of the IL-2 or biologically active variant thereof to be administered in the first period of the two-level IL-2 dosing regimen is an amount that provides at least 95% of the NK stimulatory activity of a total weekly dose of the reference IL-2 standard administered in the range of about 1834 μg (i.e., 30.0 MIU) to about 3300 μg (i.e., about 54.0 MIU), for example, at least 95% of the NK stimulatory activity of a total weekly dose of the reference IL-2 standard administered at about 1834 μg (30.0 MIU), 1956 μg (32.0 MIU), 2078 μg (34.0 MIU), 2200 μg (36.0 MIU), 2323 μg (38.0 MIU), 2445 μg (40.0 MIU), 2567 μg (42.0 MIU), 2689 μg (44.0 MIU), 2812 μg (46.0 MIU), 2934 μg (48.0 MIU), 3056 μg (50.0 MIU), 3178 μg (52.0 MIU), or 3300 μg (54.0 MIU). For these embodiments, the total weekly dose of the IL-2 or biologically active variant thereof to be administered in the second period of the two-level IL-2 dosing regimen is an amount that provides at least 60% of the NK stimulatory activity of a total weekly dose of the reference IL-2 standard administered in the range of about 1100 μg (i.e., 18.0 MIU) to about 2384 μg (i.e., about 39.0 MIU), for example, at least 60% of the NK stimulatory activity of a total weekly dose of the reference IL-2 standard administered at about 1100 μg (18.0 MIU), 1222 μg (20.0 MIU), 1406 μg (23.0 MIU), 1528 μg (25.0 MIU), 1650 μg

(27.0 MIU), 1834 μg (30.0 MIU), 1956 μg (32 MIU), 2139 μg (35.0 MIU), 2262 μg (37.0 MIU), or 2384 μg (39.0 MIU). Alternatively, the total weekly dose of the IL-2 or biologically active variant thereof to be administered in the second period of the two-level IL-2 dosing regimen is an amount that provides at least 70%, at least 80%, at least 90%, at least 95%, or even 100% of the NK stimulatory activity of a total weekly dose of the reference IL-2 standard administered in the range of about 1100 μg (i.e., 18.0 MIU) to about 2384 μg (i.e., about 39.0 MIU), for example, at least 70%, at least 80%, at least 90%, at least 95%, or even 100% of the NK stimulatory activity of a total weekly dose of the reference IL-2 standard administered at about 1100 μg (18.0 MIU), 1222 μg (20.0 MIU), 1406 μg (23.0 MIU), 1528 μg (25.0 MIU), 1650 μg (27.0 MIU), 1834 μg (30.0 MIU), 1956 μg (32 MIU), 2139 μg (35.0 MIU), 2262 μg (37.0 MIU), or 2384 μg (39.0 MIU).

In some embodiments, the total weekly dose of the IL-2 or biologically active variant thereof to be administered in the first period of the two-level IL-2 dosing regimen is an amount that provides 100% of the NK stimulatory activity of a total weekly dose of the reference IL-2 standard administered in the range of about 1834 μg (i.e., 30.0 MIU) to about 3300 μg (i.e., about 54.0 MIU), for example, 100% of the NK stimulatory activity of a total weekly dose of the reference IL-2 standard administered at about 1834 μg (30.0 MIU), 1956 μg (32.0 MIU), 2078 μg (34.0 MIU), 2200 μg (36.0 MIU), 2323 μg (38.0 MIU), 2445 μg (40.0 MIU), 2567 μg (42.0 MIU), 2689 μg (44.0 MIU), 2812 μg (46.0 MIU), 2934 μg (48.0 MIU), 3056 μg (50.0 MIU), 3178 μg (52.0 MIU), or 3300 μg (54.0 MIU). For these embodiments, the total weekly dose of the IL-2 or biologically active variant thereof to be administered in the second period of the two-level IL-2 dosing regimen is an amount that provides at least 60% of the NK stimulatory activity of a total weekly dose of the reference IL-2 standard administered in the range of about 1100 μg (i.e., 18.0 MIU) to about 2384 μg (i.e., about 39.0 MIU), for example, at least 60% of the NK stimulatory activity of a total weekly dose of the reference IL-2 standard administered at about 1100 μg (18.0 MIU), 1222 μg (20.0 MIU), 1406 μg (23.0 MIU), 1528 μg (25.0 MIU), 1650 μg (27.0 MIU), 1834 μg (30.0 MIU), 1956 μg (32 MIU), 2139 μg (35.0 MIU), 2262 μg (37.0 MIU), or 2384 μg (39.0 MIU). Alternatively, the total weekly dose of the IL-2 or biologically active variant thereof to be administered in the second period of the two-level IL-2 dosing regimen is an amount that provides at least 70%, at least 80%, at least 90%, at least 95%, or even 100% of the NK stimulatory activity of a total weekly dose of the reference IL-2 standard administered in the range of about 1100 μg (i.e., 18.0 MIU) to about 2384 μg (i.e., about 39.0 MIU), for example, at least 70%, at least 80%, at least 90%, at least 95%, or even 100% of the NK stimulatory activity of a total weekly dose of the reference IL-2 standard administered at about 1100 μg (18.0 MIU), 1222 μg (20.0 MIU), 1406 μg (23.0 MIU), 1528 μg (25.0 MIU), 1650 μg (27.0 MIU), 1834 μg (30.0 MIU), 1956 μg (32 MIU), 2139 μg (35.0 MIU), 2262 μg (37.0 MIU), or 2384 μg (39.0 MIU).

<u>Additional Oncotherapy for Use with Combination IL-2/Anti-CD40 Antibody Therapy</u>

Those skilled in the art recognize that the methods of combination therapy disclosed herein may be used before, after, or concurrently with other forms of oncotherapy. Such oncotherapy can include chemotherapy regimens such as treatment with CVP (cyclophosphamide, vincristine and prednisone), CHOP (cyclophosphamide, doxorubicin, vincristine and prednisone), ICE (ifosfamide, carboplatin, and etoposide), Mitozantrone, Cytarabine, DVP (daunorubicin, prednisone, and vincristine), ATRA (all-trans-retinoic acid), Idarubicin, hoelzer chemotherapy regime, La La chemotherapy regime, ABVD (adriamycin, bleomycin, vinblastine, and dacarbazine), CEOP (cyclophosphamide, epirubicin, vincristine, and prednisone), CEOP-BE (cyclophosphamide, epirubicin, vincristine, prednisone, bleomycin, and etoposide), 2-CdA (2-chlorodeoxyadenosine (2-CDA), FLAG & IDA (fludarabine, cytarabine, and idarubicin; with or without subsequent G-CSF treatment), VAD (vincristine, doxorubicin, and dexamethasone), M & P (melphalan and prednisone), C-Weekly (cyclophosphamide and prednisone), ABCM (adriamycin (doxorubicin), BCNU, cyclophosphamide, and melphalan), MOPP (nitrogen mustard, oncovin, procarbazine, and prednisone), DHAP (dexamethasone, high-dose ara-C, and platinol), fludarabine and cyclophosphamide. Alternatively, such oncotherapies can include surgery or surgical procedures, radiation treatment, including myleoablative therapies, or other anti-cancer monoclonal antibody therapy. Thus, the methods disclosed herein find use as a concurrent treatment to kill residual tumor cells, either *in vivo* or *ex vivo* after such oncotherapies.

In this manner, combination therapy with IL-2 (or biologically active variant thereof) and the antagonist anti-CD40 antibodies described herein, or antigen-binding fragments thereof, can be used in combination with at least one other cancer therapy, including, but not limited to, surgery or surgical procedures (e.g. splenectomy, hepatectomy, lymphadenectomy, leukophoresis, bone marrow transplantation, and the like); radiation therapy; chemotherapy, optionally in combination with autologous bone marrow transplant, where suitable chemotherapeutic agents include, but are not limited to, fludarabine or fludarabine phosphate, chlorambucil, vincristine, pentostatin, 2-chlorodeoxyadenosine (cladribine), cyclophosphamide, doxorubicin, prednisone, and combinations thereof, for example, anthracycline-containing regimens such as CAP (cyclophosphamide, doxorubicin plus prednisone), CHOP (cyclophosphamide, vincristine, prednisone plus doxorubicin), VAD (vincritsine, doxorubicin, plus dexamethasone), MP (melphalan plus prednisone), and other cytotoxic and/or therapeutic agents used in chemotherapy such as mitoxantrone, daunorubicin, idarubicin, asparaginase, and antimetabolites, including, but not limited to, cytarabine, methotrexate, 5-fluorouracil decarbazine, 6-thioguanine, 6-mercaptopurine, and nelarabine; other anti-cancer monoclonal antibody therapy (for example, alemtuzumab (Campath®) or other anti-CD52 antibody targeting the CD52 cell-surface glycoprotein on malignant B cells; rituximab (Rituxan®), the fully human antibody HuMax-CD20, R-1594, IMMU-106, TRU-015, AME-133, tositumomab/I-131 tositumomab (Bexxar®), ibritumomab tiuxetan (Zevalin®), or any other therapeutic anti-CD20 antibody targeting the CD20 antigen on malignant B cells; anti-CD19 antibody (for example, MT103, a bispecific antibody); anti-CD22 antibody (for example, the humanized monoclonal antibody epratuzumab); bevacizumab (Avastin®) or other anti-cancer antibody targeting human vascular endothelial growth factor; anti-CD22 antibody targeting the CD22 antigen on malignant B cells (for example, the monoclonal antibody BL-22, an alphaCD22 toxin); α-M-CSF antibody targeting macrophage colony stimulating factor; antibodies targeting the receptor activator of nuclear factor-kappaB (RANK) and its ligand (RANKL), which are overexpressed in multiple myeloma; anti-CD23 antibody targeting the CD23 antigen on malignant B cells (for example, IDEC-152); anti-CD80 antibody targeting the CD80 antigen (for example, IDEC-114); anti-CD38 antibody targeting the CD38 antigen on malignant B cells; antibodies targeting major histocompatibility complex class II receptors (anti-MHC antibodies) expressed on malignant B cells; other anti-CD40 antibodies (for example, SGN-40) targeting the CD40 antigen on malignant B cells; and antibodies targeting tumor necrosis factor-related apoptosis-inducing ligand receptor 1 (TRAIL-R1) (for example, the agonistic human monoclonal antibody HGS-ETR1) and TRAIL-R2 expressed on a number of solid tumors and tumors of hematopoietic origin); small molecule-based cancer therapy, including, but not limited to, microtubule and/or topoisomerase inhibitors (for example, the mitotic inhibitor dolastatin and dolastatin analogues; the tubulin-binding agent T900607; XL119; and the topoisomerase inhibitor aminocamptothecin), SDX-105 (bendamustine hydrochloride), ixabepilone (an epothilone analog, also referred to as BMS-247550), protein kinase C inhibitors, for example, midostaurin ((PKC-412, CGP 41251, N-benzoylstaurosporine), pixantrone, eloxatin (an antineoplastic agent), ganite (gallium nitrate), Thalomid® (thalidomide), immunomodulatory derivatives of thalidomide (for example, revlimid (formerly revimid)), Affinitak™ (antisense inhibitor of protein kinase C-alpha), SDX-101 (R-etodolac, inducing apoptosis of malignant lymphocytes), second-generation purine nucleoside analogs such as clofarabine, inhibitors of production of the protein Bcl-2 by cancer cells (for example, the antisense agents oblimersen and Genasense®), proteasome inhibitors (for example, Velcade™ (bortezomib)), small molecule kinase inhibitors (for example, CHIR-258), small molecule VEGF inhibitors (for example, ZD-6474), small molecule inhibitors of heat shock protein (HSP) 90 (for example, 17-AAG), small molecule inhibitors of histone deacetylases (for example, hybrid/polar cytodifferentiation HPC) agents such as suberanilohydroxamic acid (SAHA), and FR-901228) and apoptotic agents such as Trisenox® (arsenic trioxide) and Xcytrin® (motexafin gadolinium); vaccine /immunotherapy-based cancer therapies, including, but not limited to, vaccine approaches (for example, Id-KLH, oncophage, vitalethine), personalized immunotherapy or active idiotype immunotherapy (for example, MyVax® Personalized Immunotherapy, formally designated GTOP-99), Promune® (CpG 7909, a synthetic agonist for toll-like receptor 9 (TLR9)), interferon-alpha therapy, and IL-12 therapy; steroid therapy; or other cancer therapy; where the additional cancer therapy is administered prior to, during, or subsequent to the IL-2/antagonist anti-CD40 antibody combination therapy. Thus, where the combined therapies comprise IL-2/antagonist anti-CD40 antibody combination therapy in combination with administration of another therapeutic agent, as with chemotherapy, radiation therapy, other anti-cancer antibody therapy, small molecule-based cancer therapy, or vaccine/immunotherapy-based cancer therapy, the methods disclosed herein encompass coadministration, using separate formulations or a single pharmaceutical formulation, or and consecutive administration in either order. Where the methods disclosed herein comprise combined therapeutic regimens, these therapies can be given simultaneously, i.e., the IL-2/antagonist anti-CD40 antibody combination therapy is administered concurrently or within the same time frame as the other cancer therapy (i.e., the therapies are going on concurrently, but the IL-2/antagonist anti-CD40 antibody combination therapy is not administered precisely at the same time as the other cancer therapy). Alternatively, the IL-2/antagonist anti-CD40 antibody combination therapy may also be administered prior to or subsequent to the other cancer therapy. Sequential administration of the different cancer therapies may be performed regardless of whether the treated subject responds to the first course of therapy to decrease the possibility of remission or relapse.

Thus, for example, in some embodiments, a subject undergoing the IL-2/antagonist anti-CD40 antibody combination therapy disclosed herein is also undergoing therapy with FCR (fludarabine, cyclophosphamide, and rituximab). In other embodiments, a subject undergoing the IL-2/antagonist anti-CD40 antibody combination therapy disclosed herein is also undergoing therapy with CHOP-R (CHOP plus rituximab), or CFAR (fludarabine, cyclophosphamide, rituximab, and alemtuzumab). In alternative embodiments, a subject undergoing the IL-2/antagonist anti-CD40 antibody combination therapy disclosed herein is also undergoing therapy with FC (fludarabine, cyclophosphamide) and any other anti-CD20 antibody targeting the CD20 antigen on malignant B cells, including, but not limited to, the fully human antibody HuMax-CD20, R-1594, IMMU-106, TRU-015, AME-133, tositumomab/I-131 tositumomab (Bexxar®), and ibritumomab tiuxetan (Zevalin®). In yet other embodiments, a subject undergoing the IL-2/antagonist anti-CD40 antibody combination therapy disclosed herein is also undergoing therapy with CHOP plus other anti-CD20 antibody or CFA (fludarabine, cyclophosphamide, and alemtuzumab) plus other anti-CD20 antibody, where the other anti-CD20 antibody targets the CD20 antigen on malignant B cells, including, but not limited to, the fully human antibody HuMax-CD20, R-1594, IMMU-106, TRU-015, AME-133, tositumomab/I-131 tositumomab (Bexxar®), and ibritumomab tiuxetan (Zevalin®).

Interleukin-2 and Biologically Active Variants Thereof

The IL-2 described in the pharmaceutical formulations set forth herein and which is to be used in the methods disclosed herein may be native or obtained by recombinant techniques, and may be from any source, including mammalian sources such as, *e.g.*, rat, rabbit, primate, pig, and human. When the subject undergoing treatment is a human subject, preferably the IL-2 is derived from a human source, and includes human IL-2 that is recombinantly produced, such as recombinant IL-2 polypeptides produced by microbial hosts.

Human IL-2 is initially translated as a precursor polypeptide, shown in SEQ ID NO:14, which is encoded by a nucleotide sequence such as that set forth in SEQ ID NO:13. The precursor polypeptide includes a signal sequence at residues 1-20 of SEQ ID NO:14. The term "mature human IL-2" refers to the amino acid sequence set forth as SEQ ID NO:16, which is encoded by a nucleotide sequence such as that set forth as SEQ ID NO:15. The terms "des-alanyl-1, C125S human IL-2" and "des-alanyl-1, serine-125 human IL-2" refer to a mutein of mature human IL-2 that has a substitution of serine for cysteine at amino acid position 125 of the mature human IL-2 sequence and which lacks the N-terminal alanine that resides at position 1 of the mature human IL-2 sequence (i.e., at position 1 of SEQ ID NO:16). Des-alanyl-1, C125S human IL-2 has the amino acid sequence set forth in SEQ ID NO:18, which is encoded by a nucleotide sequence such as that set forth in SEQ ID NO:17. The *E. coli* recombinantly produced des-alanyl-1, C125S human IL-2 mutein, which is referred to as "aldesleukin," is available commercially as a formulation that is marketed under the tradename Proleukin® (Chiron Corporation, Emeryville, California). Proleukin® IL-2 serves as the reference IL-2 standard for determining suitable IL-2 dosing ranges for the combination IL-2/antagonist anti-CD40 antibody therapy protocols described herein.

*Biologically active variants of IL-2*. The pharmaceutical compositions useful in the methods disclosed herein may comprise biologically active variants of IL-2. Such variants should retain the desired biological activity of the native polypeptide such that the pharmaceutical composition comprising the variant polypeptide has the same therapeutic effect as the pharmaceutical composition comprising the native polypeptide when administered to a subject. That is, the variant polypeptide will serve as a therapeutically active component in the pharmaceutical composition in a manner similar to that observed for the native polypeptide. Methods are available in the art for determining whether a variant polypeptide retains the desired biological activity, and hence serves as a therapeutically active component in the pharmaceutical composition. Biological activity can be measured using assays specifically designed for measuring activity of the native polypeptide or protein, including assays described herein.

Additionally, antibodies raised against a biologically active native polypeptide can be tested for their ability to bind to the variant polypeptide, where effective binding is indicative of a polypeptide having a conformation similar to that of the native polypeptide.

For purposes of the present invention, the IL-2 biological activity of interest is the ability of IL-2 to activate and/or expand natural killer (NK) cells to mediate lymphokine activated killer (LAK) activity and antibody-dependent cellular cytotoxicity (ADCC). Thus, an IL-2 variant (for example, a mutein of human IL-2) for use in the methods disclosed herein will activate and/or expand natural killer (NK) cells to mediate lymphokine activated killer (LAK) activity and antibody-dependent cellular cytotoxicity (ADCC). NK cells mediate spontaneous or natural cytotoxicity against certain cell targets *in vitro* referred to as "NK-cell sensitive" targets, such as the human erythroleukemia K562 cell line. Following activation by IL-2, NK cells acquire LAK activity. Such LAK activity can be assayed by the ability of IL-2 activated NK cells to kill a broad variety of tumor cells and other "NK-insensitive" targets, such as the Daudi B-cell lymphoma line, that are normally resistant to lysis by resting (nonactivated) NK cells. Similarly, ADCC activity can be assayed by the ability of IL-2 activated NK cells to lyse "NK-insensitive" target cells, such as Daudi B-cell lymphoma line, or other target cells not readily lysed by resting NK cells in the presence of optimal concentrations of relevant tumor cell specific antibodies. Methods for generating and measuring cytotoxic activity of NK/LAK cells and ADCC are known in the art. See for example, Current Protocols in Immunology: Immunologic Studies in Humans, Supplement 17, Unit 7.7, 7.18, and 7.27 (John Wiley & Sons, Inc., 1996).

For purposes of the present invention, NK cells activated by an IL-2 variant for use in the methods disclosed herein demonstrate a specific lysing activity of NK-insensitive cells in the presence (ADCC activity) or absence (LAK activity) of antibody, more particularly NK-insensitive Daudi cells in the presence of B-cell specific antibodies including rituximab, that is at least about 20% greater, or at least about 25%, or 30%, or 35%, or 40% greater than baseline lysing activity of resting NK cells (i..e., nonactivated) as measured using effector to target ratios between 12.5 to 50:1 in a standard 4-hr $^{51}$Cr-release cytotoxicity assay (see Current Protocols in Immunology: Immunologic Studies in Humans, Unit 7.7, Supplement 17, Section 17.18.1 (John Wiley & Sons, Inc., 1996).

In some embodiments, the specific lysing activity of these IL-2 variant-activated NK cells is at least about 45% greater, at least about 50% greater, at least about 55% greater, at least about 60% greater, at least about 65% greater, at least about 70% greater, at least about 75% greater, or at least about 80% greater than baseline lysing activity of resting NK cells when measured as noted above.

Suitable biologically active variants of native or naturally occurring IL-2 can be fragments, analogues, and derivatives of that polypeptide. By "fragment" is intended a polypeptide consisting of only a part of the intact polypeptide sequence and structure, and can be a C-terminal deletion or N-terminal deletion of the native polypeptide. By "analogue" is intended an analogue of either the native polypeptide or of a fragment of the native polypeptide, where the analogue comprises a native polypeptide sequence and structure having one or more amino acid substitutions, insertions, or deletions. "Muteins", such as those described herein, and peptides having one or more peptoids (peptide mimics) are also encompassed by the term analogue (see International Publication No. WO 91/04282). By "derivative" is intended any suitable modification of the native polypeptide of interest, of a fragment of the native polypeptide, or of their respective analogues, such as glycosylation, phosphorylation, polymer conjugation (such as with polyethylene glycol), or other addition of foreign moieties, so long as the desired biological activity of the native polypeptide is retained. Methods for making polypeptide fragments, analogues, and derivatives are generally available in the art.

For example, amino acid sequence variants of the polypeptide can be prepared by mutations in the cloned DNA sequence encoding the native polypeptide of interest. Methods for mutagenesis and nucleotide sequence alterations are well known in the art. See, for example, Walker and Gaastra, eds. (1983) Techniques in Molecular Biology (MacMillan Publishing Company, New York); Kunkel (1985) Proc. Natl. Acad. Sci. USA 82:488-492; Kunkel et al. (1987) Methods Enzymol. 154:367-382; Sambrook et al. (1989) Molecular Cloning: A Laboratory Manual (Cold Spring Harbor, New York); U.S. Patent No. 4,873,192; and the references cited therein. Guidance as to appropriate amino acid substitutions that do not affect biological activity of the polypeptide of interest may be found in the model of Dayhoff et al. (1978) in Atlas of Protein Sequence and Structure (Natl. Biomed. Res. Found., Washington, D.C.).

Conservative substitutions, such as exchanging one amino acid with another having similar properties, may be preferred. Examples of conservative substitutions include, but are not limited to, Gly⇔Ala, Val⇔Ile⇔Leu, Asp⇔Glu, Lys⇔Arg, Asn⇔Gln, and Phe⇔Trp⇔Tyr.

In constructing variants of the IL-2 polypeptide of interest, modifications are made such that variants continue to possess the desired activity. Obviously, any mutations made in the DNA encoding the variant polypeptide must not place the sequence out of reading frame and preferably will not create complementary regions that could produce secondary mRNA structure. See EP Patent Application Publication No. 75,444.

In addition, the constant region of an antagonist anti-CD40 antibody can be mutated to alter effector function in a number of ways. For example, see U.S. Patent No. 6,737,056B1 and U.S. Patent Application Publication No. 2004/0132101A1, which disclose Fc mutations that optimize antibody binding to Fc receptors.

Biologically active variants of IL-2 will generally have at least 70%, preferably at least 80%, more preferably about 90% to 95% or more, and most preferably at least 96%, 97%, 98% or 99% sequence identity to the amino acid sequence of the reference polypeptide molecule, which serves as the basis for comparison. Thus, where the IL-2 reference molecule is human IL-2, a biologically active variant thereof will have at least 70%, preferably at least 80%, more preferably about 90% to 95% or more, and most preferably at least 96%, 97%, 98% or 99% sequence identity to the amino acid sequence for human IL-2.

For purposes of the present invention, percent sequence identity between amino acid sequences is determined using the Smith-Waterman homology search algorithm using an affine gap search with a gap open penalty of 12 and a gap extension penalty of 2, BLOSUM matrix of 62. The Smith-Waterman homology search algorithm is taught in Smith and Waterman (1981) Adv. Appl. Math. 2:482-489. A variant may, for example, differ from the reference IL-2 sequence by as few as 1 to 15 amino acid residues, as few as 1 to 10 amino acid residues, such as 6-10, as few as 5, as few as 4, 3, 2, or even 1 amino acid residue.

With respect to optimal alignment of two amino acid sequences, the contiguous segment of the variant amino acid sequence may have additional amino acid residues or deleted amino acid residues with respect to the reference amino acid sequence. The contiguous segment used for comparison to the reference amino acid sequence will include at least 20 contiguous amino acid residues, and may be 30, 40, 50, 100 or more amino acid residues. Corrections for sequence identity associated with conservative residue substitutions or gaps can be made (see Smith-Waterman homology search algorithm).

The precise chemical structure of a polypeptide having IL-2 activity depends on a number of factors. As ionizable amino and carboxyl groups are present in the molecule, a particular polypeptide may be obtained as an acidic or basic salt, or in neutral form. All such preparations that retain their biological activity when placed in suitable environmental conditions are included in the definition of polypeptides having IL-2 activity as used herein. Further, the primary amino acid sequence of the polypeptide may be augmented by derivatization using sugar moieties (glycosylation) or by other supplementary molecules such as lipids, phosphate, acetyl groups and the like. It may also be augmented by conjugation with saccharides. Certain aspects of such augmentation are accomplished through post-translational processing systems of the producing host; other such modifications may be introduced *in vitro.* In any event, such modifications are included in the definition of an IL-2 polypeptide used herein so long as the IL-2 activity of the polypeptide is not destroyed. It is expected that such modifications may quantitatively or qualitatively affect the activity, either by enhancing or diminishing the activity of the polypeptide, in the various assays. Further, individual amino acid residues in the chain may be modified by oxidation, reduction, or other derivatization, and the polypeptide may be cleaved to obtain fragments that retain activity. Such alterations that do not destroy activity do not remove the polypeptide sequence from the definition of IL-2 polypeptides of interest as used herein.

The art provides substantial guidance regarding the preparation and use of polypeptide variants. In preparing the IL-2 variants, one of skill in the art can readily determine which modifications to the native protein nucleotide or amino acid sequence will result in a variant that is suitable for use as a therapeutically active component of a pharmaceutical composition used in the methods disclosed herein.

For examples of variant IL-2 proteins, see European Patent (EP) Publication No. EP 136,489 (which discloses one or more of the following alterations in the amino acid sequence of naturally occurring IL-2: Asn26 to Gln26; Trp121 to Phe121; Cys58 to Ser58 or Ala58, Cys105 to Ser105 or Ala105; Cys125 to Ser125 or Ala125; deletion of all residues following Arg 120; and the Met-1 forms thereof); and the recombinant IL-2 muteins described in European Patent Application EP 0 109 748, which is the equivalent to Belgian Patent No. 893,016, and commonly owned U.S. Patent No. 4,518,584 (which disclose recombinant human IL-2 mutein wherein the cysteine at position 125, numbered in accordance with native human IL-2, is deleted or replaced by a neutral amino acid; alanyl-ser125-IL-2; and des-alanayl-ser125-IL-2). See also U.S. Patent No. 4,752,585 (which discloses the following variant IL-2 proteins: ala104 ser125 IL-2, ala104 IL-2, ala104 ala125 IL-2, val104 ser125 IL-2, val104 IL-2, val104 ala125 IL-2, des-ala1 ala104 ser125 IL-2, des-ala1 ala104 IL-2, des-ala1 ala104 ala125 IL-2, des-ala1 val104 ser125 IL-2, des-ala1 val104 IL-2, des-ala1 val104 ala125 IL-2, des-ala1 des-pro2 ala104 ser125 IL-2, des-ala1 des-pro2 ala104 IL-2, des-ala1 des-pro2 ala104 ala125 IL-2, des-ala1 des-pro2 val104 ser125 IL-2, des-ala1 des-pro2 val104 IL-2, des-ala1 des-pro2 val104 ala125 IL-2, des-ala1 des-pro2 des-thr3 ala104 ser125 IL-2, des-ala1 des-pro2 des-thr3 ala104 IL-2, des-ala1 des-pro2 des-thr3 ala104 ala125 IL-2, des-ala1 des-pro2 des-thr3 val104 ser125 IL-2, des-ala1 des-pro2 des-thr3 val104 IL-2, des-ala1 des-pro2 des-thr3 val104 ala125 IL-2, des-ala1 des-pro2 des-thr3 des-ser4 ala104 ser125 IL-2, des-ala1 des-pro2 des-thr3 des-ser4 ala104 IL-2, des-ala1 des-pro2 des-thr3 des-ser4 ala104 ala125 IL-2, des-ala1 des-pro2 des-thr3 des-ser4 val104 ser125 IL-2, des-ala1 des-pro2 des-thr3 des-ser4 val104 IL-2, des-ala1 des-pro2 des-thr3 des-ser4 val104 ala125 IL-2, des-alal des-pro2 des-thr3 des-ser4 des-ser5 ala104 ser125 IL-2, des-ala1 des-pro2 des-thr3 des-ser4 des-ser5 ala104 IL-2, des-ala1 des-pro2 des-thr3 des-ser4 des-ser5 ala104 ala125 IL-2, des-ala1 des-pro2 des-thr3 des-ser4 des-ser5 val104 ser125 IL-2, des-alal des-pro2 des-thr3 des-ser4 des-ser5 val 104 IL-2, des-ala1 des-pro2 des-thr3 des-ser4 des-ser5 val104 ala125 IL-2, des-ala1 des-pro2 des-thr3 des-ser4 des-ser5 des-ser6 ala104 ala125 IL-2, des-ala1 des-pro2 des-thr3 des-ser4 des-ser5 des-ser6 ala104 IL-2, des-ala1 des-pro2 des-thr3 des-ser4 des-ser5 des-ser6 ala104 ser125 IL-2, des-ala1 des-pro2 des-thr3 des-ser4 des-ser5 des-ser6 val104 ser125 IL-2, des-ala1 des-pro2 des-thr3 des-ser4 des-ser5 des-ser6 val104 IL-2, and des-ala1 des-pro2 des-thr3 des-ser4 des-ser5 des-ser6 val104 ala125 IL-2 ) and U.S. Patent No. 4,931,543 (which discloses the IL-2 mutein des-alanyl-1, serine-125 human IL-2 used in the examples herein, as well as the other IL-2 muteins).

Also see European Patent Publication No. EP 200,280 (published December 10, 1986), which discloses recombinant IL-2 muteins wherein the methionine at position 104 has been replaced by a conservative amino acid. Examples include the following muteins: ser4 des-ser5 ala104 IL-2; des-alal des-pro2 des-thr3 des-ser4 des-ser5 ala104 ala125 IL-2; des-alal des-pro2 des-thr3 des-ser4 des-ser5 glu104 ser125 IL-2; des-ala1 des-pro2 des-thr3 des-ser4 des-ser5 glu104 IL-2; des-alal des-pro2 des-thr3 des-ser4 des-ser5 glu104 ala125 IL-2; des-alal des-pro2 des-thr3 des-ser4 des-ser5 des-ser6 ala104 ala125 IL-2; des-alal des-pro2 des-thr3 des-ser4 des-ser5 des-ser6 ala104 IL-2; des-alal des-pro2 des-thr3 des-ser4 des-ser5 des-ser6 ala104 ser125 IL-2; des-alal des-pro2 des-thr3 des-ser4 des-ser5 des-ser6 glu104 ser125 IL-2; des-alal des-pro2 des-thr3 des-ser4 des-ser5 des-ser6 glu104 IL-2; and des-alal des-pro2 des-thr3 des-ser4 des-ser5 des-ser6 glu104 ala125 IL-2. See also European Patent Publication No. EP 118,617 and U.S. Patent No. 5,700,913, which disclose unglycosylated human IL-2 variants bearing alanine instead of native IL-2's methionine as the N-terminal amino acid; an unglycosylated human IL-2 with the initial methionine deleted such that proline is the N-terminal amino acid; and an unglycosylated human IL-2 with an alanine inserted between the N-terminal methionine and proline amino acids.

Other IL-2 muteins include the those disclosed in WO 99/60128 (substitutions of the aspartate at position 20 with histidine or isoleucine, the asparagine at position 88 with arginine, glycine, or isoleucine, or the glutamine at position126 with leucine or gulatamic acid), which reportedly have selective activity for high affinity IL-2 receptors expressed by cells expressing T cell receptors in preference to NK cells and reduced IL-2 toxicity; the muteins disclosed in U.S Patent No. 5,229,109 (substitutions of arginine at position 38 with alanine, or substitutions of phenylalanine at position 42 with lysine), which exhibit reduced binding to the high affinity IL-2 receptor when compared to native IL-2 while maintaining the ability to stimulate LAK cells; the muteins disclosed in International Publication No. WO 00/58456 (altering or deleting a naturally occurring (x)D(y) sequence in native IL-2 where D is aspartic acid, (x) is leucine, isoleucine, glycine, or valine, and (y) is valine, leucine or serine), which are claimed to reduce vascular leak syndrome; the IL-2 p1-30 peptide disclosed in International Publication No. WO 00/04048 (corresponding to the first 30 amino acids of IL-2, which contains the entire a-helix A of IL-2 and interacts with the b chain of the IL-2 receptor), which reportedly stimulates NK cells and induction of LAK cells; and a mutant form of the IL-2 p1-30 peptide also disclosed in WO 00/04048 (substitution of aspartic acid at position 20 with lysine), which reportedly is unable to induce vascular bleeds but remains capable of generating LAK cells. Additionally, IL-2 can be modified with polyethylene glycol to provide enhanced solubility and an altered pharmokinetic profile (see U.S. Patent No. 4,766,106).

Other muteins of IL-2 have improved functional profiles predictive of reduced toxicities. The muteins induce a lower level of pro-inflammatory cytokine production by NK cells while maintaining or increasing NK cell proliferation, maintaining NK-cell-mediated NK, LAK, and ADCC cytolytic functions, and maintaining T cell proliferative function as compared to the des-alanyl-1, C125S human IL-2 or C125S human IL-2 muteins. Examples of these improved IL-2 muteins include, but are not limited to, T7A, L36G, P65E, R81L, T7D, L36H, P65F, R81M, T7R, L36I, P65G, R81N, K8L, L36K, P65H, R81P, K9A, L36M, P65I, R81T, K9D, L36N, P65K, D84R, K9R, L36P, P65L, S87T, K9S, L36R, P65N, N88D, K9V, L36S, P65Q, N88H, K9W, L36W, P65R, N88T, T10K, L36Y, P65S, V91A, T10N, R38D, P65T, V91D, Q11A, R38G, P65V, V91E, Q11R, R38N, P65W, V91F, Q11T, R38P, P65Y, V91G, E15A, R38S, L66A, V91Q, H16D, L40D, L66F, V91W, H16E, L40G, E67A, V91N, L19D, L40N, L72G, L94A, L19E, L40S, L72N, L94I, D20E, T41E, L72T, L94T, I24L, T41G, F78S, L94V, K32A, F42A, F78W, L94Y, K32W, F42E, H79F, E95D, N33E, F42R, H79M, E95G, P34E, F42T, H79N, E95M, P34R, F42V, H79P, T102S, P34S, K43H, H79Q, T102V, P34T, F44K, H79S, M104G, P34V, M46I, H79V, E106K, K35D, E61K, L80E, Y107H, K35I, E61M, L80F, Y107K, K35L, E61R, L80G,Y107L, K35M, E62T, L80K, Y107Q, K35N, E62Y, L80N, Y107R, K35P, K64D, L80R, Y107T, K35Q, K64E, L80T, E116G, K35T, K64G, L80V, N119Q, L36A, K64L, L80W, T123S, L36D, K64Q, L80Y, T123C, L36E, K64R, R81E, Q126I, L36F, P65D, R81K, and Q126V, where residue position is relative to the position within the mature human IL-2 sequence set forth in SEQ ID NO:16. Examples of these combinatorial IL-2 muteins include, but are not limited to, 19D40D, 19D81K, 36D42R, 36D61R, 36D65L,40D36D, 40D61R, 40D65Y, 40D72N, 40D80K, 40G36D, 40G65Y, 80K36D, 80K65Y, 81K36D, 81K42E, 81K61R, 81K65Y, 81K72N, 81K88D, 81K91D, 81K107H, 81L107H, 91N95G, 107H36D, 107H42E, 107H65Y, 107R36D, 107R72N, 40D81K107H, 40G81K107H, and 91N94Y95G, where residue position is relative to the position within the mature human IL-2 sequence set forth in SEQ ID NO: 16.

The term IL-2 as used herein is also intended to include IL-2 fusions or conjugates comprising IL-2 fused to a second protein or covalently conjugated to polyproline or a water-soluble polymer to reduce dosing frequencies or to improve IL-2 tolerability. For example, the IL-2 (or a variant thereof as defined herein) can be fused to human albumin or an albumin fragment using methods known in the art (see WO 01/79258). Alternatively, the IL-2 can be covalently conjugated to polyproline or polyethylene glycol homopolymers and polyoxyethylated polyols, wherein the homopolymer is unsubstituted or substituted at one end with an alkyl group and the poplyol is unsubstituted, using methods known in the art (see, for example, U.S. Patent Nos. 4,766,106, 5,206,344, and 4,894,226).

_Pharmaceutical formulations of IL-2 or variant thereof_. Any pharmaceutical composition comprising IL-2 or suitable biologically active variant thereof as the therapeutically active component can be used in the methods disclosed herein. Such pharmaceutical compositions are known in the art and include, but are not limited to, those disclosed in U.S. Patent Nos. 4,604,377; 4,745,180; 4,766,106; 4,816,440; 4,894,226; 4,931,544; 4,992,271; 5,078,997; and 6,525,102. Thus liquid, lyophilized, or spray-dried compositions comprising IL-2 or variants thereof that are known in the art may be prepared as an aqueous or nonaqueous solution on suspension for subsequent administration to a subject in accordance with the methods disclosed herein. Each of these compositions will comprise IL-2 or variants thereof as a therapeutically or prophylactically active component. By "therapeutically or prophylactically active component" is intended the IL-2 or variants thereof is specifically incorporated into the composition to bring about a desired therapeutic or prophylactic response with regard to treatment, prevention, or diagnosis of a disease or condition within a subject when the pharmaceutical composition is administered to that subject. Preferably the pharmaceutical compositions comprise appropriate stabilizing agents, bulking agents, or both to minimize problems associated with loss of protein stability and biological activity during preparation and storage.

For example, U.S. Patent No. 4,604,377 shows an IL-2 formulation that has a therapeutic amount of IL-2, which is substantially free from non-IL-2 protein and endotoxin, a physiologically acceptable water-soluble carrier, and a sufficient amount of a surface active agent to solubilize the IL-2, such as sodium dodecyl sulfate. Other ingredients can be included, such as sugars. U.S. Patent No. 4,766,106 shows formulations including polyethylene glycol (PEG) modified IL-2. European patent application, Publication No. 268,110, shows IL-2 formulated with various non-ionic surfactants selected from the group consisting of polyoxyethylene sorbitan fatty acid esters (Tween-80), polyethylene glycol monostearate, and octylphenoxy polyethoxy ethanol compounds (Triton X405). U.S. Patent No. 4,992,271 discloses IL-2 formulations comprising human serum albumin and U.S. Patent No. 5,078,997 discloses IL-2 formulations comprising human serum albumin and amino acids. U.S. Patent No. 6,525,102 discloses IL-2 formulations comprising an amino acid base, which serves as the primary stabilizing agent of the polypeptide, and an acid and/or its salt form to buffer the solution within an acceptable pH range for stability of the polypeptide. US. 2003/0198602 discloses IL-2 formulations suitable for pulmonary delivery.

Antagonist Anti-CD40 Antibodies and Suitable Variants Thereof

The monoclonal antibodies CHIR-5.9 and CHIR-12.12 represent suitable antagonist anti-CD40 antibodies for use in the methods disclosed herein. The CHIR-5.9 and CHIR-12.2 antibodies are fully human anti-CD40 monoclonal antibodies of the $IgG_1$ isotype produced from the hybridoma cell lines 131.2F8.5.9 (referred to herein as the cell line 5.9) and 153.8E2.D10.D6.12.12 (referred to herein as the cell line 12.12). These cell lines were created using splenocytes from immunized xenotypic mice containing the human $IgG_1$ heavy chain locus and the human κ chain locus (XenoMouse® technology; Abgenix; Fremont, California). The spleen cells were fused with the mouse myeloma SP2/0 cells (Sierra BioSource). The resulting hybridomas were sub-cloned several times to create the stable monoclonal cell lines 5.9 and 12.12. Other antibodies of the invention may be prepared similarly using mice transgenic for human immunoglobulin loci or by other methods known in the art and/or described herein.

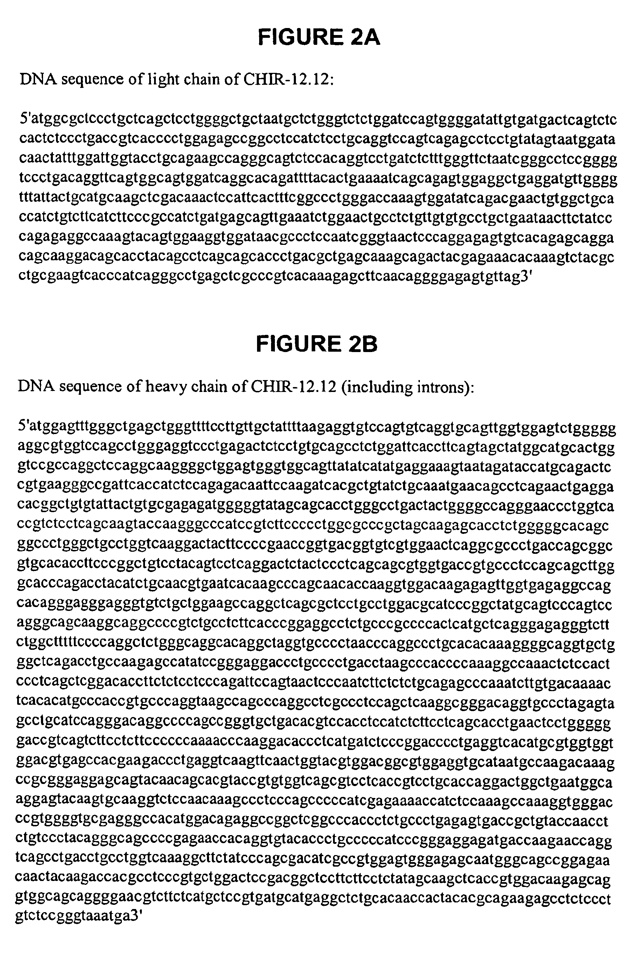

The nucleotide and amino acid sequences of the variable regions of the CHIR-12.12 antibody, and the amino acid sequences of the variable regions of the CHIR-5.9 antibody, are disclosed. More particularly, the amino acid sequences for the leader, variable, and constant regions for the light chain and heavy chain for mAb CHIR-12.12 are set forth in Figures 1A and 1B, respectively. See also SEQ ID NO:2 (complete sequence for the light chain of mAb CHIR-12.12), SEQ ID NO:4 (complete sequence for the heavy chain for mAb CHIR-12.12), and SEQ ID NO:5 (complete sequence for a variant of the heavy chain for mAb CHIR-12.12 set forth in SEQ ID NO:4, where the variant comprises a serine substitution for the alanine residue at position 153 of SEQ ID NO:4). The nucleotide sequences encoding the light chain and heavy chain for mAb CHIR-12.12 are set forth in Figures 2A and 2B, respectively. See also SEQ ID NO: 1 (coding sequence for the light chain for mAb CHIR-12.12), and SEQ ID NO:3 (coding sequence for the heavy chain for mAb CHIR-12.12). The amino acid sequences for the leader, variable, and constant regions for the light chain and heavy chain of the CHIR-5.9 mAb are set forth in Figures 3A and 3B, respectively. See also SEQ ID NO:6 (complete sequence for the light chain of mAb CHIR-5.9), SEQ ID NO:7 (complete sequence for the heavy chain of mAb CHIR-5.9), and SEQ ID NO:8 (complete sequence for a variant of the heavy chain of mAb CHIR-5.9 set forth in SEQ ID NO: 7, where the variant comprises a serine substitution for the alanine residue at position 158 of SEQ ID NO:7). Further, hybridomas expressing CHIR-5.9 and CHIR-12.12 antibodies have been deposited with the ATCC with a patent deposit designation of PTA-5542 and PTA-5543, respectively.

In addition to antagonist activity, anti-CD40 antibodies can have another mechanism of action against a tumor cell. For example, native CHIR-5.9 and CHIR-12.12 antibodies have ADCC activity. Alternatively, the variable regions of the CHIR-5.9 and CHIR-12.12 antibodies can be expressed on another antibody isotype that has ADCC activity. It is also possible to conjugate native forms, recombinant forms, or antigen-binding fragments of CHIR-5.9 or CHIR-12.12 to a cytotoxin, therapeutic agent, or radioisotope.

The CHIR-5.9 and CHIR-12.12 monoclonal antibodies bind soluble CD40 in ELISA-type assays, prevent the binding of CD40-ligand to cell-surface CD40, and displace the pre-bound CD40-ligand, as determined by flow cytometric assays. Antibodies CHIR-5.9 and CHIR-12.12 compete with each other for binding to CD40 but not with 15B8, the anti-CD40 monoclonal antibody described in WO 02/28904. When tested *in vitro* for effects on proliferation of B cells from normal human subjects, CHIR-5.9 and CHIR-12.12 act as antagonist anti-CD40 antibodies. Furthermore, CHIR-5.9 and CHIR-12.12 do not induce strong proliferation of human lymphocytes from normal subjects. These antibodies are able to kill CD40-expressing target cells by antibody dependent cellular cytotoxicity (ADCC). The binding affinity of CHIR-5.9 for human CD40 is $1.2x10^{-8}$ M and the binding affinity of CHIR-12.12 is $5x10^{-10}$ M, as determined by the Biacore™ assay.

Suitable antagonist anti-CD40 antibodies for use in the methods disclosed herein exhibit a strong single-site binding affinity for the CD40 cell-surface antigen. The monoclonal antibodies of the invention exhibit a dissociation equilibrium constant ($K_D$) for CD40 of at least $10^{-5}$ M, at least $3x10^{-5}$ M, preferably at least $10^{-6}$ M to $10^{-7}$ M, more preferably at least $10^{-8}$ M to about $10^{-12}$ M, measured using a standard assay such as Biacore™. Biacore analysis is known in the art and details are provided in the "BIAapplications handbook." Methods described in WO 01/27160 can be used to modulate the binding affinity.

By "CD40 antigen," "CD40 cell surface antigen," "CD40 receptor," or "CD40" is intended a transmembrane glycoprotein that belongs to the tumor necrosis factor (TNF) receptor family (see, for example, U.S. Patent Nos. 5,674,492 and 4,708,871; Stamenkovic et al. (1989) EMBO 8:1403; Clark (1990) Tissue Antigens 36:33; Barclay et al. (1997) The Leucocyte Antigen Facts Book (2d ed.; Academic Press, San Diego)). Two isoforms of human CD40, encoded by alternatively spliced transcript variants of this gene, have been identified. The first isoform (also known as the "long isoforms" or "isoform 1") is expressed as a 277-amino-acid precursor polypeptide (SEQ ID NO:12 (first reported as GenBank Accession No. CAA43045, and identified as isoform 1 in GenBank Accession No. NP_001241), encoded by SEQ ID NO:11 (see GenBank Accession Nos. X60592 and NM_001250)), which has a signal sequence represented by the first 19 residues. The second isoform (also known as the "short isoforms" or "isoform 2") is expressed as a 203-amino-acid precursor polypeptide (SEQ ID NO:10 (GenBank Accession No. NP_690593), encoded by SEQ ID NO:9 (GenBank Accession No. NM_152854)), which also has a signal sequence represented by the first 19 residues. The precursor polypeptides of these two isoforms of human CD40 share in common their first 165 residues (*i.e.*, residues 1-165 of SEQ ID NO:10 and SEQ ID NO:12). The precursor polypeptide of the short isoform (shown in SEQ ID NO:10) is encoded by a transcript variant (SEQ ID NO:9) that lacks a coding segment, which leads to a translation frame shift; the resulting CD40 isoform contains a shorter and distinct C-terminus (residues 166-203 of SEQ ID NO:10) from that contained in the long isoform of CD40 (C-terminus shown in residues 166-277 of SEQ ID NO: 12). For purposes of the present invention, the term "CD40 antigen," "CD40 cell surface antigen," "CD40 receptor," or "CD40" encompasses both the short and long isoforms of CD40. The anti-CD40 antibodies of the present invention bind to an epitope of human CD40 that resides at the same location within either the short isoform or long isoform of this cell surface antigen as noted herein below.

The CD40 antigen is displayed on the surface of a variety of cell types, as described elsewhere herein. By "displayed on the surface" and "expressed on the surface" is intended that all or a portion of the CD40 antigen is exposed to the exterior of the cell. The displayed or expressed CD40 antigen may be fully or partially glycosylated.

By "agonist activity" is intended that the substance functions as an agonist. An agonist combines with a cognate receptor on a cell and initiates a reaction or activity that is similar to or the same as that initiated by the receptor's natural ligand; e.g., it transduces a signal to the cell. An agonist of CD40 induces any or all of, but not limited to, the following responses: B cell proliferation and differentiation, antibody production, intercellular adhesion, B cell memory generation, isotype switching, up-regulation of cell-surface expression of MHC Class II and CD80/86, and secretion of pro-inflammatory cytokines such as IL-8, IL-12, and TNF. By "antagonist activity" is intended that the substance functions as an antagonist. An antagonist of CD40 prevents or reduces induction of any of the responses induced by binding of the CD40 receptor to an agonist ligand, particularly CD40L. The antagonist may reduce induction of any one or more of the responses to agonist binding by 5%, 10%, 15%, 20%, 25%, 30%, 35%, preferably 40%, 45%, 50%, 55%, 60%, more preferably 70%, 80%, 85%, and most preferably 90%, 95%, 99%, or 100%. Methods for measuring anti-CD40 antibody and CD40-ligand binding specificity and antagonist activity are known to one of skill in the art and include, but are not limited to, standard competitive binding assays, assays for monitoring immunoglobulin secretion by B cells, B cell proliferation assays, Banchereau-Like-B cell proliferation assays, T cell helper assays for antibody production, co-stimulation of B cell proliferation assays, and assays for up-regulation of B cell activation markers. See, for example, such assays disclosed in WO 00/75348 and U.S. Patent No. 6,087,329.

By "significant" agonist activity is intended an agonist activity of at least 30%, 35%, 40%, 45%, 50%, 60%, 70%, 75%, 80%, 85%, 90%, 95%, or 100% greater than the agonist activity induced by a neutral substance or negative control as measured in an assay of a B cell response. Preferably, "significant" agonist activity is an agonist activity that is at least 2-fold greater or at least 3-fold greater than the agonist activity induced by a neutral substance or negative control as measured in an assay of a B cell response. Thus, for example, where the B cell response of interest is B cell proliferation, "significant" agonist activity would be induction of a level of B cell proliferation that is at least 2-fold greater or at least 3-fold greater than the level of B cell proliferation induced by a neutral substance or negative control. In one embodiment, a non-specific immunoglobulin, for example IgG1, that does not bind to CD40 serves as the negative control. A substance "free of significant agonist activity" would exhibit an agonist activity of not more than about 25% greater than the agonist activity induced by a neutral substance or negative control, preferably not more than about 20% greater, 15% greater, 10% greater, 5% greater, 1% greater, 0.5% greater, or even not more than about 0.1 % greater than the agonist activity induced by a neutral substance or negative control as measured in an assay of a B cell response. The antagonist anti-CD40 antibodies useful in the methods disclosed herein are free of significant agonist activity as noted above when bound to a CD40 antigen on a human cell. In one embodiment of the invention, the antagonist anti-CD40 antibody is free of significant agonist activity in one B cell response. In another embodiment of the invention, the antagonist anti-CD40 antibody is free of significant agonist activity in assays of more than one B cell response (e.g., proliferation and differentiation, or proliferation, differentiation, and antibody production).

Monoclonal antibodies to CD40 are known in the art. See, for example, the sections dedicated to B-cell antigen in McMichael, ed. (1987; 1989) Leukocyte Typing III and IV (Oxford University Press, New York); U.S. Patent Nos. 5,674,492; 5,874,082; 5,677,165; 6,056,959; WO 00/63395; International Publication Nos. WO 02/28905 and WO 02/28904; Gordon et al. (1988) J. Immunol. 140:1425; Valle et al. (1989) Eur. J. Immunol. 19:1463; Clark et al. (1986) PNAS 83:4494; Paulie et al. (1989) J. Immunol. 142:590; Gordon et al. (1987) Eur. J. Immunol. 17:1535; Jabara et al. (1990) J. Exp. Med. 172:1861; Zhang et al. (1991) J. Immunol. 146:1836; Gascan et al. (1991) J. Immunol. 147:8; Banchereau et al. (1991) Clin. Immunol. Spectrum 3:8; and Banchereau et al. (1991) Science 251:70. Of particular interest to the present invention are the antagonist anti-CD40 antibodies disclosed herein that share the binding characteristics of the monoclonal antibodies CHIR-5.9 and CHIR-12.12 described above. Such antibodies include, but are not limited to the following: (1) the monoclonal antibodies produced by the hybridoma cell lines designated 131.2F8.5.9 (referred to herein as the cell line 5.9) and 153.8E2.D10.D6.12.12 (referred to herein as the cell line 12.12), deposited with the ATCC as Patent Deposit No. PTA-5542 and Patent Deposit No. PTA-5543, respectively; (2) a monoclonal antibody comprising an amino acid sequence selected from the group consisting of the sequence shown in SEQ ID NO: 2, the sequence shown in SEQ ID NO:4, the sequence shown in SEQ ID NO:5, both the sequences shown in SEQ ID NO:2 and SEQ ID NO:4, and both the sequences shown in SEQ ID NO:2 and SEQ ID NO:5; (3) a monoclonal antibody comprising an amino acid sequence selected from the group consisting of the sequence shown in SEQ ID NO:6, the sequence shown in SEQ ID NO:7, the sequence shown in SEQ ID NO:8, both the sequences shown in SEQ ID NO:6 and SEQ ID NO:7, and both the sequences shown in SEQ ID NO:6 and SEQ ID NO:8; (4) a monoclonal antibody having an amino acid sequence encoded by a nucleic acid molecule comprising a nucleotide sequence selected from the group consisting of the nucleotide sequence shown in SEQ ID NO:1, the nucleotide sequence shown in SEQ ID NO:3, and both the sequences shown in SEQ ID NO:1 and SEQ ID NO:3; (5) a monoclonal antibody that binds to an epitope capable of binding the monoclonal antibody produced by the hybridoma cell line 5.9 or the hybridoma cell line 12.12; (6) a monoclonal antibody that binds to an epitope comprising residues 82-87 of the amino acid sequence shown in SEQ ID NO:10 or SEQ ID NO: 12; (7) a monoclonal antibody that competes with the monoclonal antibody CHIR-5.9 or CHIR-12.12 in a competitive binding assay; and (8) a monoclonal antibody that is an antigen-binding fragment of the CHIR-12.12 or CHIR-5.9 monoclonal antibody or the foregoing monoclonal antibodies in preceding items (1)-(7), where the fragment retains the capability of specifically binding to the human CD40 antigen.

*Production of antagonist anti-CD40 antibodies*. The antagonist anti-CD40 antibodies for use in the methods disclosed herein can be produced using any antibody production method known to those of skill in the art. Thus, polyclonal sera may be prepared by conventional methods. In general, a solution containing the CD40 antigen is first used to immunize a suitable animal, preferably a mouse, rat, rabbit, or goat. Rabbits or goats are preferred for the preparation of polyclonal sera due to the volume of serum obtainable, and the availability of labeled anti-rabbit and anti-goat antibodies.

Polyclonal sera can be prepared in a transgenic animal, preferably a mouse bearing human immunoglobulin loci. In a preferred embodiment, Sf9 cells expressing CD40 are used as the immunogen. Immunization can also be performed by mixing or emulsifying the antigen-containing solution in saline, preferably in an adjuvant such as Freund's complete adjuvant, and injecting the mixture or emulsion parenterally (generally subcutaneously or intramuscularly). A dose of 50-200 μg/injection is typically sufficient. Immunization is generally boosted 2-6 weeks later with one or more injections of the protein in saline, preferably using Freund's incomplete adjuvant. One may alternatively generate antibodies by *in vitro* immunization using methods known in the art, which for the purposes of this invention is considered equivalent to *in vivo* immunization. Polyclonal antisera are obtained by bleeding the immunized animal into a glass or plastic container, incubating the blood at 25°C for one hour, followed by incubating at 4°C for 2-18 hours. The serum is recovered by centrifugation (e.g., 1,000 x g for 10 minutes). About 20-50 ml per bleed may be obtained from rabbits.

Production of the Sf 9 (*Spodoptera frugiperda*) cells is disclosed in U.S. Patent No. 6,004,552. Briefly, sequences encoding human CD40 were recombined into a baculovirus using transfer vectors. The plasmids were cotransfected with wild-type baculovirus DNA into Sf 9 cells. Recombinant baculovirus-infected Sf 9 cells were identified and clonally purified.

Preferably the antibody is monoclonal in nature. Monoclonal antibodies are highly specific, being directed against a single antigenic site, i.e., the CD40 cell surface antigen. Furthermore, in contrast to conventional (polyclonal) antibody preparations that typically include different antibodies directed against different determinants (epitopes), each monoclonal antibody is directed against a single determinant on the antigen. The modifier "monoclonal" indicates the character of the antibody as being obtained from a substantially homogeneous population of antibodies, such as those produced by a clonal population of B cells, and is not to be construed as requiring production of the antibody by any particular method. For example, the monoclonal antibodies to be used in accordance with the present invention may be made by the hybridoma method first described by Kohler et al. (1975) Nature 256:495, or may be made by recombinant DNA methods (see, *e.g.*, U.S. Patent No. 4,816,567). The "monoclonal antibodies" may also be isolated from phage antibody libraries using the techniques described in, for example, Clackson et al. (1991) Nature 352:624-628; Marks et al. (1991) J. Mol. Biol. 222:581-597; and U.S. Patent No. 5,514,548.

By "epitope" is intended the part of an antigenic molecule to which an antibody is produced and to which the antibody will bind. Epitopes can comprise linear amino acid residues (i.e., residues within the epitope are arranged sequentially one after another in a linear fashion), nonlinear amino acid residues (referred to herein as "nonlinear epitopes"; these epitopes are not arranged sequentially), or both linear and nonlinear amino acid residues.

Monoclonal antibodies can be prepared using the method of Kohler et al. (1975) Nature 256:495-496, or a modification thereof. Typically, a mouse is immunized with a solution containing an antigen. Immunization can be performed by mixing or emulsifying the antigen-containing solution in saline, preferably in an adjuvant such as Freund's complete adjuvant, and injecting the mixture or emulsion parenterally. Any method of immunization known in the art may be used to obtain the monoclonal antibodies of the invention. After immunization of the animal, the spleen (and optionally, several large lymph nodes) are removed and dissociated into single cells. The spleen cells may be screened by applying a cell suspension to a plate or well coated with the antigen of interest. The B cells expressing membrane bound immunoglobulin specific for the antigen bind to the plate and are not rinsed away. Resulting B cells, or all dissociated spleen cells, are then induced to fuse with myeloma cells to form hybridomas, and are cultured in a selective medium. The resulting cells are plated by serial dilution and are assayed for the production of antibodies that specifically bind the antigen of interest (and that do not bind to unrelated antigens). The selected monoclonal antibody (mAb)-secreting hybridomas are then cultured either *in vitro* (e.g., in tissue culture bottles or hollow fiber reactors), or *in vivo* (as ascites in mice).

Where the antagonist anti-CD40 antibodies of the invention are to be prepared using recombinant DNA methods, the DNA encoding the monoclonal antibodies is readily isolated and sequenced using conventional procedures (e.g.,

by using oligonucleotide probes that are capable of binding specifically to genes encoding the heavy and light chains of murine antibodies). The hybridoma cells described herein serve as a preferred source of such DNA. Once isolated, the DNA may be placed into expression vectors, which are then transfected into host cells such as *E. coli* cells, simian COS cells, Chinese Hamster Ovary (CHO) cells, or myeloma cells that do not otherwise produce immunoglobulin protein, to obtain the synthesis of monoclonal antibodies in the recombinant host cells. Review articles on recombinant expression in bacteria of DNA encoding the antibody include Skerra et al. (1993) Curr. Opinion in Immunol. 5:256 and Phickthun (1992) Immunol. Revs. 130:151. As an alternative to the use of hybridomas, antibody can be produced in a cell line such as a CHO cell line, as disclosed in U.S. Patent Nos. 5,545,403; 5,545,405; and 5,998,144. Briefly the cell line is transfected with vectors capable of expressing a light chain and a heavy chain, respectively. By transfecting the two proteins on separate vectors, chimeric antibodies can be produced. Another advantage is the correct glycosylation of the antibody.

In some embodiments, the antagonist anti-CD40 antibody, for example, the CHIR-12.12 or CHIR-5.9 antibody, or antigen-binding fragment thereof is produced in CHO cells using the GS gene expression system (Lonza Biologics, Portsmouth, New Hampshire), which uses glutamine synthetase as a marker. See, also U.S. Patent Nos. 5,122,464; 5,591,639; 5;658,759; 5,770,359; 5,827,739; 5,879,936; 5,891,693; and 5,981,216.

The term "antigen epitope" as used herein refers to a three dimensional molecular structure (either linear or conformational) that is capable of immunoreactivity with an anti-CD40 monoclonal antibody. Antigen epitopes may comprise proteins, protein fragments, peptides, carbohydrates, lipids, and other molecules, but for the purposes of the present invention are most commonly proteins, short oligopeptides, oligopeptide mimics (i e, organic compounds that mimic the antibody binding properties of the CD40 antigen), or combinations thereof. Suitable oligopeptide mimics are described, *inter alias,* in PCT application US 91/04282.

Additionally, the term "antibody" as used herein encompasses chimeric anti-CD40. Chimeric anti-CD40 antibodies for use in the methods disclosed herein have the binding characteristics of the anti-CD40 monoclonal antibody CHIR-12.12 or CHIR-5.9. By "chimeric" antibodies is intended antibodies that are most preferably derived using recombinant deoxyribonucleic acid techniques and which comprise both human (including immunologically "related" species, e.g., chimpanzee) and non-human components. Thus, the constant region of the chimeric antibody is most preferably substantially identical to the constant region of a natural human antibody; the variable region of the chimeric antibody is most preferably derived from a non-human source and has the desired antigenic specificity to the CD40 cell-surface antigen. The non-human source can be any vertebrate source that can be used to generate antibodies to a human CD40 cell-surface antigen or material comprising a human CD40 cell-surface antigen. Such non-human sources include, but are not limited to, rodents (e.g., rabbit, rat, mouse, etc.; see, for example, U.S. Patent No. 4,816,567) and non-human primates (e.g., Old World Monkey, Ape, etc.; see, for example, U.S. Patent Nos. 5,750,105 and 5,756,096). As used herein, the phrase "immunologically active" when used in reference to chimeric anti-CD40 antibodies means a chimeric antibody that binds human CD40.

Humanized anti-CD40 antibodies represent additional anti-CD40 antibodies suitable for use in the methods disclosed herein. By "humanized" is intended forms of anti-CD40 antibodies that contain minimal sequence derived from non-human immunoglobulin sequences. For the most part, humanized antibodies are human immunoglobulins (recipient antibody) in which residues from a hypervariable region (also known as complementarity determining region or CDR) of the recipient are replaced by residues from a hypervariable region of a non-human species (donor antibody) such as mouse, rat, rabbit, or nonhuman primate having the desired specificity, affinity, and capacity. The phrase "complementarity determining region" refers to amino acid sequences which together define the binding affinity and specificity of the natural Fv region of a native immunoglobulin binding site. See, e.g., Chothia et al ( 1987) J. Mol. Biol. 196:901-917; Kabat et al (1991) U. S. Dept. of Health and Human Services, NIH Publication No. 91-3242). The phrase "constant region" refers to the portion of the antibody molecule that confers effector functions. In previous work directed towards producing non-immunogenic antibodies for use in therapy of human disease, mouse constant regions were substituted by human constant regions. The constant regions of the subject humanized antibodies were derived from human immunoglobulins. However, these humanized antibodies still elicited an unwanted and potentially dangerous immune response in humans and there was a loss of affinity. Humanized anti-CD40 antibodies for use in the methods disclosed herein have binding characteristics similar to those exhibited by the CHIR-5.9 and CHIR-12.12 monoclonal antibodies described herein.

Humanization can be essentially performed following the method of Winter and co-workers (Jones et al. (1986) Nature 321:522-525; Riechmann et al. (1988) Nature 332:323-327; Verhoeyen et al. (1988) Science 239:1534-1536), by substituting rodent or mutant rodent CDRs or CDR sequences for the corresponding sequences of a human antibody. See also U.S. Patent Nos. 5,225,539; 5,585,089; 5,693,761; 5,693,762; 5,859,205. In some instances, residues within the framework regions of one or more variable regions of the human immunoglobulin are replaced by corresponding non-human residues (see, for example, U.S. Patent Nos. 5,585,089; 5,693,761; 5,693,762; and 6,180,370). Furthermore, humanized antibodies may comprise residues that are not found in the recipient antibody or in the donor antibody. These modifications are made to further refine antibody performance (e.g., to obtain desired affinity). In general, the humanized antibody will comprise substantially all of at least one, and typically two, variable domains, in which all or substantially all of the hypervariable regions correspond to those of a non-human immunoglobulin and all or substantially all of the framework regions are those of a human immunoglobulin sequence. The humanized antibody optionally also will comprise at least a portion of an immunoglobulin constant region (Fc), typically that of a human immunoglobulin. For further details see Jones et al. (1986) Nature 331:522-525; Riechmann et al. (1988) Nature 332:323-329; and Presta (1992) Curr. Op. Struct. Biol. 2:593-596. Accordingly, such "humanized" antibodies may include antibodies wherein substantially less than an intact human variable domain has been substituted by the corresponding sequence from a non-human species. In practice, humanized antibodies are typically human antibodies in which some CDR residues and possibly some framework residues are substituted by residues from analogous sites in rodent antibodies. See, for example, U.S. Patent Nos. 5,225,539; 5,585,089; 5,693,761; 5,693,762; 5,859,205. See also U.S. Patent No. 6,180,370, and International Publication No. WO 01/27160, where humanized antibodies and techniques for producing humanized antibodies having improved affinity for a predetermined antigen are disclosed.

Also encompassed by the term anti-CD40 antibodies are xenogeneic or modified anti-CD40 antibodies produced in a non-human mammalian host, more particularly a transgenic mouse, characterized by inactivated endogenous immunoglobulin (Ig) loci. In such transgenic animals, competent endogenous genes for the expression of light and heavy subunits of host immunoglobulins are rendered non-functional and substituted with the analogous human immunoglobulin loci. These transgenic animals produce human antibodies in the substantial absence of light or heavy host immunoglobulin subunits. See, for example, U.S. Patent Nos. 5,877,397 and 5,939,598.

Preferably, fully human antibodies to CD40 are obtained by immunizing transgenic mice. One such mouse is obtained using XenoMouse® technology (Abgenix; Fremont, California) and is disclosed in U.S. Patent Nos. 6,075,181, 6,091,001, and 6,114,598. To produce the antibodies disclosed herein, mice transgenic for the human Ig $G_1$ heavy chain locus and the human κ light chain locus can be immunized with Sf 9 cells expressing human CD40. Mice can also be transgenic for other isotypes. Fully human antibodies useful in the methods disclosed herein are characterized by binding properties similar to those exhibited by the CHIR-5.9 and CHIR-12.12 monoclonal antibodies.

Suitable antigen-binding fragments of an antibody comprise a portion of a full-length antibody, generally the antigen-binding or variable region thereof. Examples of antibody fragments include, but are not limited to, Fab, $F(ab')_2$, and Fv fragments and single-chain antibody molecules. By "Fab" is intended a monovalent antigen-binding fragment of an immunoglobulin that is composed of the light chain and part of the heavy chain. By $F(ab')_2$ is intended a bivalent antigen-binding fragment of an immunoglobulin that contains both light chains and part of both heavy chains. By "single-chain Fv" or "sFv" antibody fragments is intended fragments comprising the $V_H$ and $V_L$ domains of an antibody, wherein these domains are present in a single polypeptide chain. See, for example, U.S. Patent Nos. 4,946,778, 5,260,203, 5,455,030, and 5,856,456. Generally, the Fv polypeptide further comprises a polypeptide linker between the $V_H$ and $V_L$ domains that enables the sFv to form the desired structure for antigen-binding. For a review of sFv see Pluckthun (1994) in The Pharmacology of Monoclonal Antibodies, Vol. 113, ed. Rosenburg and Moore (Springer-Verlag, New York), pp. 269-315.

Antibodies or antibody fragments can be isolated from antibody phage libraries generated using the techniques described in, for example, McCafferty et al. (1990) Nature 348:552-554 (1990) and U.S. Patent No. 5,514,548. Clackson et al. (1991) Nature 352:624-628 and Marks et al. (1991) J Mol. Biol. 222:581-597 describe the isolation of murine and human antibodies, respectively, using phage libraries. Subsequent publications describe the production of high affinity (nM range) human antibodies by chain shuffling (Marks et al. (1992) Bio/Technology 10:779-783), as well as combinatorial infection and *in vivo* recombination as a strategy for constructing very large phage libraries (Waterhouse et al. (1993) Nucleic. Acids Res. 21:2265-2266). Thus, these techniques are viable alternatives to traditional monoclonal antibody hybridoma techniques for isolation of monoclonal antibodies.

Various techniques have been developed for the production of antibody fragments. Traditionally, these fragments were derived *via* proteolytic digestion of intact antibodies (see, e.g., Morimoto et al. (1992) Journal of Biochemical and Biophysical Methods 24:107-117 (1992) and Brennan et al. (1985) Science 229:81). However, these fragments can now be produced directly by recombinant host cells. For example, the antibody fragments can be isolated from the antibody phage libraries discussed above. Alternatively, Fab'-SH fragments can be directly recovered from *E. coli* and chemically coupled to form $F(ab')_2$ fragments (Carter et al. (1992) Bio/Technology 10:163-167). According to another approach, $F(ab')_2$ fragments can be isolated directly from recombinant host cell culture. Other techniques for the production of antibody fragments will be apparent to the skilled practitioner.

Antibodies useful in the methods disclosed herein include antagonist anti-CD40 antibodies such as the CHIR-5.9 and CHIR-12.12 monoclonal antibodies disclosed herein that differ in non-CDR regions; and antibodies with one or more amino acid addition(s), deletion(s), or substitution(s). The invention also encompasses deimmunized (humanized) antagonist anti-CD40 antibodies, which can be produced as described in, for example, International Publication Nos. WO 98/52976 and WO 0034317. In this manner, residues within the antibodies useful for the practicing the methods disclosed herein are modified so as to render the antibodies non- or less immunogenic to humans while retaining their binding specificity and biological activity, wherein such activity is measured by assays noted elsewhere herein. Also included within the scope of the claims are fusion proteins comprising antagonist anti-CD40 antibodies, or a fragment thereof, which fusion proteins can be synthesized or expressed from corresponding polynucleotide vectors, as is known in the art. Such fusion proteins are described with reference to conjugation of antibodies as noted below.

The antibodies useful in practicing the methods disclosed herein can have sequence variations produced using methods described in, for example, Patent Publication Nos. EP 0 983 303 A1, WO 00/34317, and WO 98/52976.

For example, it has been shown that sequences within the CDR can cause an antibody to bind to MHC Class II and trigger an unwanted helper T-cell response. A conservative substitution can allow the antibody to retain binding activity yet lose its ability to trigger an unwanted T-cell response. Any such conservative or non-conservative substitutions can be made using art-recognized methods, such as those noted elsewhere herein, and the resulting antibodies will fall within the scope of the invention. The variant antibodies can be routinely tested for antagonist activity, affinity, and specificity using methods described herein.

An antagonist anti-CD40 antibody produced by any of the methods described above, or any other method not disclosed herein, will fall within the scope of the invention if it possesses at least one of the following biological activities: inhibition of immunoglobulin secretion by normal human peripheral B cells stimulated by T cells; inhibition of proliferation of normal human peripheral B cells stimulated by Jurkat T cells; inhibition of proliferation of normal human peripheral B cells stimulated by CD40L-expressing cells or soluble CD40; and inhibition of proliferation of human malignant B cells as noted below.

See also the assays described in Schultze et al. (1998) Proc. Natl. Acad. Sci. USA 92:8200-8204; Denton et al. (1998) Pediatr. Transplant. 2:6-15; Evans et al. (2000) J. Immunol. 164:688-697; Noelle (1998) Agents Actions Suppl. 49:17-22; Lederman et al. (1996) Curr. Opin. Hematol. 3:77-86; Coligan et al. (1991) Current Protocols in Immunology 13:12; Kwekkeboom et al. (1993) Immunology 79:439-444; and U.S. Patent Nos. 5,674,492 and 5,847,082.

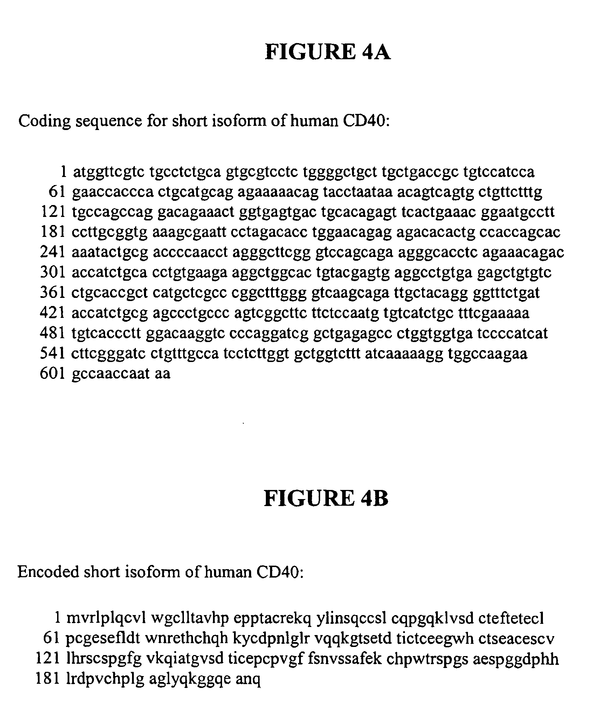

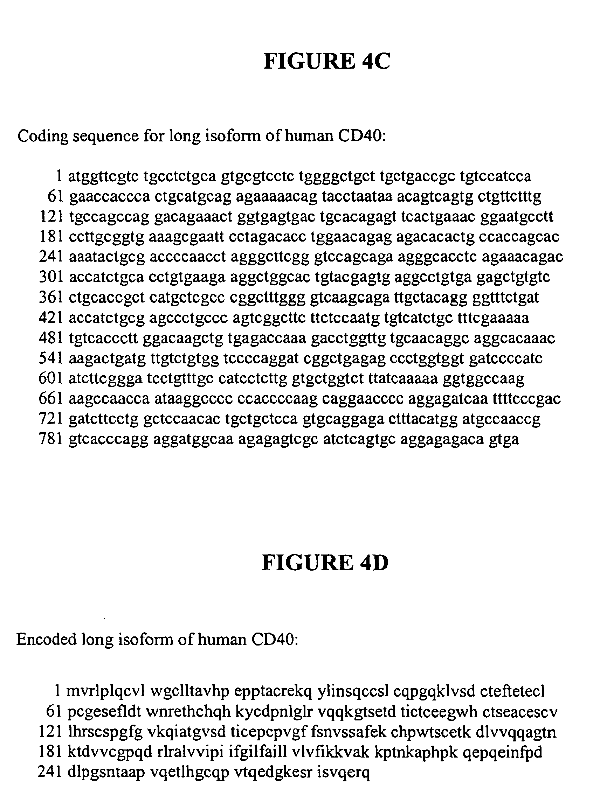

A representative assay to detect antagonist anti-CD40 antibodies specific to the CD40-antigen epitopes identified herein or is a "competitive binding assay". Competitive binding assays are serological assays in which unknowns are detected and quantitated by their ability to inhibit the binding of a labeled known ligand to its specific antibody. This is also referred to as a competitive inhibition assay. In a representative competitive binding assay, labeled CD40 polypeptide is precipitated by candidate antibodies in a sample, for example, in combination with monoclonal antibodies raised against one or more epitopes of the monoclonal antibodies of the invention. Anti-CD40 antibodies that specifically react with an epitope of interest can be identified by screening a series of antibodies prepared against a CD40 protein or fragment of the protein comprising the particular epitope of the CD40 protein of interest. For example, for human CD40, epitopes of interest include epitopes comprising linear and/or nonlinear amino acid residues of the short isoform of human CD40 (see GenBank Accession No. NP_690593) set forth in Figure 4B (SEQ ID NO:10), encoded by the sequence set forth in Figure 4A (SEQ ID NO:9; see also GenBank Accession No. NM_152854), or of the long isoform of human CD40 (see GenBank Accession Nos. CAA43045 and NP_001241) set forth in Figure 4D (SEQ ID NO:12), encoded by the sequence set forth in Figure 4C (SEQ ID NO:11; see GenBank Accession Nos. X60592 and NM_001250). Alternatively, competitive binding assays with previously identified suitable antagonist anti-CD40 antibodies could be used to select monoclonal antibodies comparable to the previously identified antibodies.

Any of the previously described antagonist anti-CD40 antibodies (or antigen-binding fragments thereof) may be conjugated prior to use in the methods disclosed herein. Methods for producing conjugated antibodies are known in the art. Thus, the anti-CD40 antibody may be labeled using an indirect labeling or indirect labeling approach. By "indirect labeling" or "indirect labeling approach" is intended that a chelating agent is covalently attached to an antibody and at least one radionuclide is inserted into the chelating agent. See, for example, the chelating agents and radionuclides described in Srivastava and Mease (1991) Nucl. Med. Bio. 18:589-603.

Suitable labels include fluorophores, chromophores, radioactive atoms (particularly $^{32}$P and $^{125}$I), electron-dense reagents, enzymes, and ligands having specific binding partners. Enzymes are typically detected by their activity. For example, horseradish peroxidase is usually detected by its ability to convert 3,3 ',5,5 '-tetramethylbenzidine (TMB) to a blue pigment, quantifiable with a spectrophotometer. "Specific binding partner" refers to a protein capable of binding a ligand molecule with high specificity, as for example in the case of an antigen and a monoclonal antibody specific therefore. Other specific binding partners include biotin and avidin or streptavidin, Ig G and protein A, and the numerous receptor-ligand couples known in the art. It should be understood that the above description is not meant to categorize the various labels into distinct classes, as the same label may serve in several different modes. For example, $^{125}$I may serve as a radioactive label or as an electron-dense reagent. HRP may serve as enzyme or as antigen for a mAb. Further, one may combine various labels for desired effect. For example, mAbs and avidin also require labels in the practice of this invention: thus, one might label a mAb with biotin, and detect its presence with avidin labeled with $^{125}$I, or with an anti-biotin mAb labeled with HRP. Other permutations and possibilities will be readily apparent to those of ordinary skill in the art, and are considered as equivalents within the scope of the instant invention.

Alternatively, the anti-CD40 antibody may be labeled using "direct labeling" or a "direct labeling approach," where a radionuclide is covalently attached directly to an antibody (typically via an amino acid residue). Preferred radionuclides are provided in Srivastava and Mease (1991) *supra.* The indirect labeling approach is particularly preferred. See also, for example, International Publication Nos. WO 00/52031 and WO 00/52473, where a linker is used to attach a radioactive label to antibodies; and the labeled forms of antibodies described in U.S. Patent No. 6,015,542.

Further, an antibody (or fragment thereof) may be conjugated to a therapeutic moiety such as a cytotoxin, a therapeutic agent, or a radioactive metal ion or radioisotope. A cytotoxin or cytotoxic agent includes any agent that is detrimental to cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents include, but are not limited to, antimetabolites (e.g., fludarabine, 2-chlorodeoxyadenosine (cladribine), methotrexate, 6-mercaptopurine, 6-thioguanine, cytarabine, 5-fluorouracil decarbazine), alkylating agents (e.g., mechlorethamine, thioepa chlorambucil, melphalan, carmustine (BSNU) and lomustine (CCNU), cyclophosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine and vinblastine). Radioisotopes include, but are not limited to, I-131, I-123, I-125, Y-90, Re-188, Re-186, At-211, Cu-67, Bi-212, Bi-213, Pd-109, Tc-99, In-111, and the like. The conjugates of the invention can be used for modifying a given biological response; the drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, interferon-alpha, interferon-beta, nerve growth factor, platelet derived growth factor, tissue plasminogen activator; or, biological response modifiers such as, for example, lymphokines, interleukin-1 ("IL-1"), interleukin-2 ("IL-2"), interleukin-6 ("IL-6"), granulocyte macrophage colony stimulating factor ("GM-CSF"), granulocyte colony stimulating factor ("G-CSF"), or other growth factors.

Techniques for conjugating such therapeutic moiety to antibodies are well known. See, for example, Arnon et al. (1985) "Monoclonal Antibodies for Immunotargeting of Drugs in Cancer Therapy," in Monoclonal Antibodies and Cancer Therapy, ed. Reisfeld et al. (Alan R. Liss, Inc.), pp. 243-256; ed. Hellstrom et al. (1987) "Antibodies for Drug Delivery," in Controlled Drug Delivery, ed. Robinson et al. (2d ed; Marcel Dekker, Inc.), pp. 623-653; Thorpe (1985) "Antibody Carriers of Cytotoxic Agents in Cancer Therapy: A Review," in Monoclonal Antibodies '84: Biological and Clinical Applications, ed. Pinchera et al. pp. 475-506 (Editrice Kurtis, Milano, Italy, 1985); "Analysis, Results, and Future Prospective of the Therapeutic Use of Radiolabeled Antibody in Cancer Therapy," in Monoclonal Antibodies for Cancer Detection and Therapy, ed. Baldwin et al. (Academic Press, New York, 1985), pp. 303-316; and Thorpe et al. (1982) Immunol. Rev. 62:119-158.

Alternatively, an antibody can be conjugated to a second antibody to form an antibody heteroconjugate as described in U.S. Patent No. 4,676,980. In addition, linkers may be used between the labels and the antibodies of the invention (see U.S. Patent No. 4,831,175). Antibodies or, antigen-binding fragments thereof may be directly labeled with radioactive iodine, indium, yttrium, or other radioactive particle known in the art (U.S. Patent No. 5,595,721). Treatment may consist of a combination of treatment with conjugated and nonconjugated antibodies administered simultaneously or sequentially, in either order, on the same or different days (WO 00/52031 and WO 00/52473).

*Biologically active variants of antagonist anti-CD40 antibodies*. The pharmaceutical compositions useful in the methods disclosed herein may comprise biologically active variants of the antagonist anti-CD40 antibodies of the invention. Such variants should retain the desired biological activity of the native polypeptide such that the pharmaceutical composition comprising the variant polypeptide has the same therapeutic effect as the pharmaceutical composition comprising the native polypeptide when administered to a subject. That is, the variant anti-CD40 antibody will serve as a therapeutically active component in the pharmaceutical composition in a manner similar to that observed for the native antagonist antibody, for example CHIR-5.9 or CHIR-12.12 as expressed by the hybridoma cell line 5.9 or 12.12, respectively. Methods are available in the art for determining whether a variant anti-CD40 antibody retains the desired biological activity, and hence serves as a therapeutically active component in the pharmaceutical composition. Biological activity of antibody variants can be measured using assays specifically designed for measuring activity of the native antagonist antibody, including assays described herein. Suitable biologically active variants of the antagonist anti-CD40 antibodies will retain the desired binding properties of the parent antibody, i.e., the parent antagonist anti-CD40 antibody. Methods for making antibody variants are generally available in the art.

For example, amino acid sequence variants of an antagonist anti-CD40 antibody, for example, the CHIR-5.9 or CHIR-12.12 monoclonal antibody described herein, can be prepared using the methods for mutagenesis and nucleotide sequence alterations that are described herein above for IL-2. See, for example, Walker and Gaastra, eds. (1983) Techniques in Molecular Biology (MacMillan Publishing Company, New York); Kunkel (1985) Proc. Natl. Acad. Sci. USA 82:488-492; Kunkel et al. (1987) Methods Enzymol. 154:367-382; Sambrook et al. (1989) Molecular Cloning: A Laboratory Manual (Cold Spring Harbor, New York); U.S. Patent No. 4,873,192; and the references cited therein.

Guidance as to appropriate amino acid substitutions that do not affect biological activity of the polypeptide of interest may be found in the model of Dayhoff *et al.* (1978) in *Atlas of Protein Sequence and Structure* (Natl. Biomed. Res. Found., Washington, D.C.). Conservative substitutions, such as exchanging one amino acid with another having similar properties, may be preferred. Examples of conservative substitutions include, but are not limited to, Gly⇔Ala, Val⇔Ile⇔Leu, Asp⇔Glu, Lys⇔Arg, Asn⇔Gln, and Phe⇔Trp⇔Tyr.

Similar to variants of IL-2, antagonist anti-CD40 antibody variants are made such that the variants continue to possess the desired activity, *i.e.,* similar binding affinity and are capable of specifically binding CD40 antigen expressed on the surface of a human cell, respectively, and in the case of anti-CD40 being free of significant agonist activity but exhibiting antagonist activity when bound to a CD40 antigen on a human CD40-expressing cell. As with IL-2 variants, any mutations made in the DNA encoding the variant anti-CD40 polypeptide must not place the sequence out of reading frame and preferably will not create complementary regions that could produce secondary mRNA structure. See EP Patent Application Publication No. 75,444.

Preferably, variants of a antagonist anti-CD40 antibody have amino acid sequences that have at least 70%, preferably at least 80% sequence identity, more preferably about 90% to 95% or more, and most preferably at least 96%, 97%, 98% or 99% sequence identity to the amino acid sequence of the reference polypeptide molecule, which serves as the basis for comparison, for example a reference antagonist anti-CD40 antibody molecule, such as CHIR-5.9 or CHIR-12.12 monoclonal antibody described herein, or to a shorter portion of the reference antibody molecule.

For purposes of the present invention, percent sequence identity for antagonist anti-CD40 antibodies is determined in the manner described for IL-2. In this manner, percent sequence identity is determined using the Smith-Waterman homology search algorithm using an affine gap search with a gap open penalty of 12 and a gap extension penalty of 2, BLOSLTM matrix of 62. A variant may, for example, differ from the reference antagonist anti-CD40 antibody by as few as 1 to 15 amino acid residues, as few as 1 to 10 amino acid residues, such as 6-10, as few as 5, as few as 4, 3, 2, or even 1 amino acid residue.

As previously noted herein above, with respect to optimal alignment of any two amino acid sequences, the contiguous segment of the variant amino acid sequence may have additional amino acid residues or deleted amino acid residues with respect to the reference amino acid sequence, for example, the antibodies CHIR-12.12 and CHIR-5.9. The contiguous segment used for comparison to the reference amino acid sequence will include at least 20 contiguous amino acid residues, and may be 30, 40, 50, or more amino acid residues. Corrections for sequence identity associated with conservative residue substitutions or gaps can be made (see Smith-Waterman homology search algorithm).

Similar to IL-2, the precise chemical structure of a polypeptide capable of specifically binding CD40 and retaining antagonist activity, particularly when bound to antigen on malignant B cells, depends on a number of factors. As ionizable amino and carboxyl groups are present in the molecule, a particular polypeptide may be obtained as an acidic or basic salt, or in neutral form. All such preparations that retain their biological activity when placed in suitable environmental conditions are included in the definition of antagonist anti-CD40 antibodies as used herein. Further, the primary amino acid sequence of the polypeptide may be augmented by derivatization using sugar moieties (glycosylation) or by other supplementary molecules such as lipids, phosphate, acetyl groups and the like. It may also be augmented by conjugation with saccharides. Certain aspects of such augmentation are accomplished through post-translational processing systems of the producing host; other such modifications may be introduced *in vitro.* In any event, such modifications are included in the definition of an anti-CD40 antibody used herein so long as the binding properties of the anti-CD40 antibody (including antagonist activity) are not destroyed. It is expected that such modifications may quantitatively or qualitatively affect the activity, either by enhancing or diminishing the activity of the polypeptide, in the various assays. Further, individual amino acid residues in the chain may be modified by oxidation, reduction, or other derivatization, and the polypeptide may be cleaved to obtain fragments that retain activity. Such alterations that do not destroy the desirable activity of the unmodified antibody do not remove the polypeptide sequence from the definition of the anti-CD40 antibody of interest as used herein.

As previously noted above, the art provides substantial guidance regarding the preparation and use of polypeptide variants. In preparing the antibody variants, one of skill in the art can readily determine which modifications to the native protein nucleotide or amino acid sequence will result in a variant that is suitable for use as a therapeutically active component of a pharmaceutical composition used in the methods disclosed herein.

*Pharmaceutical formulations of antagonist-anti-CD40 antibodies.* The antagonist anti-CD40 antibodies of this invention are administered at a concentration that is therapeutically effective to prevent or treat chronic lymphocytic leukemia. To accomplish this goal, the antibodies may be formulated using a variety of acceptable excipients known in the art. Typically, the antibodies are administered by injection, for example, either intravenously, intraperitoneally, or subcutaneously. Methods to accomplish this administration are known to those of ordinary skill in the art. It may also be possible to obtain compositions that may be topically or orally administered, or which may be capable of transmission across mucous membranes.

A pharmaceutical composition disclosed herein is formulated to be compatible with its intended route of administration. Examples of possible routes of administration include parenteral, (e.g., intravenous (IV), intramuscular (IM), intradermal, subcutaneous (SC), or infusion), oral and pulmonary (*e.g.,* inhalation), nasal, transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerin, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes, or multiple dose vials made of glass or plastic.

The antibodies are typically provided by standard technique within a pharmaceutically acceptable buffer; for example, sterile saline, sterile buffered water, propylene glycol, combinations of the foregoing, etc. The antagonist anti-CD40 antibody (or antigen-binding fragment thereof) can be formulated in separate pharmaceutical compositions, or can be formulated within a single pharmaceutical composition with IL-2 or biologically active variant thereof for simultaneous administration. Methods for preparing parenterally administrable agents are described in Remington's Pharmaceutical Sciences (18thed.; Mack Publishing Company, Eaton, Pennsylvania, 1990). See, also, for example, WO 98/56418, which describes stabilized antibody pharmaceutical formulations suitable for use in the methods disclosed herein.

The antagonist anti-CD40 antibodies or antigen-bidning fragments thereof present in the pharmaceutical compositions described herein for use in the methods disclosed herein may be native or obtained by recombinant techniques, and may be from any source, including mammalian sources such as, e.g., mouse, rat, rabbit, primate, pig, and human. Preferably such polypeptides are derived from a human source, and more preferably are recombinant, human proteins from hybridoma cell lines.

Any pharmaceutical composition comprising an antagonist anti-CD40 antibody or antigen-binding fragment thereof having the binding properties described herein as the therapeutically active component can be used in the methods disclosed herein. Thus liquid, lyophilized, or spray-dried compositions comprising one or more of the antagonist anti-CD40 antibodies of the invention, or antigen-binding fragment thereof, may be prepared as an aqueous or nonaqueous solution or suspension for subsequent administration to a subject in accordance with the methods disclosed herein. Each of these compositions will comprise at least one of the antagonist anti-CD40 antibodies of the present invention, or antigen-binding fragment thereof, as a therapeutically or prophylactically active component. By "therapeutically or prophylactically active component" is intended the anti-CD40 antibody or antigen-binding fragment thereof is specifically incorporated into the composition to bring about a desired therapeutic or prophylactic response with regard to treatment, prevention, or diagnosis of a disease or condition within a subject when the pharmaceutical composition is administered to that subject. Preferably the pharmaceutical compositions comprise appropriate stabilizing agents, bulking agents, or both to minimize problems associated with loss of protein stability and biological activity during preparation and storage.

Formulants may be added to pharmaceutical compositions comprising an antagonist anti-CD40 antibody of the invention or antigen-binding fragment thereof. These formulants may include, but are not limited to, oils, polymers, vitamins, carbohydrates, amine acids, salts, buffers, albumin, surfactants, or bulking agents. Preferably carbohydrates include sugar or sugar alcohols such as mono-, di-, or polysaccharides, or water soluble glucans. The saccharides or glucans can include fructose, glucose, mannose, sorbose, xylose, maltose, sucrose, dextran, pullulan, dextrin, $\alpha$ and $\beta$ cyclodextrin, soluble starch, hydroxyethyl starch, and carboxymethylcellulose, or mixtures thereof. "Sugar alcohol" is defined as a $C_4$ to $C_8$ hydrocarbon having a hydroxyl group and includes galactitol, inositol, mannitol, xylitol, sorbitol, glycerol, and arabitol. These sugars or sugar alcohols may be used individually or in combination. The sugar or sugar alcohol concentration is between 1.0% and 7% w/v., more preferably between 2.0% and 6.0% w/v. Preferably amino acids include levorotary (L) forms of carnitine, arginine, and betaine; however, other amino acids may be added. Preferred polymers include polyvinylpyrrolidone (PVP) with an average molecular weight between 2,000 and 3,000, or polyethylene glycol (PEG) with an average molecular weight between 3,000 and 5,000. Surfactants that can be added to the formulation are shown in EP Nos. 270,799 and 268,110.

Additionally, antibodies can be chemically modified by covalent conjugation to a polymer to increase their circulating half-life, for example. Preferred polymers, and methods to attach them to peptides, are shown in U.S. Patent Nos. 4,766,106; 4,179,337; 4,495,285; and 4,609,546. Preferred polymers are polyoxyethylated polyols and polyethylene glycol (PEG). PEG is soluble in water at room temperature and has the general formula: $R(O-CH_2 --CHO_2)_n$ O--R where R can be hydrogen, or a protective group such as an alkyl or alkanol group. Preferably, the protective group has between 1 and 8 carbons, more preferably it is methyl. The symbol n is a positive integer, preferably between 1 and 1,000, more preferably between 2 and 500. The PEG has a preferred average molecular weight between 1,000 and 40,000, more preferably between 2,000 and 20,000, most preferably between 3,000 and 12,000. Preferably, PEG has at least one hydroxy group, more preferably it is a terminal hydroxy group. It is this hydroxy group which is preferably activated to react with a free amino group on the inhibitor. However, it will be understood that the type and amount of the reactive groups may be varied to achieve a covalently conjugated PEG/antibody of the present invention.

Water-soluble polyoxyethylated polyols are also useful in the present invention. They include polyoxyethylated sorbitol, polyoxyethylated glucose, polyoxyethylated glycerol (POG), and the like. POG is preferred. One reason is because the glycerol backbone of polyoxyethylated glycerol is the same backbone occurring naturally in, for example, animals and humans in mono-, di-, triglycerides. Therefore, this branching would not necessarily be seen as a foreign agent in the body. The POG has a preferred molecular weight in the same range as PEG The structure for POG is shown in Knauf et al. (1988) J. Bio. Chem. 263:15064-15070, and a discussion of POG/IL-2 conjugates is found in U.S. Patent No. 4,766,106.

Another drug delivery system for increasing circulatory half-life is the liposome. Methods of preparing liposome delivery systems are discussed in Gabizon et al. (1982) Cancer Research 42:4734; Cafiso (1981) Biochem Biophys Acta 649:129; and Szoka (1980) Ann. Rev. Biophys. Eng. 9:467. Other drug delivery systems are known in the art and are described in, *e.g.,* Poznansky et al. (1980) Drug Delivery Systems (R.L. Juliano, ed., Oxford, N.Y.) pp. 253-315; Poznansky (1984) Pharm Revs 36:277.

The formulants to be incorporated into a pharmaceutical composition should provide for the stability of the antagonist anti-CD40 antibody or antigen-binding fragment thereof. That is, the antagonist anti-CD40 antibody or antigen-binding fragment thereof should retain its physical and/or chemical stability and have the desired biological activity, i.e., one or more of the antagonist activities defined herein above, including, but not limited to, inhibition of immunoglobulin secretion by normal human peripheral B cells stimulated by T cells; inhibition of survival and/or proliferation of normal human peripheral B cells stimulated by Jurkat T cells; inhibition of survival and/or proliferation of normal human peripheral B cells stimulated by CD40L-expressing cells or soluble CD40 ligand (sCD40L); inhibition of "survival" anti-apoptotic intracellular signals in any cell stimulated by sCD40L or solid-phase CD40L; inhibition of CD40 signal transduction in any cell upon ligation with sCD40L or solid-phase CD40L; and inhibition of proliferation of human malignant B cells as noted elsewhere herein.

Methods for monitoring protein stability are well known in the art. See, for example, Jones (1993) Adv. Drug Delivery Rev. 10:29-90; Lee, ed. (1991) Peptide and Protein Drug Delivery (Marcel Dekker, Inc., New York, New York); and the stability assays disclosed herein below. Generally, protein stability is measured at a chosen temperature for a specified period of time. In preferred embodiments, a stable antibody pharmaceutical formulation provides for stability of the antagonist anti-CD40 antibody or antigen-binding fragment thereof when stored at room temperature (about 25°C) for at least 1 month, at least 3 months, or at least 6 months, and/or is stable at about 2-8°C for at least 6 months, at least 9 months, at least 12 months, at least 18 months, at least 24 months.

A protein such as an antibody, when formulated in a pharmaceutical composition, is considered to retain its physical stability at a given point in time if it shows no visual signs (i.e., discoloration or loss of clarity) or measurable signs (for example, using size-exclusion chromatography (SEC) or UV light scattering) of precipitation, aggregation, and/or denaturation in that pharmaceutical composition. With respect to chemical stability, a protein such as an antibody, when formulated in a pharmaceutical composition, is considered to retain its chemical stability at a given point in time if measurements of chemical stability are indicative that the protein (i.e., antibody) retains the biological activity of interest in that pharmaceutical composition. Methods for monitoring changes in chemical stability are well known in the art and include, but are not limited to, methods to detect chemically altered forms of the protein such as result from clipping, using, for example, SDS-PAGE, SEC, and/or matrix-assisted laser desorption ionization/time of flight mass spectrometry; and degradation associated with changes in molecular charge (for example, associated with deamidation), using, for example, ion-exchange chromatography. See, for example, the methods disclosed herein below.

An antagonist anti-CD40 antibody or antigen-binding fragment thereof, when formulated in a pharmaceutical composition, is considered to retain a desired biological activity at a given point in time if the desired biological activity at that time is within about 30%, preferably within about 20% of the desired biological activity exhibited at the time the pharmaceutical composition was prepared as determined in a suitable assay for the desired biological activity. Assays for measuring the desired biological activity of the antagonist anti-CD40 antibodies disclosed herein, and antigen-binding fragments thereof, can be performed as described in the Examples herein. See also the assays described in Schultze et al. (1998) Proc. Natl. Acad. Sci. USA 92:8200-8204; Denton et al. (1998) Pediatr. Transplant. 2:6-15; Evans et al. (2000) J. Immunol. 164:688-697; Noelle (1998) Agents Actions Suppl. 49:17-22; Lederman et al. (1996) Curr. Opin. Hematol. 3:77-86; Coligan et al. (1991) Current Protocols in Immunology 13:12; Kwekkeboom et al. (1993) Immunology 79:439-444; and U.S. Patent Nos. 5,674,492 and 5,847,082.

In some embodiments of the invention, the antagonist anti-CD40 antibody, for example, the CHIR-12.12 or CHIR-5.9 monoclonal antibody, or antigen-binding fragment thereof is formulated in a liquid pharmaceutical formulation. The antagonist anti-CD40 antibody or antigen binding fragment thereof can be prepared using any method known in the art, including those methods disclosed herein above. In one embodiment, the antagonist anti-CD40 antibody, for example, the CHIR-12.12 or CHIR-5.9 monoclonal antibody, or antigen-binding fragment thereof is recombinantly produced in a CHO cell line.

Following its preparation and purification, the antagonist anti-CD40 antibody or antigen-binding fragment thereof can be formulated as a liquid pharmaceutical formulation in the manner set forth herein. Where the antagonist anti-CD40 antibody or antigen-binding fragment thereof is to be stored prior to its formulation, it can be frozen, ro example, at ≤20°C, and then thawed at room temperature for further formulation. The liquid pharmaceutical formulation comprises a therapeutically effective amount of the antagonist anti-CD40 antibody or antigen-binding fragment thereof. The amount of antibody or antigen-binding fragment thereof present in the formulation takes into consideration the route of administration and desired dose volume.

In this manner, the liquid pharmaceutical composition comprises the antagonist anti-CD40 antibody, for example, the CHIR-12.12 or CHIR-5.9 antibody, or antigen-binding fragment thereof at a concentration of about 0.1 mg/ml to about 50.0 mg/ml, about 0.5 mg/ml to about 40.0 mg/ml, about 1.0 mg/ml to about 30.0 mg/ml, about 5.0 mg/ml to about 25.0 mg/ml, about 5.0 mg/ml to about 20.0 mg/ml, or about 15.0 mg/ml to about 25.0 mg/ml. In some embodiments, the liquid pharmaceutical composition comprises the antagonist anti-CD40 antibody or antigen-binding fragment thereof at a concentration of about 0.1 mg/ml to about 5.0 mg/ml, about 5.0 mg/ml to about 10.0 mg/ml, about 10.0 mg/ml to about 15.0 mg/ml, about 15.0 mg/ml to about 20.0 mg/ml, about 20.0 mg/ml to about 25.0 mg/ml, about 25.0 mg/ml to about 30.0 mg/ml, about 30.0 mg/ml to about 35.0 mg/ml, about 35.0 mg/ml to about 40.0 mg/ml, about 40.0 mg/ml to about 45.0 mg/ml, or about 45.0 mg/ml to about 50.0 mg/ml. In other embodiments, the liquid pharmaceutical composition comprises the antagonist anti-CD40 antibody or antigen-binding fragment thereof at a concentration of about 15.0 mg/ml, about 16.0 mg/ml, about 17.0 mg/ml, about 18.0 mg/ml, about 19.0 mg/ml, about 20.0 mg/ml, about 21.0 mg/ml, about 22.0 mg/ml, about 23.0 mg/ml, about 24.0 mg/ml, or about 25.0 mg/ml. The liquid pharmaceutical composition comprises the antagonist anti-CD40 antibody, for example, the CHIR-12.12 or CHIR-5.9 antibody, or antigen-binding fragment thereof and a buffer that maintains the pH of the formulation in the range of about pH 5.0 to about pH 7.0, including about pH 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, and other such values within the range of about pH 5.0 to about pH 7.0. In some embodiments, the buffer maintains the pH of the formulation in the range of about pH 5.0 to about pH 6.5, about pH 5.0 to about pH 6.0, about pH 5.0 to about pH 5.5, about pH 5.5 to about 7.0, about pH 5.5 to about pH 6.5, or about pH 5.5 to about pH 6.0.

Any suitable buffer that maintains the pH of the liquid anti-CD40 antibody formulation in the range of about pH 5.0 to about pH 7.0 can be used in the formulation, so long as the physicochemical stability and desired biological activity of the antibody are retained as noted herein above. Suitable buffers include, but are not limited to, conventional acids and salts thereof, where the counter ion can be, for example, sodium, potassium, ammonium, calcium, or magnesium. Examples of conventional acids and salts thereof that can be used to buffer the pharmaceutical liquid formulation include, but are not limited to, succinic acid or succinate, citric acid or citrate, acetic acid or acetate, tartaric acid or tartarate, phosphoric acid or phosphate, gluconic acid or gluconate, glutamic acid or glutamate, aspartic acid or aspartate, maleic acid or maleate, and malic acid or malate buffers. The buffer concentration within the formulation can be from about 1 mM to about 50 mM, including about 1 mM, 2 mM, 5 mM, 8 mM, 10 mM, 15 mM, 20 mM, 25 mM, 30 mM, 35 mM, 40 mM, 45 mM, 50 mM, or other such values within the range of about 1 mM to about 50 mM. In some embodiments, the buffer concentration within the formulation is from about 5 mM to about 15 mM, including about 5 mM, 6 mM, 7 mM, 8 mM, 9 mM, 10 mM, 11 mM, 12 mM, 13 mM, 14 mM, 15 mM, or other such values within the range of about 5 mM to about 15 mM.

In some embodiments of the invention, the liquid pharmaceutical formulation comprises a therapeutically effective amount of the antagonist anti-CD40 antibody, for example, the CHIR-12.12 or CHIR-5.9 monoclonal antibody, or antigen-binding fragment thereof and succinate buffer or citrate buffer at a concentration that maintains the pH of the formulation in the range of about pH 5.0 to about pH 7.0, preferably about pH 5.0 to about pH 6.5. By "succinate buffer" or "citrate buffer" is intended a buffer comprising a salt of succinic acid or a salt of citric acid, respectively. In a preferred embodiment, the succinate or citrate counterion is the sodium cation, and thus the buffer is sodium succinate or sodium citrate, respectively. However, any cation is expected to be effective. Other possible succinate or citrate cations include, but are not limited to, potassium, ammonium, calcium, and magnesium. As noted above, the succinate or citrate buffer concentration within the formulation can be from about 1 mM to about 50 mM, including about 1 mM, 2 mM, 5 mM, 8 mM, 10 mM, 15 mM, 20 mM, 25 mM, 30 mM, 35 mM, 40 mM, 45 mM, 50 mM, or other such values within the range of about 1 mM to about 50 mM. In some embodiments, the buffer concentration within the formulation is from about 5 mM to about 15 mM, including about 5 mM, 6 mM, 7 mM, 8 mM, 9 mM, 10 mM, 11 mM, 12 mM, 13 mM, 14 mM, or about 15 mM. In other embodiments, the liquid pharmaceutical formulation comprises the antagonist anti-CD40 antibody, for example, the CHIR-12.12 or CHIR-5.9 monoclonal antibody, or antigen-binding fragment thereof at a concentration of about 0.1 mg/ml to about 50.0 mg/ml, or about 5.0 mg/ml to about 25.0 mg/ml, and succinate or citrate buffer, for example, sodium succinate or sodium citrate buffer, at a concentration of about 1 mM to about 20 mM, about 5 mM to about 15 mM, preferably about 10 mM.

Where it is desirable for the liquid pharmaceutical formulation to be near isotonic, the liquid pharmaceutical formulation comprising a therapeutically effective amount of the antagonist anti-CD40 antibody, for example, the CHIR-12.12 or CHIR-5.9 monoclonal antibody, or antigen-binding fragment thereof, and a buffer to maintain the pH of the formulation within the range of about pH 5.0 to about pH 7.0 can further comprise an amount of an isotonizing agent sufficient to render the formulation near isotonic. By "near isotonic" is intended the aqueous formulation has an osmolarity of about 240 mmol/kg to about 360 mmol/kg, preferably about 240 to about 340 mmol/kg, more preferably about 250 to about 330 mmol/kg, even more preferably about 260 to about 320 mmol/kg, still more preferably about 270 to about 310 mmol/kg. Methods of determining the isotonicity of a solution are known to those skilled in the art. See, for example, Setnikar et al. (1959) J. Am. Pharm. Assoc. 48:628.

Those skilled in the art are familiar with a variety of pharmaceutically acceptable solutes useful in providing isotonicity in pharmaceutical compositions. The isotonizing agent can be any reagent capable of adjusting the osmotic pressure of the liquid pharmaceutical formulation of the present invention to a value nearly equal to that of a body fluid. It is desirable to use a physiologically acceptable isotonizing agent. Thus, the liquid pharmaceutical formulation comprising a therapeutically effective amount of the antagonist anti-CD40 antibody, for example, the CHIR-12.12 or CHIR-5.9 monoclonal antibody, or antigen-binding fragment thereof, and a buffer to maintain the pH of the formulation within the range of about pH 5.0 to about pH 7.0, can further comprise components that can be used to provide isotonicity, for example, sodium chloride; amino acids such as alanine, valine, and glycine; sugars and sugar alcohols (polyols), including, but not limited to, glucose, dextrose, fructose, sucrose, maltose, mannitol, trehalose, glycerol, sorbitol, and xylitol; acetic acid, other organic acids or their salts, and relatively minor amounts of citrates or phosphates. The ordinary skilled person would know of additional agents that are suitable for providing optimal tonicity of the liquid formulation.

In some preferred embodiments, the liquid pharmaceutical formulation comprising a therapeutically effective amount of the antagonist anti-CD40 antibody, for example, the CHIR-12.12 or CHIR-5.9 monoclonal antibody, or antigen-binding fragment thereof, and a buffer to maintain the pH of the formulation within the range of about pH 5.0 to about pH 7.0, further comprises sodium chloride as the isotonizing agent. The concentration of sodium chloride in the formulation will depend upon the contribution of other components to tonicity. In some embodiments, the concentration of sodium chloride is about 50 mM to about 300 mM, about 50 mM to about 250 mM, about 50 mM to about 200 mM, about 50 mM to about 175 mM, about 50 mM to about 150 mM, about 75 mM to about 175 mM, about 75 mM to about 150 mM, about 100 mM to about 175 mM, about 100 mM to about 200 mM, about 100 mM to about 150 mM, about 125 mM to about 175 mM, about 125 mM to about 150 mM, about 130 mM to about 170 mM, about 130 mM to about 160 mM, about 135 mM to about 155 mM, about 140 mM to about 155 mM, or about 145 mM to about 155 mM. In one such embodiment, the concentration of sodium chloride is about 150 mM. In other such embodiments, the concentration of sodium chloride is about 150 mM, the buffer is sodium succinate or sodium citrate buffer at a concentration of about 5 mM to about 15 mM, the liquid pharmaceutical formulation comprises a therapeutically effective amount of the antagonist anti-CD40 antibody, for example, the CHIR-12.12 or CHIR-5.9 monoclonal antibody, or antigen-binding fragment thereof, and the formulation has a pH of about pH 5.0 to about pH 7.0, about pH 5.0 to about pH 6.0, or about pH 5.5 to about pH 6.5. In other embodiments, the liquid pharmaceutical formulation comprises the antagonist anti-CD40 antibody, for example, the CHIR-12.12 or CHIR-5.9 monoclonal antibody, or antigen-binding fragment thereof, at a concentration of about 0.1 mg/ml to about 50.0 mg/ml or about 5.0 mg/ml to about 25.0 mg/ml, about 150 mM sodium chloride, and about 10 mM sodium succinate or sodium citrate, at a pH of about pH 5.5.

Protein degradation due to freeze thawing or mechanical shearing during processing of a liquid pharmaceutical formulations of the present invention can be inhibited by incorporation of surfactants into the formulation in order to lower the surface tension at the solution-air interface. Thus, in some embodiments, the liquid pharmaceutical formulation comprises a therapeutically effective amount of the antagonist anti-CD40 antibody, for example, the CHIR-12.12 or CHIR-5.9 monoclonal antibody, or antigen-binding fragment thereof, a buffer to maintain the pH of the formulation within the range of about pH 5.0 to about pH 7.0, and further comprises a surfactant. In other embodiments, the liquid pharmaceutical formulation comprises a therapeutically effective amount of the antagonist anti-CD40 antibody, for example, the CHIR-12.12 or CHIR-5.9 monoclonal antibody, or antigen-binding fragment thereof, a buffer to maintain the pH of the formulation within the range of about pH 5.0 to about pH 7.0, an isotonizing agent such as sodium chloride at a concentration of about 50 mM to about 300 mM, and further comprises a surfactant.

Typical surfactants employed are nonionic surfactants, including polyoxyethylene sorbitol esters such as polysorbate 80 (Tween 80) and polysorbate 20 (Tween 20); polyoxypropylene-polyoxyethylene esters such as Pluronic F68; polyoxyethylene alcohols such as Brij 35; simethicone; polyethylene glycol such as PEG400; lysophosphatidylcholine; and polyoxyethylene-p-t-octylphenol such as Triton X-100. Classic stabilization of pharmaceuticals by surfactants or emulsifiers is described, for example, in Levine et al. (1991) J. Parenteral Sci. Technol. 45(3):160-165. A preferred surfactant employed in the practice of the present invention is polysorbate 80. Where a surfactant is included, it is typically added in an amount from about 0.001 % to about 1.0% (w/v), about 0.001% to about 0.5%, about 0.00 1 % to about 0.4%, about 0.001% to about 0.3%, about 0.001 % to about 0.2%, about 0.005% to about 0.5%, about 0.005% to about 0.2%, about 0.01% to about 0.5%, about 0.01 % to about 0.2%, about 0.03% to about 0.5%, about 0.03% to about 0.3%, about 0.05% to about 0.5%, or about 0.05% to about 0.2%.

Thus, in some embodiments, the liquid pharmaceutical formulation comprises a therapeutically effective amount of the antagonist anti-CD40 antibody, for example, the CHIR-12.12 or CHIR-5.9 monoclonal antibody, or antigen-binding fragment thereof, the buffer is sodium succinate or sodium citrate buffer at a concentration of about 1 mM to about 50 mM, about 5 mM to about 25 mM, or about 5 mM to about 15 mM; the formulation has a pH of about pH 5.0 to about pH 7.0, about pH 5.0 to about pH 6.0, or about pH 5.5 to about pH 6.5; and the formulation further comprises a surfactant, for example, polysorbate 80, in an amount from about 0.001 % to about 1.0% or about 0.001% to about 0.5%. Such formulations can optionally comprise an isotonizing agent, such as sodium chloride at a concentration of about 50 mM to about 300 mM, about 50 mM to about 200 mM, or about 50 mM to about 150 mM. In other embodiments, the liquid pharmaceutical formulation comprises the antagonist anti-CD40 antibody, for example, the CHIR-12.12 or CHIR-5.9 monoclonal antibody, or antigen-binding fragment thereof, at a concentration of about 0.1 mg/ml to about 50.0 mg/ml or about 5.0 mg/ml to about 25.0 mg/ml, including about 20.0 mg/ml; about 50 mM to about 200 mM sodium chloride, including about 150 mM sodium chloride; sodium succinate or sodium citrate at about 5 mM to about 20 mM, including about 10 mM sodium succinate or sodium citrate; sodium chloride at a concentration of about 50 mM to about 200 mM, including about 150 mM; and optionally a surfactant, for example, polysorbate 80, in an amount from about 0.001 % to about 1.0%, including about 0.001 % to about 0.5%; where the liquid pharmaceutical formulation has a pH of about pH 5.0 to about pH 7.0, about pH 5.0 to about pH 6.0, about pH 5.0 to about pH 5.5, about pH 5.5 to about pH 6.5, or about pH 5.5 to about pH 6.0.

The liquid pharmaceutical formulation can be essentially free of any preservatives and other carriers, excipients, or stabilizers noted herein above. Alternatively, the formulation can include one or more preservatives, for example, antibacterial agents, pharmaceutically acceptable carriers, excipients, or stabilizers described herein above provided they do not adversely affect the physicochemical stability of the antagonist anti-CD40 antibody or antigen-binding fragment thereof. Examples of acceptable carriers, excipients, and stabilizers include, but are not limited to, additional buffering agents, cosolvents, surfactants, antioxidants including ascorbic acid and methionine, chelating agents such as EDTA, metal complexes (for example, Zn-protein complexes), and biodegradable polymers such as polyesters. A thorough discussion of formulation and selection of pharmaceutically acceptable carriers, stabilizers, and isomolytes can be found in Remington's Pharmaceutical Sciences (18th ed.; Mack Publishing Company, Eaton, Pennsylvania, 1990).

After the liquid pharmaceutical formulation or other pharmaceutical composition described herein is prepared, it can be lyophilized to prevent degradation. Methods for lyophilizing liquid compositions are known to those of ordinary skill in the art. Just prior to use, the composition may be reconstituted with a sterile diluent (Ringer's solution, distilled water, or sterile saline, for example) that may include additional ingredients. Upon reconstitution, the composition is preferably administered to subjects using those methods that are known to those skilled in the art.

Use of Antagonist Anti-CD40 Antibodies in the Manufacture of Medicaments

The present invention also provides for the use of an antagonist anti-CD40 antibody or antigen-binding fragment thereof in the manufacture of a medicament for treating a CD40-expressing cancer in a subject, wherein the medicament is coordinated with treatment with IL-2 or biologically active variant thereof. By "CD40-expressing cancer" is intended any cancer that comprises neoplastic cells expressing the CD40 cell surface antigen. As noted herein above, such cancers include, but are not limited to, B cell-related cancers, for example, non-Hodgkin's lymphoma, chronic lymphocytic leukemia, multiple myeloma, B cell lymphoma, high-grade B cell lymphoma, intermediate-grade B cell lymphoma, low-grade B cell lymphoma, B cell acute lympohoblastic leukemia, myeloblastic leukemia, Hodgkin's disease, plasmacytoma, follicular lymphoma, follicular small cleaved lymphoma, follicular large cell lymphoma, follicular mixed small cleaved lymphoma, diffuse small cleaved cell lymphoma, diffuse small lymphocytic lymphoma, prolymphocytic leukemia, lymphoplasmacytic lymphoma, marginal zone lymphoma, mucosal associated lymphoid tissue lymphoma, monocytoid B cell lymphoma, splenic lymphoma, hairy cell leukemia, diffuse large cell lymphoma, mediastinal large B cell lymphoma, lymphomatoid granulomatosis, intravascular lymphomatosis, diffuse mixed cell lymphoma, diffuse large cell lymphoma, immunoblastic lymphoma, Burkitt's lymphoma, AIDS-related lymphoma, and mantle cell lymphoma; and solid tumors expressing the CD40 antigen, including, but not limited to, ovarian, lung (for example, non-small cell lung cancer of the squamous cell carcinoma, adenocarcinoma, and large cell carcinoma types, and small cell lung cancer), breast, colon, kidney (including, for example, renal cell carcinomas), bladder, liver (including, for example, hepatocellular carcinomas), gastric, cervical, prostate, nasopharyngeal, thyroid (for example, thyroid papillary carcinoma), and skin cancers such as melanoma, and sarcomas (including, for example, osteosarcomas and Ewing's sarcomas).

By "coordinated" is intended the medicament comprising the antagonist anti-CD40 antibody or antigen-binding fragment thereof is to be used either prior to, during, or after treatment of the subject with IL-2 or biologically active variant thereof. In one such embodiment, the present invention provides for the use of the monoclonal antibody CHIR-12.12 or CHIR-5.9 in the manufacture of a medicament for treating a CD40-expressing cancer in a subject, wherein the medicament is coordinated with treatment with IL-2 or biologically active variant thereof, for example, human IL-2 or the des-alanyl-1, serine-125 human IL-2 mutein, wherein the medicament is to be used either prior to, during, or after treatment of the subject with IL-2 or biologically active variant thereof.

In some embodiments, the medicament comprising the antagonist anti-CD40 antibody, for example, the monoclonal antibody CHIR-12.12 or CHIR-5.9 disclosed herein, or antigen-binding fragment thereof is coordinated with treatment with IL-2 or biologically active variant thereof and at least one other type of cancer therapy. Examples of other cancer therapies include, but are not limited to, those described herein above, i.e., surgery or surgical procedures (e.g. splenectomy, hepatectomy, lymphadenectomy, leukophoresis, bone marrow transplantation, and the like); radiation therapy; chemotherapy, optionally in combination with autologous bone marrow transplant, where suitable chemotherapeutic agents include, but are not limited to, fludarabine or fludarabine phosphate, chlorambucil, vincristine, pentostatin, 2-chlorodeoxyadenosine (cladribine), cyclophosphamide, doxorubicin, prednisone, and combinations thereof, for example, anthracycline-containing regimens such as CAP (cyclophosphamide, doxorubicin plus prednisone), CHOP (cyclophosphamide, vincristine, prednisone plus doxorubicin), VAD (vincritsine, doxorubicin, plus dexamethasone), MP (melphalan plus prednisone), and other cytotoxic and/or therapeutic agents used in chemotherapy such as mitoxantrone, daunorubicin, idarubicin, asparaginase, and antimetabolites, including, but not limited to, cytarabine, methotrexate, 5-fluorouracil decarbazine, 6-thioguanine, 6-mercaptopurine, and nelarabine; other anti-cancer monoclonal antibody therapy (for example, alemtuzumab (Campath®) or other anti-CD52 antibody targeting the CD52 cell-surface glycoprotein on malignant B cells; rituximab (Rituxan®), the fully human antibody HuMax-CD20, R-1594, IMMU-106, TRU-015, AME-133, tositumomab/I-131 tositumomab (Bexxar®), ibritumomab tiuxetan (Zevalin®), or any other therapeutic anti-CD20 antibody targeting the CD20 antigen on malignant B cells; anti-CD19 antibody (for example, MT103, a bispecific antibody); anti-CD22 antibody (for example, the humanized monoclonal antibody epratuzumab); bevacizumab (Avastin®) or other anti-cancer antibody targeting human vascular endothelial growth factor; anti-CD22 antibody targeting the CD22 antigen on malignant B cells (for example, the monoclonal antibody BL-22, an alphaCD22 toxin); α-M-CSF antibody targeting macrophage colony stimulating factor; antibodies targeting the receptor activator of nuclear factor-kappaB (RANK) and its ligand (RANKL), which are overexpressed in multiple myeloma; anti-CD23 antibody targeting the CD23 antigen on malignant B cells (for example, IDEC-152); anti-CD80 antibody targeting the CD80 antigen (for example, IDEC-114); anti-CD38 antibody targeting the CD38 antigen on malignant B cells; antibodies targeting major histocompatibility complex class II receptors (anti-MHC antibodies) expressed on malignant B cells; other anti-CD40 antibodies (for example, SGN-40) targeting the CD40 antigen on malignant B cells; and antibodies targeting tumor necrosis factor-related apoptosis-inducing ligand receptor 1 (TRAIL-R1) (for example, the agonistic human monoclonal antibody HGS-ETR1) and TRAIL-R2 expressed on a number of solid tumors and tumors of hematopoietic origin); small molecule-based cancer therapy, including, but not limited to, microtubule and/or topoisomerase inhibitors (for example, the mitotic inhibitor dolastatin and dolastatin analogues; the tubulin-binding agent T900607; XL119; and the topoisomerase inhibitor aminocamptothecin), SDX-105 (bendamustine hydrochloride), ixabepilone (an epothilone analog, also referred to as BMS- 247550), protein kinase C inhibitors, for example, midostaurin ((PKC-412, CGP 41251, N-benzoylstaurosporine), pixantrone, eloxatin (an antineoplastic agent), ganite (gallium nitrate), Thalomid® (thalidomide), immunomodulatory derivatives of thalidomide (for example, revlimid (formerly revimid)), Affinitak™ (antisense inhibitor of protein kinase C-alpha), SDX-101 (R-etodolac, inducing apoptosis of malignant lymphocytes), second-generation purine nucleoside analogs such as clofarabine, inhibitors of production of the protein Bcl-2 by cancer cells (for example, the antisense agents oblimersen and Genasense®), proteasome inhibitors (for example, Velcade™ (bortezomib)), small molecule kinase inhibitors (for example, CHIR-258), small molecule VEGF inhibitors (for example, ZD-6474), small molecule inhibitors of heat shock protein (HSP) 90 (for example, 17-AAG), small molecule inhibitors of histone deacetylases (for example, hybrid/polar cytodifferentiation HPC) agents such as suberanilohydroxamic acid (SAHA), and FR-901228) and apoptotic agents such as Trisenox® (arsenic trioxide) and Xcytrin® (motexafin gadolinium); vaccine /immunotherapy-based cancer therapies, including, but not limited to, vaccine approaches (for example, Id-KLH, oncophage, vitalethine), personalized immunotherapy or active idiotype immunotherapy (for example, MyVax® Personalized Immunotherapy, formally designated GTOP-99), Promune® (CpG 7909, a synthetic agonist for toll-like receptor 9 (TLR9)), interferon-alpha therapy, IL-12 therapy, IL-15 therapy, and IL-21 therapy; steroid therapy; or other cancer therapy; where treatment with the IL-2 or biologically active variant thereof and the additional cancer therapy, or additional cancer therapies, occurs prior to, during, or subsequent to treatment of the subject with the medicament comprising the antagonist anti-CD40 antibody or antigen-binding fragment thereof, as noted herein above. Where the medicament comprising the antagonist anti-CD40 antibody or antigen-binding fragment thereof is coordinated with IL-2 or biologically active variant thereof and at least one other cancer therapy, use of the medicament can be prior to, during, or after treatment of the subject with either or both of the other cancer therapies.

The present invention also provides for the use of a synergistic combination of an antagonist anti-CD40 antibody or antigen-binding fragment thereof in the manufacture of a medicament for treating a CD40-expressing cancer in a subject, as defined herein above, wherein the medicament is coordinated with treatment with IL-2 or biologically active variant thereof. By "synergistic combination" is intended the medicament comprises an amount of the antagonist anti-CD40 antibody or antigen-binding fragment thereof that provides for a synergistic therapeutic effect when the medicament is coordinated with treatment with IL-2 or biologically active variant thereof in the manner set forth herein above. "Synergistic therapeutic effect" refers to a therapeutic effect observed with a combination of two or more therapies (in this case, the antagonist anti-CD40 antibody therapy and IL-2 therapy) wherein the therapeutic effect (as measured by any of a number of parameters, including the measures of efficacy described herein above) is greater than the sum of the respective individual therapeutic effects observed with the respective individual therapies.

In one such embodiment, the present invention provides for the use of a synergistic combination of the monoclonal antibody CHIR-12.12 or CHIR-5.9 in the manufacture of a medicament for treating a CD40-expressing cancer in a subject, wherein the medicament is coordinated with treatment with IL-2 or biologically active variant thereof, for example, human IL-2 or the des-alanyl-1, serine-125 human IL-2 mutein, wherein the medicament is to be used either prior to, during, or after treatment of the subject with IL-2 or biologically active variant thereof. In some embodiments, the medicament comprising the synergistic combination of the antagonist anti-CD40 antibody, for example, the monoclonal antibody CHIR-12.12 or CHIR-5.9 disclosed herein, or antigen-binding fragment thereof is coordinated with treatment with IL-2 or biologically active variant thereof and at least one other type of cancer therapy as noted herein above.

The invention also provides for the use of an antagonist anti-CD40 antibody, for example, the monoclonal antibody CHIR-12.12 or CHIR-5.9 disclosed herein, or antigen-binding fragment thereof in the manufacture of a medicament for treating a CD40-expressing cancer in a subject, as defined herein above, wherein the medicament is used in a subject that has been pretreated with IL-2 or biologically active variant thereof. By "pretreated" or "pretreatment" is intended the subject has received IL-2 therapy (i.e., been treated with IL-2 or biologically active variant thereof) prior to receiving the medicament comprising the antagonist anti-CD40 antibody or antigen-binding fragment thereof. "Pretreated" or "pretreatment" includes subjects that have been treated with IL-2 or biologically active variant thereof, alone or in combination with other cancer therapies, within 2 years, within 18 months, within 1 year, within 6 months, within 2 months, within 6 weeks, within 1 month, within 4 weeks, within 3 weeks, within 2 weeks, within 1 week, within 6 days, within 5 days, within 4 days, within 3 days, within 2 days, or even within 1 day prior to initiation of treatment with the medicament comprising the antagonist anti-CD40 antibody, for example, the monoclonal antibody CHIR-12.12 or CHIR-5.9 disclosed herein, or antigen-binding fragment thereof. It is not necessary that the subject was a responder to pretreatment with the prior IL-2 therapy, or prior IL-2 and other cancer therapies. Thus, the subject that receives the medicament comprising the antagonist anti-CD40 antibody or antigen-binding fragment thereof could have responded, or could have failed to respond, to pretreatment with the prior IL-2 therapy, or to one or more of the prior cancer therapies where pretreatment comprised multiple cancer therapies one of which was IL-2 therapy, for example, IL-2 therapy and other anti-cancer antibody therapy, for example, rituximab or other anti-CD20 antibody therapy; IL-2 therapy and surgery; IL-2 therapy and chemotherapy; or IL-2 therapy, chemotherapy, and other anti-cancer antibody therapy.

Thus, in some embodiments, the invention provides for the use of an antagonist anti-CD40 antibody, for example the monoclonal antibody CHIR-12.12 or CHIR-5.9 disclosed herein, or antigen-binding fragment thereof in the manufacture of a medicament that is used in a subject in need of treatment for a CD40-expressing cancer defined herein above, where the subject has been pretreated with IL-2 therapy, or has been pretreated with IL-2 therapy and one or more of the following other cancer therapies: surgery or surgical procedures (e.g. splenectomy, hepatectomy, lymphadenectomy, leukophoresis, bone marrow transplantation, and the like); radiation therapy; chemotherapy, optionally in combination with autologous bone marrow transplant, where suitable chemotherapeutic agents include, but are not limited to, fludarabine or fludarabine phosphate, chlorambucil, vincristine, pentostatin, 2-chlorodeoxyadenosine (cladribine), cyclophosphamide, doxorubicin, prednisone, and combinations thereof, for example, anthracycline-containing regimens such as CAP (cyclophosphamide, doxorubicin plus prednisone), CHOP (cyclophosphamide, vincristine, prednisone plus doxorubicin), VAD (vincritsine, doxorubicin, plus dexamethasone), MP (melphalan plus prednisone), and other cytotoxic and/or therapeutic agents used in chemotherapy such as mitoxantrone, daunorubicin, idarubicin, asparaginase, and antimetabolites, including, but not limited to, cytarabine, methotrexate, 5-fluorouracil decarbazine, 6-thioguanine, 6-mercaptopurine, and nelarabine; other anti-cancer monoclonal antibody therapy (for example, alemtuzumab (Campath®) or other anti-CD52 antibody targeting the CD52 cell-surface glycoprotein on malignant B cells; rituximab (Rituxan®), the fully human antibody HuMax-CD20, R-1594, IMMU-106, TRU-015, AME-133, tositumomab/I-131 tositumomab (Bexxar®), ibritumomab tiuxetan (Zevalin®), or any other therapeutic anti-CD20 antibody targeting the CD20 antigen on malignant B cells; anti-CD 19 antibody (for example, MT103, a bispecific antibody); anti-CD22 antibody (for example, the humanized monoclonal antibody epratuzumab); bevacizumab (Avastin®) or other anti-cancer antibody targeting human vascular endothelial growth factor; anti-CD22 antibody targeting the CD22 antigen on malignant B cells (for example, the monoclonal antibody BL-22, an alphaCD22 toxin); $\alpha$-M-CSF antibody targeting macrophage colony stimulating factor; antibodies targeting the receptor activator of nuclear factor-kappaB (RANK) and its ligand (RANKL), which are overexpressed in multiple myeloma; anti-CD23 antibody targeting the CD23 antigen on malignant B cells (for example, IDEC-152); anti-CD80 antibody targeting the CD80 antigen (for example, IDEC-114); anti-CD38 antibody targeting the CD38 antigen on malignant B cells; antibodies targeting major histocompatibility complex class II receptors (anti-MHC antibodies) expressed on malignant B cells; other anti-CD40 antibodies (for example, SGN-40) targeting the CD40 antigen on malignant B cells; and antibodies targeting tumor necrosis factor-related apoptosis-inducing ligand receptor 1 (TRAIL-R1) (for example, the agonistic human monoclonal antibody HGS-ETR1) and TRAIL-R2 expressed on a number of solid tumors and tumors of hematopoietic origin); small molecule-based cancer therapy, including, but not limited to, microtubule and/or topoisomerase inhibitors (for example, the mitotic inhibitor dolastatin and dolastatin analogues; the tubulin-binding agent T900607; XL119; and the topoisomerase inhibitor aminocamptothecin), SDX-105 (bendamustine hydrochloride), ixabepilone (an epothilone analog, also referred to as BMS-247550), protein kinase C inhibitors, for example, midostaurin ((PKC-412, CGP 41251, N-benzoylstaurosporine), pixantrone, eloxatin (an antineoplastic agent), ganite (gallium nitrate), Thalomid® (thalidomide), immunomodulatory derivatives of thalidomide (for example, revlimid (formerly revimid)), Affinitak™ (antisense inhibitor of protein kinase C-alpha), SDX-101 (R-etodolac, inducing apoptosis of malignant lymphocytes), second-generation purine nucleoside analogs such as clofarabine, inhibitors of production of the protein Bcl-2 by cancer cells (for example, the antisense agents oblimersen and Genasense®), proteasome inhibitors (for example, Velcade™ (bortezomib)), small molecule kinase inhibitors (for example, CHIR-258), small molecule VEGF inhibitors (for example, ZD-6474), small molecule inhibitors of heat shock protein (HSP) 90 (for example, 17-AAG), small molecule inhibitors of histone deacetylases (for example, hybrid/polar cytodifferentiation HPC) agents such as suberanilohydroxamic acid (SAHA), and FR-901228) and apoptotic agents such as Trisenox® (arsenic trioxide) and Xcytrin® (motexafin gadolinium); vaccine /immunotherapy-based cancer therapies, including, but not limited to, vaccine approaches (for example, Id-KLH, oncophage, vitalethine), personalized immunotherapy or active idiotype immunotherapy (for example, MyVax® Personalized Immunotherapy, formally designated GTOP-99), Promune® (CpG 7909, a synthetic agonist for toll-like receptor 9 (TLR9)), interferon-alpha therapy, IL-12 therapy, IL-15 therapy, and IL-21 therapy; steroid therapy; or other cancer therapy.

The present invention also provides for the use of IL-2 or biologically active variant thereof in the manufacture of a medicament for treating a CD40-expressing cancer in a subject, as defined herein above, wherein the medicament is coordinated with treatment with an antagonist anti-CD40 antibody or antigen binding fragment thereof. In these embodiments, "coordinated" is intended the medicament comprising the IL-2 or biologically active variant thereof is to be used either prior to, during, or after treatment of the subject with the antagonist anti-CD40 antibody or antigen-binding fragment thereof. In one such embodiment, the present invention provides for the use of IL-2 or biologically active variant thereof, for example, human IL-2 or the des-alanyl-1, serine-125 human IL-2 mutein, in the manufacture of a medicament for treating a CD40-expressing cancer in a subject, wherein the medicament is coordinated with treatment with the monoclonal antibody CHIR-12.12 or CHIR-5.9, wherein the medicament is to be used either prior to, during, or after treatment of the subject with the monoclonal antibody CHIR-12.12 or CHIR-5.9.

In some embodiments, the medicament comprising the IL-2 or biologically active variant thereof is coordinated with treatment with an antagonist anti-CD40 antibody, for example, the monoclonal antibody CHIR-12.12 or CHIR-5.9 or antigen-binding fragment thereof, and at least one other type of cancer therapy. Examples of other cancer therapies include, but are not limited to, those described herein above, i.e., surgery or surgical procedures (e.g. splenectomy, hepatectomy, lymphadenectomy, leukophoresis, bone marrow transplantation, and the like); radiation therapy; chemotherapy, optionally in combination with autologous bone marrow transplant, where suitable chemotherapeutic agents include, but are not limited to, fludarabine or fludarabine phosphate, chlorambucil, vincristine, pentostatin, 2-chlorodeoxyadenosine (cladribine), cyclophosphamide, doxorubicin, prednisone, and combinations thereof, for example, anthracycline-containing regimens such as CAP (cyclophosphamide, doxorubicin plus prednisone), CHOP (cyclophosphamide, vincristine, prednisone plus doxorubicin), VAD (vincritsine, doxorubicin, plus dexamethasone), MP (melphalan plus prednisone), and other cytotoxic and/or therapeutic agents used in chemotherapy such as mitoxantrone, daunorubicin, idarubicin, asparaginase, and antimetabolites, including, but not limited to, cytarabine, methotrexate, 5-fluorouracil decarbazine, 6-thioguanine, 6-mercaptopurine, and nelarabine; other anti-cancer monoclonal antibody therapy (for example, alemtuzumab (Campath®) or other anti-CD52 antibody targeting the CD52 cell-surface glycoprotein on malignant B cells; rituximab (Rituxan®), the fully human antibody HuMax-CD20, R-1594, IMMU-106, TRU-015, AME-133, tositumomab/I-131 tositumomab (Bexxar®), ibritumomab tiuxetan (Zevalin®), or any other therapeutic anti-CD20 antibody targeting the CD20 antigen on malignant B cells; anti-CD19 antibody (for example, MT103, a bispecific antibody); anti-CD22 antibody (for example, the humanized monoclonal antibody epratuzumab); bevacizumab (Avastin®) or other anti-cancer antibody targeting human vascular endothelial growth factor; anti-CD22 antibody targeting the CD22 antigen on malignant B cells (for example, the monoclonal antibody BL-22, an alphaCD22 toxin); α-M-CSF antibody targeting macrophage colony stimulating factor; antibodies targeting the receptor activator of nuclear factor-kappaB (RANK) and its ligand (RANKL), which are overexpressed in multiple myeloma; anti-CD23 antibody targeting the CD23 antigen on malignant B cells (for example, IDEC-152); anti-CD80 antibody targeting the CD80 antigen (for example, IDEC-114); anti-CD38 antibody targeting the CD38 antigen on malignant B cells; antibodies targeting major histocompatibility complex class II receptors (anti-MHC antibodies) expressed on malignant B cells; other anti-CD40 antibodies (for example, SGN-40) targeting the CD40 antigen on malignant B cells; and antibodies targeting tumor necrosis factor-related apoptosis-inducing ligand receptor 1 (TRAIL-R1) (for example, the agonistic human monoclonal antibody HGS-ETR1) and TRAIL-R2 expressed on a number of solid tumors and tumors of hematopoietic origin); small molecule-based cancer therapy, including, but not limited to, microtubule and/or topoisomerase inhibitors (for example, the mitotic inhibitor dolastatin and dolastatin analogues; the tubulin-binding agent T900607; XL119; and the topoisomerase inhibitor aminocamptothecin), SDX-105 (bendamustine hydrochloride), ixabepilone (an epothilone analog, also referred to as BMS-247550), protein kinase C inhibitors, for example, midostaurin ((PKC-412, CGP 41251, N-benzoylstaurosporine), pixantrone, eloxatin (an antineoplastic agent), ganite (gallium nitrate), Thalomid® (thalidomide), immunomodulatory derivatives of thalidomide (for example, revlimid (formerly revimid)), Affinitak™ (antisense inhibitor of protein kinase C-alpha), SDX-101 (R-etodolac, inducing apoptosis of malignant lymphocytes), second-generation purine nucleoside analogs such as clofarabine, inhibitors of production of the protein Bcl-2 by cancer cells (for example, the antisense agents oblimersen and Genasense®), proteasome inhibitors (for example, Velcade™ (bortezomib)), small molecule kinase inhibitors (for example, CHIR-258), small molecule VEGF inhibitors (for example, ZD-6474), small molecule inhibitors of heat shock protein (HSP) 90 (for example, 17-AAG), small molecule inhibitors of histone deacetylases (for example, hybrid/polar cytodifferentiation HPC) agents such as suberanilohydroxamic acid (SAHA), and FR-901228) and apoptotic agents such as Trisenox® (arsenic trioxide) and Xcytrin® (motexafin gadolinium); vaccine /immunotherapy-based cancer therapies, including, but not limited to, vaccine approaches (for example, Id-KLH, oncophage, vitalethine), personalized immunotherapy or active idiotype immunotherapy (for example, MyVax® Personalized Immunotherapy, formally designated GTOP-99), Promune® (CpG 7909, a synthetic agonist for toll-like receptor 9 (TLR9)), interferon-alpha therapy, IL-12 therapy, IL-15 therapy, and IL-21 therapy; steroid therapy; or other cancer therapy; where treatment with the antagonist anti-CD40 antibody or antigen-binding fragment thereof and the additional cancer therapy, or additional cancer therapies, occurs prior to, during, or subsequent to treatment of the subject with the medicament comprising the IL-2 or variant thereof, as noted herein above. Where the medicament comprising the IL-2 or biologically active variant thereof is coordinated with treatment with the antagonist anti-CD40 antibody or antigen-binding fragment thereof and at least one other cancer therapy, use of the medicament can be prior to, during, or after treatment of the subject with either or both of the other cancer therapies.

The following examples are offered by way of illustration and not by way of limitation.

EXPERIMENTAL

*Introduction*

The antagonist anti-CD40 antibodies used in the examples below are CHIR-5.9 and CHIR-12.12. The CHIR-5.9 and CHIR-12.12 anti-CD40 antibodies are human $IgG_1$ subtype anti-human CD40 monoclonal antibodies (mAbs) generated by immunization of transgenic mice bearing the human $IgG_1$ heavy chain locus and the human K light chain locus (XenoMouse® (Abgenix; Fremont, California)). SF9 insect cells expressing CD40 extracellular domain were used as immunogen.

Briefly, splenocytes from immunized mice were fused with SP 2/0 or P 3 x 63Ag8.653 murine myeloma cells at a ratio of 10:1 using 50% polyethylene glycol as previously described by de Boer et al. (1988) J. Immunol. Meth. 113: 143. The fused cells were resuspended in complete IMDM medium supplemented with hypoxanthine (0.1 mM), aminopterin (0.01 mM), thymidine (0.016 mM), and 0.5 ng/ml hIL-6 (Genzyme, Cambridge, Massachusetts). The fused cells were then distributed between the wells of 96-well tissue culture plates, so that each well contained 1 growing hybridoma on average.

After 10-14 days, the supernatants of the hybridoma populations were screened for specific antibody production. For the screening of specific antibody production by the hybridoma clones, the supernatants from each well were pooled and tested for anti-CD40 activity specificity by ELISA first. The positives were then used for fluorescent cell staining of EBV-transformed B cells using a standard FACS assay. Positive hybridoma cells were cloned twice by limiting dilution in IMDM/FBS containing 0.5 ng/ml hIL-6.

A total of 31 mice spleens were fused with the mouse myeloma SP2/0 cells to generate 895 antibodies that recognize recombinant CD40 in ELISA. On average approximately 10% of hybridomas produced using Abgenix Xenomouse® technology (Abgenix; Fremont, California) may contain mouse lambda light chain instead of human kappa chain. The antibodies containing mouse light lambda chain were selected out. A subset of 260 antibodies that also showed binding to cell-surface CD40 were selected for further analysis. Stable hybridomas selected during a series of subcloning procedures were used for further characterization in binding and functional assays.

Clones from 7 other hybridomas were identified as having antagonistic activity. Based on their relative antagonistic potency and ADCC activities, two hybridoma clones were selected for further evaluation (Table 2 below). They are named 131.2F8.5.9 (5.9) and 153.8E2.D10.D6.12.12 (12.12).

Table 2. Summary of initial set of data with anti-CD40 IgG1 antibodies CHIR-5.9 and CHIR-12.12.

| Mother Hybridoma | Hybridoma clones | cell surface binding | Antagonist | ADCC | CDC | CMCC# | V-region DNA sequence |
|---|---|---|---|---|---|---|---|
| 131.2F5 | 131.2F5.8.5.9 | +++ | +++ | ++ | - | 12047 | Yes |
| 153.8E2 | 153.8E2D10D6.12.12 | +++ | +++ | ++++ | - | 12056 | Yes |

Mouse hybridoma line 131.2F8.5.9 (CMCC#12047) and hybridoma line 153.8E2.D10.D6.12.12 (CMCC#12056) have been deposited with the American Type Culture Collection (ATCC; 10801 University Blvd., Manassas, Virginia 20110-2209 (USA)) under Patent Deposit Number PTA-5542 and PTA-5543, respectively.

The cDNAs encoding the variable regions of the candidate antibodies were amplified by PCR, cloned, and sequenced. The amino acid sequences for the light chain and heavy chain of the CHIR-12.12 antibody are set forth in Figures 1A and 1B, respectively. See also SEQ ID NO:2 (light chain for mAb CHIR-12.12) and SEQ ID NO:4 (heavy chain for mAb CHIR-12.12). A variant of the heavy chain for mAb CHIR-12.12 is shown in Figure 1B (see also SEQ ID NO:5), which differs from SEQ ID NO:4 in having a serine residue substituted for the alanine residue at position 153 of SEQ ID NO:4. The nucleotide sequences encoding the light chain and heavy chain of the CHIR-12.12 antibody are set forth in Figures 2A and 2B, respectively. See also SEQ ID NO:1 (coding sequence for light chain for mAb CHIR-12.12) and SEQ ID NO:3 (coding sequence for heavy chain for mAb CHIR-12.12). The amino acid sequences for the light chain and heavy chain of the CHIR-5.9 antibody are set forth in Figures 3A and 3B, respectively. See also SEQ ID NO:6 (light chain for mAb CHIR-5.9) and SEQ ID NO:7 (heavy chain for mAb CHIR-5.9). A variant of the heavy chain for mAb CHIR-5.9 is shown in Figure 3B (see also SEQ ID NO:8), which differs from SEQ ID NO:7 in having a serine residue substituted for the alanine residue at position 158 of SEQ ID NO:7.

As expected for antibodies derived from independent hybridomas, there is substantial variation in the nucleotide sequences in the complementarity determining regions (CDRs). The diversity in the CDR3 region of $V_H$ is believed to most significantly determine antibody specificity.

As shown by FACS analysis, CHIR-5.9 and CHIR-12.12 bind specifically to human CD40 and can prevent CD40-ligand binding. Both mAbs can compete off CD40-ligand pre-bound to cell surface CD40. The binding affinity of CHIR-5.9 to human CD40 is $1.2 \times 10^{-8}$ M and the binding affinity of CHIR-12.12 to human CD40 is $5 \times 10^{-10}$M.

The CHIR-12.12 and CHIR-5.9 monoclonal antibodies are strong antagonists and inhibit *in vitro* CD40 ligand-mediated proliferation of normal B cells, as well as inhibiting *in vitro* CD40 ligand-mediated proliferation of cancer cells from NHL and CLL patients. *In vitro,* both antibodies kill primary cancer cells from NHL patients by ADCC. Dose-dependent anti-tumor activity was seen in a xenograft human lymphoma model.

Example 1: CHIR-5.9 and CHIR-12.12 Bind to a Different Epitope on CD40 than 15B8

The candidate monoclonal antibodies CHIR-5.9 and CHIR-12.12 compete with each other for binding to CD40 but not with 15B8, an $IgG_2$ anti-CD40 mAb (see International Publication No. WO 02/28904). Antibody competition binding studies using Biacore were designed using CM5 biosensor chips with protein A immobilized via amine coupling, which was used to capture either anti-CD40, CHIR-12.12, or 15B8. Normal association/dissociation binding curves are observed with varying concentrations of CD40-his (data not shown). For competition studies, either CHIR-12.12 or 15B8 were captured onto the protein A surface. Subsequently a CD40-his /CHIR-5.9 Fab complex (100 nM CD40:1 μM CHIR-5.9 Fab), at varying concentrations, was flowed across the modified surface. In the case of CHIR-12.12, there was no association of the complex observed, indicating CHIR-5.9 blocks binding of CHIR-12.12 to CD40-his. For 15B8, association of the Fab CHIR-5.9 complex was observed indicating CHIR-5.9 does not block binding of 15B8 to CD40 binding site. However, the off rate of the complex dramatically increased (data not shown).

It has also been determined that 15B8 and CHIR-12.12 do not compete for CD40-his binding. This experiment was set up by capturing CHIR-12.12 on the protein A biosensor chip, blocking residual protein A sites with control $hIgG_1$, binding CD40-his and then flowing 15B8 over the modified surface. 15B8 did bind under these conditions indicating CHIR-12.12 does not block 15B8 from binding to CD40.

Example 2: Binding Properties of CHIR-12.12 and CHIR-5.9 mAB

Protein A was immobilized onto CM5 biosensor chips via amine coupling. Human anti-CD40 monoclonal antibodies, at 1.5 μg/ml, were captured onto the modified biosensor surface for 1.5 minutes at 10 μl/min. Recombinant soluble CD40-his was flowed over the biosensor surface at varying concentrations. Antibody and antigen were diluted in 0.01 M HEPES pH 7.4, 0.15 M NaCl, 3 mM EDTA, 0.005% Surfactant P20 (HBS-EP). Kinetic and affinity constants were determined using the Biaevaluation software with a 1:1 interaction model/global fit.

As shown in Table 3 below, there is 121-fold difference in the off rate of CHIR-5.9 and CHIR-12.12 resulting in 24-fold higher affinity for CHIR-12.12.

Table 3. Summary of binding properties of CHIR-5.9 and CHIR-12.12 anti-CD40 antibodies.

| Antibody | Ka(M-1 sec-1)) | kd (sec-1) | KD (nM) |
|---|---|---|---|
| Anti-CD40, CHIR-5.9 | $(12.35 \pm 0.64) \times 10^5$ | $(15.0 \pm 1.3) \times 10^{-3}$ | $12.15 \pm 0.35$ |
| Anti-CD40, CHIR-12.12 | $(2.41 \pm 0.13) \times 10^5$ | $(1.24 \pm 0.06) \times 10^{-4}$ | $0.51 \pm 0.02$ |

Example 3: Characterization of the Epitope for Monoclonal Antibodies CHIR-12.12 and CHIR-5.9

To determine the location of the epitope on CD40 recognized by monoclonal antibodies CHIR-12.12 and CHIR-5.9, SDS-PAGE and Western blot analysis were performed. Purified CD40 (0.5 μg) was separated on a 4-12% NUPAGE gel under reducing and non-reducing conditions, transferred to PVDF membranes, and probed with monoclonal antibodies at 10 μg/ml concentration. Blots were probed with alkaline phosphatase conjugated anti-human IgG and developed using the Western Blue® stabilized substrate for alkaline phosphatase (Promega).

Results indicate that anti-CD40 monoclonal antibody CHIR-12.12 recognizes epitopes on both the non-reduced and reduced forms of CD40, with the non-reduced form of CD40 exhibiting greater intensity than the reduced form of CD40 (Table 4; blots not shown). The fact that recognition was positive for both forms of CD40 indicates that this antibody interacts with a conformational epitope part of which is a linear sequence. Monoclonal antibody CHIR-5.9 primarily recognizes the non-reduced form of CD40 suggesting that this antibody interacts with a primarily conformational epitope (Table 4; blots not shown).

Table 4. Domain identification.

| | Domain 1 | Domain 2 | Domain 3 | Domain 4 |
|---|---|---|---|---|
| mAb CHIR-12.12 | - | + | - | - |
| mAb CHIR-5.9 | - | + | - | - |
| mAb 15B8 | + | - | - | - |

To map the antigenic region on CD40, the four extracellular domains of CD40 were cloned and expressed in insect cells as GST fusion proteins. The secretion of the four domains was ensured with a GP67 secretion signal. Insect cell supernatant was analyzed by SDS-PAGE and western blot analysis to identify the domain containing the epitope.

Monoclonal antibody CHIR-12.12 recognizes an epitope on Domain 2 under both reducing and non-reducing conditions (Table 5; blots not shown). In contrast, monoclonal antibody CHIR-5.9 exhibits very weak recognition to Domain 2 (Table 5; blots not shown). Neither of these antibodies recognize Domains 1, 3, or 4 in this analysis.

Table 5. Domain 2 analysis.

| | Reduced | Non-reduced |
|---|---|---|
| mAb CHIR-12.12 | ++ | +++ |
| mAb CHIR-5.9 | + | + |

To define more precisely the epitope recognized by mAb CHIR-12.12, peptides were synthesized from the extracellular Domain 2 of CD40, which corresponds to the sequence PCGESEFLDTWNRETHCHQHKYCDPNLGLRVQQKGTSETDTICT (residues 61-104 of the sequence shown in SEQ ID NO:10 or SEQ ID NO:12). SPOTs membranes (Sigma) containing thirty-five 10mer peptides with a 1-amino-acid offset were generated. Western blot analysis with mAb CHIR-12.12 and anti-human IgG beta-galactosidase as secondary antibody was performed. The blot was stripped and reprobed with mAb CHIR-5.9 to determine the region recognized by this antibody

SPOTs analysis probing with anti-CD40 monoclonal antibody CHIR-12.12 at 10 μg/ml yielded positive reactions with spots 18 through 22. The sequence region covered by these peptides is shown in Table 6.

Table 6. Results of SPOTs analysis probing with anti-CD40 monoclonal antibody CHIR-12.12.

| **Spot Number** | Sequence Region |
|---|---|
| 18 | HQHK**YCDPNL** (residues 78-87 of SEQ ID NO:10 or 12) |
| 19 | QHK**YCDPNLG** (residues 79-88 of SEQ ID NO:10 or12) |
| 20 | HK**YCDPNL**GL (residues 80-89 of SEQ ID NO:10 or 12) |
| 21 | K**YCDPNL**GLR (residues 81-90 of SEQ ID NO:10 or 12) |
| 22 | **YCDPNL**GLRV (residues 82-91 of SEQ ID NO:10 or 12) |

These results correspond to a linear epitope of: YCDPNL (residues 82-87 of the sequence shown in SEQ ID NO:10 or SEQ ID NO:12). This epitope contains Y82, D84, and N86, which have been predicted to be involved in the CD40-CD40 ligand interaction.

SPOTs analysis with mAb CHIR-5.9 showed a weak recognition of peptides represented by spots 20-22 shown in Table 7, suggesting involvement of the region YCDPNLGL (residues 82-89 of the sequence shown in SEQ ID NO:10 or SEQ ID NO:12) in its binding to CD40. It should be noted that the mAbs CHIR-12.12 and CHIR-5.9 compete with each other for binding to CD40 in BIACORE analysis.

Table 7. Results of SPOTs analysis probing with anti-CD40 monoclonal antibody CHIR-5.9.

| **Spot Number** | Sequence Region |
|---|---|
| 20 | HK**YCDPNLGL** (residues 80-89 of SEQ ID NO:10 or 12) |
| 21 | K**YCDPNLGL**R (residues 81-90 of SEQ ID NO:10 or 12) |
| 22 | **YCDPNLGL**RV (residues 82-91 of SEQ ID NO:10 or 12) |

The linear epitopes identified by the SPOTs analyses are within the CD40 B 1 module. The sequence of the CD40 B1 module is:

HKYCDPNLGLRVQQKGTSETDTIC (residues 80-103 of SEQ ID NO:10 or 12).

Within the linear epitope identified for CHIR-12.12 is C83. It is known that this cysteine residue forms a disulphide bond with C103. It is likely that the conformational epitope of the CHIR-12.12 mAb contains this disulfide bond (C83-C103) and/or surrounding amino acids conformationally close to C103.

Example 4: Combination Treatment with CHIR-12.12 and IL-2 Shows Additive Anti-Tumor Activity in Animal Models

The CHIR-12.12 mAb is expected to produce desired pharmacological effects to reduce tumor burden by either/both of two anti-tumor mechanisms, blockade of proliferation/survival signal and induction of ADCC. The currently available human lymphoma xenograft models use long-term lymphoma cell lines that, in contrast to primary cancer cells, do not depend on CD40 stimulation for their growth and survival. Therefore, the component of these mAbs' anti-tumor activity based on blocking the tumor proliferation/survival signal is not expected to contribute to anti-tumore efficacy in these models. The efficacy in these models is dependent on the ADCC, the second anti-tumor mechanism associated with the CHIR-12.12 mAb.

Combination treatment with CHIR-12.12 mAb and IL-2 were evaluated in a staged human NHL Namalwa xenograft model. To ensure consistent tumor growth, T cell-deficient nude mice were whole-body irradiated at 3 Gy to further suppress the immune system one day before tumor inoculation. Tumor cells were inoculated subcutaneously in the right flank at $5x10^6$ cells per mouse. Treatment was initiated when tumor volume reached about 100-200 $mm^3$ (about 7 days after tumor inoculation). Tumor-bearing mice were treated as follows: (1) IgG1 at 10 mg/kg, intraperitoneally, once a week for 4 weeks; (2) CHIR-12.12 mAb at 1 mg/kg, intraperitoneally, once a week for 4 weeks; (3) IL-2 at 0.5 mg/kg, subcutaneously, daily for 8 days; (4) IL-2 at 0.5 mg/kg, subcutaneously, daily for 8 days + CHIR-12.12 mAb at 1 mg/kg, intraperitoneally, once a week for 4 weeks, and (5) IL-2 at 0.5 mg/kg, subcutaneously, daily for 8 days + CHIR-12.12 mAb at 10 mg/kg, intraperitoneally, once a week for 4 weeks. Tumor volumes were recorded twice a week. Data were analyzed using ANOVA.

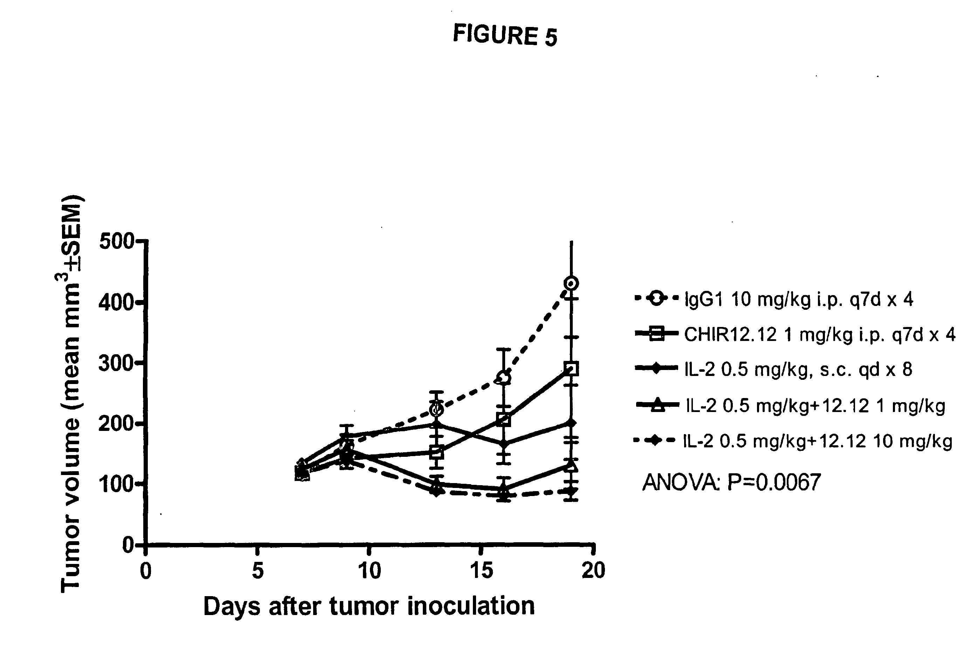

CHIR-12.12 and IL-2 alone both inhibited the growth of Namalwa tumors (N = 10 mice/group). The combination of CHIR-12.12 and IL-2 resulted in additive anti-tumor activity (Figure 5).

Example 5: CHIR-12.12 Blocks CD40L-Mediated CD40 Survival and Signaling Pathways in Normal Human B Cells

Soluble CD40 ligand (CD40L) activates B cells and induces various aspects of functional responses, including enhancement of survival and proliferation, and activation of NFκB, ERK/MAPK, PI3K/Akt, and p38 signaling pathways. In addition, CD40L-mediated CD40 stimulation provides survival signals by reduction of cleaved PARP and induction of the anti-apoptotic proteins, XIAP and Mcl-1, in normal B cells. CD40L-mediated CD40 stimulation also recruits TRAF2 and TRAF3 to bind CD40 cytoplasmic domain.

The following studies demonstrate that CHIR-12.12 directly inhibited all of these stimulation effects on normal human B cells. For example, CHIR-12.12 treatment resulted in increased cleavage of caspase-9, caspase-3, and PARP as well as reduction of XIAP and Mcl-1 in a time- and dose-dependent manner, restoring B cell apoptosis. Treatment with CHIR-12.12 also inhibited phosphorylation of IκB kinase (IKK) $\alpha$ and $\beta$ (NFκB pathway), ERK, Akt, and p38 in response to CD40L-mediated CD40 stimulation. Further, it was found that CHIR-12.12 did not trigger these apoptotic effects without initial CD40L-mediated CD40 stimulation.

*CHIR-12.12 inhibited survival mediated by CD40 ligand by inducing cleavage of PARP.*

In these experiments, 0.6 x $10^6$ normal human B cells from healthy donors (percent purity between 85-95%) were stimulated with 1 μg/ml sCD40L (Alexis Corp., Bingham, Nottinghamshire, UK). CHIR-12.12 (10 μg/ml) and control IgG were then added. Cells were collected at 0, 20 minutes, 2 hours, 6 hours, 18 hours, and 26 hours. Cleaved caspase-9, cleaved caspase-3, cleaved PARP, and β-actin controls were detected in cell lysates by Western blot.

Briefly, it was observed that CD40L-mediated CD40 stimulation provided survival signals as it did not result in increases of cleaved caspase-9, cleaved caspase-3, or cleaved PARP over time, indicating that the cells were not undergoing apoptosis. However, treatment with CHIR-12.12 resulted in an increase of these cleavage products, indicating that CHIR-12.12 treatment abrogated the effects of CD40L binding on survival signaling in sCD40L-stimulated normal B cells, restoring B cell apoptosis (data not shown).

*CHIR-12.12 inhibited expression of "survival" anti-apoptotic proteins.*

In these experiments, 0.6 x $10^6$ normal human B cells from healthy donors (percent purity between 85-95%) were stimulated with 1 μg/ml sCD40L (Alexis Corp., Bingham, Nottinghamshire, UK). CHIR-12.12 (10 μg/ml) and control IgG were then added. Cells were collected at 0, 20 minutes, 2 hours, 6 hours, 18 hours, and 26 hours. Mcl-1, XIAP, CD40, and β-actin controls were detected in cell lysates by Western blot.

Briefly, sCD40L stimulation resulted in sustained expression of Mcl-1 and XIAP over time. However, treatment of the sCD40L-stimulated cells with CHIR 12.12 resulted in a decrease in expression of these proteins overtime (data not shown). Since Mcl-1 and XIAP are "survival" signals capable of blocking the apoptotic pathway, these results demonstrate that CHIR-12.12 treatment removes the blockade against apoptosis in sCD40L-stimulated normal B cells.

*CHIR-12.12 treatment inhibited phosphorylation of IKKα (Ser180) and IKK β (Ser 181) in normal B cells.*

In these experiments, 1.0 x $10^6$ normal human B cells from healthy donors (percent purity between 85-95%) were stimulated with 1 μg/ml sCD40L (Alexis Corp., Bingham, Nottinghamshire, UK). CHIR-12.12 (10 μg/ml) and control IgG were then added. Cells were collected at 0 and 20 minutes. Phosphorylated IKKα (Ser180) and IKK β (Ser 181) and total IKKβ controls were detected in cell lysates by Western blot.

Briefly, stimulation by sCD40L resulted in phosphorylation of IKKα (Ser180) and IKK β (Ser 181) over time; however, treatment with CHIR-12.12 abrogated this response to sCD40L stimulation in normal B cells (data not shown).

*CHIR-12.12 treatment inhibited survival mediated by CD40 ligand in a dose-dependent manner.*

In these experiments, 0.6 x $10^6$ normal human B cells from healthy donors percent purity between 85-95%) were stimulated with 1 μg/ml sCD40L (Alexis Corp., Bingham, Nottinghamshire, UK). CHIR-12.12 (0.01, 0.1, 0.2, 0.5, 1.0 μg/ml) and control IgG were then added. Cells were collected at 24 hours. Cleaved PARP, and β-actin controls were detected in cell lysates by Western blot.

Briefly, CHIR-12.12 treatment resulted in increase of PARP cleavage in sCD40L stimulated cells in a dose-dependent manner and therefore abrogated the survival signaling pathway in sCD40L-stimulated normal B cells (data not shown).

*CHIR-12.12 inhibited expression of "survival" anti-apoptotic proteins in a dose-dependent manner.*

In these experiments, 0.6 x $10^6$ normal human B cells from healthy donors (percent purity between 85-95%) were stimulated with 1 μg/ml sCD40L (Alexis Corp., Bingham, Nottinghamshire, UK). CHIR-12.12 (0.5, 2, and 10 μg/ml) and control IgG were then added. Cells were collected at 22 hours. Mcl-1, XIAP, cleaved PARP, and β-actin controls were detected in cell lysates by Western blot.

Briefly, CHIR-12.12 treatment reduced Mcl-1 and XIAP expression and increased cleaved PARP expression in sCD40L-stimulated cells in a dose-dependent manner, and thus abrogated these blockades to the apoptotic pathway in sCD40L-stimulated normal B cells (data not shown).

*CHIR-12.12 did not affect expression of anti-apoptotic proteins, cleaved-PARP, and XIAP, in the absence of soluble CD40L signaling.*

In these experiments, 1.0 x $10^6$ normal human B cells from healthy donors (percent purity between 85-95%) were treated with CHIR-12.12 (10 μg/ml) and control IgG only (i.e., cells were not pre-stimulated with sCD40L before adding antibody). Cells were collected at 0, 4, 14, and 16 hours. XIAP, cleaved PARP, and β-actin controls were detected in cell lysates by Western blot.

Briefly, the results show that without sCD40L stimulation, the cells expressed increased concentrations of cleaved PARP, while expression of XIAP remained constant, in both IgG treated control cells and CHIR-12.12 cells (data not shown). These data indicate that CHIR-12.12 does not trigger apoptosis in normal human B cells without CD40L stimulation.

*CHIR-12.12 inhibits phosphorylation of IKKα (Ser180) and IKKβ (Ser181), Akt, ERK, and p38 in normal B cells.*

In these experiments, 1.0 x $10^6$ normal human B cells from healthy donors (percent purity between 85-95%) were serum starved in 1% FBS-containing media and stimulated with 1 μg/ml sCD40L (Alexis Corp., Bingham, Nottinghamshire, UK). The cultures were treated with CHIR-12.12 (1 and 10 μg/ml) and control IgG. Cells were collected at 0 and 20 minutes. Phospho-IKKα, phospho-IKKβ, total IKKβ, phospho-ERK, total ERK, phospho-Akt, total Akt, phospho-p38, and total p38 were detected in cell lysates by Western blot.

Briefly, sCD40L stimulation resulted in increases in IKKα/β phosphorylation, ERK phosphorylation, Akt phosphorylation, and p38 phosphorylation, thus leading to survival and or proliferation of the cells. Treatment of the cells with CHIP-12.12 abrogated the effects of sCD40L stimulation on these signaling pathways in normal B cells (data not shown).

*CHIR 12.12 inhibits multiple signaling pathways such as PI3K and MEK/ERK in the CD40 signaling cascade.*

In these experiments, 1.0 x $10^6$ normal human B cells from healthy donors (percent purity between 85-95%) were serum starved in 1% FBS-containing media and stimulated with 1 μg/ml sCD40L (Alexis Corp., Bingham, Nottinghamshire, UK). The cultures were also treated with CHIR-12.12 (1 and 10 μg/ml), Wortmanin, (a PI3K/Akt inhibitor; 1 and 10 μM), LY 294002 (a PI3K/Akt inhibitor; 10 and 30 μM), and PD 98095 (a MEK inhibitor; 10 and 30 μg/ml). Cells were collected at 0 and 20 minutes. Phospho-ERK, phospho-Akt, total Akt, phospho-IKKα/β and total were detected in cell lysates by Western blot.

Briefly, the results show that CH1R-12.12 abrogated the phosphorylation of all of these signal transduction molecules, whereas the signal transduction inhibitors showed only specific abrogation of signaling, indicating that CHIR-12.12 likely inhibits upstream of these signal transduction molecules mediated by CD40L stimulation (data not shown).

*CHIR-12.12 inhibits the binding of signaling molecules TRAF2 and TRAF3 to the cytoplasmic domain of CD40 in normal B cells.*

In these experiments, 4.0 x $10^6$ normal human B cells from healthy donors (percent purity between 85-95%) were serum starved for four hours in 1% FBS-containing media and stimulated with 1 μg/ml sCD40L (Alexis Corp., Bingham, Nottinghamshire, UK) for 20 minutes. Cells were collected at 0 and 20 minutes. CD40 was immunoprecipitated using polyclonal anti-CD40 (Santa Cruz Biotechnology, CA), and was probed in a Western blot with anti-TRAF2 mAb (Santa Cruz Biotechnology, CA), anti-TRAF3 mAb (Santa Cruz Biotechnology, CA), and anti-CD40 mAb (Santa Cruz Biotechnology, CA).

Briefly, the results show that TRAF2 and TRAF3 co-precipitated with CD40 after sCD40L stimulation. In contrast, treatment with CHIR-12.12 abrogated formation of the CD40-TRAF2/3 signaling complex in sCD40L-stimulated normal B cells. There were no changes in CD40 expression (data not shown).

Without being bound by theory, the results of these experiments, and the results in the examples outlined above, indicate that the CHIR-12.12 antibody is a dual action antagonist anti-CD40 monoclonal antibody having a unique combination of attributes. This fully human monoclonal antibody blocks CD40L-mediated CD40 signaling pathways for survival and proliferation of B cells; this antagonism leads to ultimate cell death. CHIR-12.12 also mediates recognition and binding by effector cells, initiating antibody dependent cellular cytotoxicity (ADCC). Once CHIR-12.12 is bound to effector cells, cytolytic enzymes are released, leading to B-cell apoptosis and lysis. CHIR-12.12 is a more potent anti-tumor antibody than is rituximab when compared in pre-clinical tumor models.

Example 6: Liquid Pharmaceutical Formulation for Antagonist Anti-CD40 Antibodies

Protein stability is known to be sensitive to solution pH as surface charge of a protein varies with pH of solution, resulting in stabilization or destabilization. Thus, non-optimal formulation pHs can alter electrostatic interaction, leading to physical degradation of an antagonist anti-CD40 antibody, such as aggregation, precipitation, and the like. Change in pH conditions could also cause breakage of covalent bonds, leading to chemical degradation, such as fragmentation, deamidation, and the like. This study was therefore designed to identify an optimal solution pH to minimize antagonist anti-CD40 antibody degradation due to aggregation, fragmentation, and deamidation.

The objective of this study was to investigate the effects of solution pH on stability of the antagonist anti-CD40 antibody CHIR-12.12 by both biophysical and biochemical methods in order to select the optimum solution environment for this antibody. Differential Scanning Calorimetry (DSC) results showed that the conformation stability of CHIR-12.12 is optimal in formulations having pH 5.5-6.5. Based on a combination of SDS-PAGE, Size-Exclusion HPLC (SEC-HPLC), and Cation-Exchange HPLC (CEX-HPLC) analysis, the physicochemical stability of CHIR-12.12 is optimal at about pH 5.0-5.5. In view of these results, one recommended liquid pharmaceutical formulation comprising this antibody is a formulation comprising CHIR-12.12 at about 20 mg/ml formulated in about 10 mM sodium succinate, about 150 mM sodium chloride, and having a pH of about pH 5.5.

Materials and Methods

The CHIR-12.12 antibody used in the formulation studies is a human monoclonal antibody produced by a CHO cell culture process. This MAb has a molecular weight of 150 kDa and consists of two light chains and two heavy chains linked together by disulfide bands. It is targeted against the CD40 cell surface receptor on CD40-expressing cells, including normal and malignant B cells, for treatment of various cancers and autoimmune/inflammatory diseases.

The anti-CD40 drug substance used for this study was a CHO-derived purified anti-CD40 (CHIR-12.12) bulk lot. The composition of the drug substance was 9.7 mg/ml CHIR-12.12 antibody in 10 mM sodium citrate, 150 mM sodium chloride, at pH 6.5. The control sample in the study was the received drug substance, followed by freezing at ≤ - 60°C, thawing at RT and testing along with stability samples at predetermined time points. The stability samples were prepared by dialysis of the drug substance against different pH solutions and the CHIR-12.12 concentration in each sample was determined by UV 280 as presented in Table 8.

Table 8. CHIR-12.12 formulations.

| Buffer Composition | pH | CHIR-12.12 Concentration ( mg/ml) |
|---|---|---|
| 10 mM sodium citrate, 150 mM sodium chloride | 4.5 | 9.0 |
| 10 mM sodium succinate, 150 mM sodium chloride | 5.0 | 9.3 |
| 10 mM sodium succinate, 150 mM sodium chloride | 5.5 | 9.2 |
| 10 mM sodium citrate, 150 mM sodium chloride | 6.0 | 9.7 |
| 10 mM sodium citrate, 150 mM sodium chloride | 6.5 | 9.4 |
| 10 mM sodium phosphate, 150 mM sodium chloride | 7.0 | 9.4 |
| 10 mM sodium phosphate, 150 mM sodium chloride | 7.5 | 9.5 |
| 10 mM glycine, 150 mM sodium chloride | 9.0 | 9.5 |

Physicochemical stability of the CHIR-12.12 antibody in the various formulations was assayed using the following protocols.

*Differential Scanning Calorimetry (DSC*

Conformational stability of different formulation samples was monitored using a MicroCal VP-DSC upon heating 15°C to 90°C at 1°C/min.

*SDS-PAGE*

Fragmentation and aggregation were estimated using 4-20% Tris-Glycine Gel under non-reducing and reducing conditions. Protein was detected by Coomassie blue staining.

*Size Exclusion Chromatograph (SEC-HPLC)*

Protein fragmentation and aggregation were also measured by a Water Alliance HPLC with a Tosohaas TSK-GEL 3000SWXL column, 100 mM sodium phosphate, pH 7.0 as mobile phase at a flow rate of 0.7 ml/min.

*Cation Exchange Chromatography (CEX-HPLC)*

Charge change related degradation was measured using Waters 600s HPLC system with a Dionex Propac WCX-10 column, 50 mM HEPEs, pH 7.3 as mobile phase A and 50 mM HEPES containing 500 mM NaCl, pH 7.3 as mobile phase B at a flow rate of 0.5°C/min.

Results and Discussion

*Conformational stability study.*

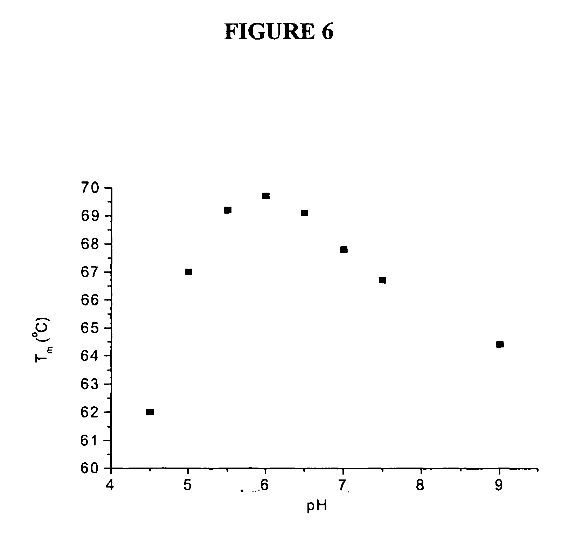

Thermal unfolding of CHIR-12.12 revealed at least two thermal transitions, probably representing unfolding melting of the Fab and the Fc domains, respectively. At higher temperatures, the protein presumably aggregated, resulting in loss of DSC signal. For the formulation screening purpose, the lowest thermal transition temperature was defined as the melting temperature, Tm, in this study. Figure 6 shows the thermal melting temperature as a function of formulation pHs. Formulations at pH 5.5-6.5 provided anti-CD40 with higher conformational stability as demonstrated by the higher thermal melting temperatures.

*SDS-PAGE analysis.*

The CHIR-12.12 formulation samples at pH 4.5-9.0 were incubated at 40°C for 2 months and subjected to SDS-PAGE analysis (data not shown). Under non-reducing conditions, species with molecular weight (MW) of 23 kDa and 27 kDa were observed in formulations above pH 5.5, and species with MW of 51 kDa were observed in all formulations, but appeared less at pH 5.0-5.5. A species with MW of 100 kDa could be seen at pH 7.5 and pH 9.0.

Under reducing conditions, CHIR-12.12 was reduced into free heavy chains and light chains with MW of 50 kDa and 24 kDa, respectively. The 100 kDa species seemed not fully reducible and increased with increasing solution pH, suggesting non-disulfide covalent association might occur in the molecules. Since there were other species with unknown identities on SDS-PAGE, stability comparison of each formulation is based on the remaining purity of CHIR-12.12. Formulations at pH 5.0-6.0 provided a more stable environment to CHIR-12.12. Few aggregates were detected by SDS-PAGE (data not shown).

*SEC-HPLC analysis .*

SEC-HPLC analysis detected the intact CHIR-12.12 as the main peak species, an aggregation species as a front peak species separate from the main peak species, a large fragment species as a shoulder peak on the back of the main peak species, and small fragment species were detected post-main peak species. After incubation at 5°C and 25°C for 3 months, negligible amounts of protein fragments and aggregates (<1.0% ) were detected in the above formulations and the CHIR-12.12 main peak species remained greater than 99% purity (data not shown). However, protein fragments gradually developed upon storage at 40°C and more fragments formed at pH 4.5 and pH 6.5-9.0, as shown in Table 9. After incubating the CHIR-12.12 formulations at 40°C for 3 months, about 2-3% aggregates were detected in pH 7.5 and pH 9.0, while less than 1% aggregates were detected in other pH formulations (data not shown). The SEC-HPLC results indicate CHIR-12.12 is more stable at about pH 5.0-6.0.

Table 9. SEC-HPLC results of CHIR-12.12 stability samples under real-time and accelerated storage conditions.

| Sample | Main peak % | | | | Fragments % | | | |
|---|---|---|---|---|---|---|---|---|
| | t=0 | 40°C 1 m | 40°C 2m | 40°C 3m | t=0 | 40°C 1 m | 40°C 2m | 40°C 3m |
| Control | 99.4 | 99.2 | 99.9 | 99.5 | <1.0 | <1.0 | <1.0 | <1.0 |
| pH 4.5 | 99.4 | 93.2 | 86.0 | 81.3 | <1.0 | 6.4 | 13.2 | 18.1 |
| pH 5.0 | 99.8 | 98.7 | 91.3 | 89.2 | <1.0 | <1.0 | 7.8 | 10.2 |
| pH 5.5 | 99.8 | 98.9 | 91.4 | 90.6 | <1.0 | <1.0 | 7.6 | 8.8 |

(continued)

| Sample | Main peak % | | | | Fragments % | | | |
|---|---|---|---|---|---|---|---|---|
| | t=0 | 40°C 1 m | 40°C 2m | 40°C 3m | t=0 | 40°C 1 m | 40°C 2m | 40°C 3m |
| pH 6.0 | 99.6 | 97.7 | 90.4 | 87.3 | <1.0 | 1.9 | 8.2 | 11.7 |
| pH 6.5 | 99.3 | 93.4 | 89.0 | 86.9 | <1.0 | 5.6 | 9.9 | 12.4 |
| pH 7.0 | 99.2 | 93.9 | 87.4 | 85.1 | <1.0 | 5.5 | 11.1 | 13.5 |
| pH 7.5 | 99.1 | 92.8 | 84.4 | 81.9 | <1.0 | 6.4 | 12.9 | 16.2 |
| pH 9.0 | 99.3 | 82.4 | 61.6 | 50.6 | <1.0 | 15.4 | 36.2 | 47.6 |

*CEX-HPLC analysis.*

CEX-HPLC analysis detected the intact CHIR-12.12 as the main peak species, acidic variants eluted earlier than the main peak species, and C-terminal lysine addition variants eluted post-main peak species. Table 10 shows the dependence of the percentages of the remaining main peak CHIR-12.12 species and acidic variants on solution pH. The control sample already contained a high degree of acidic species (~33%), probably due to early-stage fermentation and purification processes. The susceptibility of CHIR-12.12 to higher pH solutions is evidenced by two facts. First, the initial formulation sample at pH 9.0 (t=0) already generated 12% more acidic species than the control. Second, the percentage of acidic species increased sharply with increasing pH. The charge change-related degradation is likely due to deamidation. The above data indicate that this type of degradation of CHIR-12.12 was minimized at about pH 5.0-5.5.

Table 10. Percentage of peak area by CEX-HPLC for CHIR-12.12 in different pH formulations under real-time and accelerated storage conditions.

| Sample | Main peak % | | | | | Acidic variants % | | | | |
|---|---|---|---|---|---|---|---|---|---|---|
| | t=0 | 5°C 3m | 25°C 3 m | 40°C 1 m | 40°C 2 m | t=0 | 5°C 3m | 25°C 3 m | 40°C 1 m | 40°C 2 m |
| Control | 49.2 | 49.8 | 49.8 | 49.2 | 50.3 | 32.0 | 33.7 | 33.7 | 32.0 | 33.6 |
| pH 4.5 | 48.5 | 49.7 | 43.7 | 39.7 | 30.0 | 32.5 | 32.6 | 38.0 | 44.2 | 56.4 |
| pH 5.0 | 49.6 | 49.8 | 48.3 | 40.6 | 31.4 | 32.7 | 31.8 | 35.0 | 44.3 | 57.1 |
| pH 5.5 | 50.7 | 50.3 | 48.1 | 40.0 | 30.2 | 32.6 | 31.8 | 37.8 | 48.9 | 63.3 |
| pH 6.0 | 50.2 | 49.9 | 47.9 | 37.4 | 23.9 | 33.1 | 33.6 | 38.5 | 54.9 | 72.7 |
| pH 6.5 | 49.4 | 49.9 | 42.3 | 29.7 | 14.6 | 33.3 | 33.6 | 47.7 | 65.2 | 84.6 |
| pH 7.0 | 49.7 | 49.9 | 21.9 | - | - | 34.4 | 36.4 | 64.4 | - | - |
| pH 7.5 | 49.3 | 48.3 | 12.7 | - | - | 35.5 | 40.1 | 79.2 | - | - |
| pH 9.0 | 41.3 | 31.8 | - | - | - | 44.7 | 59.9 | - | - | - |

Conclusion

The pH has a significant effect on conformational and physicochemical stabilities of CHIR-12.12. Charge change-related degradation was determined to be the main degradation pathway for CHIR-12.12, which was minimized at pH 5.0-5.5. Based on overall stability data, one recommended liquid pharmaceutical formulation comprising this antibody is a formulation comprising CHIR-12.12 at about 20 mg/ml formulated in about 10 mM sodium succinate, about 150 mM sodium chloride, and having a pH of about pH 5.5.

Example 7: Clinical Studies with IL-2 and Antagonisitic Anti-CD40 Antibodies ***Clinical Objectives***

The overall objective is to provide an effective therapy for CD40-expressing solid tumors and B-cell related cancers by targeting them with a combination of an antagonist anti-CD40 antibody and IL-2 or biologically active variant thereof. These tumors include solid tumors such as urinary bladder carcinoma, breast carcinoma, prostate cancer, renal cell carcinoma, nasopharyngeal carcinoma, squamous cell carcinoma, thyroid papillary carcinoma, melanoma, ovarian carcinoma, lung carcinoma, cervical carcinoma, gastric carcinoma, liver carcinoma, and sarcomas. B-cell related cancers include, but are not limited to, B cell lymphoma, chronic lymphocytic lyphoma (CLL), acute lymphoblastic leukemia (ALL), multiple myeloma (MM), Waldenstrom's Macroglobulinemia, and Systemic Castleman's Disease. The signal for these diseases is determined in phase II although some measure of activity may be obtained in phase I. The initial antagonist anti-CD40 antibody is the mAb CHIR-12.12, and the initial IL-2 product Proleukin®, which contains the recombinantly produced IL-2 mutein des-alanyl-1, serine-125 human IL-2.

**Phase I**

- Evaluate safety and pharmacokinetics - dose escalation of these two oncotherapies in malignancies.
- Choose dose of each oncotherapy based on safety, tolerability, and change in serum markers of respective targets, *i.e.*, CD40. In general an MTD for each of these antibodies when used in combination is sought but other indications of efficacy (depletion of $CD40^+$, etc.) may be adequate for dose finding.
- Consideration of more than one combination of doses especially for different indications, e.g., the CLL combination dose may be different than that for NHL. Thus, some dose finding may be necessary in phase II.
- Patients are dosed weekly with real-time pharmacokinetic (Pk) sampling. Initially a 4-week cycle is the maximum dosing allowed. The Pk may be highly variable depending on the disease studied, density of CD40, etc.
- This trial(s) is open to subjects with $CD40^+$ solid tumors and B-cell lymphoma, CLL, and potentially other malignancies.
- Decision to discontinue or continue studies is based on safety, dose, and preliminary evidence of anti-tumor activity, particularly synergistic in nature.
- Activity of combination therapy as determined by response rate is determined in Phase II.
- Identify combination dose(s) for Phase II.

**Phase II**

Several trials will be initiated in the above-mentioned tumor types with concentration on solid tumors and B-cell lymphoma, CLL, and Multiple Myeloma (MM). Separate trials may be required in low grade and intermediate/high grade NHL as CD40 may have a different function depending on the grade of lymphoma. More than one combination of doses, and more than one schedule may be tested in a randomized phase II setting.

In each disease, target a population that has failed current standard of care:

- CLL: patients who were resistant to Campath® and chemotherapy.
- Low grade NHL: Rituxan® or CHOP-R failures
- Intermediate NHL: CHOP-R failures
- Multiple Myeloma: Chemotherapy failures, dexamethazone plus thalidomide failures, or high-dose chemotherapy plus stem cell transplant failures

  ✓ Decision to discontinue or continue with study is based on proof of therapeutic concept in Phase II
  ✓ Determine whether surrogate marker can be used as early indication of clinical efficacy
  ✓ Identify doses for Phase III

**Phase III**

Phase III will depend on where the signal is detected in phase II, and what competing therapies are considered to be the standard. If the signal is in a stage of disease where there is no standard of therapy, then a single arm, well-controlled study could serve as a pivotal trial. If there are competing agents that are considered standard, then head-to-head studies are conducted.

<u>Example 8: Calculation IL-2 Serum Concentration-Time Curves for Pharmaceutical Formulations of IL-2</u>

The area under the serum concentration-time curve (AUC) of Proleukin® IL-2 administered subcutaneously (SC) at 4.5 million international units (MIU) (equivalent to approximately 275 $\mu$g protein) was determined using data from an unpublished HIV study. Serum concentration time profiles were measured in 8 IL-2 naïve, HIV patients following an initial exposure to IL-2 dosing in this study. For each patient, the AUC was calculated using the linear trapezoidal rule up to the last measurable concentrations and extrapolated to 24 hours (Winnonlin software version 3.1, Pharsight Corporation, California). The average $AUC_{0-24}$, SD, and the lower and upper 95% confidence limits at 4.5 MIU dose are presented in Table 11.

The $AUC_{0-24}$ value of Proleukin® IL-2 administered SC at doses equivalent to 18 MIU (1100 μg) was estimated using data from three different studies where this IL-2 product was administered SC. Two are published studies, one in HIV patients (N=3) (Piscitelli et al. (1996) Pharmacotherapy 16(5):754-759) and one in cancer patients (N=7) (Kirchner et al. (1998) Br. J. Clin. Pharmacol. 46:5-10). The third is an unpublished study in which serum concentration time data were available from 6 cancer patients after SC doses of IL-2. The similarity of the AUC in cancer and HIV patients was previously established (unpublished data). The actual doses administered in these three studies ranged between 18 and 34 MIU. For the two published trials, the AUC up to 24 hours ($AUC_{0-24}$) values were normalized to 18 MIU dose by multiplying the AUC with the quotient of 18 and actual dose in MIU. For example, if the $AUC_{0-24}$ for a 20 MIU dose was calculated to be 400, the normalized $AUC_{0-24}$ would be 400∗18/20=360. For the unpublished cancer-patient study, individual AUC values were calculated from the serum concentration time data using the linear trapezoidal rule up to the last measurable concentrations and extrapolated to 24 hours (Winnonlin software version 3.1, Pharsight Corporation, California) then were normalized to 18 MIU dose as noted above. The overall mean and SD for all three studies was calculated as the weighted average of the means and variances, respectively, using equations 1 and 2.

1. $$\overline{X}_P = \frac{\left(n_1\overline{X}_1 + n_2\overline{X}_2 + n_3\overline{X}_3\right)}{\left(n_1 + n_2 + n_3\right)}$$

2. $$SD_P = \sqrt{\frac{\left(n_1 - 1\right)s_1^2 + \left(n_2 - 1\right)s_2^2 + \left(n_3 - 1\right)s_3^2}{\left(n_1 + n_2 + n_3 - 3\right)}}$$

Where $n_1$,$n_2$, $n_3$, $\overline{X}_1$, $\overline{X}_2$, $\overline{X}_3$ and $s_1^2, s_2^2, s_3^2$ are the number of subjects, means, and variances for each of the three studies, respectively. $\overline{X}_P$ and $SD_P$ are estimates of the overall mean and standard deviation. The overall average AUC, SD, and the lower and upper 95% confidence limits at 18 MIU are also presented in Table 11.

Table 11: Average (± SD) $AUC_{0-24}$ obtained after initial exposure to a single dose administration of Proleukin® IL-2 administered subcutaneously.

| Proleukin® IL-2 Dose (MIU/μg) | $AUC_{0-24}$ (IU∗hr/ml) | SD | LL of 95% CI[1] | UL of 95% CI[1] |
|---|---|---|---|---|
| | | | | |
| | | | | |
| 4.5/275 | 51 | 14 | 23 | 78 |
| 6.0/367[2] | 65 | | 22 | 107 |
| 7.5/458 | 79 | 29 | 21 | 137 |
| 18/1100 | 344 | 127 | 90 | 598 |
| | | | | |

[1] Upper (UL) and lower (LL) limits of the 95% confidence intervals (CI). 95% CI were calculated as the mean ± 2 SD.
[2] Values for 6.0 MIU are estimated based on actual values for 4.5 MIU and 7.5 MIU.

Similar to Proleukin®IL-2, L2-7001, a liquid formulation of monomeric IL-2, was administered to HIV patients at doses ranging from 50 to 180 μg (unpublished data). The exposures obtained from this study as measured by AUC are shown in Table 12. These exposure values were within the range of the exposure values generated using Proleukin®

IL-2 (Table 11).

Table 12: Average (± SD) $AUC_{0-24}$ obtained after an initial exposure to a single dose administration of the monomeric IL-2 formulation L2-7001.

| L2-7001 Dose (MIU/μg) | $AUC_{0-24}$ (IU∗hr/ml) | SD |
|---|---|---|
| | | |
| 0.82/50 | 60 | 11 |
| 1.5/90 | 110 | 36 |
| 2.2/135 | 143 | 41 |
| 2.9/180 | 275 | 99 |

The IL-2 exposure data (AUC) was obtained from the published literature where recombinant human native IL-2 was administered SC to 8 cancer patients at doses ranging from 0.1 MU to 3.0 MU. The reported average (%CV) AUCs for the 0.3, 1, and 3 MU dose levels were 120 (38), 177 (36), and 359 (46) U∗hr/ml (Gustavson (1998) J. Biol. Response Modifiers 1998:440-449). As indicated in Thompson et al. 1987 Cancer Research 47:4202-4207, the units measured in this study were normalized to BRMP units (Rossio et al. (1986) Lymphokine Research 5 (suppl 1):S13-S18), which was adopted later as international units (IU) by WHO (Gearing and Thorpe (1988) J. Immunological Methods 114:3-9). The AUC values generated under the study conditions also agree well with the established Proleukin® IL-2 exposure.

Many modifications and other embodiments of the inventions set forth herein will come to mind to one skilled in the art to which these inventions pertain having the benefit of the teachings presented in the foregoing descriptions and the associated drawings. Therefore, it is to be understood that the inventions are not to be limited to the specific embodiments disclosed, and that modifications and other embodiments are intended to be included within the scope of the appended claims and embodiments disclosed herein. Although specific terms are employed herein, they are used in a generic and descriptive sense only and not for purposes of limitation.

## Claims

1. An anti-CD40 antibody or antigen-binding fragment thereof, for use in a method of treating a human subject for a cancer comprising neoplastic cells expressing CD40 antigen, said method comprising administering to said subject combination therapy, said therapy comprising administration of an effective amount of the anti-CD40 antibody or antigen-binding fragment thereof in combination with an interleukin-2 (IL-2) or biologically active variant thereof, wherein said anti-CD40 antibody or antigen-binding fragment thereof is free of significant agonist activity when bound to CD40 antigen and is selected from the group consisting of:

   a) an antibody or antigen-binding fragment thereof that binds to an epitope capable of binding the monoclonal antibody CHIR-5.9 obtainable from the hybridoma cell line deposited with the ATCC as Patent Deposit No. PTA-5542 or the monoclonal antibody CHIR-12.12 obtainable from the hybridoma cell line deposited with the ATCC as Patent Deposit No. PTA-5543;
   b) an antibody or antigen-binding fragment thereof that binds to an epitope comprising residues 82-87 of the human CD40 sequence shown in SEQ ID NO:10 or SEQ ID NO:12;
   c) an antibody or antigen-binding fragment thereof that binds to an epitope comprising residues 82-89 of the human CD40 sequence shown in SEQ ID NO:10 or SEQ ID NO: 12; and
   d) an antibody or antigen-binding fragment thereof that competes with the monoclonal antibody CHIR-5.9 obtainable from the hybridoma cell line deposited with the ATCC as Patent Deposit No. PTA-5542 or the monoclonal antibody CHIR-12.12 obtainable from the hybridoma cell line deposited with the ATCC as Patent Deposit No. PTA-5543 in a competitive binding assay.

2. Use of an effective amount of an anti-CD40 antibody or antigen-binding fragment thereof in combination with an interleukin-2 (IL-2) or biologically active variant thereof in the manufacture of a medicament for treating a human subject for a cancer comprising neoplastic cells expressing CD40 antigen by combination therapy, wherein said anti-CD40 antibody or antigen-binding fragment thereof is free of significant agonist activity when bound to CD40 antigen and is selected from the group consisting of:

   a) an antibody or antigen-binding fragment thereof that binds to an epitope capable of binding the monoclonal antibody CHIR-5.9 obtainable from the hybridoma cell line deposited with the ATCC as Patent Deposit No. PTA-5542 or the monoclonal antibody CHIR-12.12 obtainable from the hybridoma cell line deposited with the ATCC as Patent Deposit No. PTA-5543;
   b) an antibody or antigen-binding fragment thereof that binds to an epitope comprising residues 82-87 of the human CD40 sequence shown in SEQ ID NO:10 or SEQ ID NO:12;
   c) an antibody or antigen-binding fragment thereof that binds to an epitope comprising residues 82-89 of the human CD40 sequence shown in SEQ ID NO:10 or SEQ ID NO: 12; and
   d) an antibody or antigen-binding fragment thereof that competes with the monoclonal antibody CHIR-5.9 obtainable from the hybridoma cell line deposited with the ATCC as Patent Deposit No. PTA-5542 or the monoclonal antibody CHIR-12.12 obtainable from the hybridoma cell line deposited with the ATCC as Patent Deposit No. PTA-5543 in a competitive binding assay.

3. The anti-CD40 antibody or antigen-binding fragment of claim 1 or the use of claim 2, wherein said anti-CD40 antibody is a human antibody.

4. The anti-CD40 antibody or antigen-binding fragment of claim 1 or the use of claim 2, wherein said anti-CD40 antibody is selected from the group consisting of the monoclonal antibody CHIR-5.9 obtainable from the hybridoma cell line deposited with the ATCC as Patent Deposit No. PTA-5542 and the monoclonal antibody CHIR-12.12 obtainable from the hybridoma cell line deposited with the ATCC as Patent Deposit No. PTA-5543.

5. The anti-CD40 antibody or antigen-binding fragment of claim 1 or the use of claim 2, wherein said anti-CD40 antibody or antigen-binding fragment thereof is selected from the group consisting of:

   (i) an antibody or antigen-binding fragment thereof comprising a light chain variable domain containing residues 44-54, 70-76 and 109-117 of SEQ ID NO:2 or SEQ ID NO:6;
   (ii) an antibody or antigen-binding fragment thereof comprising a heavy chain variable domain containing residues 50-54, 69-84 and 114-121 of SEQ ID NO:4 or SEQ ID NO:7;
   (iii) an antibody or antigen-binding fragment thereof comprising a light chain variable domain containing residues 46-52, 70-72 and 111-116 of SEQ ID NO:2 or SEQ ID NO:6; and

(iv) an antibody or antigen-binding fragment thereof comprising a heavy chain variable domain containing residues 45-51, 72-74, 115-120 of SEQ ID NO:4 or SEQ ID NO:7.

**6.** The anti-CD40 antibody or antigen-binding fragment or use of claim 5, wherein said anti-CD40 antibody or antigen-binding fragment thereof comprises an amino acid sequence selected from the group consisting of:

(i) residues 21-132 of SEQ ID NO:2;
(ii) residues 21-239 of SEQ ID NO:2;
(iii) SEQ ID NO:2;
(iv) residues 20-139 of SEQ ID NO:4;
(v) residues 20-469 of SEQ ID NO:4;
(vi) SEQ ID NO:4;
(vii) residues 20-469 of SEQ ID NO:5;
(viii) SEQ ID NO:5;
(ix) residues 21-132 of SEQ ID NO:2 and residues 20-139 of SEQ ID NO:4;
(x) residues 21-239 of SEQ ID NO:2 and residues 20-469 of SEQ ID NO:4;
(xi) residues 21-239 of SEQ ID NO:2 and residues 20-469 of SEQ ID NO:5;
(xii) SEQ ID NO:2 and SEQ ID NO:4; and
(xiii) SEQ ID NO:2 and SEQ ID NO:5.

**7.** The anti-CD40 antibody or antigen-binding fragment or use of claim 5, wherein said anti-CD40 antibody or antigen-binding fragment thereof comprises an amino acid sequence selected from the group consisting of:

(i) residues 21-132 of SEQ ID NO:6;
(ii) residues 21-239 of SEQ ID NO:6;
(iii) SEQ ID NO:6;
(iv) residues 20-144 of SEQ ID NO:7;
(v) residues 20-474 of SEQ ID NO:7;
(vi) SEQ ID NO:7;
(vii) residues 20-474 of SEQ ID NO:8;
(viii) SEQ ID NO:8;
(ix) residues 21-132 of SEQ ID NO:6 and residues 20-144 of SEQ ID NO:7;
(x) residues 21-239 of SEQ ID NO:6 and residues 20-474 of SEQ ID NO:7;
(xi) residues 21-239 of SEQ ID NO:6 and residues 20-474 of SEQ ID NO:8
(xii) SEQ ID NO:6 and SEQ ID NO:7; and
(xiii) SEQ ID NO:6 and SEQ ID NO:8.

**8.** The anti-CD40 antibody or antigen-binding fragment or use of any preceding claim, wherein said antibody or antigen-binding fragment thereof binds to human CD40 antigen with an affinity ($K_D$) of at least $10^{-6}$ M.

**9.** The anti-CD40 antibody or antigen-binding fragment or use of claim 8, wherein said antibody or antigen-binding fragment thereof binds to human CD40 antigen with an affinity ($K_D$) of at least $10^{-8}$ M.

**10.** The anti-CD40 antibody or antigen-binding fragment or use of any preceding claim, wherein said antibody or antigen-binding fragment thereof is produced in a CHO cell line.

**11.** The anti-CD40 antibody or antigen-binding fragment of claim 1 or the use of claim 2, wherein said combination therapy provides a synergistic therapeutic effect.

**12.** The anti-CD40 antibody or antigen-binding fragment of claim 1 or the use of claim 2, wherein said antigen-binding fragment of said anti-CD40 antibody is selected from the group consisting of an Fab fragment, an $F(ab')_2$ fragment, an Fv fragment, and a single-chain Fv fragment.

**13.** The anti-CD40 antibody or antigen-binding fragment of claim 1 or the use of claim 2, wherein said IL-2 is human IL-2 or biologically active variant thereof.

**14.** The anti-CD40 antibody or antigen-binding fragment or use of claim 13, wherein said variant of human IL-2 is des-alanyl-1, serine-125 human IL-2.

**15.** The anti-CD40 antibody or antigen-binding fragment of claim 1 or the use of claim 2, wherein the cancer is a B cell-related cancer or solid tumor.

**16.** The anti-CD40 antibody or antigen-binding fragment or use of claim 15, wherein the B cell-related cancer is selected from the group consisting of non-Hodgkin's lymphoma, chronic lymphocytic leukemia, multiple myeloma, B cell lymphoma, high-grade B cell lymphoma, intermediate-grade B cell lymphoma, low-grade B cell lymphoma, B cell acute lymphoblastic leukemia, myeloblastic leukemia, Hodgkin's disease, plasmacytoma, follicular lymphoma, follicular small cleaved lymphoma, follicular large cell lymphoma, follicular mixed small cleaved lymphoma, diffuse small cleaved cell lymphoma, diffuse small lymphocytic lymphoma, prolymphocytic leukemia, lymphoplasmacytic lymphoma, marginal zone lymphoma, mucosal associated lymphoid tissue lymphoma, monocytoid B cell lymphoma, splenic lymphoma, hairy cell leukemia, diffuse large cell lymphoma, mediastinal large B cell lymphoma, lymphomatoid granulomatosis, intravascular lymphomatosis, diffuse mixed cell lymphoma, diffuse large cell lymphoma, immunoblastic lymphoma, Burkitt's lymphoma, AIDS-related lymphoma, and mantle cell lymphoma.

**17.** The anti-CD40 antibody or antigen-binding fragment or use of claim 15, wherein said solid tumor is selected from the group consisting of urinary bladder carcinoma, breast carcinoma, liver carcinoma, gastric carcinoma, colon carcinoma, prostate cancer, renal cell carcinoma, nasopharyngeal carcinoma, squamous cell carcinoma, thyroid papillary carcinoma, melanoma, ovarian carcinoma, lung carcinoma, cervical carcinoma, and sarcomas.

**18.** The anti-CD40 antibody or antigen-binding fragment of claim 1 or the use of claim 2, wherein said IL-2 or variant thereof and said anti-CD40 antibody or antigen-binding fragment thereof are administered sequentially.

**19.** The anti-CD40 antibody or antigen-binding fragment of claim 1 or the use of claim 2, wherein said IL-2 or variant thereof and said anti-CD40 antibody or antigen-binding fragment thereof are administered simultaneously.

**20.** The anti-CD40 antibody or antigen-binding fragment of claim 1 or the use of claim 2, wherein said anti-CD40 antibody or antigen-binding fragment thereof is administered according to a dosing schedule selected from the group consisting of once per week, once every two weeks, once every three weeks, and once every four weeks throughout a treatment period or for a fixed duration of 4 weeks to 16 weeks within said treatment period in combination with the administration of one or more cycles of a constant IL-2 dosing regimen during said treatment period, wherein said constant IL-2 dosing regimen comprises a first time period, wherein a constant total weekly dose of IL-2 or biologically active variant thereof is administered to said subject, and a second time period, wherein administration of said IL-2 or biologically variant thereof is withheld from said subject.

**21.** The anti-CD40 antibody or antigen-binding fragment or use of claim 20, wherein said first time period has a duration of about 2 weeks to about 12 weeks, and wherein said second time period has a duration of about 1 week to about 4 weeks.

**22.** The anti-CD40 antibody or antigen-binding fragment or use of claim 21, wherein said first time period has a duration of 4 weeks, and wherein said second time period has a duration of 1 week.

**23.** The anti-CD40 antibody or antigen-binding fragment or use of claim 21, wherein a first administration of said anti-CD40 antibody or antigen-binding fragment thereof begins on day 1 of said treatment period, and wherein a first cycle of said constant IL-2 dosing regimen is initiated within 10 days of said first administration of said anti-CD40 antibody or antigen-binding fragment thereof.

**24.** The anti-CD40 antibody or antigen-binding fragment or use of claim 23, wherein said first cycle of said constant IL-2 dosing regimen is initiated on day 8 of said treatment period.

**25.** The anti-CD40 antibody or antigen-binding fragment or use of claim 23, wherein said treatment period comprises one or more subsequent cycles of said constant IL-2 dosing regimen that is initiated within 4 weeks following completion of said first cycle of said constant IL-2 dosing regimen or completion of any subsequent cycle of said constant IL-2 dosing regimen, wherein said administration of said anti-CD40 antibody or antigen-binding fragment thereof continues throughout said treatment period.

**26.** The anti-CD40 antibody or antigen-binding fragment or use of claim 20, wherein said therapeutically effective dose of said anti-CD40 antibody or antigen-binding fragment thereof is in the range from about 0.5 mg/kg to about 30.0 mg/kg.

**27.** The anti-CD40 antibody or antigen-binding fragment or use of claim 20, wherein said constant total weekly dose of IL-2 or biologically active variant thereof is administered as a single dose or is partitioned into a first series of equivalent doses that are administered according to a two-, three-, four-, five-, six- or seven-times-a-week dosing schedule.

**28.** The anti-CD40 antibody or antigen-binding fragment or use of claim 27, wherein said IL-2 or biologically active variant thereof is administered by a route selected from the group consisting of intravenous, intramuscular, and subcutaneous.

**29.** The anti-CD40 antibody or antigen-binding fragment or use of claim 20, wherein said constant total weekly dose of said IL-2 or biologically active variant thereof is an amount equivalent to a total weekly dose of a reference IL-2 standard administered by the same route and with the same dosing schedule, wherein said total weekly dose of the reference IL-2 standard is in a range from about 1100 μg (18.0 MIU) to about 3300 μg (54.0 MIU), said constant total weekly dose of said IL-2 or biologically active variant thereof providing at least 70% of the natural killer (NK) cell activity that is provided by said total weekly dose of the reference IL-2 standard.

**30.** The anti-CD40 antibody or antigen-binding fragment or use of claim 29, wherein said total weekly dose of the reference IL-2 standard is about 1100 μg (18.0 MIU) to about 2567 μg (42.0 MIU), and wherein said total weekly dose of the reference IL-2 standard is partitioned into three equivalent doses that are administered according to a three-times-a-week dosing schedule.

**31.** The anti-CD40 antibody or antigen-binding fragment of claim 1 or the use of claim 2, wherein said anti-CD40 antibody or antigen-binding fragment thereof is administered according to a dosing schedule selected from the group consisting of once per week, once every two weeks, once every three weeks, and once every four weeks throughout a treatment period or for a fixed duration of 4 weeks to 16 weeks within said treatment period in combination with the administration of one or more cycles of a two-level IL-2 dosing regimen during said treatment period, wherein said two-level IL-2 dosing regimen comprises a first time period, wherein a higher total weekly dose of an IL-2 or biologically active variant thereof is administered to said subject, followed by a second time period, wherein a lower total weekly dose of said IL-2 or biologically active variant thereof is administered to said subject.

**32.** The anti-CD40 antibody or antigen-binding fragment or use of claim 31, wherein a first dose of said IL-2 or biologically active variant thereof is administered to said subject prior to administering a first dose of said anti-CD40 antibody or antigen-binding fragment thereof.

**33.** The anti-CD40 antibody or antigen-binding fragment or use of claim 32, wherein said first dose of said IL-2 or biologically active variant thereof is administered up to one month before said first dose of said anti-CD40 antibody or antigen-binding fragment thereof is administered to said subject.

**34.** The anti-CD40 antibody or antigen-binding fragment or use of claim 33, wherein said first dose of said IL-2 or biologically active variant thereof is administered one week before said first dose of said anti-CD40 antibody or antigen-binding fragment thereof is administered to said subject.

**35.** The anti-CD40 antibody or antigen-binding fragment or use of claim 31, wherein a first dose of said IL-2 or biologically active variant thereof is administered to said subject concurrently with a first dose of said anti-CD40 antibody or antigen-binding fragment thereof.

**36.** The anti-CD40 antibody or antigen-binding fragment or use of claim 31, wherein a first dose of said IL-2 or biologically active variant thereof is administered to said subject one week after a first dose of said anti-CD40 antibody or antigen-binding fragment thereof is administered to said subject.

**37.** The anti-CD40 antibody or antigen-binding fragment or use of claim 31, wherein said therapeutically effective dose of said anti-CD40 antibody or antigen-binding fragment thereof is in the range from about 0.5 mg/kg to about 30.0 mg/kg.

**38.** The anti-CD40 antibody or antigen-binding fragment or use of claim 31, wherein said two-level IL-2 dosing regimen has a combined duration of 4 weeks to 16 weeks.

**39.** The anti-CD40 antibody or antigen-binding fragment or use of claim 38, wherein said first time period of said two-level IL-2 dosing regimen has a duration of at least 1 week out of said combined duration of 4 weeks to 16 weeks.

**40.** The anti-CD40 antibody or antigen-binding fragment or use of claim 38, wherein said first time period of said two-level dosing regimen of IL-2 has a duration that is one-half of said combined duration of 4 weeks to 16 weeks.

**41.** The anti-CD40 antibody or antigen-binding fragment or use of claim 31, wherein said higher total weekly dose of said IL-2 or biologically active variant thereof is administered as a single dose or is partitioned into a first series of equivalent doses that are administered according to a two-, three-, four-, five-, six- or seven-times-a-week dosing schedule, and wherein said lower total weekly dose of said IL-2 or biologically active variant thereof is administered as a single dose or is partitioned into a second series of equivalent doses that are administered according to a two-, three-, four-, five-, six- or seven-times-a-week dosing schedule.

**42.** The anti-CD40 antibody or antigen-binding fragment or use of claim 41, wherein said IL-2 or biologically active variant thereof is administered by a route selected from the group consisting of intravenous, intramuscular, and subcutaneous.

**43.** The anti-CD40 antibody or antigen-binding fragment or use of claim 42, wherein said first series of equivalent doses is administered according to three-times-a-week dosing schedule, and wherein said second series of equivalent doses is administered according to a three-times-a-week dosing schedule.

**44.** The anti-CD40 antibody or antigen-binding fragment or use of claim 31, wherein:

a) said higher total weekly dose of said IL-2 or biologically active variant thereof is an amount equivalent to a higher total weekly dose of a reference IL-2 standard administered by the same route and with the same dosing schedule, wherein said higher total weekly dose of the reference IL-2 standard is in a range from about 1834 μg (30.0 MIU) to about 3300 μg (54.0 MIU), said higher total weekly dose of said IL-2 or biologically active variant thereof providing at least 70% of the natural killer (NK) activity that is provided by said higher total weekly dose of the reference IL-2 standard;
b) said lower total weekly dose of said IL-2 or biologically active variant thereof is an amount equivalent to a lower total weekly dose of the reference IL-2 standard administered by the same route and with the same dosing schedule, wherein said lower total weekly dose of the reference IL-2 standard is in a range from about 1100 μg (18.0 MIU) to about 2384 μg (39.0 MIU), said lower total weekly dose of said IL-2 or biologically active variant thereof providing at least 70% of the natural killer (NK) activity that is provided by said lower total weekly dose of the reference IL-2 standard; and
c) said lower total weekly dose of said IL-2 or biologically active variant thereof is lower than said higher total weekly dose of said IL-2 or biologically active variant thereof.

**45.** The anti-CD40 antibody or antigen-binding fragment or use of claim 44, wherein said higher total weekly dose of the reference IL-2 standard is about 1834 μg (30.0 MIU) to about 2567 μg (42.0 MIU) and said lower total weekly dose of the reference IL-2 standard is about 1100 μg (18.0 MIU) to about 1834 μg (30.0 MIU).

**46.** The anti-CD40 antibody or antigen-binding fragment or use of claim 45, wherein said higher total weekly dose of the reference IL-2 standard is about 2567 μg (42.0 MIU), said lower total weekly dose of the reference IL-2 standard is about 1834 μg (30.0 MIU), and wherein said higher total weekly dose of the reference IL-2 standard and said lower total weekly dose of the reference IL-2 standard are each partitioned into three equivalent doses that are administered according to a three-times-a-week dosing schedule.

**47.** The anti-CD40 antibody or antigen-binding fragment or use of claim 31, further comprising an interruption in said two-level IL-2 dosing regimen, said interruption comprising a time period off of administration of said IL-2 or biologically active variant thereof between said first time period and said second time period of said two-level IL-2 dosing regimen.

**48.** The anti-CD40 antibody or antigen-binding fragment or use of claim 47, wherein said interruption has a duration of about 1 week to about 4 weeks.

**49.** The anti-CD40 antibody or antigen-binding fragment or use of claim 31, wherein said treatment period comprises one or more subsequent cycles of said two-level IL-2 dosing regimen that is initiated about 1 week to about 4 weeks following completion of a first cycle of said two-level IL-2 dosing regimen or completion of any subsequent cycle of said two-level IL-2 dosing regimen, wherein said weekly administration of said anti-CD40 antibody or antigen-binding fragment thereof continues throughout said treatment period.

**50.** The anti-CD40 antibody or antigen-binding fragment or use of claim 49, wherein at least one cycle of said two-level IL-2 dosing regimen comprises an interruption in said two-level IL-2 dosing regimen, said interruption comprising a time period off of administration of said IL-2 or biologically active variant thereof between said first time period and said second time period of any given cycle of said two-level IL-2 dosing regimen that comprises said interruption.

**51.** The anti-CD40 antibody or antigen-binding fragment or use according to any one of claims 1-50, wherein said anti-CD40 antibody or antigen-binding fragment thereof is administered intravenously or subcutaneously.

**Patentansprüche**

**1.** Anti-CD40-Antikörper oder Antigen-bindendes Fragment davon, zur Verwendung in einem Verfahren zur Behandlung eines humanen Subjekts im Hinblick auf einen Krebs, der neoplastische Zellen umfasst, welche CD40-Antigen exprimieren, wobei das Verfahren die Verabreichung einer Kombinationstherapie an das Subjekt umfasst, wobei die Therapie die Verabreichung einer wirksamen Menge des Anti-CD40-Antikörpers oder des Antigen-bindenden Fragments davon in Kombination mit einem Interleukin-2 (IL-2) oder einer biologisch aktiven Variante davon umfasst, wobei der Anti-CD40-Antikörper oder das Antigen-bindende Fragment davon frei von signifikanter agonistischer Aktivität ist, wenn er/es an CD40-Antigen gebunden ist, und ausgewählt ist aus der Gruppe bestehend aus:

(a) einem Antikörper oder einem Antigen-bindenden Fragment davon, der/das an ein Epitop bindet, welches in der Lage ist, den monoklonalen Antikörper CHIR-5.9 zu binden, welcher von der Hybridom-Zelllinie erhältlich ist, die bei der ATCC als Patent-Hinterlegungsnummer PTA-5542 hinterlegt ist, oder den monoklonalen Antikörper CHIR-12.12, welcher von der Hybridom-Zelllinie erhältlich ist, die bei der ATCC als Patent-Hinterlegungsnummer PTA-5543 hinterlegt ist;
(b) einem Antikörper oder einem Antigen-bindenden Fragment davon, der/das an ein Epitop bindet, welches die Reste 82-87 der in SEQ ID NO:10 oder SEQ ID NO:12 gezeigten humanen CD40-Sequenz umfasst;
(c) einem Antikörper oder einem Antigen-bindenden Fragment davon, der/das an ein Epitop bindet, welches die Reste 82-89 der in SEQ ID NO:10 oder SEQ ID NO:12 gezeigten humanen CD40-Sequenz umfasst; und
(d) einem Antikörper oder einem Antigen-bindenden Fragment davon, der/das mit dem monoklonalen Antikörper CHIR-5.9, welcher von der Hybridom-Zelllinie erhältlich ist, die bei der ATCC als Patent-Hinterlegungsnummer PTA-5542 hinterlegt ist, oder mit dem monoklonalen Antikörper CHIR-12.12, welcher von der Hybridom-Zelllinie erhältlich ist, die bei der ATCC als Patent-Hinterlegungsnummer PTA-5543 hinterlegt ist, in einem kompetitiven Bindungsassay konkurriert.

**2.** Verwendung einer wirksamen Menge eines Anti-CD40-Antikörpers oder eines Antigen-bindenden Fragments davon in Kombination mit einem Interleukin-2 (IL-2) oder einer biologisch aktiven Variante davon bei der Herstellung eines Medikaments zur Behandlung eines humanen Subjekts im Hinblick auf einen Krebs, der neoplastische Zellen umfasst, welche CD40-Antigen exprimieren, durch Kombinationstherapie, wobei der Anti-CD40-Antikörper oder das Antigen-bindende Fragment davon frei von signifikanter agonistischer Aktivität ist, wenn er/es an CD40-Antigen gebunden ist, und ausgewählt ist aus der Gruppe bestehend aus:

(a) einem Antikörper oder einem Antigen-bindenden Fragment davon, der/das an ein Epitop bindet, welches in der Lage ist, den monoklonalen Antikörper CHIR-5.9 zu binden, welcher von der Hybridom-Zelllinie erhältlich ist, die bei der ATCC als Patent-Hinterlegungsnummer PTA-5542 hinterlegt ist, oder den monoklonalen Antikörper CHIR-12.12, welcher von der Hybridom-Zelllinie erhältlich ist, die bei der ATCC als Patent-Hinterlegungsnummer PTA-5543 hinterlegt ist;
(b) einem Antikörper oder einem Antigen-bindenden Fragment davon, der/das an ein Epitop bindet, welches die Reste 82-87 der in SEQ ID NO:10 oder SEQ ID NO:12 gezeigten humanen CD40-Sequenz umfasst;
(c) einem Antikörper oder einem Antigen-bindenden Fragment davon, der/das an ein Epitop bindet, welches die Reste 82-89 der in SEQ ID NO:10 oder SEQ ID NO:12 gezeigten humanen CD40-Sequenz umfasst; und
(d) einem Antikörper oder einem Antigen-bindenden Fragment davon, der/das mit dem monoklonalen Antikörper CHIR-5.9, welcher von der Hybridom-Zelllinie erhältlich ist, die bei der ATCC als Patent-Hinterlegungsnummer PTA-5542 hinterlegt ist, oder mit dem monoklonalen Antikörper CHIR-12.12, welcher von der Hybridom-Zelllinie erhältlich ist, die bei der ATCC als Patent-Hinterlegungsnummer PTA-5543 hinterlegt ist, in einem kompetitiven Bindungsassay konkurriert.

3. Anti-CD40-Antikörper oder Antigen-bindendes Fragment davon nach Anspruch 1 oder Verwendung nach Anspruch 2, wobei der Anti-CD40-Antikörper ein humaner Antikörper ist.

4. Anti-CD40-Antikörper oder Antigen-bindendes Fragment davon nach Anspruch 1 oder Verwendung nach Anspruch 2, wobei der Anti-CD40-Antikörper ausgewählt ist aus der Gruppe bestehend aus dem monoklonalen Antikörper CHIR-5.9, welcher von der Hybridom-Zelllinie erhältlich ist, die bei der ATCC als Patent-Hinterlegungsnummer PTA-5542 hinterlegt ist, und dem monoklonalen Antikörper CHIR-12.12, welcher von der Hybridom-Zelllinie erhältlich ist, die bei der ATCC als Patent-Hinterlegungsnummer PTA-5543 hinterlegt ist.

5. Anti-CD40-Antikörper oder Antigen-bindendes Fragment davon nach Anspruch 1 oder Verwendung nach Anspruch 2, wobei der Anti-CD40-Antikörper oder ein Antigen-bindendes Fragment davon ausgewählt ist aus der Gruppe bestehend aus:

   (i) einem Antikörper oder einem Antigen-bindenden Fragment davon, der/das eine variable Domäne einer leichten Kette umfasst, welche die Reste 44-54, 70-76 und 109-117 von SEQ ID NO:2 oder SEQ ID NO:6 enthält;
   (ii) einem Antikörper oder einem Antigen-bindenden Fragment davon, der/das eine variable Domäne einer schweren Kette umfasst, welche die Reste 50-54, 69-84 und 114-121 von SEQ ID NO:4 oder SEQ ID NO:7 enthält;
   (iii) einem Antikörper oder einem Antigen-bindenden Fragment davon, der/das eine variable Domäne einer leichten Kette umfasst, welche die Reste 46-52, 70-72 und 111-116 von SEQ ID NO:2 oder SEQ ID NO:6 enthält; und
   (iv) einem Antikörper oder einem Antigen-bindenden Fragment davon, der/das eine variable Domäne einer schweren Kette umfasst, welche die Reste 45-51, 72-74 und 115-120 von SEQ ID NO:4 oder SEQ ID NO:7 umfasst.

6. Anti-CD40-Antikörper oder Antigen-bindendes Fragment davon oder Verwendung nach Anspruch 5, wobei der Anti-CD40-Antikörper oder das Antigen-bindende Fragment davon eine Aminosäuresequenz umfasst, die ausgewählt ist aus der Gruppe bestehend aus:

   (i) den Resten 21-132 von SEQ ID NO:2;
   (ii) den Resten 21-239 von SEQ ID NO:2;
   (iii) SEQ ID NO:2;
   (iv) den Resten 20-139 von SEQ ID NO:4;
   (v) den Resten 20-469 von SEQ ID NO:4;
   (vi) SEQ ID NO:4;
   (vii) den Resten 20-469 von SEQ ID NO:5;
   (viii) SEQ ID NO:5;
   (ix) den Resten 21-132 von SEQ ID NO:2 und den Resten 20-139 von SEQ ID NO:4;
   (x) den Resten 21-239 von SEQ ID NO:2 und den Resten 20-469 von SEQ ID NO:4;
   (xi) den Resten 21-239 von SEQ ID NO:2 und den Resten 20-469 von SEQ ID NO:5;
   (xii) SEQ ID NO:2 und SEQ ID NO:4; und
   (xiii) SEQ ID NO:2 und SEQ ID NO:5.

7. Anti-CD40-Antikörper oder Antigen-bindendes Fragment davon oder Verwendung nach Anspruch 5, wobei der Anti-CD40-Antikörper oder das Antigen-bindende Fragment davon eine Aminosäuresequenz umfasst, die ausgewählt ist aus der Gruppe bestehend aus:

   (i) den Resten 21-132 von SEQ ID NO:6;
   (ii) den Resten 21-239 von SEQ ID NO:6;
   (iii) SEQ ID NO:6;
   (iv) den Resten 20-144 von SEQ ID NO:7;
   (v) den Resten 20-474 von SEQ ID NO:7;
   (vi) SEQ ID NO:7;
   (vii) den Resten 20-474 von SEQ ID NO:8;
   (viii) SEQ ID NO:8;
   (ix) den Resten 21-132 von SEQ ID NO:6 und den Resten 20-144 von SEQ ID NO:7;
   (x) den Resten 21-239 von SEQ ID NO:6 und den Resten 20-474 von SEQ ID NO:7;
   (xi) den Resten 21-239 von SEQ ID NO:6 und den Resten 20-474 von SEQ ID NO:8;

(xii) SEQ ID NO:6 und SEQ ID NO:7; und
(xiii) SEQ ID NO:6 und SEQ ID NO:8.

**8.** Anti-CD40-Antikörper oder Antigen-bindendes Fragment davon oder Verwendung nach einem der vorhergehenden Ansprüche, wobei der Antikörper oder das Antigen-bindende Fragment davon mit einer Affinität ($K_D$) von mindestens $10^{-6}$ M an das humane CD40-Antigen bindet.

**9.** Anti-CD40-Antikörper oder Antigen-bindendes Fragment davon oder Verwendung nach Anspruch 8, wobei der Antikörper oder das Antigen-bindende Fragment davon mit einer Affinität ($K_D$) von mindestens $10^{-8}$ M an das humane CD40-Antigen bindet.

**10.** Anti-CD40-Antikörper oder Antigen-bindendes Fragment davon oder Verwendung nach einem der vorhergehenden Ansprüche, wobei der Antikörper oder das Antigen-bindende Fragment davon in einer CHO-Zelllinie produziert wird.

**11.** Anti-CD40-Antikörper oder Antigen-bindendes Fragment davon nach Anspruch 1 oder Verwendung nach Anspruch 2, wobei die Kombinationstherapie einen synergistischen therapeutischen Effekt bereitstellt.

**12.** Anti-CD40-Antikörper oder Antigen-bindendes Fragment davon nach Anspruch 1 oder Verwendung nach Anspruch 2, wobei das Antigen-bindende Fragment des Anti-CD40-Antikörpers ausgewählt ist aus der Gruppe bestehend aus einem Fab-Fragment, einem $F(ab')_2$ Fragment, einem Fv-Fragment und einem einzelkettigen Fv-Fragment.

**13.** Anti-CD40-Antikörper oder Antigen-bindendes Fragment davon nach Anspruch 1 oder Verwendung nach Anspruch 2, wobei das IL-2 humanes IL-2 oder eine biologisch aktive Variante davon ist.

**14.** Anti-CD40-Antikörper oder Antigen-bindendes Fragment davon oder Verwendung nach Anspruch 13, wobei die Variante des humanen IL-2 humanes des-Alanyl-1, Serin-125-IL-2 ist.

**15.** Anti-CD40-Antikörper oder Antigen-bindendes Fragment davon nach Anspruch 1 oder Verwendung nach Anspruch 2, wobei der Krebs ein B-Zell-assoziierter Krebs oder ein solider Tumor ist.

**16.** Anti-CD40-Antikörper oder Antigen-bindendes Fragment davon oder Verwendung nach Anspruch 15, wobei der B-Zellassoziierte Krebs ausgewählt ist aus der Gruppe bestehend aus Non-Hodgkin-Lymphom, chronischer lymphozytischer Leukämie, multiplem Myelom, B-Zell-Lymphom, hochmalignem B-Zell-Lymphom, mittelmalignem B-Zell-Lymphom, niedrigmalignem B-Zell-Lymphom, akuter lymphoblastischer B-Zell-Leukämie, myeloblastischer Leukämie, Hodgkin-Erkrankung, Plasmazytom, follikulärem Lymphom, follikulärem kleinzellig-gekerbtem Lymphom, follikulärem großzelligem Lymphom, follikulärem gemischtem kleinzellig-gekerbtem Lymphom, diffus kleinzellig-gekerbtem Zell-Lymphom, diffus kleinem lymphozytischem Lymphom, prolymphozytischer Leukämie, lymphoplasmazytischem Lymphom, Marginalzonen-Lymphom, mukosal-assoziiertem Lymphgewebe-Lymphom, monozytoidem B-Zell-Lymphom, Milz-Lymphom, Haarzellen-Leukämie, diffus großzelligem Lymphom, mediastinalem großem B-Zell-Lymphom, lymphomatoider Granulomatose, intravaskulärer Lymphomatose, diffus mischzelligem Lymphom, diffus großzelligem Lymphom, immunoblastischem Lymphom, Burkitt-Lymphom, AIDSbedingtem Lymphom und Mantelzellen-Lymphom.

**17.** Anti-CD40-Antikörper oder Antigen-bindendes Fragment davon oder Verwendung nach Anspruch 15, wobei der solide Tumor ausgewählt ist aus der Gruppe bestehend aus Harnblasenkarzinom, Brustkarzinom, Leberkarzinom, Magenkarzinom, Kolonkarzinom, Prostatakrebs, Nierenzellkarzinom, nasopharyngales Karzinom, Plattenepithelkarzinom, papilläres Schilddrüsenkarzinom, Melanom, Ovarialkarzinom, Lungenkarzinom, Gebärmutterhalskrebs und Sarkome.

**18.** Anti-CD40-Antikörper oder Antigen-bindendes Fragment davon nach Anspruch 1 oder Verwendung nach Anspruch 2, wobei das IL-2 oder die Variante davon und der Anti-CD40-Antikörper oder das Antigen-bindende Fragment davon nacheinander verabreicht werden.

**19.** Anti-CD40-Antikörper oder Antigen-bindendes Fragment davon nach Anspruch 1 oder Verwendung nach Anspruch 2, wobei das IL-2 oder die Variante davon und der Anti-CD40-Antikörper oder das Antigen-bindende Fragment davon gleichzeitig verabreicht werden.

**20.** Anti-CD40-Antikörper oder Antigen-bindendes Fragment davon nach Anspruch 1 oder Verwendung nach Anspruch 2, wobei der Anti-CD40-Antikörper oder das Antigen-bindende Fragment davon nach einem Dosierungsplan verabreicht wird, der ausgewählt ist aus der Gruppe bestehend aus einmal pro Woche, einmal alle zwei Wochen, einmal alle drei Wochen, einmal alle vier Wochen für einen Behandlungszeitraum oder für eine festgelegte Dauer von 4 Wochen bis 16 Wochen innerhalb des Behandlungszeitraums, in Kombination mit der Verabreichung von einem oder mehreren Zyklen eines konstanten IL-2-Dosierungsplans während des Behandlungszeitraums, wobei der konstante IL-2-Dosierungsplan einen ersten Zeitraum umfasst, in dem eine konstante wöchentliche Gesamtdosis von IL-2 oder der biologisch aktiven Variante davon an das Subjekt verabreicht wird, und einen zweiten Zeitraum, in dem das IL-2 oder die biologisch aktive Variante davon dem Subjekt vorenthalten wird.

**21.** Anti-CD40-Antikörper oder Antigen-bindendes Fragment davon oder Verwendung nach Anspruch 20, wobei der erste Zeitraum eine Dauer von etwa 2 Wochen bis etwa 12 Wochen aufweist, und wobei der zweite Zeitraum eine Dauer von etwa 1 Woche bis etwa 4 Wochen aufweist.

**22.** Anti-CD40-Antikörper oder Antigen-bindendes Fragment davon oder Verwendung nach Anspruch 21, wobei der erste Zeitraum eine Dauer von 4 Wochen aufweist, und wobei der zweite Zeitraum eine Dauer von 1 Woche aufweist.

**23.** Anti-CD40-Antikörper oder Antigen-bindendes Fragment davon oder Verwendung nach Anspruch 21, wobei eine erste Verabreichung des Anti-CD40-Antikörpers oder des Antigen-bindenden Fragments davon am Tag 1 des Behandlungszeitraums beginnt, und wobei ein erster Zyklus des konstanten IL-2-Dosierungsplans innerhalb von 10 Tagen nach der ersten Verabreichung des Anti-CD40-Antikörpers oder des Antigen-bindenden Fragments davon initiiert wird.

**24.** Anti-CD40-Antikörper oder Antigen-bindendes Fragment davon oder Verwendung nach Anspruch 23, wobei der erste Zyklus des konstanten IL-2-Dosierungsplans am Tag 8 des Behandlungszeitraums initiiert wird.

**25.** Anti-CD40-Antikörper oder Antigen-bindendes Fragment davon oder Verwendung nach Anspruch 23, wobei der Behandlungszeitraum einen oder mehrere aufeinanderfolgende Zyklen des konstanten IL-2-Dosierungsplans umfasst, der innerhalb von vier Wochen nach Abschluss des ersten Zyklus des konstanten IL-2-Dosierungsplans oder nach Abschluss eines beliebigen nachfolgenden Zyklus des konstanten IL-2-Dosierungsplans initiiert wird, wobei die Verabreichung des Anti-CD40-Antikörpers oder des Antigen-bindenden Fragments davon während des Behandlungszeitraums fortgeführt wird.

**26.** Anti-CD40-Antikörper oder Antigen-bindendes Fragment davon oder Verwendung nach Anspruch 20, wobei die therapeutisch wirksame Dosis des Anti-CD40-Antikörpers oder des Antigen-bindenden Fragments davon im Bereich von etwa 0,5 mg/kg bis etwa 30,0 mg/kg liegt.

**27.** Anti-CD40-Antikörper oder Antigen-bindendes Fragment davon oder Verwendung nach Anspruch 20, wobei die konstante wöchentliche Gesamtdosis von IL-2 oder der biologisch aktiven Variante davon als Einzeldosis verabreicht wird, oder aufgeteilt wird in eine erste Reihe von äquivalenten Dosen, die nach einem Dosierungsplan zwei-, drei-, vier, fünf-, sechs- oder siebenmal pro Woche verabreicht werden.

**28.** Anti-CD40-Antikörper oder Antigen-bindendes Fragment davon oder Verwendung nach Anspruch 27, wobei das IL-2 oder die biologisch aktive Variante davon auf einem Weg verabreicht wird, der ausgewählt ist aus der Gruppe bestehend aus intranvenös, intramuskulär und subkutan.

**29.** Anti-CD40-Antikörper oder Antigen-bindendes Fragment davon oder Verwendung nach Anspruch 20, wobei die konstante wöchentliche Gesamtdosis von IL-2 oder der biologisch aktiven Variante davon eine Menge ist, die äquivalent zu einer wöchentlichen Gesamtdosis eines IL-2-Referenzstandards ist, der auf gleichem Wege und nach dem gleichen Dosierungsplan verabreicht wird, wobei die wöchentliche Gesamtdosis des IL-2-Referenzstandards in einem Bereich von etwa 1100 μg (18,0 MIU) bis etwa 3300 μg (54,0 MIU) liegt, und wobei die wöchentliche Gesamtdosis von IL-2 oder der biologisch aktiven Variante davon mindestens 70% der Aktivität der natürlichen Killerzellen bereitstellt, welche von der wöchentlichen Gesamtdosis des IL-2-Referenzstandards bereitgestellt wird.

**30.** Anti-CD40-Antikörper oder Antigen-bindendes Fragment davon oder Verwendung nach Anspruch 29, wobei die wöchentliche Gesamtdosis des IL-2-Referenzstandards etwa 1100 μg (18,0 MIU) bis etwa 2567 μg (42,0 MIU) beträgt, und wobei die wöchentliche Gesamtdosis des IL-2-Referenzstandards in drei äquivalente Dosen aufgeteilt ist, welche gemäß eines Dosierungsplans dreimal pro Woche verabreicht werden.

**31.** Anti-CD40-Antikörper oder Antigen-bindendes Fragment davon nach Anspruch 1 oder Verwendung nach Anspruch 2, wobei der Anti-CD40-Antikörper oder das Antigen-bindende Fragment davon nach einem Dosierungsplan verabreicht wird, welcher ausgewählt ist aus der Gruppe bestehend aus einmal pro Woche, einmal alle zwei Wochen, einmal alle drei Wochen und einmal alle vier Wochen für einen Behandlungszeitraum oder für eine festgelegte Dauer von 4 Wochen bis 16 Wochen innerhalb des Behandlungszeitraums, in Kombination mit der Verabreichung von einem oder mehreren Zyklen eines zweistufigen IL-2 Dosierungsplans während des Behandlungszeitraums, wobei der zweistufige IL-2 Dosierungsplan einen ersten Zeitraum umfasst, in dem eine höhere wöchentliche Gesamtdosis von IL-2 oder der biologisch aktiven Variante davon an das Subjekt verabreicht wird, gefolgt von einem zweiten Zeitraum, in dem eine geringere wöchentliche Gesamtdosis von IL-2 oder der biologisch aktiven Variante davon an das Subjekt verabreicht wird.

**32.** Anti-CD40-Antikörper oder Antigen-bindendes Fragment davon oder Verwendung nach Anspruch 31, wobei eine erste Dosis von IL-2 oder der biologisch aktiven Variante davon vor der Verabreichung einer ersten Dosis des Anti-CD40-Antikörpers oder des Antigen-bindenden Fragments davon an das Subjekt verabreicht wird.

**33.** Anti-CD40-Antikörper oder Antigen-bindendes Fragment davon oder Verwendung nach Anspruch 32, wobei die erste Dosis von IL-2 oder der biologisch aktiven Variante davon bis zu einem Monat vor der ersten Dosis des Anti-CD40-Antikörpers oder des Antigen-bindenden Fragments davon an das Subjekt verabreicht wird.

**34.** Anti-CD40-Antikörper oder Antigen-bindendes Fragment davon oder Verwendung nach Anspruch 33, wobei die erste Dosis von IL-2 oder der biologisch aktiven Variante davon eine Woche vor der ersten Dosis des Anti-CD40-Antikörpers oder des Antigen-bindenden Fragments davon an das Subjekt verabreicht wird.

**35.** Anti-CD40-Antikörper oder Antigen-bindendes Fragment davon oder Verwendung nach Anspruch 31, wobei die erste Dosis von IL-2 oder der biologisch aktiven Variante davon gleichzeitig mit einer ersten Dosis des Anti-CD40-Antikörpers oder des Antigen-bindenden Fragments davon an das Subjekt verabreicht wird.

**36.** Anti-CD40-Antikörper oder Antigen-bindendes Fragment davon oder Verwendung nach Anspruch 31, wobei eine erste Dosis von IL-2 oder der biologisch aktiven Variante davon eine Woche nach einer ersten Dosis des Anti-CD40-Antikörpers oder des Antigen-bindenden Fragments davon an das Subjekt verabreicht wird.

**37.** Anti-CD40-Antikörper oder Antigen-bindendes Fragment davon oder Verwendung nach Anspruch 31, wobei die therapeutisch wirksame Dosis des Anti-CD40-Antikörpers oder des Antigen-bindenden Fragments davon in einem Bereich von etwa 0,5 mg/kg bis etwa 30,0 mg/kg liegt.

**38.** Anti-CD40-Antikörper oder Antigen-bindendes Fragment davon oder Verwendung nach Anspruch 31, wobei der zweistufige IL-2-Dosierungsplan eine kombinierte Dauer von 4 Wochen bis 16 Wochen aufweist.

**39.** Anti-CD40-Antikörper oder Antigen-bindendes Fragment davon oder Verwendung nach Anspruch 38, wobei der erste Zeitraum des zweistufigen IL-2-Dosierungsplans eine Dauer von mindestens einer Woche der kombinierten Dauer von 4 Wochen bis 16 Wochen aufweist.

**40.** Anti-CD40-Antikörper oder Antigen-bindendes Fragment davon oder Verwendung nach Anspruch 38, wobei der erste Zeitraum des zweistufigen IL-2-Dosierungsplans eine Dauer aufweist, die der Hälfte der kombinierten Dauer von 4 Wochen bis 16 Wochen entspricht.

**41.** Anti-CD40-Antikörper oder Antigen-bindendes Fragment davon oder Verwendung nach Anspruch 31, wobei die höhere wöchentliche Gesamtdosis von IL-2 oder der biologisch aktiven Variante davon als Einzeldosis an das Subjekt verabreicht wird oder aufgeteilt wird in eine erste Reihe von äquivalenten Dosen, die nach einem Dosierungsplan zwei-, drei-, vier, fünf-, sechs- oder siebenmal pro Woche verabreicht werden, und wobei die geringere wöchentliche Gesamtdosis von IL-2 oder der biologisch aktiven Variante davon als Einzeldosis verabreicht wird oder aufgeteilt wird in eine zweite Reihe von äquivalenten Dosen, die nach einem Dosierungsplan zwei-, drei-, vier, fünf-, sechs- oder siebenmal pro Woche verabreicht werden.

**42.** Anti-CD40-Antikörper oder Antigen-bindendes Fragment davon oder Verwendung nach Anspruch 41, wobei das IL-2 oder die biologisch aktive Variante davon auf einem Weg verabreicht wird, der ausgewählt ist aus der Gruppe bestehend aus intravenös, intramuskulär und subkutan.

**43.** Anti-CD40-Antikörper oder Antigen-bindendes Fragment davon oder Verwendung nach Anspruch 42, wobei die erste Reihe von äquivalenten Dosen nach einem Dosierungsplan dreimal pro Woche verabreicht wird, und wobei die zweite Reihe von äquivalenten Dosen nach einem Dosierungsplan dreimal pro Woche verabreicht wird.

**44.** Anti-CD40-Antikörper oder Antigen-bindendes Fragment davon oder Verwendung nach Anspruch 31, wobei

(a) die höhere wöchentliche Gesamtdosis von IL-2 oder der biologisch aktiven Variante davon eine Menge ist, die äquivalent zu einer höheren wöchentlichen Gesamtdosis des IL-2-Referenzstandards ist, der auf dem gleichen Weg und nach dem gleichen Dosierungsplan verabreicht wird, wobei die höhere wöchentliche Gesamtdosis des IL-2-Referenzstandards in einem Bereich von etwa 1834 μg (30,0 MIU) bis etwa 3300 μg (54,0 MIU) liegt, und wobei die höhere wöchentliche Gesamtdosis von IL-2 oder der biologisch aktiven Variante davon mindestens 70% der Aktivität der natürlichen Killerzellen bereitstellt, welche von der wöchentlichen Gesamtdosis des IL-2-Referenzstandards bereitgestellt wird;
(b) und wobei die geringere wöchentliche Gesamtdosis von IL-2 oder der biologisch aktiven Variante davon eine Menge ist, die äquivalent zu einer geringeren wöchentlichen Gesamtdosis eines IL-2-Referenzstandards ist, der auf gleichem Weg und nach dem gleichen Dosierungsplan verabreicht wird, wobei die geringere wöchentliche Gesamtdosis des IL-2-Referenzstandards in einem Bereich von etwa 1100 μg (18,0 MIU) bis etwa 2384 μg (39,0 MIU) liegt, und wobei die geringere wöchentliche Gesamtdosis von IL-2 oder der biologisch aktiven Variante davon mindestens 70% der Aktivität der natürlichen Killerzellen bereitstellt, welche von der wöchentlichen Gesamtdosis des IL-2-Referenzstandards bereitgestellt wird; und
(c) die geringere wöchentliche Gesamtdosis von IL-2 oder der biologisch aktiven Variante davon geringer ist als die höhere wöchentliche Gesamtdosis von IL-2 oder der biologisch aktiven Variante davon.

**45.** Anti-CD40-Antikörper oder Antigen-bindendes Fragment davon oder Verwendung nach Anspruch 44, wobei die höhere wöchentliche Gesamtdosis des IL-2-Referenzstandards etwa 1834 μg (30,0 MIU) bis etwa 2567 μg (42,0 MIU) beträgt, und wobei die geringere wöchentliche Gesamtdosis des IL-2-Referenzstandards etwa 1100 μg (18,0 MIU) bis etwa 1834 μg (30,0 MIU) beträgt.

**46.** Anti-CD40-Antikörper oder Antigen-bindendes Fragment davon oder Verwendung nach Anspruch 45, wobei die höhere wöchentliche Gesamtdosis des IL-2-Referenzstandards etwa 2567 μg (42,0 MIU) beträgt, und wobei die geringere wöchentliche Gesamtdosis des IL-2-Referenzstandards etwa 1834 μg (30,0 MIU) beträgt, und wobei die höhere wöchentliche Gesamtdosis des IL-2-Referenzstandards und die geringere wöchentliche Gesamtdosis des IL-2-Referenzstandards jeweils in drei äquivalente Dosen aufgeteilt sind, welche nach einem Dosierungsplan dreimal pro Woche verabreicht werden.

**47.** Anti-CD40-Antikörper oder Antigen-bindendes Fragment davon oder Verwendung nach Anspruch 31, ferner umfassend eine Unterbrechung in dem zweistufigen IL-2-Dosierungsplan, wobei die Unterbrechung eine im Hinblick auf IL-2 oder die biologisch aktive Variante davon verabreichungsfreie Zeit zwischen dem ersten Zeitraum und dem zweiten Zeitraum des zweistufigen IL-2-Dosierungsplans umfasst.

**48.** Anti-CD40-Antikörper oder Antigen-bindendes Fragment davon oder Verwendung nach Anspruch 47, wobei die Unterbrechung eine Dauer von etwa einer Woche bis etwa 4 Wochen hat.

**49.** Anti-CD40-Antikörper oder Antigen-bindendes Fragment davon oder Verwendung nach Anspruch 31, wobei der Behandlungszeitraum einen oder mehrere aufeinanderfolgende Zyklen des zweistufigen IL-2-Dosierungsplans umfasst, der/die etwa 1 Woche bis etwa 4 Wochen nach Abschluss des ersten Zyklus des zweistufigen IL-2-Dosierungsplans oder nach Abschluss eines beliebigen nachfolgenden Zyklus des zweistufigen IL-2-Dosierungsplans initiiert wird/werden, wobei die wöchentliche Verabreichung des Anti-CD40-Antikörpers oder des Antigen-bindenden Fragment davon während des Behandlungszeitraums fortgeführt wird.

**50.** Anti-CD40-Antikörper oder Antigen-bindendes Fragment davon oder Verwendung nach Anspruch 49, wobei mindestens ein Zyklus des zweistufigen IL-2-Dosierungsplans eine Unterbrechung in dem zweistufigen IL-2-Dosierungsplan umfasst, wobei die Unterbrechung eine im Hinblick auf IL-2 oder die biologisch aktive Variante davon verabreichungsfreie Zeit zwischen dem ersten Zeitraum und dem zweiten Zeitraum eines beliebigen Zyklus des zweistufigen IL-2 Dosierungsplans umfasst, welcher diese Unterbrechung umfasst.

**51.** Anti-CD40-Antikörper oder Antigen-bindendes Fragment davon oder Verwendung nach einem der Ansprüche 1-50, wobei der Anti-CD40-Antikörper oder das Antigen-bindende Fragment davon intravenös oder subkutan verabreicht wird.

## Revendications

1. Anticorps anti-CD40 ou un de ses fragments liant l'antigène, destiné à être utilisé dans une méthode de traitement d'un sujet humain pour un cancer comprenant des cellules néoplasiques exprimant l'antigène CD40, ladite méthode comprenant l'administration audit sujet d'une thérapie de combinaison, ladite thérapie comprenant l'administration d'une quantité efficace de l'anticorps anti-CD40 ou d'un de ses fragments liant l'antigène en combinaison avec une interleukine-2 (IL-2) ou un de ses variants biologiquement actifs, ledit anticorps anti-CD40 ou un de ses fragments liant l'antigène étant dépourvu d'activité agoniste significative lorsqu'il est lié à l'antigène CD40 et étant choisi dans le groupe consistant en :

   a) un anticorps ou un de ses fragments liant l'antigène qui se lie à un épitope capable de se lier à l'anticorps monoclonal CHIR-5.9 susceptible d'être obtenu à partir de la lignée de cellules d'hybridome déposée auprès de l'ATCC sous le numéro de dépôt de brevet PTA-5542 ou à l'anticorps monoclonal CHIR-12.12 susceptible d'être obtenu à partir de la lignée de cellules d'hybridome déposée auprès de l'ATCC sous le numéro de dépôt de brevet PTA-5543 ;
   b) un anticorps ou un de ses fragments liant l'antigène qui se lie à un épitope comprenant les résidus 82-87 de la séquence du CD40 humain présentée dans SEQ ID NO:10 ou SEQ ID NO:12 ;
   c) un anticorps ou un de ses fragments liant l'antigène qui se lie à un épitope comprenant les résidus 82-89 de la séquence du CD40 humain présentée dans SEQ ID NO:10 ou SEQ ID NO:12 ; et
   d) un anticorps ou un de ses fragments liant l'antigène qui entre en compétition avec l'anticorps monoclonal CHIR-5.9 susceptible d'être obtenu à partir de la lignée de cellules d'hybridome déposée auprès de l'ATCC sous le numéro de dépôt de brevet PTA-5542 ou avec l'anticorps monoclonal CHIR-12.12 susceptible d'être obtenu à partir de la lignée de cellules d'hybridome déposée auprès de l'ATCC sous le numéro de dépôt de brevet PTA-5543 dans un essai par liaison compétitive.

2. Utilisation d'une quantité efficace d'un anticorps anti-CD40 ou d'un de ses fragments liant l'antigène en association avec une interleukine-2 (IL-2) ou un de ses variants biologiquement actifs, pour la fabrication d'un médicament destiné au traitement d'un sujet humain pour un cancer comprenant des cellules néoplasiques exprimant l'antigène CD40 par une thérapie de combinaison, dans laquelle ledit anticorps anti-CD40 ou fragment de celui-ci liant l'antigène est dépourvu d'activité agoniste significative lorsqu'il est lié à l'antigène CD40 et est choisi dans le groupe consistant en :

   a) un anticorps ou un de ses fragments liant l'antigène qui se lie à un épitope capable de se lier à l'anticorps monoclonal CHIR-5.9 susceptible d'être obtenu à partir de la lignée de cellules d'hybridome déposée auprès de l'ATCC sous le numéro de dépôt de brevet PTA-5542 ou à l'anticorps monoclonal CHIR-12.12 susceptible d'être obtenu à partir de la lignée de cellules d'hybridome déposée auprès de l'ATCC sous le numéro de dépôt de brevet PTA-5543 ;
   b) un anticorps ou un de ses fragments liant l'antigène qui se lie à un épitope comprenant les résidus 82-87 de la séquence du CD40 humain présentée dans SEQ ID NO:10 ou SEQ ID NO:12 ;
   c) un anticorps ou un de ses fragments liant l'antigène qui se lie à un épitope comprenant les résidus 82-89 de la séquence du CD40 humain présentée dans SEQ ID NO:10 ou SEQ ID NO:12 ; et
   d) un anticorps ou un de ses fragments liant l'antigène qui entre en compétition avec l'anticorps monoclonal CHIR-5.9 susceptible d'être obtenu à partir de la lignée de cellules d'hybridome déposée auprès de l'ATCC sous le numéro de dépôt de brevet PTA-5542 ou avec l'anticorps monoclonal CHIR-12.12 susceptible d'être obtenu à partir de la lignée de cellules d'hybridome déposée auprès de l'ATCC sous le numéro de dépôt de brevet PTA-5543 dans un essai par liaison compétitive.

3. Anticorps anti-CD40 ou fragment liant l'antigène selon la revendication 1 ou utilisation selon la revendication 2, ledit anticorps anti-CD40 étant un anticorps humain.

4. Anticorps anti-CD40 ou fragment liant l'antigène selon la revendication 1 ou utilisation selon la revendication 2, ledit anticorps anti-CD40 étant choisi dans le groupe consistant en l'anticorps monoclonal CHIR-5.9 susceptible d'être obtenu à partir de la lignée de cellules d'hybridome déposée auprès de l'ATCC sous le numéro de dépôt de brevet PTA-5542 et l'anticorps monoclonal CHIR-12.12 susceptible d'être obtenu à partir de la lignée de cellules d'hybridome déposée auprès de l'ATCC sous le numéro de dépôt de brevet PTA-5543.

**5.** Anticorps anti-CD40 ou fragment liant l'antigène selon la revendication 1 ou utilisation selon la revendication 2, ledit anticorps anti-CD40 ou fragment de celui-ci liant l'antigène étant choisi dans le groupe consistant en :

(i) un anticorps ou fragment de celui-ci liant l'antigène comprenant un domaine variable de chaîne légère contenant les résidus 44-54, 70-76 et 109-117 de SEQ ID NO:2 ou SEQ ID NO:6 ;
(ii) un anticorps ou fragment de celui-ci liant l'antigène comprenant un domaine variable de chaîne lourde contenant les résidus 50-54, 69-84 et 114-121 de SEQ ID NO:4 ou SEQ ID NO:7 ;
(iii) un anticorps ou fragment de celui-ci liant l'antigène comprenant un domaine variable de chaîne légère contenant les résidus 46-52, 70-72 et 111-116 de SEQ ID NO:2 ou SEQ ID NO:6 ; et
(iv) un anticorps ou fragment de celui-ci liant l'antigène comprenant un domaine variable de chaîne lourde contenant les résidus 45-51, 72-74, 115-120 de SEQ ID NO:4 ou SEQ ID NO:7.

**6.** Anticorps anti-CD40 ou fragment liant l'antigène ou utilisation selon la revendication 5, ledit anticorps anti-CD40 ou fragment de celui-ci liant l'antigène comprenant une séquence d'acides aminés choisie dans le groupe consistant en :

(i) les résidus 21-132 de SEQ ID NO:2 ;
(ii) les résidus 21-239 de SEQ ID NO:2 ;
(iii) SEQ ID NO:2 ;
(iv) les résidus 20-139 de SEQ ID NO:4 ;
(v) les résidus 20-469 de SEQ ID NO:4 ;
(vi) SEQ ID NO:4 ;
(vii) les résidus 20-469 de SEQ ID NO:5 ;
(viii) SEQ ID NO:5 ;
(ix) les résidus 21-132 de SEQ ID NO:2 et les résidus 20-139 de SEQ ID NO:4 ;
(x) les résidus 21-239 de SEQ ID NO:2 et les résidus 20-469 de SEQ ID NO:4 ;
(xi) les résidus 21-239 de SEQ ID NO:2 et les résidus 20-469 de SEQ ID NO:5 ;
(xii) SEQ ID NO:2 et SEQ ID NO:4 ; et
(xiii) SEQ ID NO:2 et SEQ ID NO:5.

**7.** Anticorps anti-CD40 ou fragment liant l'antigène ou utilisation selon la revendication 5, ledit anticorps anti-CD40 ou fragment de celui-ci liant l'antigène comprenant une séquence d'acides aminés choisie dans le groupe consistant en :

(i) les résidus 21-132 de SEQ ID NO:6 ;
(ii) les résidus 21-239 de SEQ ID NO:6 ;
(iii) SEQ ID NO:6 ;
(iv) les résidus 20-144 de SEQ ID NO:7 ;
(v) les résidus 20-474 de SEQ ID NO:7 ;
(vi) SEQ ID NO:7 ;
(vii) les résidus 20-474 de SEQ ID NO:8 ;
(viii) SEQ ID NO:8 ;
(ix) les résidus 21-132 de SEQ ID NO:6 et les résidus 20-144 de SEQ ID NO:7 ;
(x) les résidus 21-239 de SEQ ID NO:6 et les résidus 20-474 de SEQ ID NO:7 ;
(xi) les résidus 21-239 de SEQ ID NO:6 et les résidus 20-474 de SEQ ID NO:8 ;
(xii) SEQ ID NO:6 et SEQ ID NO:7 ; et
(xiii) SEQ ID NO:6 et SEQ ID NO:8.

**8.** Anticorps anti-CD40 ou fragment liant l'antigène ou utilisation selon l'une quelconque des revendications précédentes, ledit anticorps ou fragment de celui-ci liant l'antigène se liant à l'antigène CD40 humain avec une affinité ($K_D$) d'au moins $10^{-6}$ M.

**9.** Anticorps anti-CD40 ou fragment liant l'antigène ou utilisation selon la revendication 8, ledit anticorps ou fragment de celui-ci liant l'antigène se liant à l'antigène CD40 humain avec une affinité ($K_D$) d'au moins $10^{-8}$ M.

**10.** Anticorps anti-CD40 ou fragment liant l'antigène ou utilisation selon l'une quelconque des revendications précédentes, ledit anticorps ou fragment de celui-ci liant l'antigène étant produit dans une lignée de cellules CHO.

**11.** Anticorps anti-CD40 ou fragment liant l'antigène selon la revendication 1 ou utilisation selon la revendication 2, ladite thérapie de combinaison exerçant un effet thérapeutique synergique.

**12.** Anticorps anti-CD40 ou fragment liant l'antigène selon la revendication 1 ou utilisation selon la revendication 2, ledit fragment liant l'antigène dudit anticorps anti-CD40 étant choisi dans le groupe consistant en un fragment Fab, un fragment $F(ab')_2$, un fragment Fv, et un fragment Fv simple chaîne.

**13.** Anticorps anti-CD40 ou fragment liant l'antigène selon la revendication 1 ou utilisation selon la revendication 2, ou ladite IL-2 étant une IL-2 humaine ou un de ses variants biologiquement actifs.

**14.** Anticorps anti-CD40 ou fragment liant l'antigène ou utilisation selon la revendication 13, ledit variant d'IL-2 humaine étant la des-alanyl-1-,sérine-125 IL-2 humaine.

**15.** Anticorps anti-CD40 ou fragment liant l'antigène selon la revendication 1 ou utilisation selon la revendication 2, le cancer étant un cancer lié aux lymphocytes B ou une tumeur solide.

**16.** Anticorps anti-CD40 ou fragment liant l'antigène ou utilisation selon la revendication 15, le cancer lié aux lymphocytes B étant choisi dans le groupe consistant en le lymphome non hodgkinien, la leucémie chronique lymphoïde, myélome multiple, lymphome malin à cellules B, lymphome à cellules B de haut grade de malignité, lymphome à cellules B de grade intermédiaire de malignité, lymphome à cellules B de bas grade de malignité, la leucémie aiguë lymphoblastique à cellules B, la leucémie myéloblastique, la maladie de Hodgkin, plasmocytome, lymphome folliculaire, lymphome folliculaire à petites cellules clivées, lymphome folliculaire à grandes cellules, lymphome folliculaire mixte à petites cellules clivées, du lymphome diffus à petites cellules clivées, lymphome diffus à petites cellules lymphoïdes, la leucémie prolymphocytaire, lymphome lymphoplasmatique, lymphome de la zone marginale, lymphome de type MALT, lymphome à cellules B monocytoïdes, lymphome splénique, la leucémie à tricholeucocytes, lymphome diffus à grandes cellules, lymphome médiastinal à grandes cellules B, la granulomatose lymphomatoïde, la lymphomatose intravasculaire, lymphome diffus à cellules mixtes, lymphome diffus à grandes cellules, lymphome immunoblastique, lymphome de Burkitt, lymphome associé au SIDA, et lymphome à cellules du manteau.

**17.** Anticorps anti-CD40 ou fragment liant l'antigène ou utilisation selon la revendication 15, ladite tumeur solide étant choisie dans le groupe consistant en le carcinome de la vessie, carcinome mammaire, carcinome hépatique, carcinome gastrique, carcinome du côlon, cancer de la prostate, carcinome des cellules rénales, carcinome nasopharyngien, carcinome épidermoïde, carcinome papillaire thyroïdien, mélanome, carcinome ovarien, carcinome pulmonaire, carcinome du col de l'utérus, et sarcomes.

**18.** Anticorps anti-CD40 ou fragment liant l'antigène selon la revendication 1 ou utilisation selon la revendication 2, ladite IL-2 ou ledit variant de celle-ci et ledit anticorps anti-CD40 ou fragment de celui-ci liant l'antigène étant administrés séquentiellement.

**19.** Anticorps anti-CD40 ou fragment liant l'antigène selon la revendication 1 ou utilisation selon la revendication 2, ladite IL-2 ou ledit variant de celle-ci et ledit anticorps anti-CD40 ou fragment de celui-ci liant l'antigène étant administrés simultanément.

**20.** Anticorps anti-CD40 ou fragment liant l'antigène selon la revendication 1 ou utilisation selon la revendication 2, ledit anticorps anti-CD40 ou fragment de celui-ci liant l'antigène étant administré selon un schéma posologique sélectionné dans le groupe consistant en une fois par semaine, une fois toutes les deux semaines, une fois toutes les trois semaines, et une fois toutes les quatre semaines sur toute une période de traitement ou pendant une durée fixe de 4 semaines à 16 semaines au cours de ladite période de traitement en combinaison avec l'administration d'un ou plusieurs cycles d'un programme d'administration d'IL-2 à dose constante pendant ladite période de traitement, ledit programme d'administration d'IL-2 à dose constante comprenant une première période de temps, au cours de laquelle une dose hebdomadaire totale constante d'IL-2 ou d'un de ses variants biologiquement actifs est administrée audit sujet, et une seconde période de temps, au cours de laquelle l'administration de ladite IL-2 ou dudit variant de celle-ci biologiquement actif n'est pas effectuée chez ledit sujet.

**21.** Anticorps anti-CD40 ou fragment liant l'antigène ou utilisation selon la revendication 20, ladite première période de temps ayant une durée d'environ 2 semaines à environ 12 semaines, et ladite seconde période de temps ayant une durée d'environ 1 semaine à environ 4 semaines.

**22.** Anticorps anti-CD40 ou fragment liant l'antigène ou utilisation selon la revendication 21, ladite première période de temps ayant une durée de 4 semaines, et ladite seconde période de temps ayant une durée de 1 semaine.

**23.** Anticorps anti-CD40 ou fragment liant l'antigène ou utilisation selon la revendication 21, une première administration dudit anticorps anti-CD40 ou fragment de celui-ci liant l'antigène commençant le 1$^{er}$ jour de ladite période de traitement, et un premier cycle dudit programme d'administration d'IL-2 à dose constante étant démarré dans les 10 jours qui suivent ladite première administration dudit anticorps anti-CD40 ou fragment de celui-ci liant l'antigène.

**24.** Anticorps anti-CD40 ou fragment liant l'antigène ou utilisation selon la revendication 23, ledit premier cycle dudit programme d'administration d'IL-2 à dose constante étant démarré le 8$^{ème}$ jour de ladite période de traitement.

**25.** Anticorps anti-CD40 ou fragment liant l'antigène ou utilisation selon la revendication 23, ladite période de traitement comprenant un ou plusieurs cycles ultérieurs dudit programme d'administration d'IL-2 à dose constante qui est démarré dans les 4 semaines qui suivent l'achèvement dudit premier cycle dudit programme d'administration d'IL-2 à dose constante ou l'achèvement de tout cycle ultérieur dudit programme d'administration d'IL-2 à dose constante, ladite administration dudit anticorps anti-CD40 ou fragment de celui-ci liant l'antigène se poursuivant sur toute ladite période de traitement.

**26.** Anticorps anti-CD40 ou fragment liant l'antigène ou utilisation selon la revendication 20, ladite dose thérapeutiquement efficace dudit anticorps anti-CD40 ou fragment de celui-ci liant l'antigène se situant dans la gamme d'environ 0,5 mg/kg à environ 30,0 mg/kg.

**27.** Anticorps anti-CD40 ou fragment liant l'antigène ou utilisation selon la revendication 20, ladite dose hebdomadaire totale constante d'IL-2 ou d'un variant de celle-ci biologiquement actif étant administrée sous forme de dose unique ou étant fractionnée en une première série de doses équivalentes qui sont administrées selon un schéma posologique de deux, trois, quatre, cinq, six ou sept fois par semaine.

**28.** Anticorps anti-CD40 ou fragment liant l'antigène ou utilisation selon la revendication 27, ladite IL-2 ou ledit variant de celle-ci biologiquement actif étant administré(e) par une voie choisie dans le groupe consistant en les voies intraveineuse, intramusculaire, et sous-cutanée.

**29.** Anticorps anti-CD40 ou fragment liant l'antigène ou utilisation selon la revendication 20, ladite dose hebdomadaire totale constante de ladite IL-2 ou dudit variant de celle-ci biologiquement actif étant une quantité équivalente à une dose hebdomadaire totale d'un étalon IL-2 de référence administré par la même voie et selon le même schéma posologique, ladite dose hebdomadaire totale de l'étalon IL-2 de référence se situant dans une gamme d'environ 1100 $\mu$g (18,0 MUI) à environ 3300 $\mu$g (54,0 MUI), ladite dose hebdomadaire totale constante de ladite IL-2 ou dudit variant de celle-ci biologiquement actif apportant au moins 70 % de l'activité des cellules tueuses naturelles (cellules NK) qui est apportée par ladite dose hebdomadaire totale de l'étalon IL-2 de référence.

**30.** Anticorps anti-CD40 ou fragment liant l'antigène ou utilisation selon la revendication 29, ladite dose hebdomadaire totale de l'étalon IL-2 de référence étant d'environ 1100 $\mu$g (18,0 MUI) à environ 2567 $\mu$g (42,0 MUI), et ladite dose hebdomadaire totale de l'étalon IL-2 de référence étant fractionnée en trois doses équivalentes qui sont administrées selon un schéma posologique de trois fois par semaine.

**31.** Anticorps anti-CD40 ou fragment liant l'antigène selon la revendication 1 ou utilisation selon la revendication 2, ledit anticorps anti-CD40 ou fragment de celui-ci liant l'antigène étant administré selon un schéma posologique sélectionnée dans le groupe consistant en une fois par semaine, une fois toutes les deux semaines, une fois toutes les trois semaines, et une fois toutes les quatre semaines sur toute une période de traitement ou pendant une durée fixe de 4 semaines à 16 semaines au cours de ladite période de traitement en combinaison avec l'administration d'un ou plusieurs cycles d'un programme d'administration d'IL-2 à 2 niveaux de dose pendant ladite période de traitement, ledit programme d'administration d'IL-2 à 2 niveaux de dose comprenant une première période de temps, au cours de laquelle une dose hebdomadaire totale supérieure d'une IL-2 ou d'un de ses variants biologiquement actifs est administrée audit sujet, suivie par une seconde période de temps, au cours de laquelle une dose hebdomadaire totale inférieure de ladite IL-2 ou dudit variant de celle-ci biologiquement actif est administrée audit sujet.

**32.** Anticorps anti-CD40 ou fragment liant l'antigène ou utilisation selon la revendication 31, une première dose de ladite IL-2 ou dudit variant de celle-ci biologiquement actif étant administrée audit sujet avant l'administration d'une première dose dudit anticorps anti-CD40 ou fragment de celui-ci liant l'antigène.

**33.** Anticorps anti-CD40 ou fragment liant l'antigène ou utilisation selon la revendication 32, ladite première dose de ladite IL-2 ou dudit variant de celle-ci biologiquement actif étant administrée jusqu'à un mois avant que ladite première dose dudit anticorps anti-CD40 ou fragment de celui-ci liant l'antigène ne soit administrée audit sujet.

**34.** Anticorps anti-CD40 ou fragment liant l'antigène ou utilisation selon la revendication 33, ladite première dose de ladite IL-2 ou dudit variant de celle-ci biologiquement actif étant administrée une semaine avant que ladite première dose dudit anticorps anti-CD40 ou fragment de celui-ci liant l'antigène ne soit administrée audit sujet.

**35.** Anticorps anti-CD40 ou fragment liant l'antigène ou utilisation selon la revendication 31, une première dose de ladite IL-2 ou dudit variant de celle-ci biologiquement actif étant administrée audit sujet simultanément avec une première dose dudit anticorps anti-CD40 ou fragment de celui-ci liant l'antigène.

**36.** Anticorps anti-CD40 ou fragment liant l'antigène ou utilisation selon la revendication 31, une première dose de ladite IL-2 ou dudit variant de celle-ci biologiquement actif étant administrée audit sujet une semaine après qu'une première dose dudit anticorps anti-CD40 ou fragment de celui-ci liant l'antigène a été administrée audit sujet.

**37.** Anticorps anti-CD40 ou fragment liant l'antigène ou utilisation selon la revendication 31, ladite dose thérapeutiquement efficace dudit anticorps anti-CD40 ou fragment de celui-ci liant l'antigène se situant dans la gamme d'environ 0,5 mg/kg à environ 30,0 mg/kg.

**38.** Anticorps anti-CD40 ou fragment liant l'antigène ou utilisation selon la revendication 31, ledit programme d'administration d'IL-2 à 2 niveaux de dose ayant une durée combinée de 4 semaines à 16 semaines.

**39.** Anticorps anti-CD40 ou fragment liant l'antigène ou utilisation selon la revendication 38, ladite première période de temps dudit programme d'administration d'IL-2 à 2 niveaux de dose ayant une durée d'au moins 1 semaine sur ladite durée combinée de 4 semaines à 16 semaines.

**40.** Anticorps anti-CD40 ou fragment liant l'antigène ou utilisation selon la revendication 38, ladite première période de temps dudit programme d'administration d'IL-2 à 2 niveaux de dose ayant une durée égale à la moitié de ladite durée combinée de 4 semaines à 16 semaines.

**41.** Anticorps anti-CD40 ou fragment liant l'antigène ou utilisation selon la revendication 31, ladite dose hebdomadaire totale supérieure de ladite IL-2 ou dudit variant de celle-ci biologiquement actif étant administrée sous forme de dose unique ou est fractionnée en une première série de doses équivalentes qui sont administrées selon un schéma posologique de deux, trois, quatre, cinq, six ou sept fois par semaine, et ladite dose hebdomadaire totale inférieure de ladite IL-2 ou dudit variant de celle-ci biologiquement actif étant administrée sous forme de dose unique ou est fractionnée en une seconde série de doses équivalentes qui sont administrées selon un schéma posologique de deux, trois, quatre, cinq, six ou sept fois par semaine.

**42.** Anticorps anti-CD40 ou fragment liant l'antigène ou utilisation selon la revendication 41, ladite IL-2 ou ledit variant de celle-ci biologiquement actif étant administré(e) par une voie choisie dans le groupe consistant en les voies intraveineuse, intramusculaire, et sous-cutanée.

**43.** Anticorps anti-CD40 ou fragment liant l'antigène ou utilisation selon la revendication 42, ladite première série de doses équivalentes étant administrée selon un schéma posologique de trois fois par semaine, et ladite seconde série de doses équivalentes étant administrée selon un schéma posologique de trois fois par semaine.

**44.** Anticorps anti-CD40 ou fragment liant l'antigène ou utilisation selon la revendication 31, dans lequel ou laquelle :

a) ladite dose hebdomadaire totale supérieure de ladite IL-2 ou dudit variant de celle-ci biologiquement actif est une quantité équivalente à une dose hebdomadaire totale supérieure d'un étalon IL-2 de référence administré par la même voie et selon le même schéma posologique, ladite dose hebdomadaire totale supérieure de l'étalon IL-2 de référence se situant dans une gamme d'environ 1834 $\mu$g (30,0 MUI) à environ 3300 $\mu$g (54,0 MUI), ladite dose hebdomadaire totale supérieure de ladite IL-2 ou dudit variant de celle-ci biologiquement actif apportant au moins 70 % de l'activité des cellules tueuses naturelles (cellules NK) qui est apportée par ladite dose hebdomadaire totale supérieure de l'étalon IL-2 de référence ;
b) ladite dose hebdomadaire totale inférieure de ladite IL-2 ou dudit variant de celle-ci biologiquement actif est une quantité équivalente à une dose hebdomadaire totale inférieure de l'étalon IL-2 de référence administré par la même voie et selon le même schéma posologique, ladite dose hebdomadaire totale inférieure de l'étalon IL-2 de référence se situant dans une gamme d'environ 1100 $\mu$g (18,0 MUI) à environ 2384 $\mu$g (39,0 MUI),

ladite dose hebdomadaire totale inférieure de ladite IL-2 ou dudit variant de celle-ci biologiquement actif apportant au moins 70 % de l'activité des cellules tueuses naturelles (cellules NK) qui est apportée par ladite dose hebdomadaire totale inférieure de l'étalon IL-2 de référence ; et

c) ladite dose hebdomadaire totale inférieure de ladite IL-2 ou dudit variant de celle-ci biologiquement actif est inférieure à ladite dose hebdomadaire totale supérieure de ladite IL-2 ou dudit variant de celle-ci biologiquement actif.

**45.** Anticorps anti-CD40 ou fragment liant l'antigène ou utilisation selon la revendication 44, ladite dose hebdomadaire totale supérieure de l'étalon IL-2 de référence étant d'environ 1834 $\mu$g (30,0 MUI) à environ 2567 $\mu$g (42,0 MUI) et ladite dose hebdomadaire totale inférieure de l'étalon IL-2 de référence étant d'environ 1100 $\mu$g (18,0 MUI) à environ 1834 $\mu$g (30,0 MUI).

**46.** Anticorps anti-CD40 ou fragment liant l'antigène ou utilisation selon la revendication 45, ladite dose hebdomadaire totale supérieure de l'étalon IL-2 de référence étant d'environ 2567 $\mu$g (42,0 MUI), ladite dose hebdomadaire totale inférieure de l'étalon IL-2 de référence étant d'environ 1834 $\mu$g (30,0 MUI), et ladite dose hebdomadaire totale supérieure de l'étalon IL-2 de référence et ladite dose hebdomadaire totale inférieure de l'étalon IL-2 de référence étant chacune fractionnées en trois doses équivalentes qui sont administrées selon un schéma posologique de trois fois par semaine.

**47.** Anticorps anti-CD40 ou fragment liant l'antigène ou utilisation selon la revendication 31, comprenant en outre une interruption dans ledit programme d'administration d'IL-2 à 2 niveaux de dose, ladite interruption comprenant une période de temps sans administration de ladite IL-2 ou dudit variant de celle-ci biologiquement actif entre ladite première période de temps et ladite seconde période de temps dudit programme d'administration d'IL-2 à 2 niveaux de dose.

**48.** Anticorps anti-CD40 ou fragment liant l'antigène ou utilisation selon la revendication 47, ladite interruption ayant une durée d'environ 1 semaine à environ 4 semaines.

**49.** Anticorps anti-CD40 ou fragment liant l'antigène ou utilisation selon la revendication 31, ladite période de traitement comprenant un ou plusieurs cycles ultérieurs dudit programme d'administration d'IL-2 à 2 niveaux de dose qui est démarré environ 1 semaine à environ 4 semaines après l'achèvement d'un premier cycle dudit programme d'administration d'IL-2 à 2 niveaux de dose ou l'achèvement de tout cycle ultérieur dudit programme d'administration d'IL-2 à 2 niveaux de dose, ladite administration hebdomadaire dudit anticorps anti-CD40 ou fragment de celui-ci liant l'antigène se poursuivant sur toute ladite période de traitement.

**50.** Anticorps anti-CD40 ou fragment liant l'antigène ou utilisation selon la revendication 49, au moins un cycle dudit programme d'administration d'IL-2 à 2 niveaux de dose comprenant une interruption dans ledit programme d'administration d'IL-2 à 2 niveaux de dose, ladite interruption comprenant une période de temps sans administration de ladite IL-2 ou dudit variant de celle-ci biologiquement actif entre ladite première période de temps et ladite seconde période de temps de n'importe quel cycle donné dudit programme d'administration d'IL-2 à 2 niveaux de dose qui comprend ladite interruption.

**51.** Anticorps anti-CD40 ou fragment liant l'antigène ou utilisation selon l'une quelconque des revendications 1-50, ledit anticorps anti-CD40 ou fragment de celui-ci liant l'antigène étant administré par voie intraveineuse ou sous-cutanée.

# FIGURE 1A

CHIR 12.12 light chain:

leader:

MALPAQLLGLLMLWVSGSSG

variable:

DIVMTQSPLSLTVTPGEPASISCRSSQSLLYSNGYNYLDWYLQKPGQSPQVLISLGS
NRASGVPDRFSGSGSGTDFTLKISRVEAEDVGVYYCMQARQTPFTFGPGTKVDIR

constant:

RTVAAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC

# FIGURE 1B

CHIR-12.12 heavy chain:

leader:

MEFGLSWVFLVAILRGVQC

variable:

QVQLVESGGGVVQPGRSLRLSCAASGFTFSSYGMHWVRQAPGKGLEWVAVISYEESN
RYHADSVKGRFTISRDNSKITLYLQMNSLRTEDTAVYYCARDGGIAAPGPDYWGQGT
LVTVSS

constant:

ASTKGPSVFPLAPASKSTSGGTAALGCLVKDYFPEPVTVSWNSGALTSGVHTFPAVL
QSSGLYSLSSVVTVPSSSLGTQTYICNVNHKPSNTKVDKRVEPKSCDKTHTCPPCPA
PELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPRE
PQVYTLPPSREEMTKNQVSLTCLVKGFYPSDIAVEWESNGQPENNYKTTPPVLDSDG
SFFLYSKLTVDKSRWQQGNVFSCSVMHEALHNHYTQKSLSLSPGK

alternative constant region:

ASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKRVEPKSCDKTHTCPPCPA
PELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPRE
PQVYTLPPSREEMTKNQVSLTCLVKGFYPSDIAVEWESNGQPENNYKTTPPVLDSDG
SFFLYSKLTVDKSRWQQGNVFSCSVMHEALHNHYTQKSLSLSPGK

# FIGURE 2A

DNA sequence of light chain of CHIR-12.12:

5'atggcgctccctgctcagctcctggggctgctaatgctctgggtctctggatccagtggggatattgtgatgactcagtctc
cactctccctgaccgtcacccctggagagccggcctccatctcctgcaggtccagtcagagcctcctgtatagtaatggata
caactatttggattggtacctgcagaagccagggcagtctccacaggtcctgatctctttgggttctaatcgggcctccgggg
tccctgacaggttcagtggcagtggatcaggcacagattttacactgaaaatcagcagagtggaggctgaggatgttgggg
tttattactgcatgcaagctcgacaaactccattcactttcggccctgggaccaaagtggatatcagacgaactgtggctgca
ccatctgtcttcatcttcccgccatctgatgagcagttgaaatctggaactgcctctgttgtgtgcctgctgaataacttctatcc
cagagaggccaaagtacagtggaaggtggataacgccctccaatcgggtaactcccaggagagtgtcacagagcagga
cagcaaggacagcacctacagcctcagcagcaccctgacgctgagcaaagcagactacgagaaacacaaagtctacgc
ctgcgaagtcacccatcagggcctgagctcgcccgtcacaaagagcttcaacaggggagagtgttag3'

# FIGURE 2B

DNA sequence of heavy chain of CHIR-12.12 (including introns):

5'atggagtttgggctgagctgggttttccttgttgctattttaagaggtgtccagtgtcaggtgcagttggtggagtctggggg
aggcgtggtccagcctgggaggtccctgagactctcctgtgcagcctctggattcaccttcagtagctatggcatgcactgg
gtccgccaggctccaggcaaggggctggagtgggtggcagttatatcatatgaggaaagtaatagataccatgcagactc
cgtgaagggccgattcaccatctccagagacaattccaagatcacgctgtatctgcaaatgaacagcctcagaactgagga
cacggctgtgtattactgtgcgagagatgggggtatagcagcacctgggcctgactactggggccagggaaccctggtca
ccgtctcctcagcaagtaccaagggcccatccgtcttccccctggcgcccgctagcaagagcacctctgggggcacagc
ggccctgggctgcctggtcaaggactacttccccgaaccggtgacggtgtcgtggaactcaggcgccctgaccagcggc
gtgcacaccttcccggctgtcctacagtcctcaggactctactccctcagcagcgtggtgaccgtgccctccagcagcttgg
gcacccagacctacatctgcaacgtgaatcacaagcccagcaacaccaaggtggacaagagagttggtgagaggccag
cacagggagggagggtgtctgctggaagccaggctcagcgctcctgcctggacgcatcccggctatgcagtcccagtcc
agggcagcaaggcaggccccgtctgcctcttcacccggaggcctctgcccgccccactcatgctcagggagagggtctt
ctggctttttccccaggctctgggcaggcacaggctaggtgcccctaacccaggccctgcacacaaaggggcaggtgctg
ggctcagacctgccaagagccatatccgggaggaccctgcccctgacctaagcccaccccaaaggccaaactctccact
ccctcagctcggacaccttctctcctcccagattccagtaactcccaatcttctctctgcagagcccaaatcttgtgacaaaac
tcacacatgcccaccgtgcccaggtaagccagcccaggcctcgccctccagctcaaggcgggacaggtgccctagagta
gcctgcatccagggacaggccccagccgggtgctgacacgtccacctccatctcttcctcagcacctgaactcctgggggg
gaccgtcagtcttcctcttccccccaaaacccaaggacaccctcatgatctcccggacccctgaggtcacatgcgtggtggt
ggacgtgagccacgaagaccctgaggtcaagttcaactggtacgtggacggcgtggaggtgcataatgccaagacaaag
ccgcgggaggagcagtacaacagcacgtaccgtgtggtcagcgtcctcaccgtcctgcaccaggactggctgaatggca
aggagtacaagtgcaaggtctccaacaaagccctcccagcccccatcgagaaaaccatctccaaagccaaaggtgggac
ccgtggggtgcgagggccacatggacagaggccggctcggcccaccctctgccctgagagtgaccgctgtaccaacct
ctgtccctacagggcagccccgagaaccacaggtgtacaccctgcccccatcccgggaggagatgaccaagaaccagg
tcagcctgacctgcctggtcaaaggcttctatcccagcgacatcgccgtggagtgggagagcaatgggcagccggagaa
caactacaagaccacgcctcccgtgctggactccgacggctccttcttcctctatagcaagctcaccgtggacaagagcag
gtggcagcaggggaacgtcttctcatgctccgtgatgcatgaggctctgcacaaccactacacgcagaagagcctctccct
gtctccgggtaaatga3'

# FIGURE 3A

CHIR-5.9 light chain:

leader:

MALLAQLLGLLMLWVPGSSG

variable:

AIVMTQPPLSSPVTLGQPASISCRSSQSLVHSDGNTYLNWLQQRPGQPPRLLIYKFF
RRLSGVPDRFSGSGAGTDFTLKISRVEAEDVGVYYCMQVTQFPHTFGQGTRLEIK

constant:

RTVAAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC

# FIGURE 3B

CHIR-5.9 heavy chain:

leader:

MGSTAILALLLAVLQGVCA

variable:

EVQLVQSGAEVKKPGESLKISCKGSGYSFTSYWIGWVRQMPGKGLEWMGIIYPGDSD
TRYSPSFQGQVTISADKSISTAYLQWSSLKASDTAMYYCARGTAAGRDYYYYYGMDV
WGQGTTVTVSS

constant:

ASTKGPSVFPLAPASKSTSGGTAALGCLVKDYFPEPVTVSWNSGALTSGVHTFPAVL
QSSGLYSLSSVVTVPSSSLGTQTYICNVNHKPSNTKVDKRVEPKSCDKTHTCPPCPA
PELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPRE
PQVYTLPPSREEMTKNQVSLTCLVKGFYPSDIAVEWESNGQPENNYKTTPPVLDSDG
SFFLYSKLTVDKSRWQQGNVFSCSVMHEALHNHYTQKSLSLSPGK

alternative constant region:

ASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKRVEPKSCDKTHTCPPCPA
PELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPRE
PQVYTLPPSREEMTKNQVSLTCLVKGFYPSDIAVEWESNGQPENNYKTTPPVLDSDG
SFFLYSKLTVDKSRWQQGNVFSCSVMHEALHNHYTQKSLSLSPGK

# FIGURE 4A

Coding sequence for short isoform of human CD40:

1 atggttcgtc tgcctctgca gtgcgtcctc tggggctgct tgctgaccgc tgtccatcca
61 gaaccaccca ctgcatgcag agaaaaacag tacctaataa acagtcagtg ctgttctttg
121 tgccagccag gacagaaact ggtgagtgac tgcacagagt tcactgaaac ggaatgcctt
181 ccttgcggtg aaagcgaatt cctagacacc tggaacagag agacacactg ccaccagcac
241 aaatactgcg accccaacct agggcttcgg gtccagcaga agggcacctc agaaacagac
301 accatctgca cctgtgaaga aggctggcac tgtacgagtg aggcctgtga gagctgtgtc
361 ctgcaccgct catgctcgcc cggctttggg gtcaagcaga ttgctacagg ggtttctgat
421 accatctgcg agccctgccc agtcggcttc ttctccaatg tgtcatctgc tttcgaaaaa
481 tgtcaccctt ggacaaggtc cccaggatcg gctgagagcc ctggtggtga tccccatcat
541 cttcgggatc ctgtttgcca tcctcttggt gctggtcttt atcaaaaagg tggccaagaa
601 gccaaccaat aa

# FIGURE 4B

Encoded short isoform of human CD40:

1 mvrlplqcvl wgclltavhp epptacrekq ylinsqccsl cqpgqklvsd cteftetecl
61 pcgesefldt wnrethchqh kycdpnlglr vqqkgtsetd tictceegwh ctseacescv
121 lhrscspgfg vkqiatgvsd ticepcpvgf fsnvssafek chpwtrspgs aespggdphh
181 lrdpvchplg aglyqkggqe anq

# FIGURE 4C

Coding sequence for long isoform of human CD40:

1 atggttcgtc tgcctctgca gtgcgtcctc tggggctgct tgctgaccgc tgtccatcca
61 gaaccaccca ctgcatgcag agaaaaacag tacctaataa acagtcagtg ctgttctttg
121 tgccagccag gacagaaact ggtgagtgac tgcacagagt tcactgaaac ggaatgcctt
181 ccttgcggtg aaagcgaatt cctagacacc tggaacagag agacacactg ccaccagcac
241 aaatactgcg accccaacct agggcttcgg gtccagcaga agggcacctc agaaacagac
301 accatctgca cctgtgaaga aggctggcac tgtacgagtg aggcctgtga gagctgtgtc
361 ctgcaccgct catgctcgcc cggctttggg gtcaagcaga ttgctacagg ggtttctgat
421 accatctgcg agccctgccc agtcggcttc ttctccaatg tgtcatctgc tttcgaaaaa
481 tgtcaccctt ggacaagctg tgagaccaaa gacctggttg tgcaacaggc aggcacaaac
541 aagactgatg ttgtctgtgg tccccaggat cggctgagag ccctggtggt gatccccatc
601 atcttcggga tcctgtttgc catcctcttg gtgctggtct ttatcaaaaa ggtggccaag
661 aagccaacca ataaggcccc ccaccccaag caggaacccc aggagatcaa ttttcccgac
721 gatcttcctg gctccaacac tgctgctcca gtgcaggaga ctttacatgg atgccaaccg
781 gtcacccagg aggatggcaa agagagtcgc atctcagtgc aggagagaca gtga

# FIGURE 4D

Encoded long isoform of human CD40:

1 mvrlplqcvl wgclltavhp epptacrekq ylinsqccsl cqpgqklvsd cteftetecl
61 pcgesefldt wnrethchqh kycdpnlglr vqqkgtsetd tictceegwh ctseacescv
121 lhrscspgfg vkqiatgvsd ticepcpvgf fsnvssafek chpwtscetk dlvvqqagtn
181 ktdvvcgpqd rlralvvipi ifgilfaill vlvfikkvak kptnkaphpk qepqeinfpd
241 dlpgsntaap vqetlhgcqp vtqedgkesr isvqerq

FIGURE 5

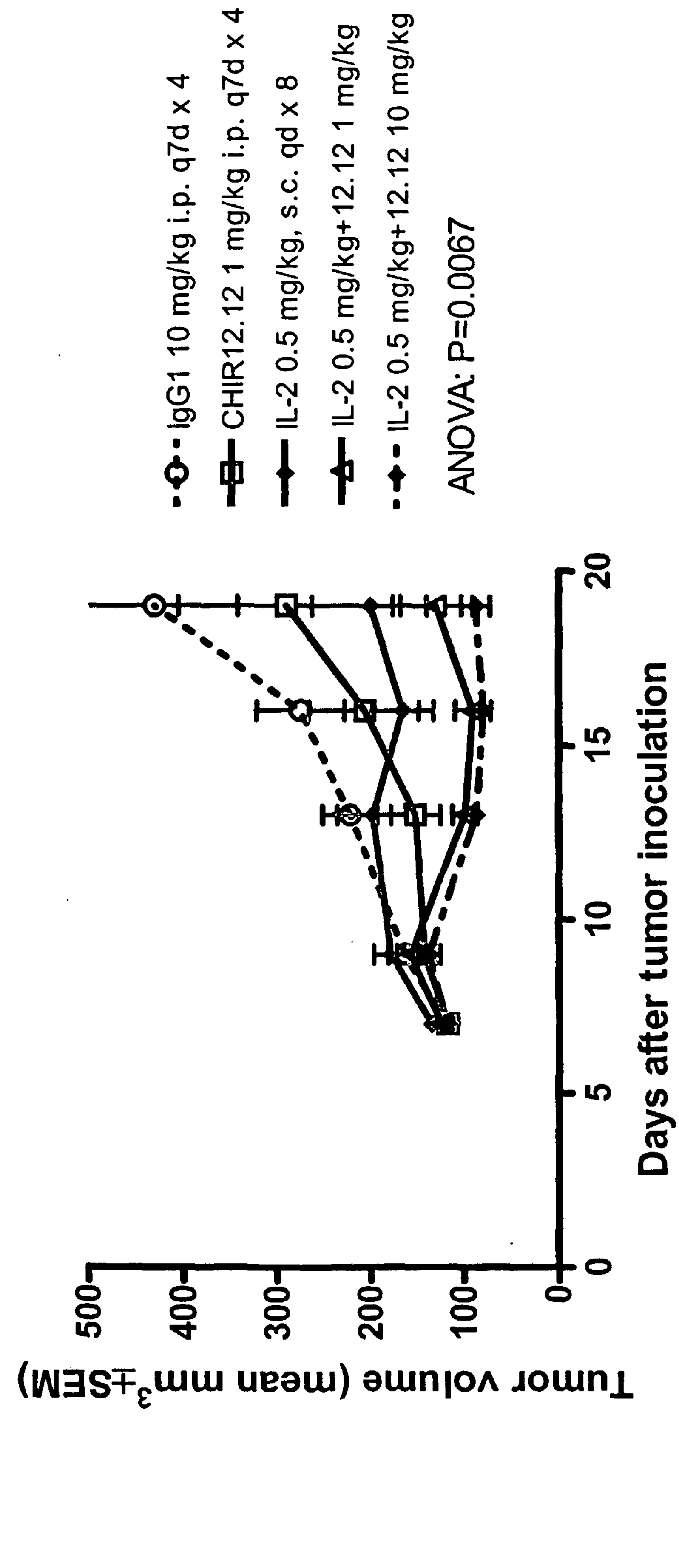
IgG1 10 mg/kg i.p. q7d x 4
CHIR12.12 1 mg/kg i.p. q7d x 4
IL-2 0.5 mg/kg, s.c. qd x 8
IL-2 0.5 mg/kg+12.12 1 mg/kg
IL-2 0.5 mg/kg+12.12 10 mg/kg
ANOVA: P=0.0067
Tumor volume (mean mm3±SEM)
0
100
200
300
400
500
Days after tumor inoculation
0
5
10
15
20

# FIGURE 6

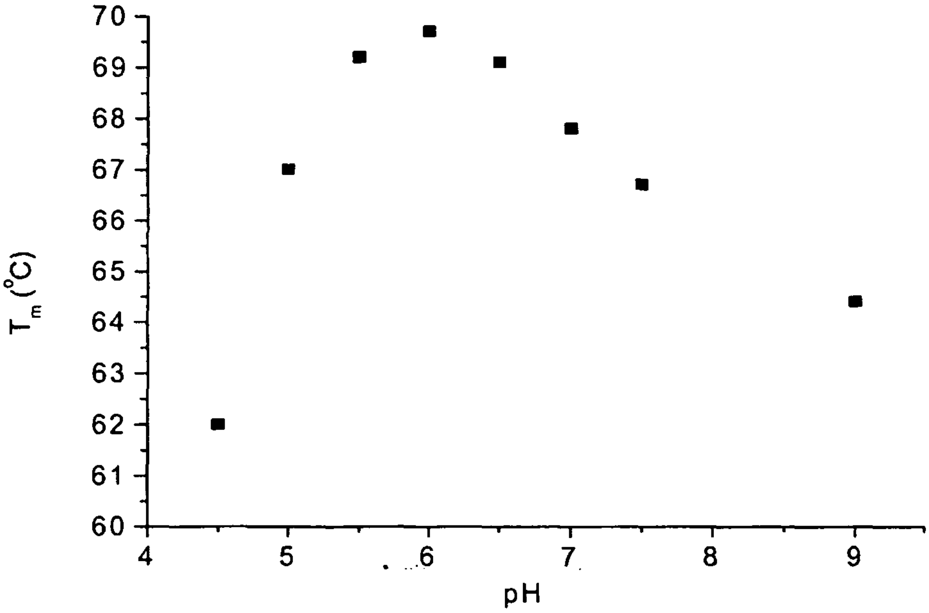

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0183755 A
- WO 0228480 A
- WO 0228904 A
- US 4604377 A
- US 4738927 A
- US 4656132 A
- US 4569790 A
- US 4748234 A
- US 4530787 A
- US 4572798 A
- US 4931543 A
- EP 0136489 A
- EP 0091539 A
- EP 0088195 A
- EP 0109748 A
- BE 893016
- US 4518584 A
- US 4752585 A
- WO 9960128 A
- US 4766106 A
- WO 9104282 A
- US 4873192 A
- EP 75444 A
- US 6737056 B1
- US 20040132101 A1
- EP 136489 A
- EP 200280 A
- EP 118617 A
- US 5700913 A
- US 5229109 A
- WO 0058456 A
- WO 0004048 A
- WO 0179258 A
- US 5206344 A
- US 4894226 A
- US 4745180 A
- US 4816440 A
- US 4931544 A
- US 4992271 A
- US 5078997 A
- US 6525102 A
- EP 268110 A
- US 6525102 B
- US 20030198602 A
- WO 0127160 A
- US 5674492 A
- US 4708871 A
- WO 0075348 A
- US 6087329 A
- US 5874082 A
- US 5677165 A
- US 6056959 A
- WO 0063395 A
- WO 0228905 A
- US 6004552 A
- US 4816567 A
- US 5514548 A
- US 5545403 A
- US 5545405 A
- US 5998144 A
- US 5122464 A
- US 5591639 A
- US 5658759 A
- US 5770359 A
- US 5827739 A
- US 5879936 A
- US 5891693 A
- US 5981216 A
- US 9104282 W
- US 5750105 A
- US 5756096 A
- US 5225539 A
- US 5585089 A
- US 5693761 A
- US 5693762 A
- US 5859205 A
- US 6180370 A
- US 6180370 B
- US 5877397 A
- US 5939598 A
- US 6075181 A
- US 6091001 A
- US 6114598 A
- US 4946778 A
- US 5260203 A
- US 5455030 A
- US 5856456 A
- WO 9852976 A
- WO 0034317 A
- EP 0983303 A1
- US 5847082 A
- WO 0052031 A
- WO 0052473 A
- US 6015542 A
- US 4676980 A
- US 4831175 A
- US 5595721 A

- WO 9856418 A
- EP 270799 A
- US 4179337 A
- US 4495285 A
- US 4609546 A
- US 60565710 B
- US 60525579 B
- US 60517337 B

**Non-patent literature cited in the description**


- **Paulie et al.** *J. Immunol,* 1989, vol. 142, 590-595
- **Braesch-Andersen et al.** *J. Immunol.,* 1989, vol. 142, 562-567
- **Hirano et al.** *Blood,* 1999, vol. 93, 2999-3007
- **Wingett et al.** *Breast Cancer Res. Treat.,* 1998, vol. 50, 27-36
- **Rokhlin et al.** *Cancer Res.,* 1997, vol. 57, 1758-1768
- **Kluth et al.** *Cancer Res.,* 1997, vol. 57, 891-899
- **Agathanggelou et al.** *Am. J. Pathol.,* 1995, vol. 147, 1152-1160
- **Amo et al.** *Eur. J. Dermatol.,* 2000, vol. 10, 438-442
- **Posner et al.** *Clin. Cancer Res.,* 1999, vol. 5, 2261-2270
- **Smith et al.** *Thyroid,* 1999, vol. 9, 749-755
- **van den Oord et al.** *Am. J. Pathol.,* 1996, vol. 149, 1953-1961
- **Ciaravino et al.** *Eur. J. Gynaecol. Oncol.,* 2004, vol. 25, 27-32
- **Sabel et al.** *Cancer Immunol. Immunother.,* 2000, vol. 49, 101-8
- **Altenberg et al.** *J. Immunol,* vol. 162, 4140-4147
- **Yamaguchi et al.** *Int. J. Oncol.,* 2003, vol. 23 (6), 1697-702
- **Lollini et al.** *Clin. Cancer Res.,* 1998, vol. 4 (8), 1843-849
- **Sugimoto et al.** *Hepatology,* 1999, vol. 30 (4), 920-26
- **Sabel et al.** *Cancer Immuno. Immunother.,* 2000, vol. 49 (2), 101-108
- **Ottaiano et al.** *Clin. Cancer Res.,* 2004, vol. 10 (8), 2824-2831
- **Morgan et al.** *Science,* 1976, vol. 193, 1007-1011
- **Weil-Hillman et al.** *Cancer Res.,* 1989, vol. 49 (13), 3680-3688
- **Rosenberg et al.** *N. Engl. J. Med.,* 1987, vol. 316, 889-897
- **Rosenberg.** *Ann. Surg.,* 1988, vol. 208, 121-135
- **Topalian et al.** *J. Clin. Oncol.,* 1988, vol. 6, 839-853
- **Rosenberg et al.** *N. Engl. J. Med.,* 1988, vol. 319, 1676-1680
- **Weber et al.** *J. Clin. Oncol.,* 1992, vol. 10, 33-40
- **Dutcher ; Wiernik.** *Sem. Oncol,* 1993, vol. 20 (6), 33-40
- **Duggan et al.** *J. Immunotherapy,* 1992, vol. 12, 115-122
- **Gisselbrecht et al.** *Blood,* 1994, vol. 83, 2081-2085
- **Sznol ; Parkinson.** *Blood,* 1994, vol. 83, 2020-2022
- **Palmer et al.** *Ann. Oncol.,* 1992, vol. 3, 475-80
- **Ellmark et al.** *Immunology,* 2002, vol. 106, 456-463
- **Gillis ; Watson.** *J. Exp. Med.,* 1980, vol. 159, 1709
- **Morgan et al.** *Science,* 1976, vol. 193, 1007-1008
- **Kabat et al.** *NIH Publ. No. 91-3242,* 1991, vol. I, 647-669
- **Kabat et al.** Sequences of Proteins of Immunological Interest. National Institute of Health, 1991
- **Clothia ; Lesk.** *J. Mol. Biol,* 1987, vol. 196, 901-917
- **Zapata et al.** *Protein Eng.,* 1995, vol. 10, 1057-1062
- **Daeron.** *Annu. Rev. Immunol.,* 1997, vol. 15, 203-234
- **Ravetch ; Kinet.** *Annu. Rev. Immunol,* 1991, vol. 9, 457-92
- **Capel et al.** *Immunomethods,* 1994, vol. 4, 25-34
- **de Haas et al.** *J. Lab. Clin. Med.,* 1995, vol. 126, 330-41
- **Guyer et al.** *J. Immunol,* 1976, vol. 117, 587
- **Kim et al.** *J. Immunol,* 1994, vol. 24, 249
- The Non-Hodgkin's Lymphoma Pathologic Classification Project. *Cancer,* 1982, vol. 49, 2112-2135
- **Taniguchi et al.** *Nature,* 1983, vol. 302, 305-310
- **Devos.** *Nucleic Acids Res.,* 1983, vol. 11, 4307-4323
- **Wang et al.** *Science,* 1984, vol. 224, 1431-1433
- **Allison et al.** *J. Clin. Oncol.,* 1989, vol. 7 (1), 75-80
- **Hernandez-Illzaliturri et al.** *Clin. Cancer Res.,* 2003, vol. 9, 5866-5873
- **Uchida et al.** *J. Exp. Med.,* 2004, vol. 199 (12), 1659-1669
- **Gearing ; Thorpe.** *J. Immunological Methods,* 1988, vol. 114, 3-9

- **Nakanishi et al.** *J. Exp. Med,* 1984, vol. 160 (6), 1605-1621
- **Gustavson.** *J Biol. Response Modifiers,* 1998, 440-449
- **Thompson et al.** *Cancer Research,* 1987, vol. 47, 4202-4207
- **Kirchner et al.** *Br. J. Clin. Pharmacol.,* 1998, vol. 46, 5-10
- **Piscitelli et al.** *Pharmacotherapy,* 1996, vol. 16 (5), 754-759
- **Nagler et al.** *J. Immunol,* 1989, vol. 143, 3183-3191
- Current Protocols in Immunology: Immunologic Studies in Humans. John Wiley & Sons, Inc, 1996
- Techniques in Molecular Biology. MacMillan Publishing Company, 1983
- **Kunkel.** *Proc. Natl. Acad. Sci. USA,* 1985, vol. 82, 488-492
- **Kunkel et al.** *Methods Enzymol.,* 1987, vol. 154, 367-382
- **Sambrook et al.** Molecular Cloning: A Laboratory Manual. Cold Spring Harbor, 1989
- **Dayhoff et al.** Atlas of Protein Sequence and Structure. Natl. Biomed. Res. Found, 1978
- **Smith ; Waterman.** *Adv. Appl. Math.,* 1981, vol. 2, 482-489
- **Stamenkovic et al.** *EMBO,* 1989, vol. 8, 1403
- **Clark.** *Tissue Antigens,* 1990, vol. 36, 33
- **Barclay et al.** The Leucocyte Antigen Facts Book. Academic Press, 1997
- Leukocyte Typing III and IV. Oxford University Press, 1987
- **Gordon et al.** *J. Immunol.,* 1988, vol. 140, 1425
- **Valle et al.** *Eur. J. Immunol.,* 1989, vol. 19, 1463
- **Clark et al.** *PNAS,* 1986, vol. 83, 4494
- **Paulie et al.** *J. Immunol.,* 1989, vol. 142, 590
- **Gordon et al.** *Eur. J. Immunol.,* 1987, vol. 17, 1535
- **Jabara et al.** *J. Exp. Med.,* 1990, vol. 172, 1861
- **Zhang et al.** *J. Immunol.,* 1991, vol. 146, 1836
- **Gascan et al.** *J. Immunol.,* 1991, vol. 147, 8
- **Banchereau et al.** *Clin. Immunol. Spectrum,* 1991, vol. 3, 8
- **Banchereau et al.** *Science,* 1991, vol. 251, 70
- **Kohler et al.** *Nature,* 1975, vol. 256, 495
- **Clackson et al.** *Nature,* 1991, vol. 352, 624-628
- **Marks et al.** *J. Mol. Biol.,* 1991, vol. 222, 581-597
- **Kohler et al.** *Nature,* 1975, vol. 256, 495-496
- **Skerra et al.** *Curr. Opinion in Immunol.,* 1993, vol. 5, 256
- **Phickthun.** *Immunol. Revs.,* 1992, vol. 130, 151
- **Chothia et al.** *J. Mol. Biol.,* 1987, vol. 196, 901-917
- **Kabat et al.** NIH Publication No. 91-3242. U. S. Dept. of Health and Human Services, 1991
- **Jones et al.** *Nature,* 1986, vol. 321, 522-525
- **Riechmann et al.** *Nature,* 1988, vol. 332, 323-327
- **Verhoeyen et al.** *Science,* 1988, vol. 239, 1534-1536
- **Jones et al.** *Nature,* 1986, vol. 331, 522-525
- **Riechmann et al.** *Nature,* 1988, vol. 332, 323-329
- **Presta.** *Curr. Op. Struct. Biol.,* 1992, vol. 2, 593-596
- **Pluckthun.** The Pharmacology of Monoclonal Antibodies. Springer-Verlag, 1994, vol. 113, 269-315
- **McCafferty et al.** *Nature,* 1990, vol. 348, 552-554
- **Marks et al.** *J Mol. Biol.,* 1991, vol. 222, 581-597
- **Marks et al.** *Bio/Technology,* 1992, vol. 10, 779-783
- **Waterhouse et al.** *Nucleic. Acids Res.,* 1993, vol. 21, 2265-2266
- **Morimoto et al.** *Journal of Biochemical and Biophysical Methods,* 1992, vol. 24, 107-117
- **Brennan et al.** *Science,* 1985, vol. 229, 81
- **Carter et al.** *Bio/Technology,* 1992, vol. 10, 163-167
- **Schultze et al.** *Proc. Natl. Acad. Sci. USA,* 1998, vol. 92, 8200-8204
- **Denton et al.** *Pediatr. Transplant.,* 1998, vol. 2, 6-15
- **Evans et al.** *J. Immunol.,* 2000, vol. 164, 688-697
- **Noelle.** *Agents Actions,* 1998, vol. 49, 17-22
- **Lederman et al.** *Curr. Opin. Hematol.,* 1996, vol. 3, 77-86
- **Coligan et al.** *Current Protocols in Immunology,* 1991, vol. 13, 12
- **Kwekkeboom et al.** *Immunology,* 1993, vol. 79, 439-444
- **Srivastava ; Mease.** *Nucl. Med. Bio.,* 1991, vol. 18, 589-603
- Monoclonal Antibodies for Immunotargeting of Drugs in Cancer Therapy. **Arnon et al.** Monoclonal Antibodies and Cancer Therapy. Alan R. Liss, Inc, 1985, 243-256
- Antibodies for Drug Delivery. Controlled Drug Delivery. 1987, 623-653
- Antibody Carriers of Cytotoxic Agents in Cancer Therapy: A Review. **Thorpe et al.** Monoclonal Antibodies '84: Biological and Clinical Applications. 1985, 475-506

- Analysis, Results, and Future Prospective of the Therapeutic Use of Radiolabeled Antibody in Cancer Therapy. Monoclonal Antibodies for Cancer Detection and Therapy. Academic Press, 1985, 303-316
- **Thorpe et al.** *Immunol. Rev.,* 1982, vol. 62, 119-158
- Remington's Pharmaceutical Sciences. Mack Publishing Company, 1990
- **Knauf et al.** *J. Bio. Chem.,* 1988, vol. 263, 15064-15070
- **Gabizon et al.** *Cancer Research,* 1982, vol. 42, 4734
- **Cafiso.** *Biochem Biophys Acta,* 1981, vol. 649, 129
- **Szoka.** *Ann. Rev. Biophys. Eng.,* 1980, vol. 9, 467
- **Poznansky et al.** Drug Delivery Systems. 1980, 253-315
- **Poznansky.** *Pharm Revs,* 1984, vol. 36, 277
- **Jones.** *Adv. Drug Delivery Rev.,* 1993, vol. 10, 29-90
- Peptide and Protein Drug Delivery. Marcel Dekker, Inc, 1991
- **Setnikar et al.** *J. Am. Pharm. Assoc.,* 1959, vol. 48, 628
- **Levine et al.** *J. Parenteral Sci. Technol.,* 1991, vol. 45 (3), 160-165
- **Boer et al.** *J. Immunol. Meth.,* 1988, vol. 113, 143
- **Gustavson.** *J. Biol. Response Modifiers,* 1998, 440-449
- **Rossio et al.** *Lymphokine Research,* 1986, vol. 5 (1), S13-S18